(19) Europäisches Patentamt / European Patent Office / Office européen des brevets

(11) **EP 4 321 515 A1**

(12) **EUROPEAN PATENT APPLICATION**
published in accordance with Art. 153(4) EPC


(43) Date of publication:
**14.02.2024 Bulletin 2024/07**

(21) Application number: **22784119.4**

(22) Date of filing: **08.04.2022**

(51) International Patent Classification (IPC):
***C07D 417/14*** *(2006.01)* ***C07D 291/08*** *(2006.01)*
***C07D 273/00*** *(2006.01)* ***A61K 31/519*** *(2006.01)*
***A61P 35/00*** *(2006.01)*

(52) Cooperative Patent Classification (CPC):
**A61K 31/519; A61P 35/00; C07D 273/00; C07D 291/08; C07D 417/14**

(86) International application number:
**PCT/CN2022/085703**

(87) International publication number:
**WO 2022/214053 (13.10.2022 Gazette 2022/41)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority:
**09.04.2021 CN 202110403760**
**22.07.2021 CN 202110829701**
**30.09.2021 CN 202111166322**
**23.12.2021 CN 202111591747**
**11.03.2022 CN 202210242231**



(71) Applicant: **Simcere Zaiming Pharmaceutical Co., Ltd.**
**Hainan 570311 (CN)**

(72) Inventors:
- **ZHU, Wei**
  **Shanghai 201321 (CN)**
- **ZOU, Hao**
  **Shanghai 201321 (CN)**
- **MAI, Wansun**
  **Shanghai 201321 (CN)**
- **ZHU, Dongxing**
  **Shanghai 201321 (CN)**
- **CHEN, Xiang**
  **Shanghai 201321 (CN)**
- **WANG, Tao**
  **Shanghai 201321 (CN)**
- **SUN, Tianwen**
  **Nanjing, Jiangsu 210042 (CN)**
- **LI, Zhengtao**
  **Shanghai 201321 (CN)**

(74) Representative: **V.O.**
**P.O. Box 87930**
**2508 DH Den Haag (NL)**


(54) **UBIQUITIN-SPECIFIC PROTEASE 1 (USP1) INHIBITOR**


(57) The present application discloses a compound of formula (I) as a USP1 inhibitor, or a pharmaceutically acceptable salt thereof, a pharmaceutical composition comprising the compound or the pharmaceutically acceptable salt thereof, and use thereof in prevention or treatment of diseases associated with USP1.


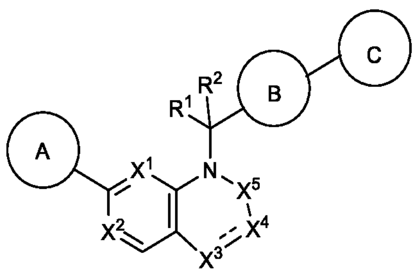

(I)

EP 4 321 515 A1

## Description

### CROSS-REFERENCE TO RELATED APPLICATIONS

The present application claims benefit and priority to the following five Chinese patent applications, which are incorporated herein by reference in their entirety:

Patent Application No. 202110403760.7, filed with China National Intellectual Property Administration on April 9, 2021;
Patent Application No. 202110829701.6, filed with China National Intellectual Property Administration on July 22, 2021;
Patent Application No. 202111166322.X, filed with China National Intellectual Property Administration on September 30, 2021;
Patent Application No. 202111591747.5, filed with China National Intellectual Property Administration on December 23, 2021; and
Patent Application No. 202210242231.8, filed with China National Intellectual Property Administration on March 11, 2022.

### TECHNICAL FIELD

The present application belongs to the field of pharmaceutical technologies, and relates to a compound as a ubiquitin-specific protease 1 (USP1) inhibitor, or an optical isomer and a pharmaceutically acceptable salt thereof, a pharmaceutical composition containing the same, and use of the compound as a USP1 inhibitor in the prevention or treatment of diseases associated with USP1.

### BACKGROUND

Ubiquitination is a reversible process which involves a family of deubiquitinating enzymes (DUBs) that regulate a variety of cellular processes by deubiquitinating substrates. DUBs are encoded by approximately 100 human genes and are classified into six families, with the largest family being the ubiquitin-specific proteases (USPs) having more than 50 members. The phenomenon that DUBs and their substrate proteins are often deregulated in cancers supports the hypothesis that targeting specific DUBs can enhance the ubiquitination and degradation of oncogenic substrates, and regulate the activity of other key proteins involved in tumor growth, survival, differentiation and maintenance of the tumor microenvironment (Hussain, S., et. al., DUBs and cancer: The role of deubiquitinating enzymes as oncogenes, non-oncogenes and tumor suppressors, Cell Cycle 8, 1688-1697 (2009)). USP1 is a cysteine isopeptidase of the USP subfamily of DUBs. Full-length human USP1 consists of 785 amino acids, including a catalytic triad consisting of Cys90, His593 and Asp751. USP1 plays a role in DNA damage repair. USP1 is relatively inactive in itself, and its full enzymatic activity can only be obtained by binding to the cofactor UAF1 to form a complex required for the activity of deubiquitinating enzymes. The two proteins, deubiquitinated monoubiquitinated PCNA (proliferating cell nuclear antigen) and monoubiquitinated FANCD2 (Fanconi anemia group complementary group D2) by the USP1/UAF1 complex, play important roles in translational synthesis (TLS) and Fanconi anemia (FA) pathways, respectively. The two pathways are essential for the repair of DNA damage induced by DNA cross-linking agents such as cisplatin and mitomycin C (MMC). The USP1/UAF1 complex also deubiquitinates FANCI (Fanconi anemia complementation group I). The importance of these findings was further confirmed experimentally by the fact that USP1-deficient mice were observed to be highly sensitive to DNA damage. Interestingly, the expression of USP1 is significantly increased in a number of cancers. Blocking USP1 to inhibit DNA repair can induce apoptosis in multiple myeloma cells, and can also enhance the sensitivity of lung cancer cells to cisplatin. These results indicate that USP1 is a promising target for chemotherapy for some cancers.

In conclusion, targeted inhibition of USP1 protein is a potential approach to the prevention and treatment of cancers and other diseases. Therefore, the development of small molecule inhibitors of USP1 is essential.

### SUMMARY

According to one aspect, the present application relates to a compound of formula (I) or a pharmaceutically acceptable salt thereof,

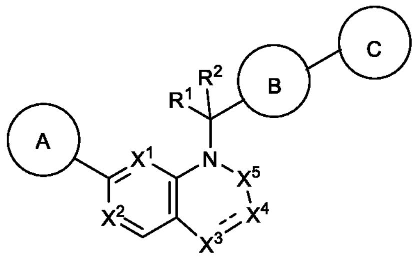

(I)

wherein,

$X^1$ is selected from $CR^3$ and N;
$X^2$ is selected from N;
$X^3$ and $X^4$ are each independently selected from $C(R^4)(R^5)$, $CR^4$, $NR^6$, N, O, S, S=O, and $S(=O)_2$; $X^5$ is independently selected from $C(R^4)(R^5)$, $NR^6$, and O;
$R^3$, $R^4$, $R^5$, and $R^6$ are each independently selected from H, halogen, CN, OH, $NH_2$, $C_1$-$C_6$ alkyl, $C_2$-$C_6$ alkenyl, $C_2$-$C_6$ alkynyl, -C(O)-$C_1$-$C_6$ alkyl, -C(O)O-$C_1$-$C_6$ alkyl, $C_3$-$C_{10}$ cycloalkyl, and 4- to 7-membered heterocyclyl, wherein the OH, $NH_2$, $C_1$-$C_6$ alkyl, $C_2$-$C_6$ alkenyl, $C_2$-$C_6$ alkynyl, $C_3$-$C_{10}$ cycloalkyl, or 4- to 7-membered heterocyclyl is optionally substituted with $R^a$,
or $R^4$ and $R^5$ are merged into =O or =S; or $R^4$ and $R^5$ together with the C to which they are attached form $C_3$-$C_{10}$ cycloalkyl, wherein the $C_3$-$C_{10}$ cycloalkyl is optionally substituted with $R^a$; or $R^4$ and $R^5$ together with the atoms to which they are attached form $C_3$-$C_{10}$ heterocyclyl, wherein the $C_3$-$C_{10}$ heterocyclyl is optionally substituted with $R^a$; or $R^4$ and $R^6$ at different positions in the ring, together with the atoms to which they are attached form $C_3$-$C_{10}$ heterocyclyl, wherein the $C_3$-$C_{10}$ heterocyclyl is optionally substituted with $R^a$;
ring A is selected from aryl and 5- to 10-membered heteroaryl, wherein the aryl or the 5- to 10-membered heteroaryl is optionally substituted with $R^b$;
ring B is selected from aryl, 5- to 10-membered heteroaryl, 4- to 10-membered heterocyclyl, $C_3$-$C_{10}$ cycloalkyl, and $C_3$-$C_{10}$ cycloalkenyl, wherein the aryl, 5- to 10-membered heteroaryl, 4- to 10-membered heterocyclyl, $C_3$-$C_{10}$ cycloalkyl, or $C_3$-$C_{10}$ cycloalkenyl is optionally substituted with $R^c$; $R^b$ and $R^c$ are each independently selected from halogen, CN, OH, $NH_2$, SH, $C_1$-$C_6$ alkyl, $C_3$-$C_{10}$ cycloalkyl or 4- to 7-membered heterocyclyl,

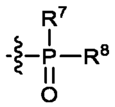 , 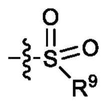 , 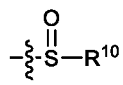 , 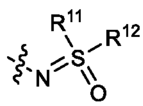 ,

and

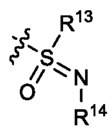 ,

wherein the OH, $NH_2$, SH, $C_1$-$C_6$ alkyl, $C_3$-$C_{10}$ cycloalkyl, or 4- to 7-membered heterocyclyl is optionally substituted with $R^a$;
$R^7$, $R^8$, $R^9$, $R^{10}$, $R^{11}$, $R^{12}$, $R^{13}$ and $R^{14}$ are each independently selected from $NH_2$, $C_1$-$C_6$ alkyl, $C_3$-$C_{10}$ cycloalkyl, and 4- to 7-membered heterocyclyl, wherein the $NH_2$, $C_1$-$C_6$ alkyl, $C_3$-$C_{10}$ cycloalkyl, or 4- to 7-membered heterocyclyl is optionally substituted with $R^a$;
or $R^7$ and $R^8$ together with the P to which they are attached form 4- to 7-membered heterocyclyl, wherein the 4- to 7-membered heterocyclyl is optionally substituted with $R^a$;
or $R^{13}$ and $R^{14}$ together with the atoms to which they are attached form 4- to 7-membered heterocyclyl, wherein the 4- to 7-membered heterocyclyl is optionally substituted with $R^a$;
ring C is selected from aryl, 5- to 10-membered heteroaryl, and 4- to 10-membered heterocyclyl, wherein the aryl, 5- to 10-membered heteroaryl, or 4- to 10-membered heterocyclyl is optionally substituted with $R^d$;
$R^d$ is selected from halogen, CN, OH, $NH_2$, $C_1$-$C_6$ alkyl, $C_3$-$C_{10}$ cycloalkyl, and 4- to 7-membered heterocyclyl,

wherein the OH, $NH_2$, $C_1$-$C_6$ alkyl, $C_3$-$C_{10}$ cycloalkyl, or 4- to 7-membered heterocyclyl is optionally substituted with $R^a$; or $R^c$ and $R^d$ together with the atoms to which they are attached form 5- to 8-membered heterocyclyl or 5- to 6-membered heteroaryl, wherein the 5- to 8-membered heterocyclyl or the 5- to 6-membered heteroaryl is optionally substituted with $R^a$;

$R^1$ and $R^2$ are each independently selected from H, halogen, CN, OH, $NH_2$, $C_1$-$C_6$ alkyl, $C_3$-$C_{10}$ cycloalkyl, and 4- to 7-membered heterocyclyl, wherein the OH, $NH_2$, $C_1$-$C_6$ alkyl, $C_3$-$C_{10}$ cycloalkyl, or 4- to 7-membered heterocyclyl is optionally substituted with $R^a$,

or $R^1$ and $R^2$ together with the atoms to which they are attached form $C_3$-$C_{10}$ cycloalkyl or 4- to 7-membered heterocyclyl, wherein the $C_3$-$C_{10}$ cycloalkyl or the 4- to 7-membered heterocyclyl is optionally substituted with $R^a$;

each $R^a$ is independently selected from halogen, CN, =O, OH, $NH_2$, $C_1$-$C_6$ alkyl, $C_3$-$C_{10}$ cycloalkyl, and 4- to 7-membered heterocyclyl, wherein the OH, $NH_2$, $C_1$-$C_6$ alkyl, $C_3$-$C_{10}$ cycloalkyl, or 4- to 7-membered heterocyclyl is optionally substituted with $R^e$;

$R^e$ is selected from halogen, CN, =O, OH, $NH_2$, $C_1$-$C_6$ alkyl, $C_3$-$C_6$ cycloalkyl, and 4- to 7-membered heterocyclyl, wherein the OH, $NH_2$, $C_1$-$C_6$ alkyl, $C_3$-$C_6$ cycloalkyl, or 4- to 7-membered heterocyclyl is optionally substituted with $R^f$; and

$R^f$ is selected from halogen, CN, OH, $NH_2$, $C_1$-$C_6$ alkyl, $C_3$-$C_6$ cycloalkyl, and 4- to 7-membered heterocyclyl.

The expression "$R^4$ and $R^6$ at different positions in the ring, together with the atoms to which they are attached form $C_3$-$C_{10}$ heterocyclyl" means that when groups $C(R^4)(R^5)$ or $CR^4$ and $NR^6$ are located at different positions in the ring, $R^4$ and $R^6$ together with the atoms to which they are attached may form $C_3$-$C_{10}$ heterocyclyl. For example, but not limited to, when $X^4$ is $NR^6$ and $X^5$ is $C(R^4)(R^5)$, $R^4$ and $R^6$ together with the atoms to which they are attached form $C_3$-$C_{10}$ heterocyclyl.

In some embodiments, $X^1$ is selected from N, and $X^2$ is selected from N.

In some embodiments, $X^1$ is selected from $CR^3$, and $X^2$ is selected from N.

In some embodiments, $X^1$ is selected from CH, and $X^2$ is selected from N.

In some embodiments, $X^1$ is selected from $CR^3$ and N, wherein $R^3$ is H.

In some embodiments, $X^3$ and $X^4$ are each independently selected from $C(R^4)(R^5)$, $CR^4$, $NR^6$, and O. In some embodiments, $X^3$ is selected from $C(R^4)(R^5)$, $CR^4$, $NR^6$, N, O, and S; or $X^3$ is selected from $C(R^4)(R^5)$, $NR^6$, and O; wherein $R^4$ and $R^5$ are each independently selected from H, halogen, $C_1$-$C_6$ alkyl, and $C_2$-$C_6$ alkynyl, wherein the $C_1$-$C_6$ alkyl or the $C_2$-$C_6$ alkynyl is optionally substituted with $R^a$; or $R^4$ and $R^5$ are merged into =O; or $R^4$ and $R^5$ together with the C to which they are attached form $C_3$-$C_{10}$ cycloalkyl, wherein the $C_3$-$C_{10}$ cycloalkyl is optionally substituted with $R^a$; or $R^4$ and $R^5$ together with the atoms to which they are attached form $C_3$-$C_{10}$ heterocyclyl, wherein the $C_3$-$C_{10}$ heterocyclyl is optionally substituted with $R^a$; or $R^4$ and $R^5$ are each independently selected from H, halogen, $C_1$-$C_6$ alkyl, and $C_2$-$C_6$ alkynyl, wherein the $C_1$-$C_6$ alkyl is optionally substituted with $R^a$; or $R^4$ and $R^5$ are merged into =O; or $R^4$ and $R^5$ together with the C to which they are attached form $C_3$-$C_{10}$ cycloalkyl; or $R^4$ and $R^5$ together with the atoms to which they are attached form $C_3$-$C_{10}$ heterocyclyl; or $R^4$ and $R^5$ are each independently selected from H, halogen, and $C_1$-$C_6$ alkyl, wherein the $C_1$-$C_6$ alkyl is optionally substituted with $R^a$; or $R^4$ and $R^5$ are merged into =O; or $R^4$ and $R^5$ together with the C to which they are attached form $C_3$-$C_{10}$ cycloalkyl; wherein $R^6$ is selected from H, $C_1$-$C_6$ alkyl, $C_2$-$C_6$ alkenyl, -C(O)-$C_1$-$C_6$ alkyl, -C(O)O-$C_1$-$C_6$ alkyl, and $C_3$-$C_{10}$ cycloalkyl, wherein the $C_1$-$C_6$ alkyl, $C_2$-$C_6$ alkenyl, or $C_3$-$C_{10}$ cycloalkyl is optionally substituted with $R^a$; or $R^6$ is selected from H, $C_1$-$C_6$ alkyl, and $C_3$-$C_{10}$ cycloalkyl, wherein the $C_1$-$C_6$ alkyl is optionally substituted with $R^a$. In the embodiments, each $R^a$ is independently selected from halogen and OH; and each halogen is independently selected from F and Cl.

In some embodiments, $X^4$ is selected from $C(R^4)(R^5)$, $CR^4$, $NR^6$, and O; or $X^4$ is selected from $C(R^4)(R^5)$, $NR^6$, and O; wherein $R^4$ and $R^5$ are each independently selected from H, halogen, and $C_1$-$C_6$ alkyl, wherein the $C_1$-$C_6$ alkyl is optionally substituted with $R^a$; or $R^4$ and $R^5$ are merged into =O or =S; or $R^4$ and $R^5$ together with the C to which they are attached form $C_3$-$C_{10}$ cycloalkyl, wherein the $C_3$-$C_{10}$ cycloalkyl is optionally substituted with $R^a$; or $R^4$ and $R^6$ at different positions in the ring, together with the atoms to which they are attached form $C_3$-$C_{10}$ heterocyclyl, wherein the $C_3$-$C_{10}$ heterocyclyl is optionally substituted with $R^a$; or $R^4$ and $R^5$ are each independently selected from H and $C_1$-$C_6$ alkyl, wherein the $C_1$-$C_6$ alkyl is optionally substituted with $R^a$; or $R^4$ and $R^5$ are merged into =O or =S; or $R^4$ and $R^5$ together with the C to which they are attached form $C_3$-$C_{10}$ cycloalkyl, wherein the $C_3$-$C_{10}$ cycloalkyl is optionally substituted with $R^a$; or $R^4$ and $R^5$ are each independently selected from H and $C_1$-$C_6$ alkyl; or $R^4$ and $R^5$ are merged into =O or =S; or $R^4$ and $R^5$ together with the C to which they are attached form $C_3$-$C_{10}$ cycloalkyl; wherein $R^6$ is selected from H and $C_1$-$C_6$ alkyl, wherein the $C_1$-$C_6$ alkyl is optionally substituted with $R^a$; or when $X^4$ is $NR^6$ and $X^5$ is $C(R^4)(R^5)$, $R^4$ and $R^6$ together with the atoms to which they are attached form $C_3$-$C_{10}$ heterocyclyl, wherein the $C_3$-$C_{10}$ heterocyclyl is optionally substituted with $R^a$; or $R^6$ is selected from H and $C_1$-$C_6$ alkyl; or when $X^4$ is $NR^6$ and $X^5$ is $C(R^4)(R^5)$, $R^4$ and $R^6$ together with the atoms to which they are attached form $C_3$-$C_{10}$ heterocyclyl. In the embodiments, each $R^a$ is halogen; and the halogen may be selected from F and Cl.

In some embodiments, $X^3$ is selected from S.

In some embodiments, $X^5$ is selected from $C(R^4)(R^5)$.

In some embodiments, $X^5$ is selected from $C(R^4)(R^5)$ and $NR^6$; or $X^5$ is selected from $C(R^4)(R^5)$; wherein $R^4$ and $R^5$ are each independently selected from H and $C_1$-$C_6$ alkyl, wherein the $C_1$-$C_6$ alkyl is optionally substituted with $R^a$; or $R^4$ and $R^5$ are merged into =O; or $R^4$ and $R^5$ together with the C to which they are attached form $C_3$-$C_{10}$ cycloalkyl, wherein the $C_3$-$C_{10}$ cycloalkyl is optionally substituted with $R^a$; or $R^4$ and $R^5$ together with the atoms to which they are attached form $C_3$-$C_{10}$ heterocyclyl, wherein the $C_3$-$C_{10}$ heterocyclyl is optionally substituted with $R^a$; or when $X^4$ is $NR^6$ and $X^5$ is $C(R^4)(R^5)$, $R^4$ and $R^6$ together with the atoms to which they are attached form $C_3$-$C_{10}$ heterocyclyl, wherein the $C_3$-$C_{10}$ heterocyclyl is optionally substituted with $R^a$; or $R^4$ and $R^5$ are each independently selected from H and $C_1$-$C_6$ alkyl, wherein the $C_1$-$C_6$ alkyl is optionally substituted with $R^a$; or $R^4$ and $R^5$ are merged into =O; or $R^4$ and $R^5$ together with the C to which they are attached form $C_3$-$C_{10}$ cycloalkyl, wherein the $C_3$-$C_{10}$ cycloalkyl is optionally substituted with $R^a$; or $R^4$ and $R^5$ are each independently selected from H and $C_1$-$C_6$ alkyl; or $R^4$ and $R^5$ are merged into =O; or $R^4$ and $R^5$ together with the C to which they are attached form $C_3$-$C_{10}$ cycloalkyl; wherein $R^6$ is selected from H and $C_1$-$C_6$ alkyl, wherein the $C_1$-$C_6$ alkyl is optionally substituted with $R^a$; or $R^6$ is selected from H and $C_1$-$C_6$ alkyl.

In some implementations, $R^3$, $R^4$, $R^5$, and $R^6$ are independently selected from H, halogen, CN, OH, $NH_2$, $C_1$-$C_6$ alkyl, $C_3$-$C_{10}$ cycloalkyl, and 4- to 7-membered heterocyclyl, wherein the OH, $NH_2$, $C_1$-$C_6$ alkyl, $C_3$-$C_{10}$ cycloalkyl, or 4- to 7-membered heterocyclyl is optionally substituted with $R^a$; or $R^4$ and $R^5$ are merged into =O; or $R^4$ and $R^5$ together with the C to which they are attached form $C_3$-$C_{10}$ cycloalkyl, wherein the $C_3$-$C_{10}$ cycloalkyl is optionally substituted with $R^a$; or $R^4$ and $R^5$ together with the atoms to which they are attached form $C_3$-$C_{10}$ heterocyclyl, wherein the $C_3$-$C_{10}$ heterocyclyl is optionally substituted with $R^a$; or $R^4$ and $R^6$ together with the atoms to which they are attached form $C_3$-$C_{10}$ heterocyclyl, wherein the $C_3$-$C_{10}$ heterocyclyl is optionally substituted with $R^a$.

In some embodiments, $R^3$, $R^4$, $R^5$, and $R^6$ are independently selected from H, halogen, CN, $C_1$-$C_6$ alkyl, $C_2$-$C_6$ alkenyl, -C(O)-$C_1$-$C_6$ alkyl, -C(O)O-$C_1$-$C_6$ alkyl, $C_3$-$C_{10}$ cycloalkyl, and 4- to 7-membered heterocyclyl, wherein the $C_1$-$C_6$ alkyl, $C_2$-$C_6$ alkenyl, $C_3$-$C_{10}$ cycloalkyl, or 4- to 7-membered heterocyclyl is optionally substituted with $R^a$.

In some embodiments, $R^3$, $R^4$, $R^5$, and $R^6$ are independently selected from H, halogen, $C_1$-$C_6$ alkyl, $C_2$-$C_6$ alkenyl, C(O)-$C_1$-$C_6$ alkyl, -C(O)O-$C_1$-$C_6$ alkyl, and $C_3$-$C_{10}$ cycloalkyl, wherein the $C_1$-$C_6$ alkyl, $C_2$-$C_6$ alkenyl, or $C_3$-$C_{10}$ cycloalkyl is optionally substituted with $R^a$.

In some embodiments, $R^4$ and $R^5$ are each independently selected from H, halogen, $C_1$-$C_6$ alkyl, and $C_2$-$C_6$ alkynyl, wherein the $C_1$-$C_6$ alkyl or the $C_2$-$C_6$ alkynyl is optionally substituted with $R^a$; or $R^4$ and $R^5$ are merged into =O or =S; or $R^4$ and $R^5$ together with the C to which they are attached form $C_3$-$C_{10}$ cycloalkyl, wherein the $C_3$-$C_{10}$ cycloalkyl is optionally substituted with $R^a$; or $R^4$ and $R^5$ together with the atoms to which they are attached form $C_3$-$C_{10}$ heterocyclyl, wherein the $C_3$-$C_{10}$ heterocyclyl is optionally substituted with $R^a$; or $R^4$ and $R^6$ at different positions in the ring, together with the atoms to which they are attached form $C_3$-$C_{10}$ heterocyclyl, wherein the $C_3$-$C_{10}$ heterocyclyl is optionally substituted with $R^a$.

In some embodiments, $R^4$ and $R^5$ are each independently selected from H, halogen, $C_1$-$C_6$ alkyl, and $C_2$-$C_6$ alkynyl, wherein the $C_1$-$C_6$ alkyl is optionally substituted with $R^a$; or $R^4$ and $R^5$ are merged into =O or =S; or $R^4$ and $R^5$ together with the C to which they are attached form $C_3$-$C_{10}$ cycloalkyl; or $R^4$ and $R^5$ together with the atoms to which they are attached form $C_3$-$C_{10}$ heterocyclyl; or $R^4$ and $R^6$ at different positions in the ring, together with the atoms to which they are attached form $C_3$-$C_{10}$ heterocyclyl.

In some embodiments, $R^4$ and $R^5$ are each independently selected from H, halogen, $C_1$-$C_4$ alkyl, and $C_2$-$C_3$ alkynyl, wherein the $C_1$-$C_4$ alkyl is optionally substituted with $R^a$; or $R^4$ and $R^5$ are merged into =O or =S; or $R^4$ and $R^5$ together with the C to which they are attached form $C_3$-$C_7$ cycloalkyl; or $R^4$ and $R^5$ together with the atoms to which they are attached form $C_3$-$C_6$ heterocyclyl; or $R^4$ and $R^6$ at different positions in the ring, together with the atoms to which they are attached form $C_3$-$C_6$ heterocyclyl. In the embodiments, each $R^a$ is independently selected from halogen and OH; and each halogen is independently selected from F and Cl.

In some embodiments, $R^6$ is selected from H, $C_1$-$C_6$ alkyl, $C_2$-$C_6$ alkenyl, -C(O)-$C_1$-$C_6$ alkyl, -C(O)O-$C_1$-$C_6$ alkyl, and $C_3$-$C_{10}$ cycloalkyl, wherein the $C_1$-$C_6$ alkyl, $C_2$-$C_6$ alkenyl, or $C_3$-$C_{10}$ cycloalkyl is optionally substituted with $R^a$; or $R^6$ is selected from H, $C_1$-$C_6$ alkyl, and $C_3$-$C_{10}$ cycloalkyl, wherein the $C_1$-$C_6$ alkyl is optionally substituted with $R^a$; or $R^6$ is selected from H, $C_1$-$C_4$ alkyl, and $C_3$-$C_6$ cycloalkyl, wherein the $C_1$-$C_4$ alkyl is optionally substituted with $R^a$; or $R^6$ is selected from H, $C_1$-$C_4$ alkyl, and $C_3$-$C_4$ cycloalkyl, wherein the $C_1$-$C_4$ alkyl is optionally substituted with $R^a$. In the embodiments, each $R^a$ is independently selected from halogen and OH; and each halogen is independently selected from F and Cl.

In some embodiments, when $X^4$ is $NR^6$ and $X^5$ is $C(R^4)(R^5)$, $R^4$ and $R^6$ together with the atoms to which they are attached form $C_3$-$C_6$ heterocyclyl, wherein the $C_3$-$C_6$ heterocyclyl is optionally substituted with $R^a$; or when $X^4$ is $NR^6$ and $X^5$ is $C(R^4)(R^5)$, $R^4$ and $R^6$ together with the atoms to which they are attached form $C_4$-$C_5$ heterocyclyl, wherein the $C_4$-$C_5$ heterocyclyl is optionally substituted with $R^a$.

In some embodiments, ring A is selected from phenyl and 5- to 6-membered heteroaryl, wherein the phenyl or the 5- to 6-membered heteroaryl is optionally substituted with $R^b$.

In some embodiments, ring A is selected from phenyl, pyridyl, pyrimidinyl, and pyrazolyl, wherein the phenyl,

pyridyl, pyrimidinyl, or pyrazolyl is optionally substituted with $R^b$.

In some embodiments, ring A is selected from phenyl, pyridyl, and pyrimidinyl, wherein the phenyl, pyridyl, or pyrimidinyl is optionally substituted with $R^b$.

In some embodiments, ring A is selected from

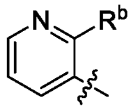 , 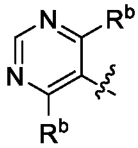 , 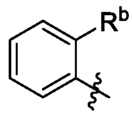 , and 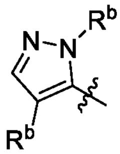 .

In some embodiments, ring A is selected from

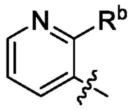 , 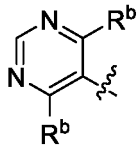 , and 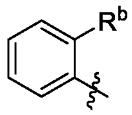 .

In the embodiments described above, each $R^b$ is independently selected from halogen, OH, $C_1$-$C_6$ alkyl, $C_3$-$C_{10}$ cycloalkyl, and

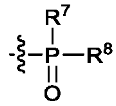 ,

wherein the OH, $C_1$-$C_6$ alkyl, or $C_3$-$C_{10}$ cycloalkyl is optionally substituted with $R^a$, wherein $R^a$ is selected from $C_1$-$C_6$ alkyl and $C_3$-$C_{10}$ cycloalkyl, the $C_1$-$C_6$ alkyl or the $C_3$-$C_{10}$ cycloalkyl is optionally substituted with $R^e$, and $R^e$ is selected from halogen; or each $R^b$ is independently selected from halogen, OH, $C_1$-$C_4$ alkyl, and $C_3$-$C_6$ cycloalkyl, wherein the OH, $C_1$-$C_4$ alkyl, or $C_3$-$C_6$ cycloalkyl is optionally substituted with $R^a$, wherein $R^a$ is selected from $C_1$-$C_4$ alkyl and $C_3$-$C_6$ cycloalkyl, the $C_1$-$C_4$ alkyl or the $C_3$-$C_6$ cycloalkyl is optionally substituted with $R^e$, and $R^e$ is selected from halogen; or each $R^b$ is independently selected from F, Cl, OH, $C_1$-$C_3$ alkyl, and $C_3$ cycloalkyl, wherein the OH is substituted with $R^a$, wherein $R^a$ is selected from $C_1$-$C_2$ alkyl and $C_3$ cycloalkyl, the $C_1$-$C_2$ alkyl is optionally substituted with $R^e$, and $R^e$ is selected from F and Cl. In some embodiments, ring B is selected from aryl, 5- to 6-membered heteroaryl, 4- to 10-membered heterocyclyl, $C_3$-$C_{10}$ cycloalkyl, and $C_3$-$C_{10}$ cycloalkenyl, wherein the aryl, 5- to 6-membered heteroaryl, 4- to 10-membered heterocyclyl, $C_3$-$C_{10}$ cycloalkyl, or $C_3$-$C_{10}$ cycloalkenyl is optionally substituted with $R^c$.

In some embodiments, ring B is selected from phenyl, 5- to 6-membered heteroaryl, 4- to 6-membered heterocyclyl, $C_3$-$C_8$ cycloalkyl, and $C_4$-$C_6$ cycloalkenyl, wherein the phenyl, 5- to 6-membered heteroaryl, 4- to 6-membered heterocyclyl, $C_3$-$C_8$ cycloalkyl, or $C_4$-$C_6$ cycloalkenyl is optionally substituted with $R^c$.

In some embodiments, ring B is selected from phenyl, 4- to 6-membered heterocyclyl, $C_3$-$C_8$ cycloalkyl, and $C_4$-$C_6$ cycloalkenyl, wherein the phenyl, 4- to 6-membered heterocyclyl, $C_3$-$C_8$ cycloalkyl, or $C_4$-$C_6$ cycloalkenyl is optionally substituted with $R^c$.

In some embodiments, ring B is selected from

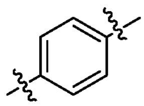 , 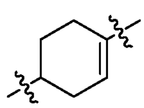 , 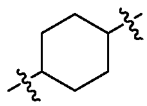 , 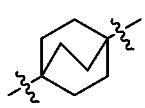 ,

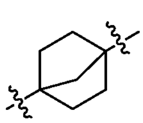 , 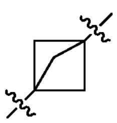 , 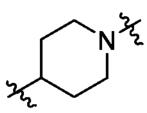 , 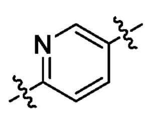 , and 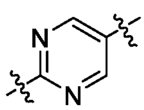 ,

wherein the

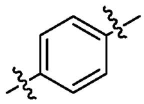,

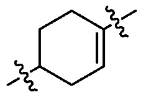, 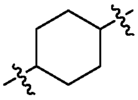, 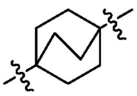, 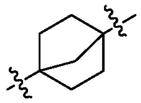, 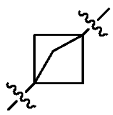, 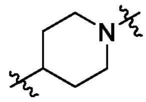, 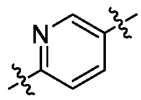, or 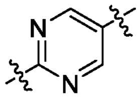

is optionally substituted with $R^c$.

In some embodiments, ring B is selected from

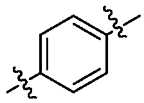, 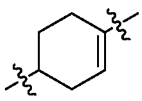, 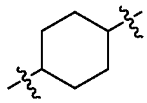, 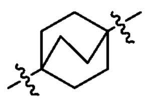,

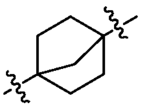, 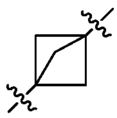, 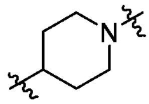, and 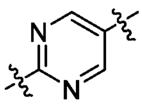,

wherein the

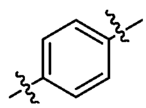, 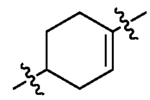,

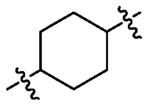, 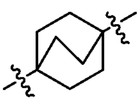, 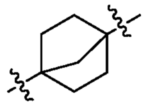, 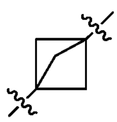, 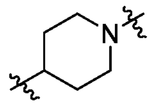, or 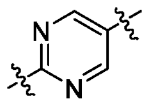

is optionally substituted with $R^c$.

In the embodiments described above, each $R^c$ is independently selected from halogen and $C_1$-$C_6$ alkyl, wherein the $C_1$-$C_6$ alkyl is optionally substituted with $R^a$; or each $R^c$ is independently selected from halogen and $C_1$-$C_4$ alkyl, wherein the $C_1$-$C_4$ alkyl is optionally substituted with $R^a$; or each $R^c$ is independently selected from F, Cl, and $C_1$-$C_4$ alkyl, wherein the $C_1$-$C_4$ alkyl is optionally substituted with $R^a$; or each $R^c$ is independently selected from F, Cl, and $C_1$-$C_2$ alkyl, wherein the $C_1$-$C_2$ alkyl is optionally substituted with $R^a$. $R^a$ is selected from halogen, such as F or Cl.

In some embodiments, $R^b$ and $R^c$ are each independently selected from halogen, OH, $NH_2$, SH, $C_1$-$C_6$ alkyl, $C_3$-$C_{10}$ cycloalkyl, 4- to 7-membered heterocyclyl,

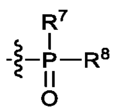, 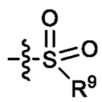, 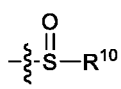,

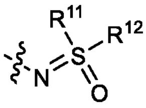, and 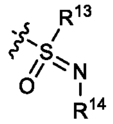,

wherein the OH, $NH_2$, SH, $C_1$-$C_6$ alkyl, $C_3$-$C_{10}$ cycloalkyl, or 4- to 7-membered heterocyclyl is optionally substituted with $R^a$.

In some embodiments, $R^b$ and $R^c$ are each independently selected from halogen, OH, $NH_2$, SH, $C_1$-$C_6$ alkyl, $C_3$-$C_{10}$ cycloalkyl, 4- to 7-membered heterocyclyl, and

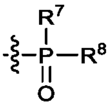,

wherein the OH, $NH_2$, SH, $C_1$-$C_6$ alkyl, $C_3$-$C_{10}$ cycloalkyl, or 4- to 7-membered heterocyclyl is optionally substituted with $R^a$.

In some embodiments, $R^7$, $R^8$, $R^9$, $R^{10}$, $R^{11}$, $R^{12}$, $R^{13}$, and $R^{14}$ are each independently selected from $C_1$-$C_6$ alkyl, $C_3$-$C_{10}$ cycloalkyl, and 4- to 7-membered heterocyclyl, wherein the $C_1$-$C_6$ alkyl, $C_3$-$C_{10}$ cycloalkyl, or 4- to 7-membered heterocyclyl is optionally substituted with $R^a$;

or $R^7$ and $R^8$ together with the P to which they are attached form 4- to 7-membered heterocyclyl, wherein the 4- to 7-membered heterocyclyl is optionally substituted with $R^a$;
or $R^{13}$ and $R^{14}$ together with the atoms to which they are attached form 4- to 7-membered heterocyclyl, wherein the 4- to 7-membered heterocyclyl is optionally substituted with $R^a$.

In some embodiments, $R^b$ and $R^c$ are each independently selected from

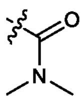, 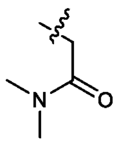, 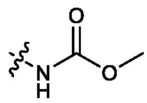,

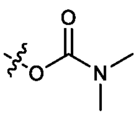, 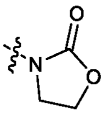, 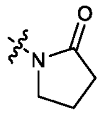, 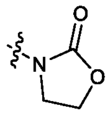, 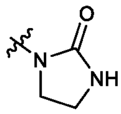, 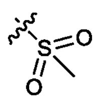, 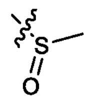, 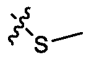, 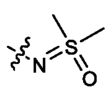, 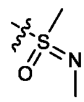,

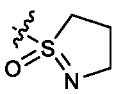, 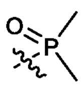, 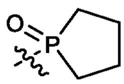, 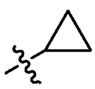, and 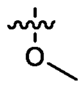.

In some embodiments, ring C is selected from aryl and 5- to 10-membered heteroaryl, wherein the aryl or the 5- to 10-membered heteroaryl is optionally substituted with $R^d$.

In some embodiments, ring C is selected from 5- to 6-membered heteroaryl, wherein the 5- to 6-membered heteroaryl is optionally substituted with $R^d$.

In some embodiments, ring C is selected from 4- to 10-membered heterocyclyl, wherein the 4- to 10-membered heterocyclyl is optionally substituted with $R^d$.

In some embodiments, $R^d$ is selected from halogen, CN, OH, $NH_2$, $C_1$-$C_6$ alkyl, and $C_3$-$C_{10}$ cycloalkyl, wherein the OH, $NH_2$, $C_1$-$C_6$ alkyl, or $C_3$-$C_{10}$ cycloalkyl is optionally substituted with $R^a$.

In some embodiments, $R^d$ is selected from $C_1$-$C_6$ alkyl and $C_3$-$C_{10}$ cycloalkyl, wherein the $C_1$-$C_6$ alkyl or the $C_3$-$C_{10}$ cycloalkyl is optionally substituted with $R^a$; or $R^d$ is selected from $C_1$-$C_4$ alkyl and $C_3$-$C_6$ cycloalkyl, wherein the $C_1$-$C_4$ alkyl is optionally substituted with $R^a$; or $R^d$ is selected from $C_1$-$C_4$ alkyl and $C_3$-$C_6$ cycloalkyl, wherein the $C_1$-$C_4$ alkyl is optionally substituted with $R^a$, wherein $R^a$ is selected from halogen, such as F or Cl; or $R^d$ is selected from $C_1$-$C_4$

alkyl optionally substituted with $R^a$, wherein $R^a$ is selected from halogen, such as F or Cl.

In some embodiments, $R^c$ and $R^d$ together with the atoms to which they are attached form 6- to 7-membered heterocyclyl or 5- to 6-membered heteroaryl, wherein the 6- to 7-membered heterocyclyl or the 5- to 6-membered heteroaryl is optionally substituted with $R^a$.

In some embodiments, ring C is selected from

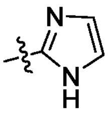, 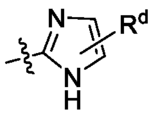, 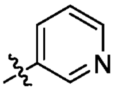, 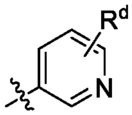, 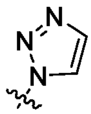,

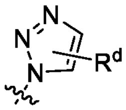, 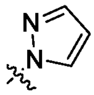, 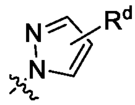, 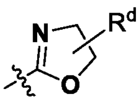, 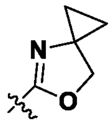, 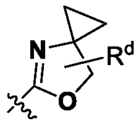, 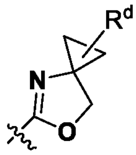, 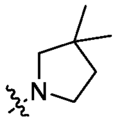,

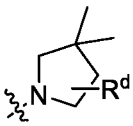, 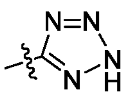, 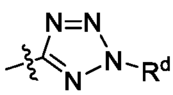, 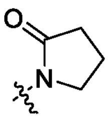, and 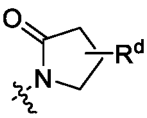.

In some embodiments, ring C is selected from

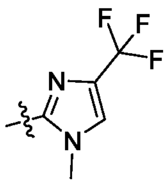.

In some embodiments, ring C is selected from

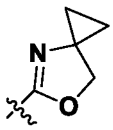, 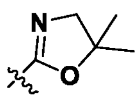, 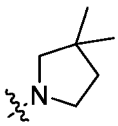, 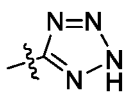,

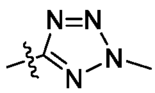, and 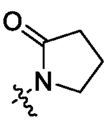.

In some embodiments, $R^1$ and $R^2$ are each independently selected from H, halogen, CN, OH, $NH_2$, and $C_1$-$C_6$ alkyl, wherein the OH, $NH_2$, or $C_1$-$C_6$ alkyl is optionally substituted with $R^a$;

or $R^1$ and $R^2$ together with the atoms to which they are attached form $C_3$-$C_6$ cycloalkyl or 4- to 7-membered heterocyclyl, wherein the $C_3$-$C_6$ cycloalkyl or the 4- to 7-membered heterocyclyl is optionally substituted with $R^a$.

In some embodiments, $R^1$ and $R^2$ are each independently selected from H, methyl, and ethyl; or $R^1$ and $R^2$ together with the atoms to which they are attached form the following rings:

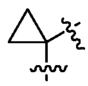, 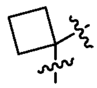,

and 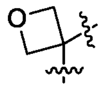,

wherein the

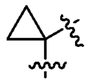, 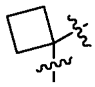, and 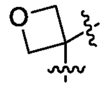

are optionally substituted with $R^a$.

In some embodiments, $R^1$ and $R^2$ are each independently selected from H; or $R^1$ and $R^2$ together with the atoms to which they are attached form the following rings:

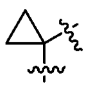, 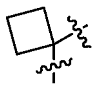, and 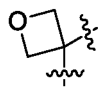,

wherein the

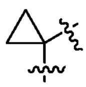, 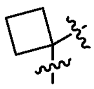, or 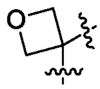

are optionally substituted with $R^a$.

In some embodiments, both $R^1$ and $R^2$ are H.

In some embodiments, each $R^a$ is independently selected from halogen, =O, OH, $NH_2$, $C_1$-$C_6$ alkyl, $C_3$-$C_6$ cycloalkyl, and 4- to 7-membered heterocyclyl, wherein the OH, $NH_2$, $C_1$-$C_6$ alkyl, $C_3$-$C_6$ cycloalkyl, or 4- to 7-membered heterocyclyl is optionally substituted with $R^e$.

In some embodiments, each $R^a$ is independently selected from halogen, OH, $C_1$-$C_6$ alkyl, and $C_3$-$C_6$ cycloalkyl, wherein the OH, $C_1$-$C_6$ alkyl, or $C_3$-$C_6$ cycloalkyl is optionally substituted with $R^e$; or each $R^a$ is independently selected from F, Cl, OH, $C_1$-$C_6$ alkyl, and $C_3$-$C_6$ cycloalkyl, wherein the OH or the $C_1$-$C_6$ alkyl is optionally substituted with $R^e$.

In some embodiments, $R^e$ is selected from halogen, =O, OH, $NH_2$, $C_1$-$C_6$ alkyl, $C_3$-$C_6$ cycloalkyl, and 4- to 7-membered heterocyclyl, wherein the OH, $NH_2$, $C_1$-$C_6$ alkyl, $C_3$-$C_6$ cycloalkyl, or 4- to 7-membered heterocyclyl is optionally substituted with $R^f$.

In some embodiments, $R^e$ is halogen, such as F or Cl.

In some embodiments, $R^f$ is selected from halogen, OH, $NH_2$, $C_1$-$C_6$ alkyl, $C_3$-$C_6$ cycloalkyl, and 4-to 7-membered heterocyclyl.

In some embodiments, the compound of formula (I) or the pharmaceutically acceptable salt thereof provided in the present application is selected from a compound of formula (II) and a pharmaceutically acceptable salt thereof,

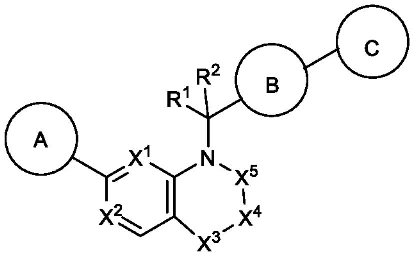

(II)

wherein $X^3$ and $X^4$ are independently selected from $C(R^4)(R^5)$, $NR^6$, O, S, and $S(=O)_2$; and ring A, ring B, ring C, $X^1$, $X^2$, $X^5$, $R^1$, $R^2$, $R^4$, $R^5$, and $R^6$ are as defined in formula (I).

In some embodiments, the compound of formula (I) or the pharmaceutically acceptable salt thereof is selected from the following compounds and pharmaceutically acceptable salts thereof,

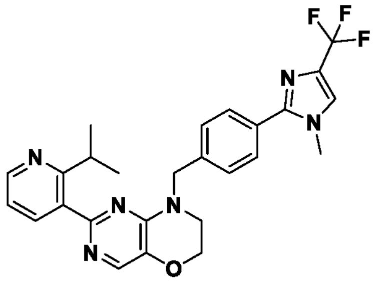 , 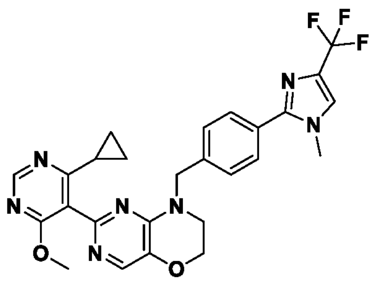 , 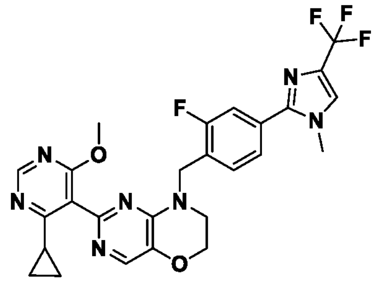 ,

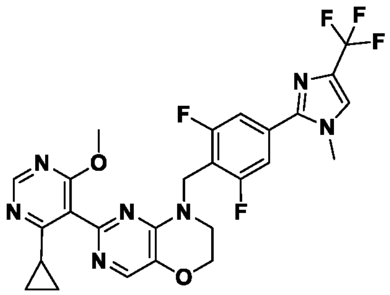 , 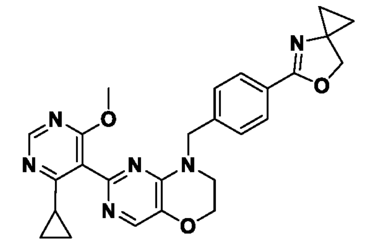 , 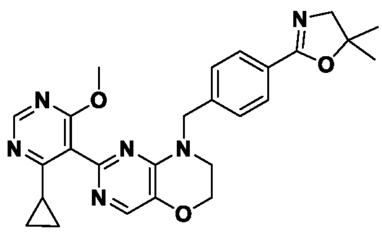 ,

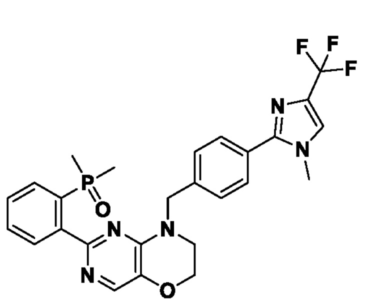 , 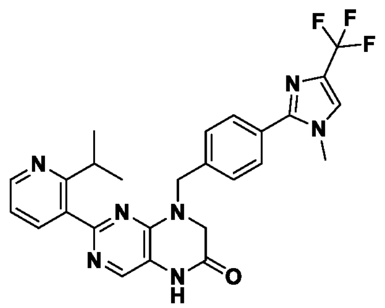 , 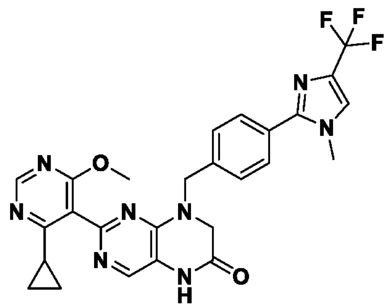 ,

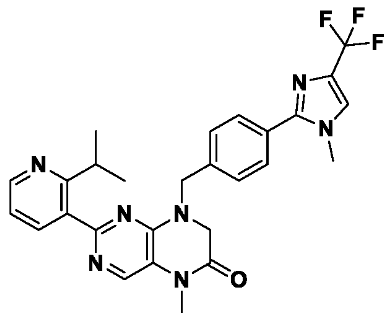
,
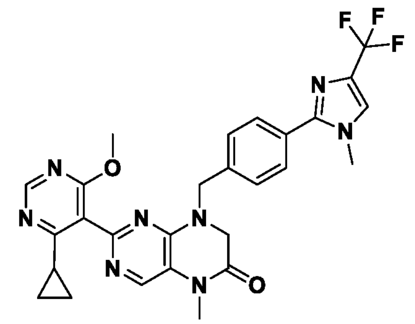
,
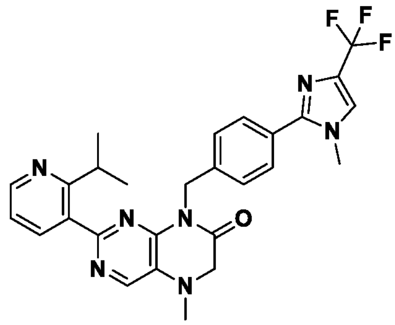
,

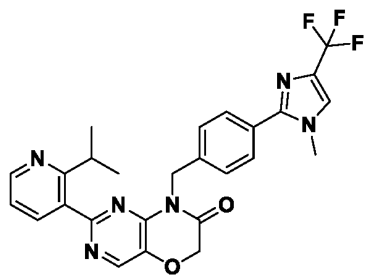
,
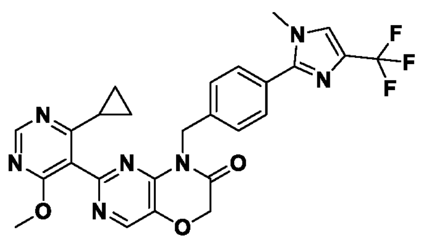
,
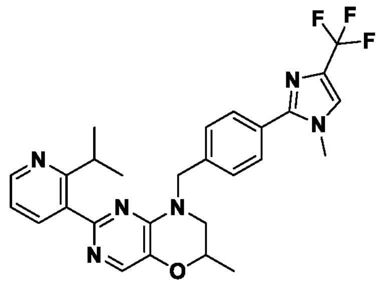
,

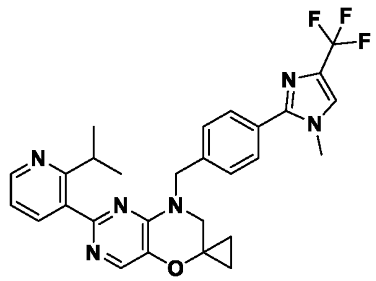
,
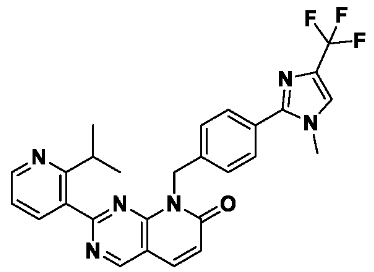
,
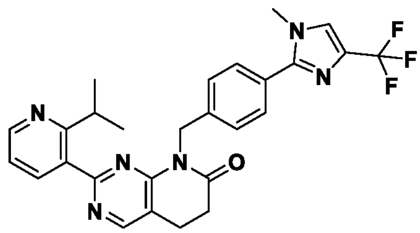
,

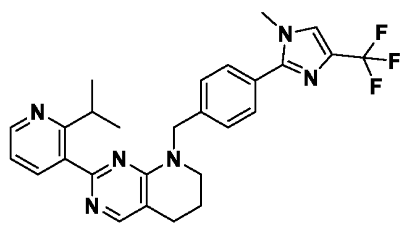
,
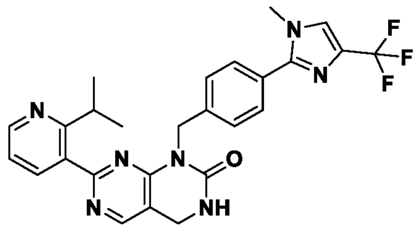
,

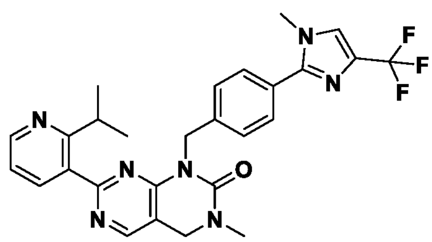
,
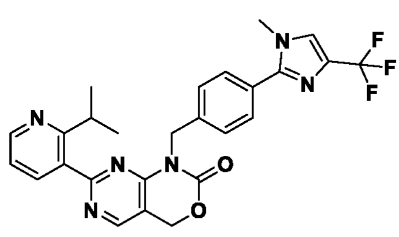
,
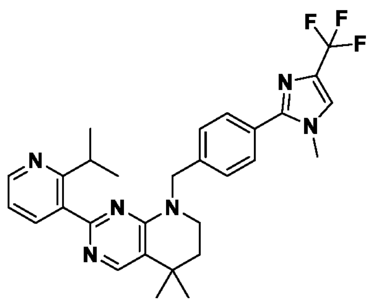
,

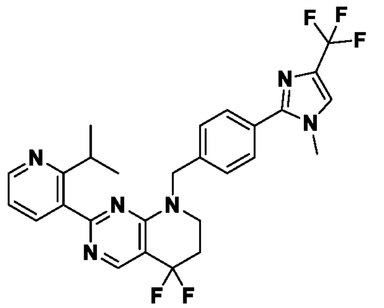,

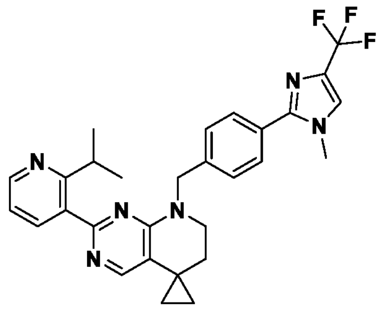,

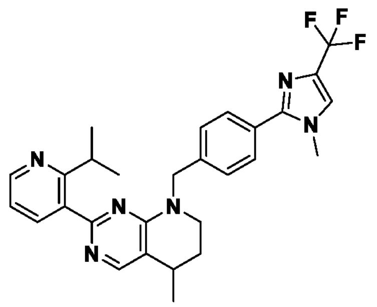,

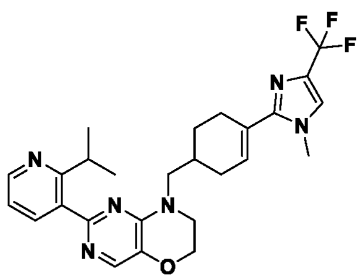,

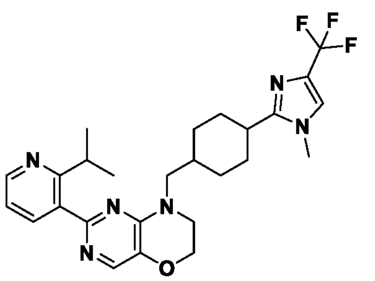,

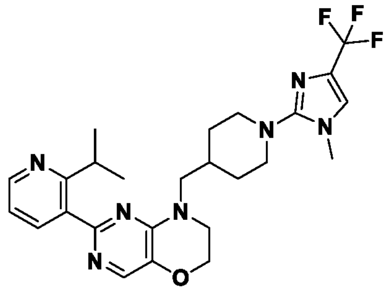,

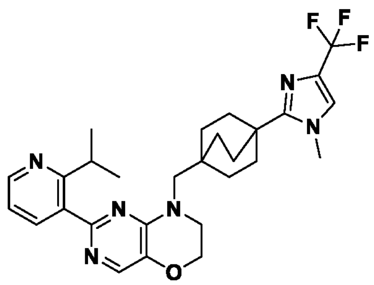,

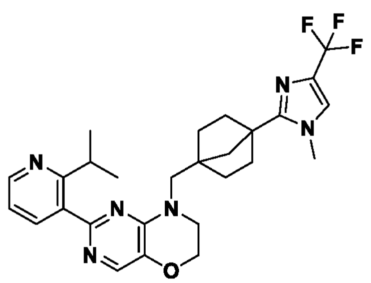,

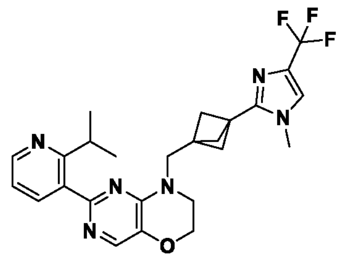,

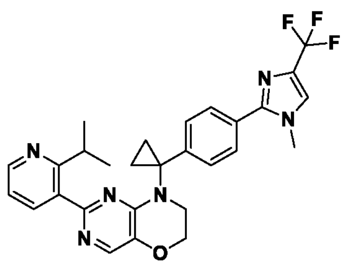,

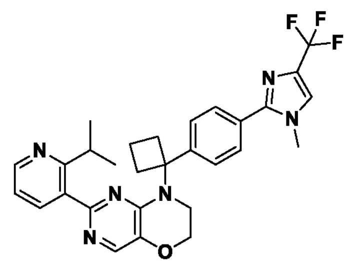,

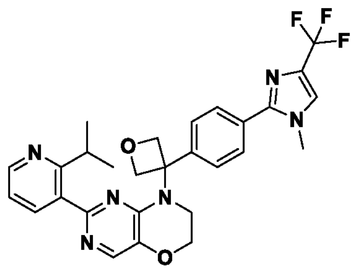,

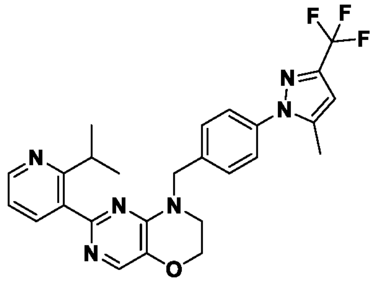,

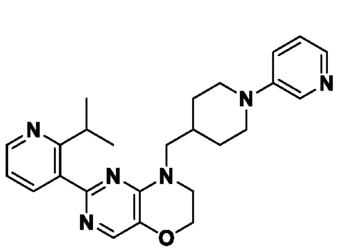,

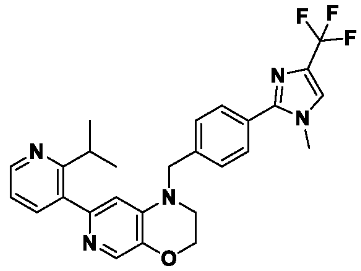,

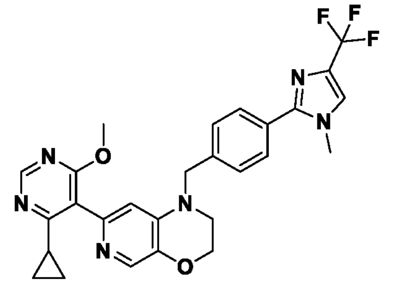 , 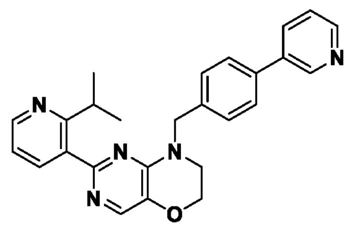 , 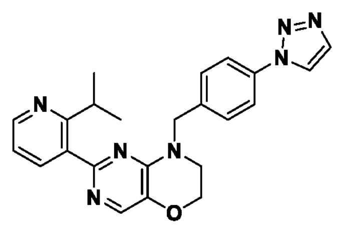 ,

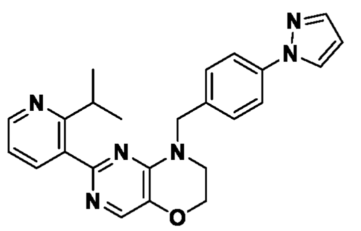 , 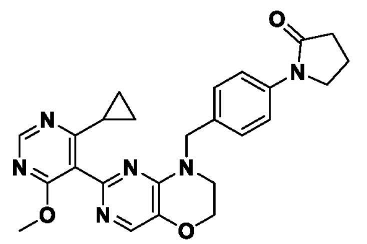 , 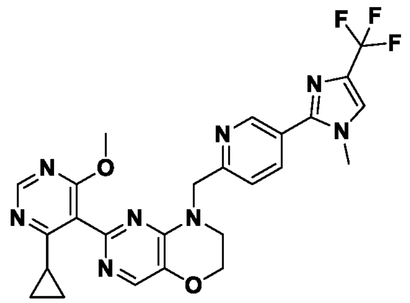 ,

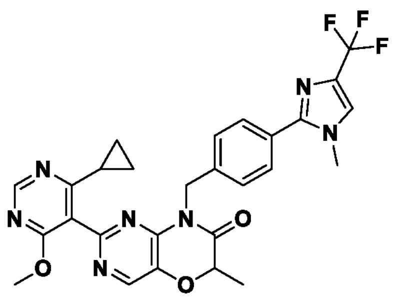 , 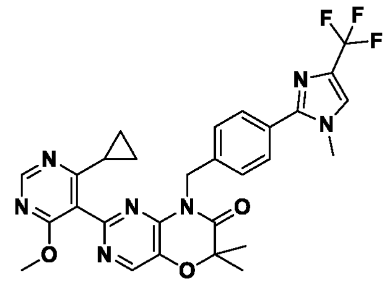 , 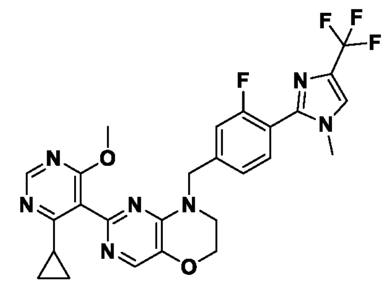 ,

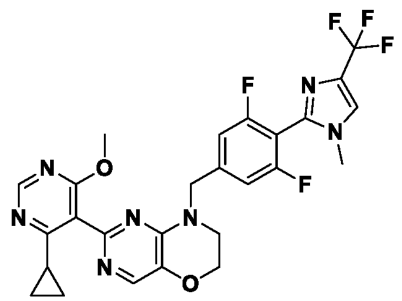 , 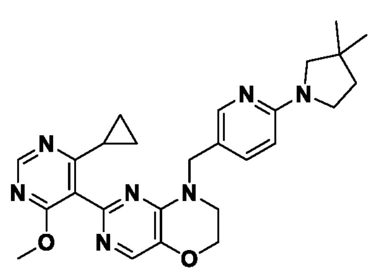 , 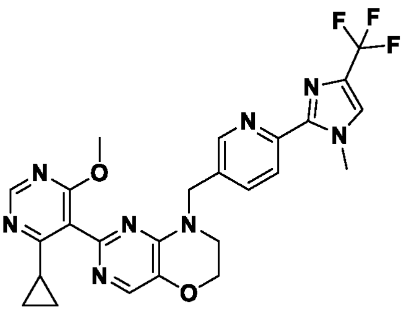 ,

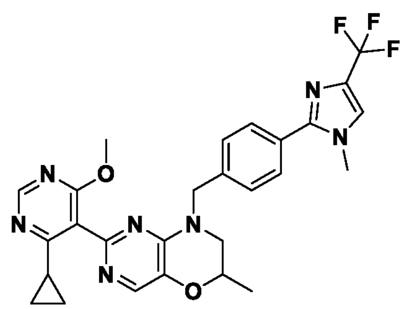 , 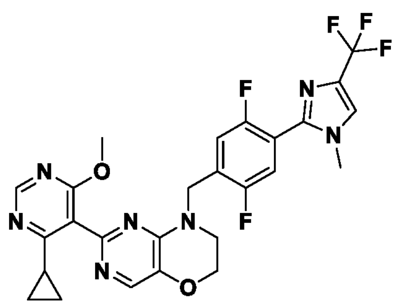 , 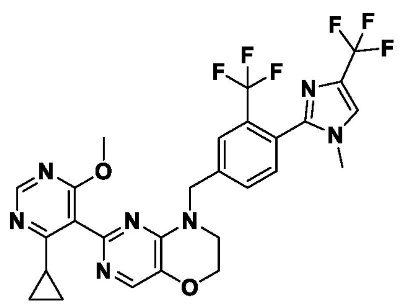 ,

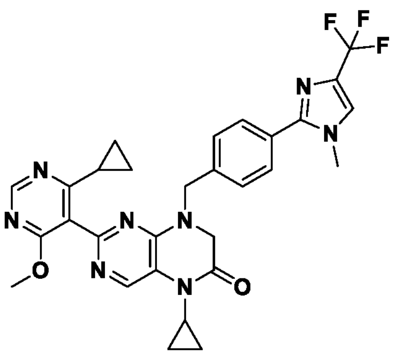 , 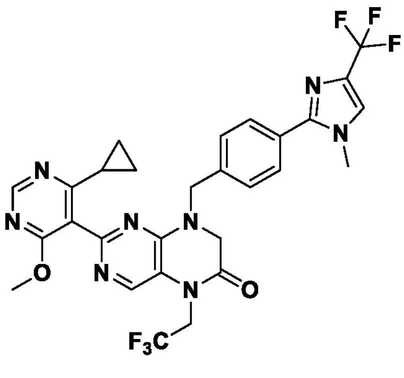 , 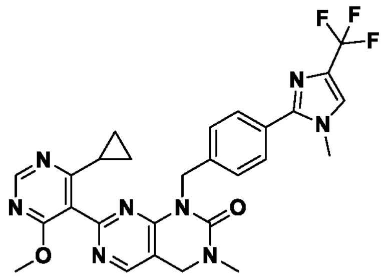 ,

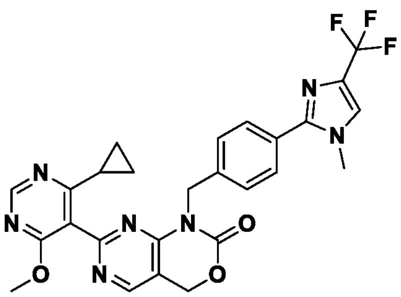 , 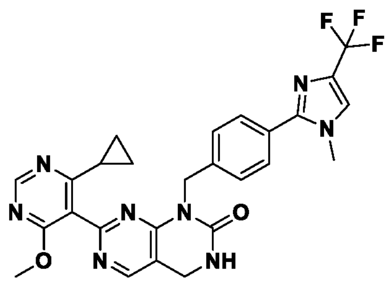 , 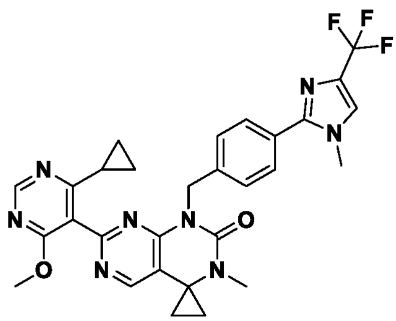 ,

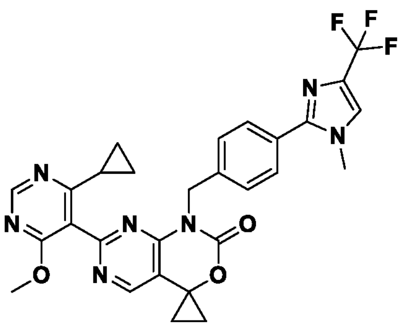 , 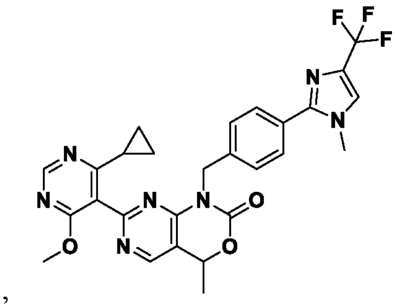 , 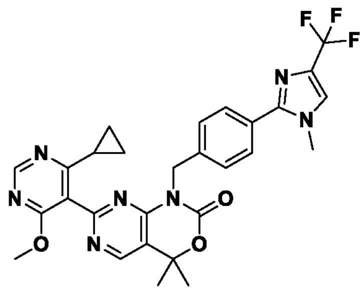 ,

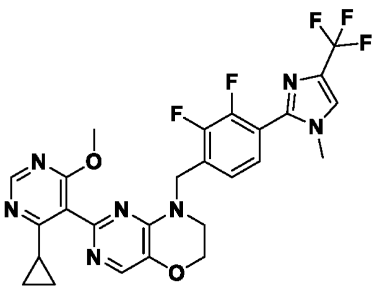 , 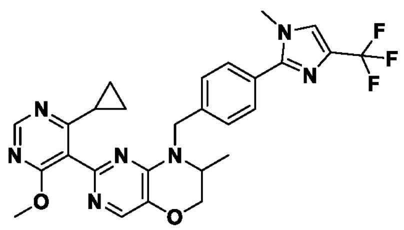 , 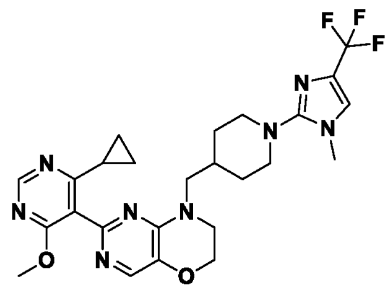 ,

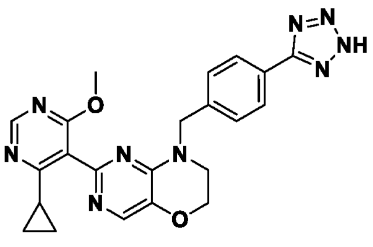 , 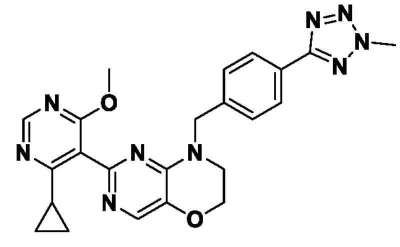 , 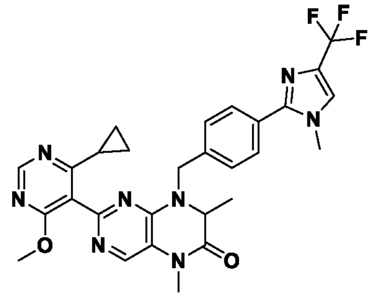 ,

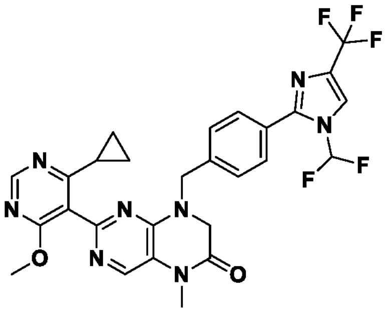,
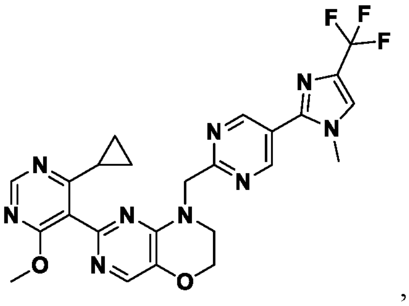,
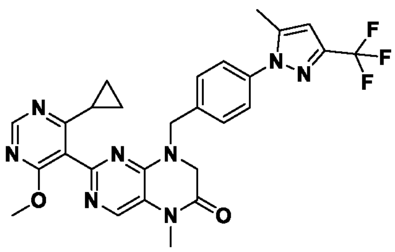,

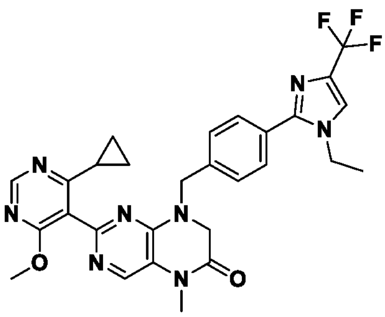,
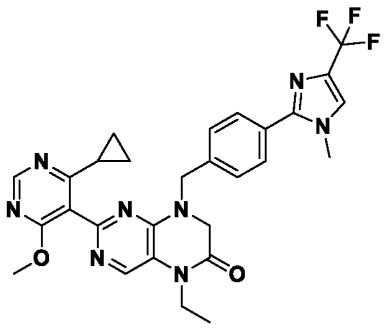,
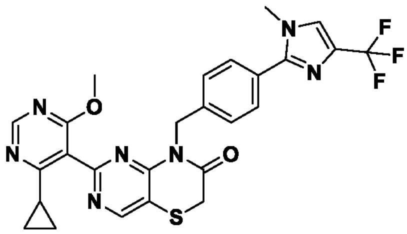,

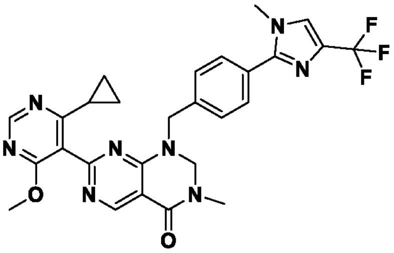,
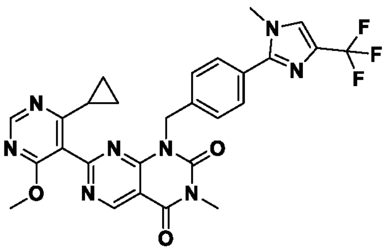,
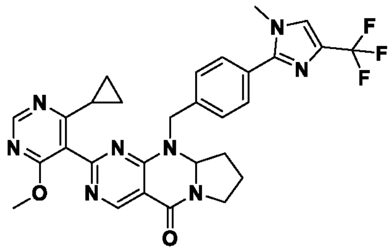,

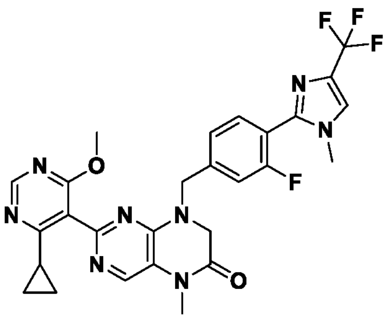,
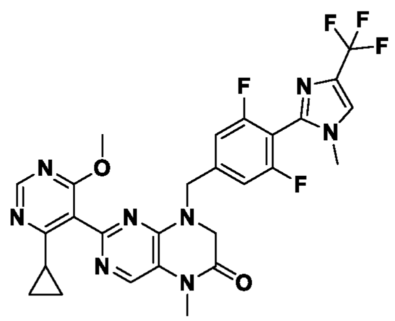,
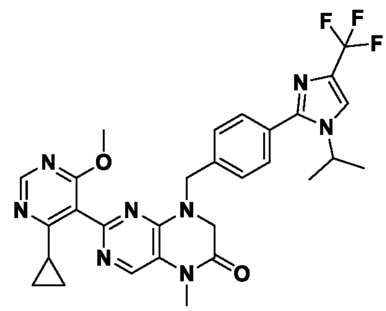,

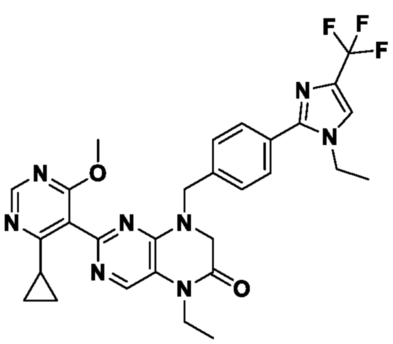,
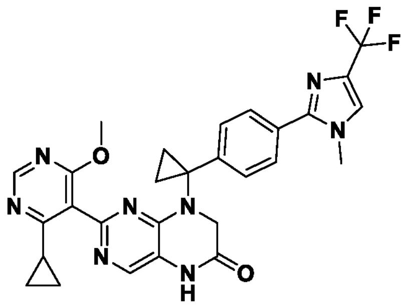,
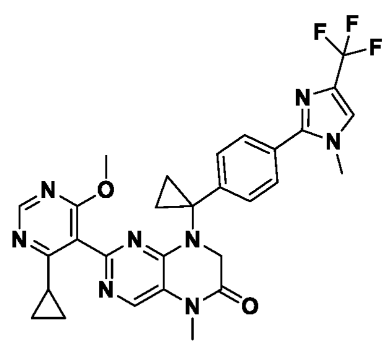,

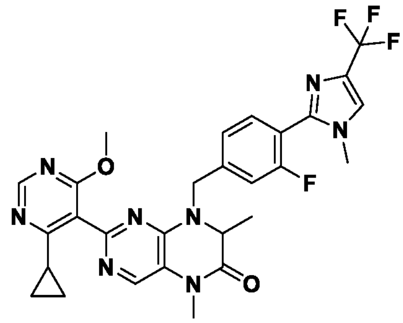,
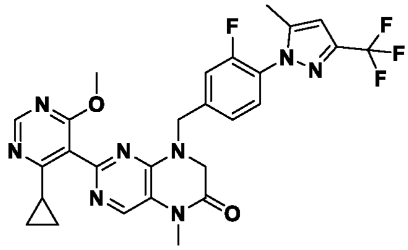,
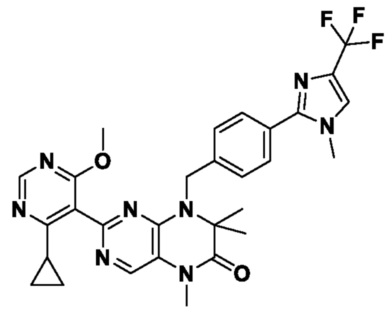,

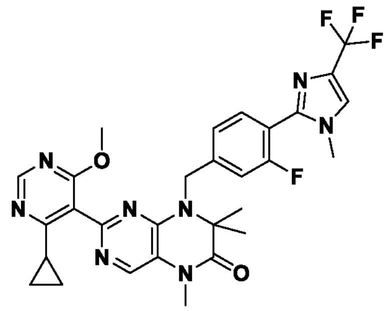,
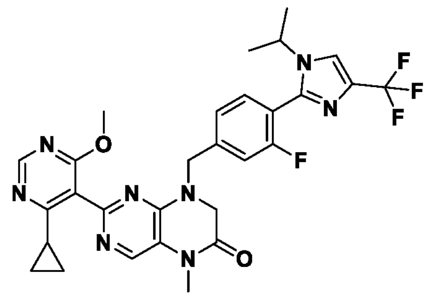,
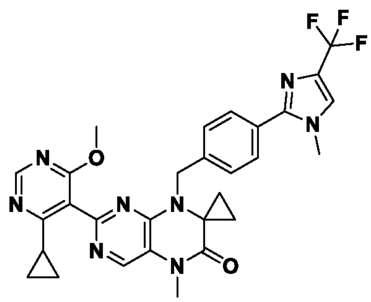,

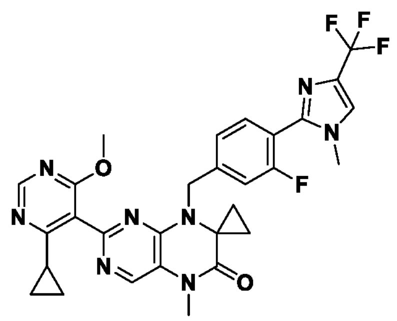,
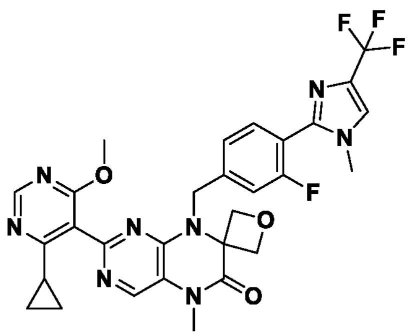,
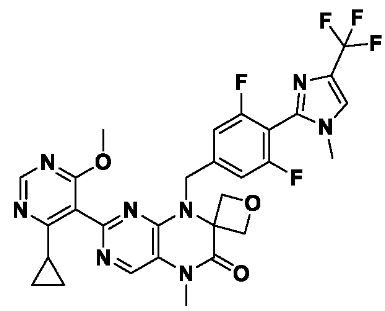,

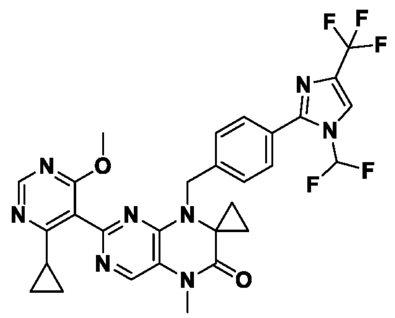,
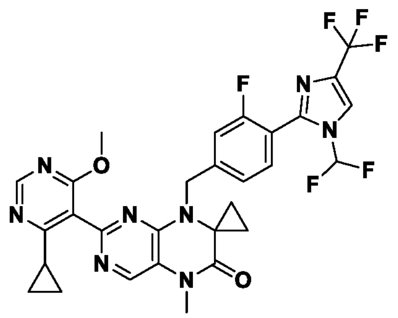,
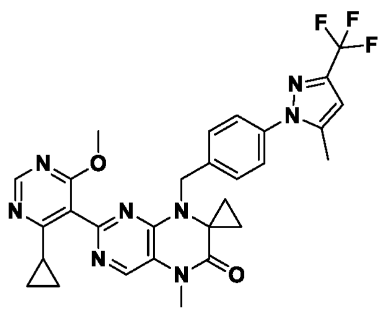,

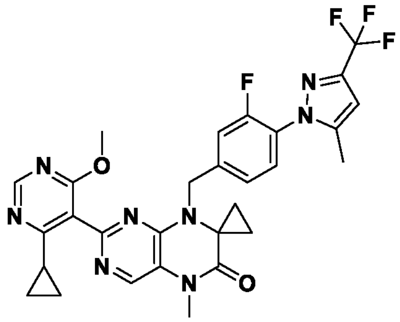
,
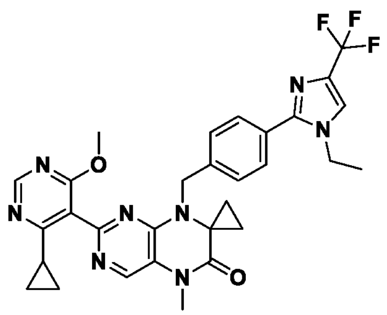
,
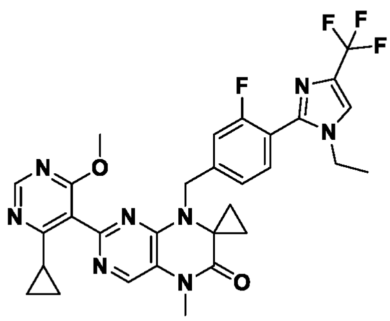
,
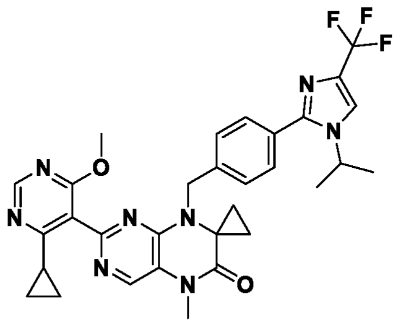
,
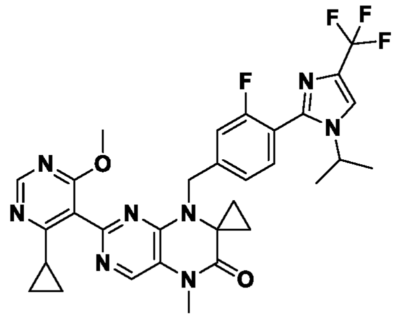
,
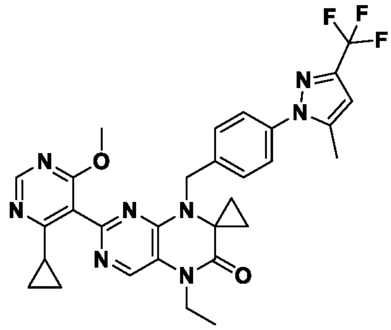
,
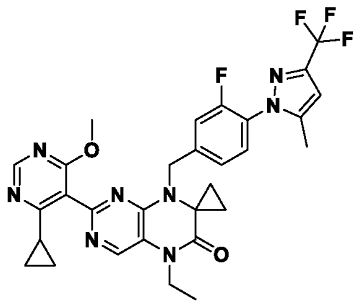
,
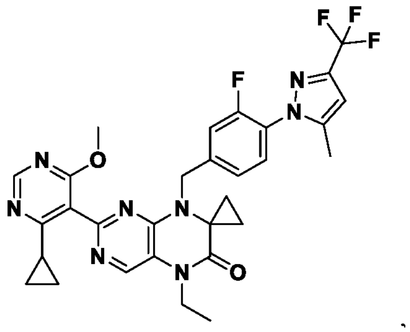
,
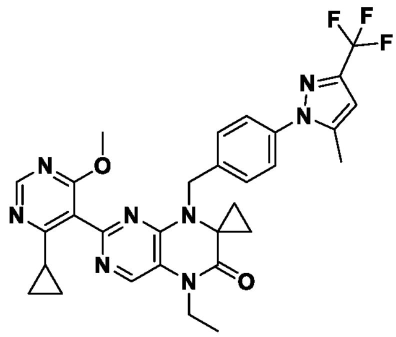
,
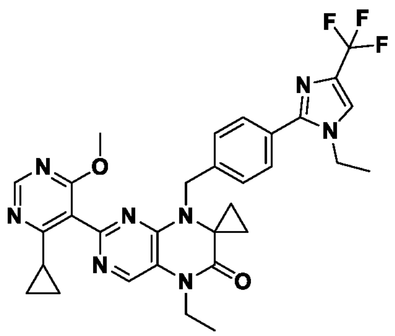
,
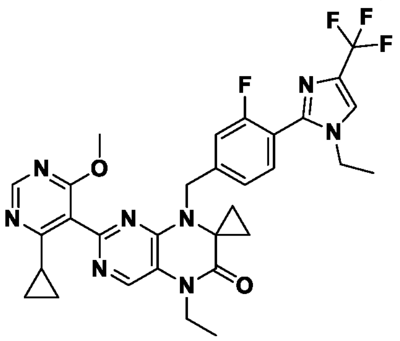
,
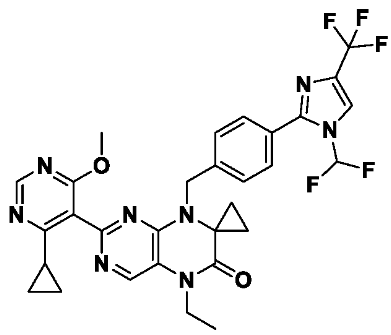
,

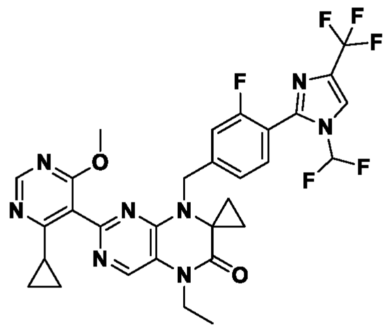,
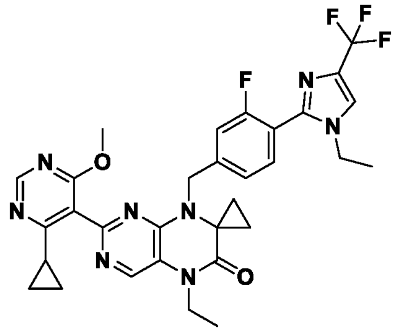,
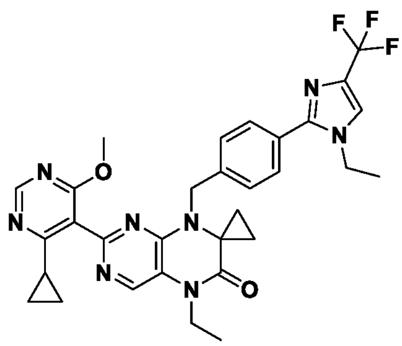,

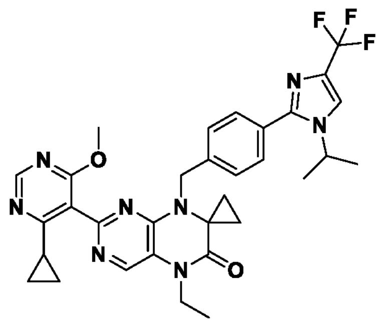,
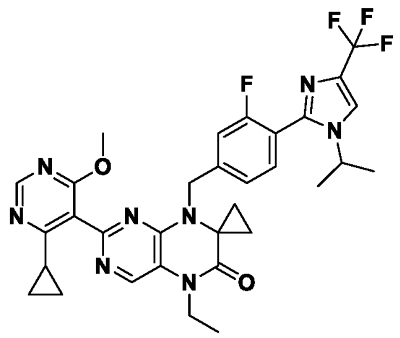,
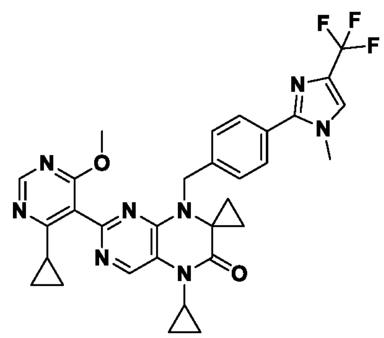,

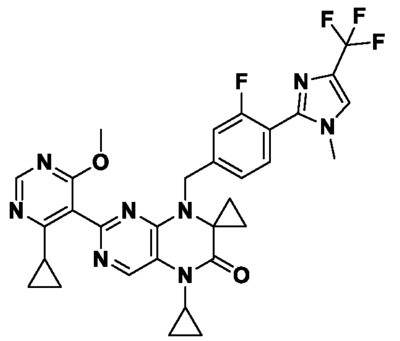,
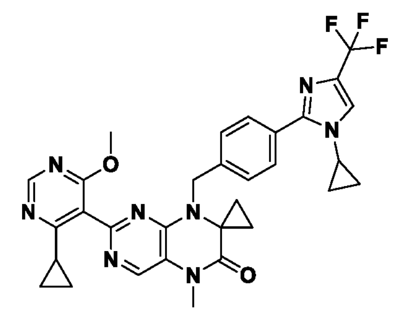,
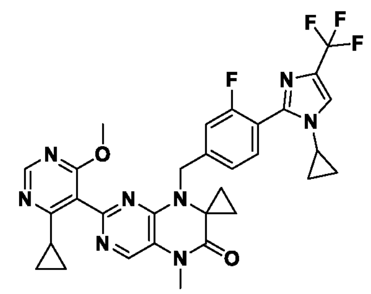,

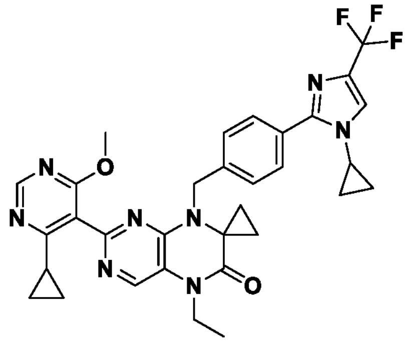,
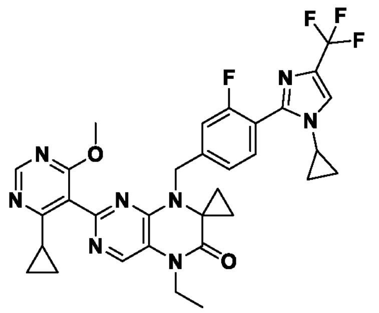,
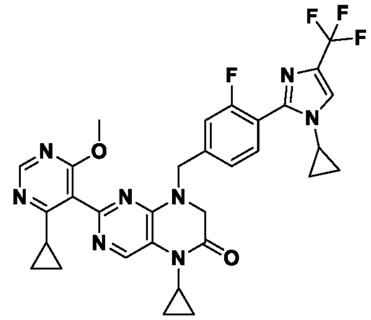,

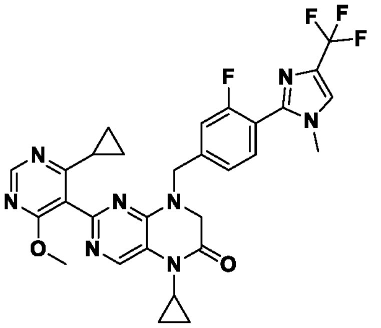
,
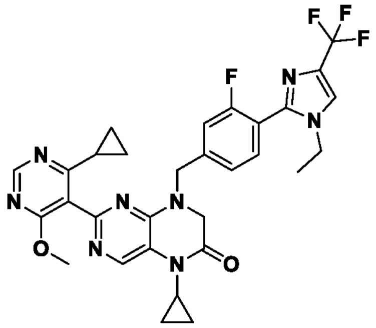
,
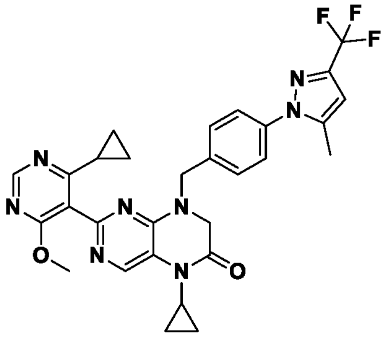
,

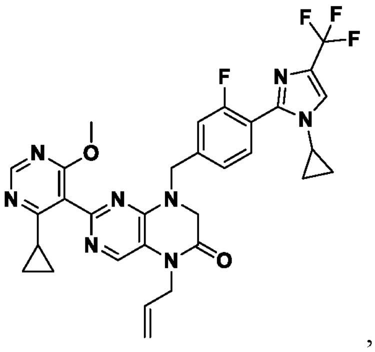
,
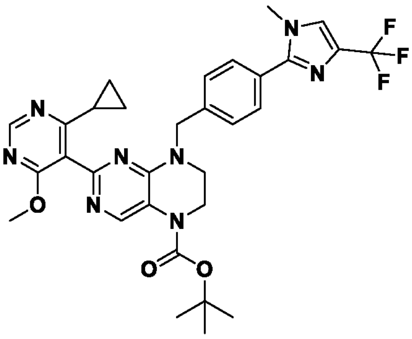
,
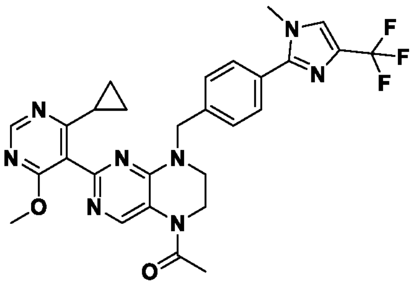
,

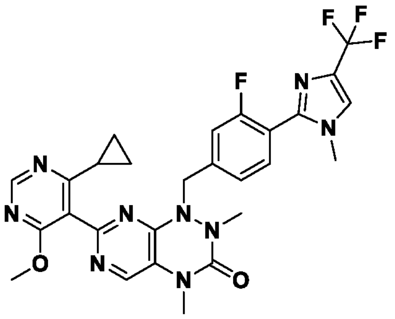
,
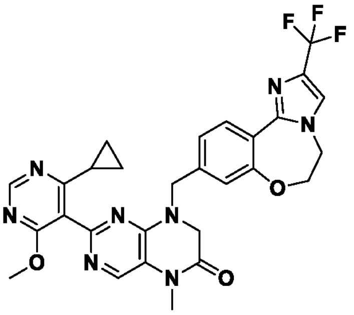
,
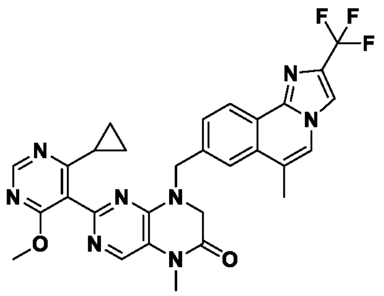
,

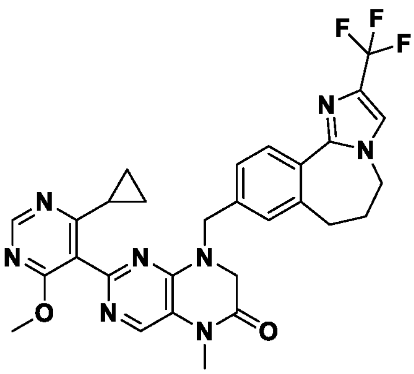
,
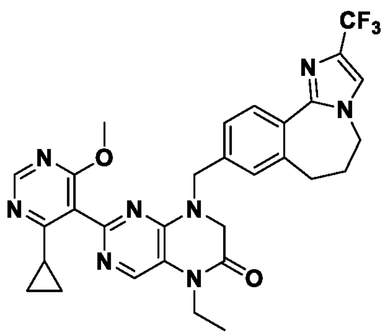
,
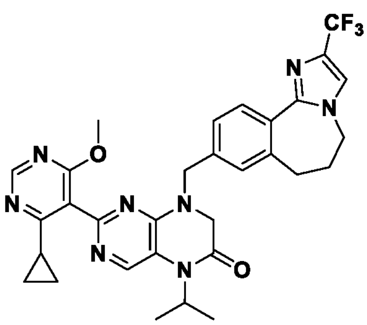
,

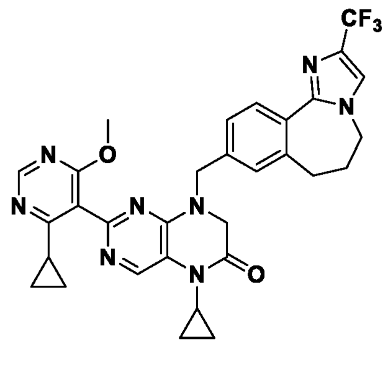
,
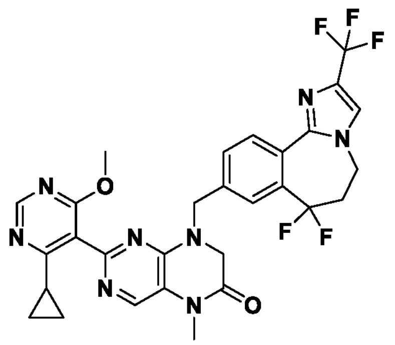
,
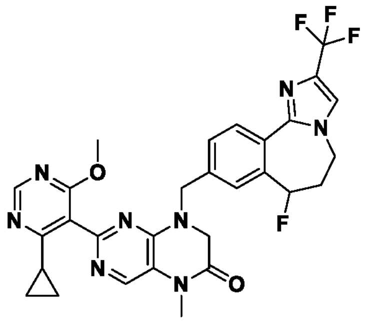
,

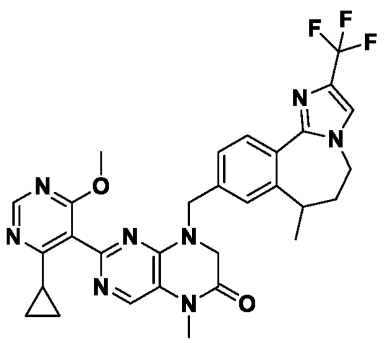
,
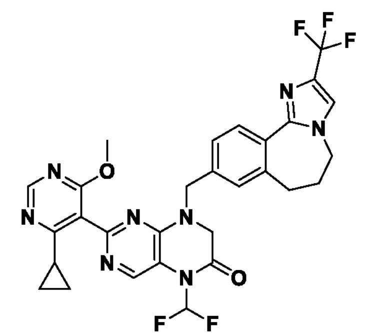
,
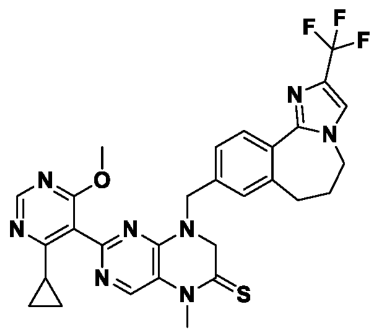
,

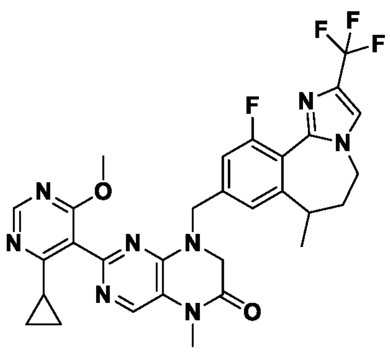
,
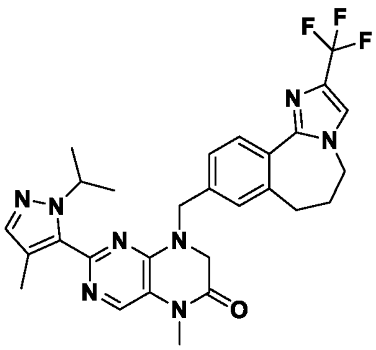
,
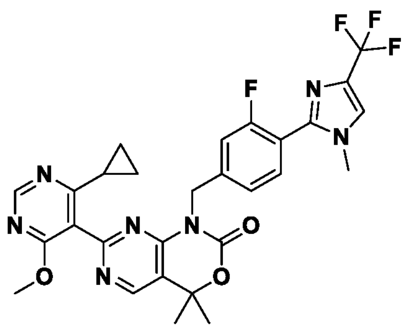
,

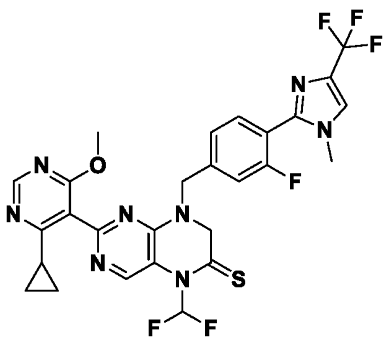
,
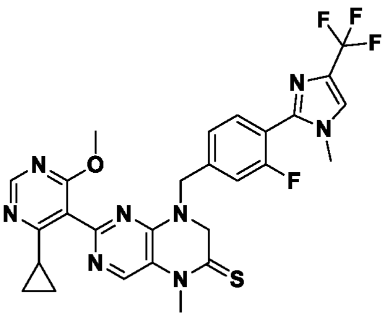
,
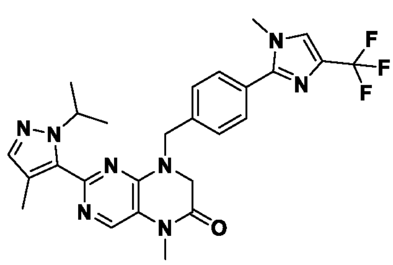
,

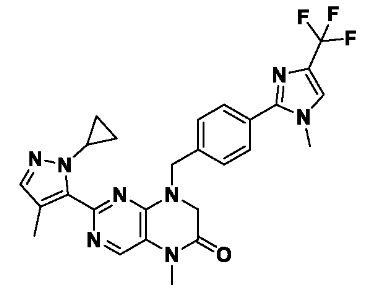,

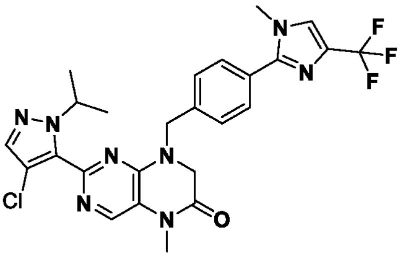,

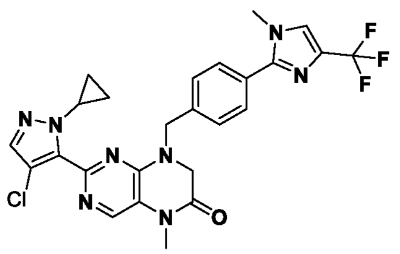,

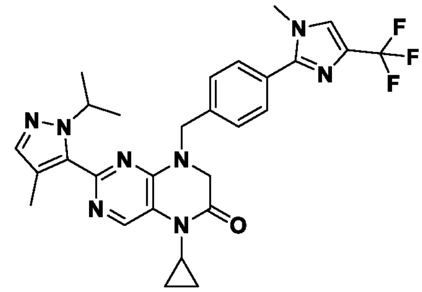,

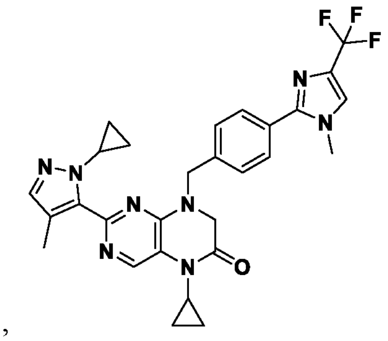,

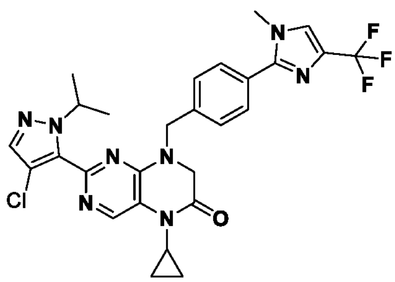,

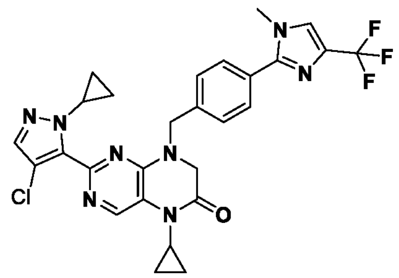,

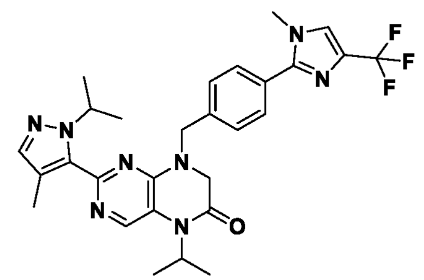,

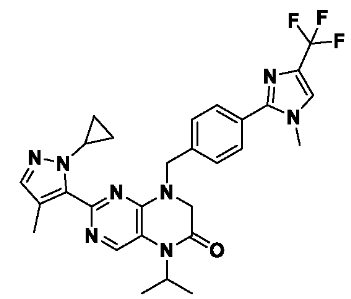,

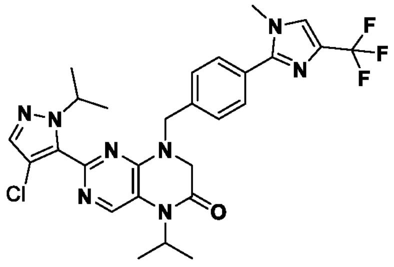,

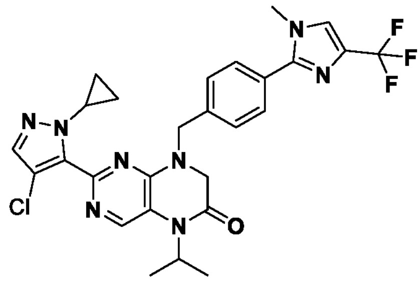,

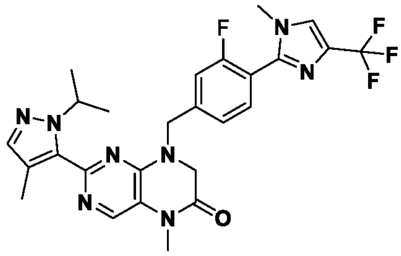,

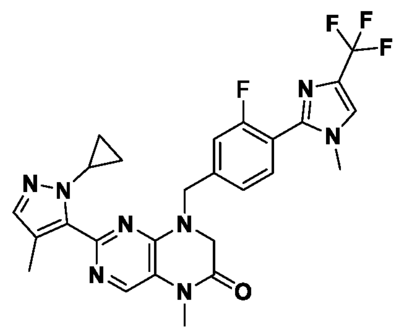,

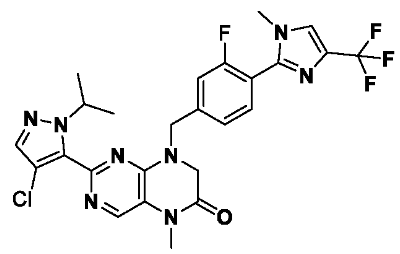,

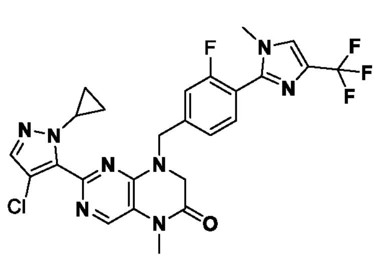 , 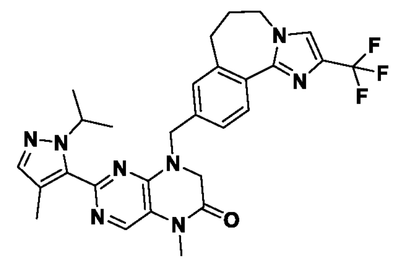 , 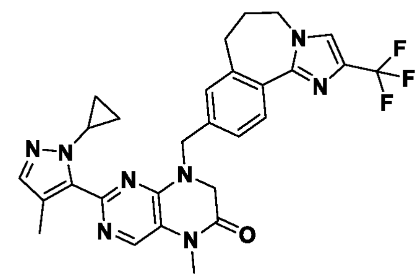 ,

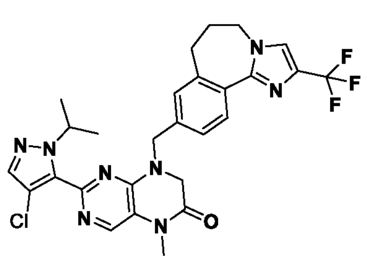 , 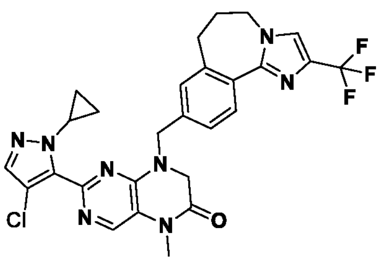 , 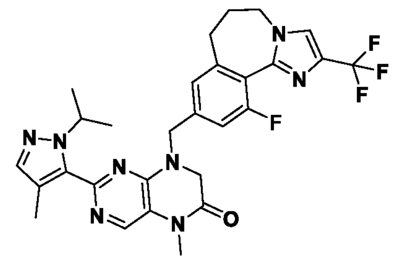 ,

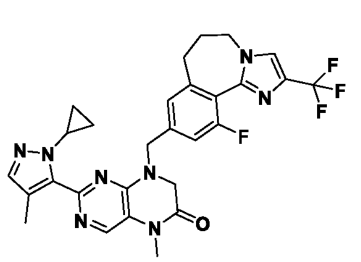 , 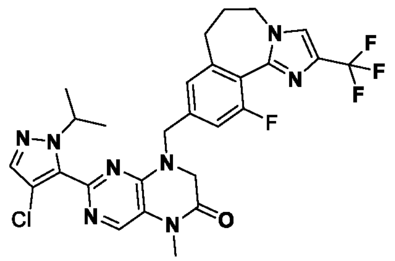 , 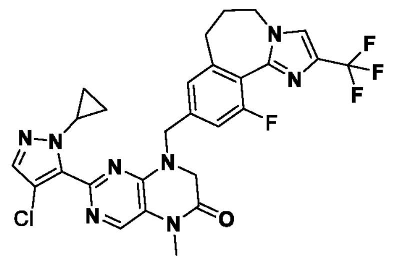 ,

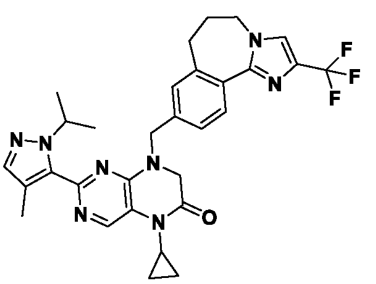 , 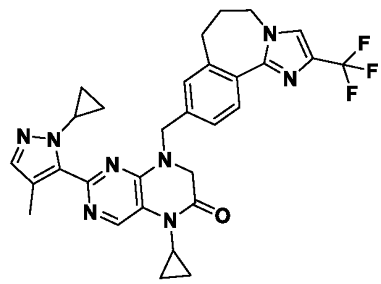 , 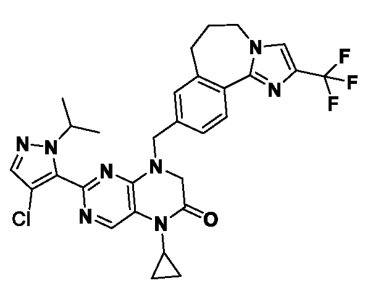 ,

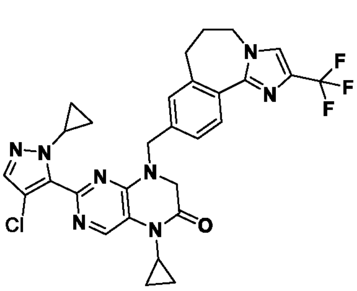 , 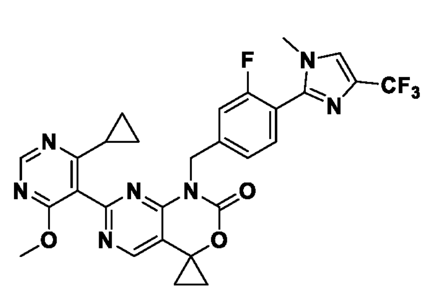 , 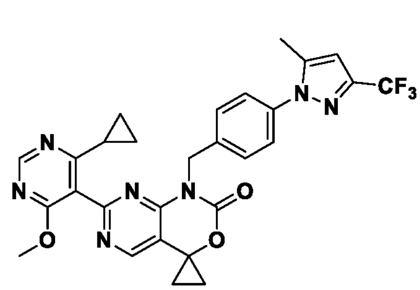 ,

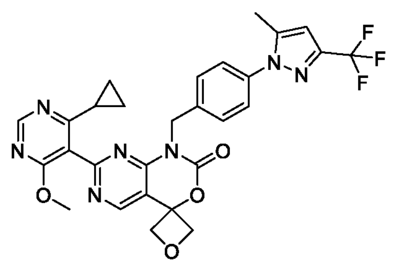 , 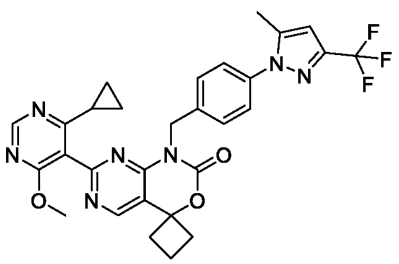 , 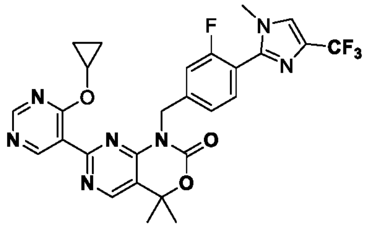 ,

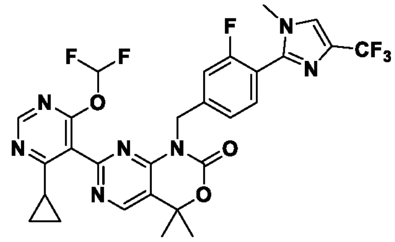 , 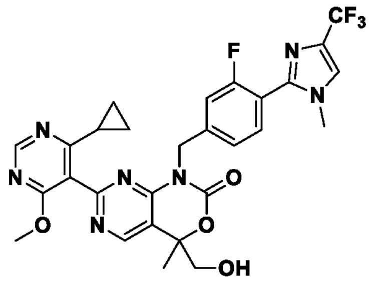 , 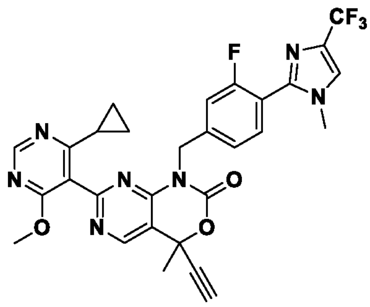 ,

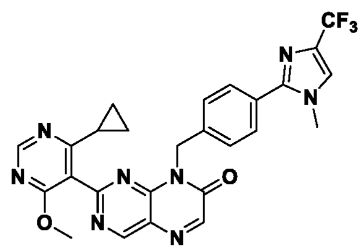 , 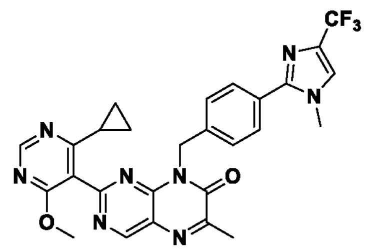 , 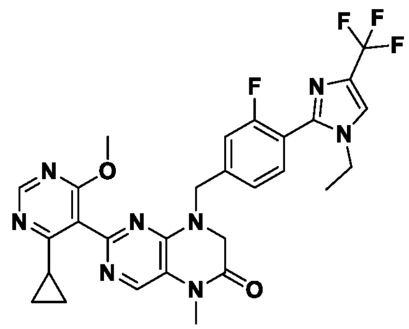 ,

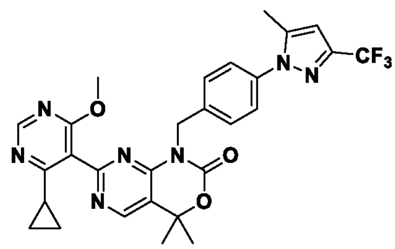 , 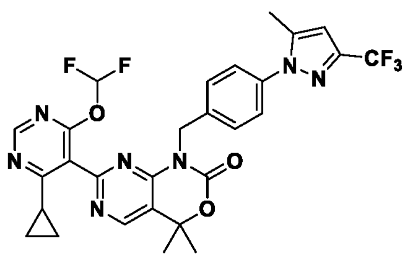 , 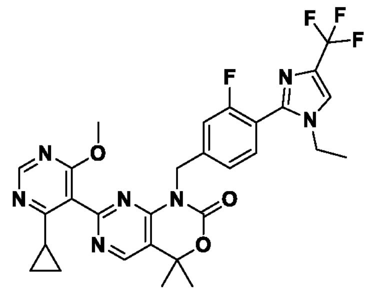 ,

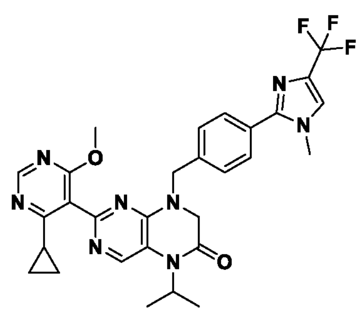 , 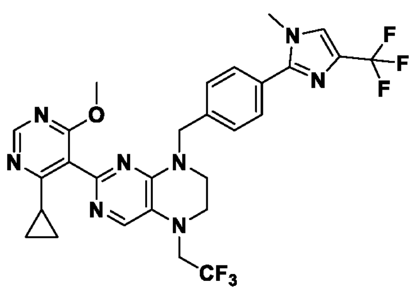 , or 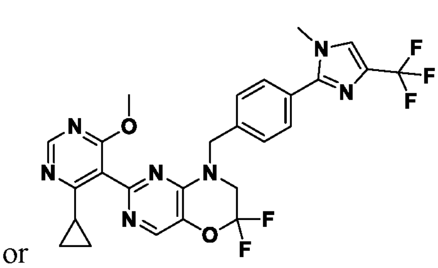 .

According to another aspect, the present application relates to a method for preparing the compound of formula (I) or the pharmaceutically acceptable salt thereof, as follows:

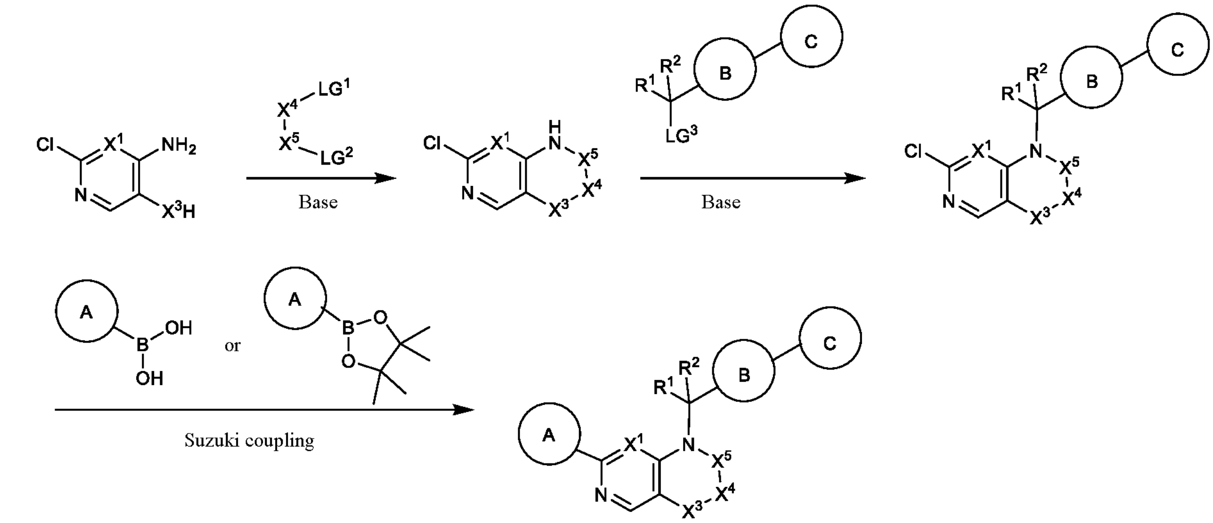


wherein

$X^3$ is selected from O, S, and $NR^6$;
$X^4$ is selected from $C(R^4)(R^5)$, $NR^6$, O, S, and $S(=O)_2$;
ring A, ring B, ring C, $X^1$, $X^5$, $R^1$, $R^2$, $R^4$, $R^5$, and $R^6$ are as defined in formula (I); and the $LG^1$, the $LG^2$, and the $LG^3$ represent leaving groups commonly used in the art.

According to another aspect, the present application relates to a method for preparing the compound of formula (I) or the pharmaceutically acceptable salt thereof, as follows:

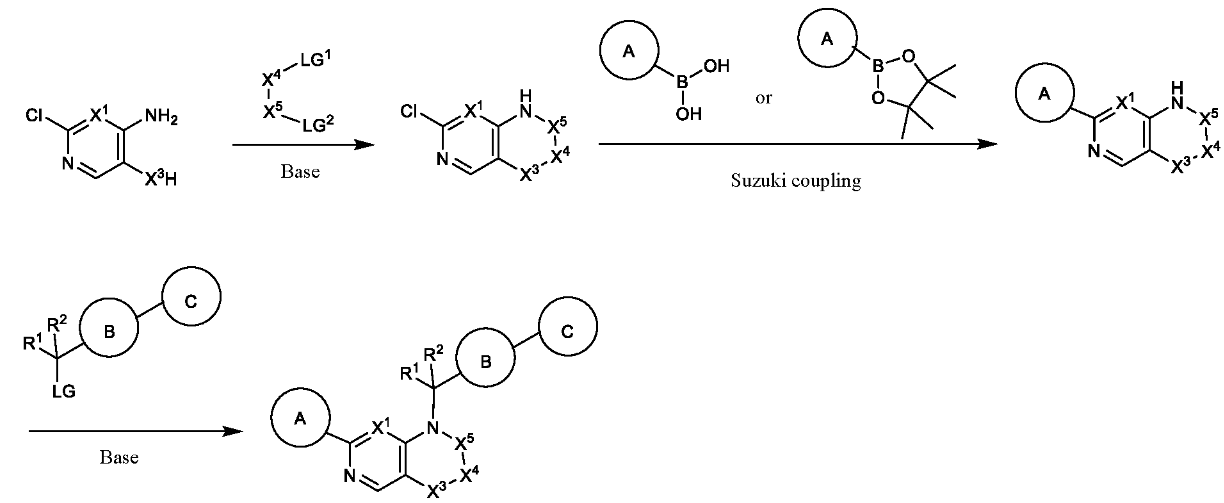


wherein $X^3$ is selected from O, S, and $NR^6$;
$X^4$ is selected from $C(R^4)(R^5)$, $NR^6$, O, S, and $S(=O)_2$;
ring A, ring B, ring C, $X^1$, $X^5$, $R^1$, $R^2$, $R^4$, $R^5$, and $R^6$ are as defined in formula (I); and the LG, the $LG^1$, and the $LG^2$ represent leaving groups commonly used in the art.

According to another aspect, the present application provides a pharmaceutical composition, comprising the compound of formula (I) or the pharmaceutically acceptable salt thereof provided in the present application and a pharmaceutically acceptable excipient.

According to another aspect, the present application provides a method for treating a USP1-mediated disease in a mammal, comprising administering to the mammal, preferably a human, in need of the treatment a therapeutically effective amount of the compound of formula (I) or the pharmaceutically acceptable salt thereof or the pharmaceutical composition thereof.

According to another aspect, the present application provides use of the compound of formula (I) or the pharmaceutically acceptable salt thereof or the pharmaceutical composition thereof in preparing a medicament for use in preventing or treating a USP1-mediated disease.

According to another aspect, the present application provides use of the compound of formula (I) or the pharmaceutically acceptable salt thereof or the pharmaceutical composition thereof in preventing or treating a USP1-mediated disease.

According to another aspect, the present application provides the compound of formula (I) or the pharmaceutically acceptable salt thereof or the pharmaceutical composition thereof preventing or treating a USP1-mediated disease.

In some embodiments, the USP1-mediated disease is a tumor.

In some embodiments, the tumor is, for example, a solid tumor, an adenocarcinoma, or a hematological cancer, such as breast cancer.

## TERMINOLOGY AND DEFINITIONS

Unless otherwise stated, the following terms used herein have the following meanings, and the definitions of groups and terms described herein, including definitions thereof as examples, exemplary definitions, preferred definitions, definitions described in tables, definitions of specific compounds in examples, and the like, may be combined with each other in any way. A particular term should not be considered uncertain or unclear in the absence of a particular definition, but should be construed according to the ordinary meaning in the art. A trade name herein is intended to refer to a corresponding product or active ingredient thereof.

As used herein, the

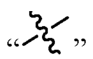

indicates a ligation site.

As used herein, the double arrow

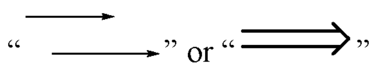

in a synthesis route indicates a multistep reaction.

As used herein, the bond "- - -" depicted by solid and dashed lines is a single bond or a double bond.

For example, the component

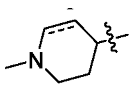

covers both:

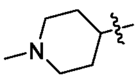

and

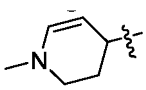

.

The compounds in the present application may have asymmetric atoms such as carbon atoms, sulfur atoms, nitrogen atoms, phosphorus atoms, or asymmetric double bonds, and thus the compounds provided in the present application may exist in the forms of particular geometric isomers or stereoisomers. The forms of particular geometric isomers or stereoisomers may be cis- and trans-isomers, E- and Z-geometric isomers, (-)- and (+)-enantiomers, (R)- and (S)-enantiomers, diastereomers, (D)-isomers, (L)-isomers, and racemic mixtures or other mixtures thereof, such as enantiomerically or diastereomerically enriched mixtures, all the foregoing isomers and mixtures thereof fall within the scope of definitions of the compounds in the present application. Additional asymmetric carbon atoms, asymmetric sulfur atoms, asymmetric nitrogen atoms, or asymmetric phosphorus atoms may be present in substituents such as alkyl, and all of these isomers involved in the substituents, and mixtures thereof, are also included within the scope of definitions of the compounds in the present application. The compounds, containing asymmetric atoms, in the present application, can be isolated in an optically active pure form or in a racemic form, and the optically active pure form can be resolved from racemic mixtures, or synthesized by using chiral raw materials or chiral reagents.

The term "substituted" means that any one or more hydrogen atoms on a particular atom are substituted with a substituent that may be a variant of deuterium and hydrogen, provided that the valence state of the particular atom is

normal and the substituted compound is stable. When the substituent is oxo (i.e., =O), it means that two hydrogen atoms are substituted, and oxo does not occur on aryl.

The term "optional" or "optionally" means that a subsequently described event or circumstance may or may not occur, and the description includes occurrence and non-occurrence of the event or circumstance. For example, ethyl is "optionally" substituted with halogen, meaning that the ethyl may be unsubstituted ($CH_2CH_3$), monosubstituted (e.g., $CH_2CH_2F$ and $CH_2CH_2Cl$), polysubstituted (e.g., $CHFCH_2F$, $CH_2CHF_2$, $CHFCH_2Cl$, and $CH_2CHCl_2$), or fully substituted ($CF_2CF_3$, $CF_2CCl_3$, $CCl_2CCl_3$, and the like). It will be understood by those skilled in the art that any group containing one or more substituents will not introduce any substitution or substitution pattern that is spatially impossible to exist and/or cannot be synthesized.

When any variable (e.g., $R^a$ and $R^b$) appears more than once in the composition or structure of a compound, its definition in each case is independent. For example, if a group is substituted with 2 $R^b$, each $R^b$ has an independent option.

When the linking direction is not specified for a linking group involved herein, the linking direction is arbitrary. For example, when $L^1$ in the structural unit

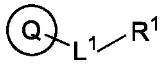

is selected from "$C_1$-$C_3$ alkylene-O", the $L^1$ may either be linked to ring Q and $R^1$ in the same direction as the reading order from left to right to form "ring Q-$C_1$-$C_3$ alkylene-O-$R^1$", or be linked to ring Q and $R^1$ in the opposite direction of the reading order from left to right to form "ring Q-O-$C_1$-$C_3$ alkylene-$R^1$".

When a substituent bond is cross-linked to two atoms on a ring, such a substituent may be bonded to any atom on the ring. For example, the structural unit

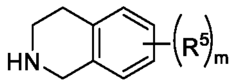

indicates that $R^5$ may be substituted at any position on a benzene ring, and $R^5$ may also be substituted at any position on a piperidine ring.

The term "halo" or "halogen" refers to fluorine, chlorine, bromine, or iodine.

$C_m$-$C_n$ herein refers to a group having an integer number of carbon atoms in the range of m-n. For example, "$C_1$-$C_{10}$" means that the group can have 1 carbon atom, 2 carbon atoms, 3 carbon atoms, 4 carbon atoms, 5 carbon atoms, 6 carbon atoms, 7 carbon atoms, 8 carbon atoms, 9 carbon atoms, or 10 carbon atoms.

LG herein is short for a leaving group, and specific examples include, but are not limited to, halogen, methanesulfonyloxy, *p*-toluenesulfonyl, alkoxy, haloalkoxy, O-*N*-succinimidyl, pentafluorophenoxyl, 4-nitrophenoxyl, and the like.

The term "alkyl" refers to a hydrocarbon group with a general formula of $C_nH_{2n+1}$. The alkyl may be a straight-chain or branched group. For example, the term "$C_1$-$C_{10}$ alkyl" is to be understood as a straight-chain or branched saturated monovalent hydrocarbon group having 1, 2, 3, 4, 5, 6, 7, 8, 9, or 10 carbon atoms. The alkyl is, for example, methyl, ethyl, propyl, butyl, pentyl, hexyl, isopropyl, isobutyl, *sec*-butyl, *tert*-butyl, isoamyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, or 1,2-dimethylbutyl; and the term "$C_1$-$C_6$ alkyl" refers to a alkyl containing 1 to 6 carbon atoms (e.g., methyl, ethyl, *n*-propyl, isopropyl, *n*-butyl, isobutyl, *sec*-butyl, *tert*-butyl, *n*-pentyl, 1-methylbutyl, 2-methylbutyl, 3-methylbutyl, neopentyl, hexyl, and 2-methylpentyl). Similarly, alkyl portions (i.e., alkyl) of alkoxy, alkylamino, and dialkylamino have the same definition as described above. When alkyl is described as being optionally substituted with an R group, this means that the alkyl is optionally substituted with one or more R groups. When alkoxy, alkylamino, and dialkylamino are described as being optionally substituted with R groups, this means that the alkoxy, the alkylamino, and the dialkylamino are optionally substituted with one or more R groups.

The "$C_1$-$C_{10}$ alkyl" described herein may comprise "$C_1$-$C_6$ alkyl", "$C_1$-$C_4$ alkyl", "$C_1$-$C_3$ alkyl", or "$C_1$-$C_2$ alkyl", and the "$C_1$-$C_6$ alkyl" may further comprise "$C_1$-$C_4$ alkyl", "$C_1$-$C_3$ alkyl", or "$C_1$-$C_2$ alkyl".

The term "alkenyl" refers to a straight-chain or branched unsaturated aliphatic hydrocarbon group consisting of carbon atoms and hydrogen atoms and having at least one double bond. For example, the term "$C_2$-$C_{10}$ alkenyl" is to be understood preferably as a straight-chain or branched monovalent hydrocarbon group containing one or more double bonds and having 2, 3, 4, 5, 6, 7, 8, 9, or 10 carbon atoms, and "$C_2$-$C_{10}$ alkenyl" is preferably "$C_2$-$C_6$ alkenyl", further preferably "$C_2$-$C_4$ alkenyl", and still further preferably $C_2$ or $C_3$ alkenyl. It should be understood that where the

alkenyl contains more than one double bonds, the double bonds may be separated from or conjugated to each other. The alkenyl is, for example, vinyl, allyl, (E)-2-methylvinyl, (Z)-2-methylvinyl, (E)-but-2-enyl, (Z)-but-2-enyl, (E)-but-1-enyl, (Z)-but-1-enyl, isopropenyl, 2-methylprop-2-enyl, 1-methylprop-2-enyl, 2-methylprop-1-enyl, (E)-1-methylprop-1-enyl, or (Z)-1-methylprop-1-enyl. When alkenyl is described as being optionally substituted with an R group, this means that the alkenyl is optionally substituted with one or more R groups.

The term "alkynyl" refers to a straight-chain or branched unsaturated aliphatic hydrocarbon group consisting of carbon atoms and hydrogen atoms and having at least one triple bond. The term "$C_2$-$C_{10}$ alkynyl" is to be understood as a straight-chain or branched unsaturated hydrocarbon group containing one or more triple bonds and having 2, 3, 4, 5, 6, 7, 8, 9, or 10 carbon atoms. Examples of "$C_2$-$C_{10}$ alkynyl" include, but are not limited to, ethynyl (-C=CH), propynyl (-C≡$CCH_3$, -$CH_2$C≡CH), but-1-ynyl, but-2-ynyl, or but-3-ynyl. "$C_2$-$C_{10}$ alkynyl" may include "$C_2$-$C_3$ alkynyl", and examples of "$C_2$-$C_3$ alkynyl" include ethynyl (-C≡CH), prop-1-ynyl (-C≡$CCH_3$), and prop-2-ynyl (-$CH_2$C≡CH). When alkynyl is described as being optionally substituted with an R group, this means that the alkynyl is optionally substituted with one or more R groups.

The term "cycloalkyl" refers to a carbocyclic ring that is fully saturated and may exist as a monocyclic ring, a fused ring, a bridged ring, or a spirocyclic ring. Unless otherwise indicated, the carbocyclic ring is typically a 3- to 10-membered ring. For example, the term "$C_3$-$C_{10}$ cycloalkyl" is to be understood as a saturated monovalent carbocyclic group having 3, 4, 5, 6, 7, 8, 9, or 10 carbon atoms, in the form of a monocyclic ring, a fused ring, a spirocyclic ring, or a bridged ring. Non-limiting examples of cycloalkyl include, but are not limited to, cyclopropyl, cyclobutyl, cyclopentyl, cyclohexyl, cycloheptyl, cyclooctyl, cyclononyl, cyclodecyl, norbornyl (bicyclo[2.2.1]heptyl), bicyclo[2.2.2]octyl, adamantyl, spiro[4.5]decane, and the like. Spirocycloalkyl refers to cycloalkyl that exists as a spirocyclic ring. The term "$C_3$-$C_{10}$ cycloalkyl" may include "$C_3$-$C_6$ cycloalkyl", and "$C_3$-$C_6$ cycloalkyl" is to be understood as a saturated monovalent monocyclic or bicyclic hydrocarbon ring having 3, 4, 5, or 6 carbon atoms, specific examples include cyclopropyl, cyclobutyl, cyclopentyl, cyclohexyl, and the like. When cycloalkyl is described as being optionally substituted with an R group, this means that the cycloalkyl is optionally substituted with one or more R groups.

The term "cycloalkenyl" refers to a non-aromatic carbocyclic ring that is partially saturated and may exist as a monocyclic ring, a fused ring, a bridged ring, or a spirocyclic ring. Unless otherwise indicated, the carbocyclic ring is typically a 5-, 6-, 7-, or 8-membered ring. Non-limiting examples of cycloalkenyl include, but are not limited to, cyclopentenyl, cyclopentadienyl, cyclohexenyl, cyclohexadienyl, cycloheptenyl, cycloheptadienyl, and the like. When cycloalkenyl is described as being optionally substituted with an R group, this means that the cycloalkenyl is optionally substituted with one or more R groups.

The term "heterocyclyl" refers to a fully saturated or partially saturated (but not aromatic heteroaromatic as a whole) monovalent monocyclic, fused-ring, spirocyclic, or bridged-ring group, and cyclic atoms of the group contain 1, 2, 3, 4, or 5 heteroatoms or heteroatom groups, the "heteroatoms or heteroatom groups" being independently selected from -N-, -O-, -S-, -P-, -O-N=, - C(=O)O-, -C(=O)-, -C(=S)-, -S(=O)$_2$-, -S(=O)-, -C(=O)NH-, -C(=NH)-, -S(=O)$_2$NH-, -S(=O)NH-, and -NHC(=O)NH-. The term "4- to 10-membered heterocyclyl" refers to heterocyclyl having 4, 5, 6, 7, 8, 9, or 10 cyclic atoms containing 1, 2, 3, 4, or 5 heteroatoms or heteroatom groups independently selected from those described above, and preferably, "4- to 10-membered heterocyclyl" includes "4- to 7-membered heterocyclyl", wherein non-limiting examples of 4-membered heterocyclyl include, but are not limited to, azetidinyl and oxetanyl; examples of 5-membered heterocyclyl include, but are not limited to, tetrahydrofuranyl, dioxolyl, pyrrolidinyl, imidazolidinyl, pyrazolidinyl, pyrrolinyl, 4,5-dihydrooxazole, or 2,5-dihydro-1H-pyrrolyl; examples of 6-membered heterocyclyl include, but are not limited to, tetrahydropyranyl, piperidinyl, morpholinyl, dithianyl, thiomorpholinyl, piperazinyl, trithianyl, tetrahydropyridyl, or 4H-[1,3,4]thiadiazinyl; and examples of 7-membered heterocyclyl include, but are not limited to, diazepanyl. The heterocyclyl may also be a bicyclic group, wherein examples of 5,5-membered bicyclic heterocyclyl include, but are not limited to, hexahydrocyclopenta[c]pyrrol-2(1H)-yl ring, and examples of 5,6-membered bicyclic heterocyclyl include, but are not limited to, hexahydropyrrolo[1,2-a]pyrazin-2(1H)-yl ring, 5,6,7,8-tetrahydro-[1,2,4]triazolo[4,3-a]pyrazinyl ring, or 5,6,7,8-tetrahydroimidazo[1,5-a]pyrazine. Optionally, the heterocyclyl may be a benzo-fused cyclic group of the 4- to 7-membered heterocyclyl described above, and examples include, but are not limited to, dihydroisoquinolinyl and the like. Preferably, "4- to 7-membered heterocyclyl" may include "4- to 6-membered heterocyclyl", "5- to 6-membered heterocyclyl", "4- to 7-membered heterocycloalkyl", "4- to 6-membered heterocycloalkyl", "5- to 6-membered heterocycloalkyl", and the like. According to the present application, although some bicyclic heterocyclyl contains in part a benzene ring or a heteroaromatic ring, the heterocyclyl is still non-aromatic as a whole. When heterocyclyl is described as being optionally substituted with an R group, this means that the heterocyclyl is optionally substituted with one or more R groups.

The term "heterocycloalkyl" refers to a 4- to 10-membered cyclic group that is fully saturated and may exist as a monocyclic ring, a fused ring, a bridged ring, or a spirocyclic ring. Unless otherwise indicated, cyclic atoms of the heterocyclic ring contain 1, 2, 3, 4, or 5 heteroatoms or heteroatom groups, the "heteroatoms or heteroatom groups" being independently selected from -N-, -O-, -S-, - P-, -O-N=, -C(=O)O-, -C(=O)-, -C(=S)-, -S(=O)$_2$-, -S(=O)-, -C(=O)NH-, -C(=NH)-, -S(=O)$_2$NH-, - S(=O)NH-, and -NHC(=O)NH-. The term "4- to 10-membered heterocycloalkyl" refers to heterocycloalkyl having 4, 5, 6, 7, 8, 9, or 10 cyclic atoms containing 1, 2, 3, 4, or 5 heteroatoms or heteroatom groups

independently selected from those described above, preferably,"4- to 10-membered heterocycloalkyl" includes "4- to 7-membered heterocycloalkyl", wherein non-limiting examples of 4-membered heterocycloalkyl include, but are not limited to, azetidinyl, oxetanyl, and thietanyl; examples of 5-membered heterocycloalkyl include, but are not limited to, tetrahydrofuranyl, tetrahydrothienyl, pyrrolidinyl, isoxazolidinyl, oxazolidinyl, isothiazolidinyl, thiazolidinyl, imidazolidinyl, and tetrahydropyrazolyl; examples of 6-membered heterocycloalkyl include, but are not limited to, piperidinyl, tetrahydropyranyl, tetrahydrothiopyranyl, morpholinyl, piperazinyl, 1,4-thioxanyl, 1,4-dioxanyl, thiomorpholinyl, 1,3-dithianyl, and 1,4-dithianyl,; and examples of 7-membered heterocycloalkyl include, but are not limited to, azepanyl, oxepanyl, and thiepanyl. When heterocycloalkyl is described as being optionally substituted with an R group, this means that the heterocycloalkyl is optionally substituted with one or more R groups.

The term "aryl" refers to an all-carbon monocyclic or fused polycyclic aromatic cyclic group with a conjugated $\pi$-electron system. For example, aryl may have 6-20 carbon atoms, 6-14 carbon atoms, or 6-12 carbon atoms. The term "$C_6$-$C_{20}$ aryl" is to be understood preferably as a monovalent aromatic or partially aromatic monocyclic, bicyclic, or tricyclic hydrocarbon ring having 6-20 carbon atoms, particularly a ring having 6 carbon atoms ("$C_6$ aryl"), such as phenyl; or a ring having 9 carbon atoms ("$C_9$ aryl"), such as indanyl or indenyl; or a ring having 10 carbon atoms ("$C_{10}$ aryl"), such as tetrahydronaphthyl, dihydronaphthyl, or naphthyl; or a ring having 13 carbon atoms ("$C_{13}$ aryl"), such as fluorenyl; or a ring having 14 carbon atoms ("$C_{14}$ aryl"), such as anthracenyl. The term "$C_6$-$C_{10}$ aryl" is to be understood preferably as a monovalent aromatic or partially aromatic all-carbon monocyclic or bicyclic group having 6-10 carbon atoms, particularly a ring having 6 carbon atoms ("$C_6$ aryl"), such as phenyl; or a ring having 9 carbon atoms ("$C_9$ aryl"), such as indanyl or indenyl; or a ring having 10 carbon atoms ("$C_{10}$ aryl"), such as tetrahydronaphthyl, dihydronaphthyl, or naphthyl. When aryl is described as being optionally substituted with an R group, this means that the aryl is optionally substituted with one or more R groups.

The term "heteroaryl" refers to an aromatic monocyclic or fused polycyclic ring system containing at least one cyclic atom selected from N, O, and S, and the remaining ring atoms being aromatic ring groups of C. The term "5- to 10-membered heteroaryl" is understood to include monovalent monocyclic or bicyclic aromatic ring systems having 5, 6, 7, 8, 9, or 10 cyclic atoms, particularly 5, 6, 9, or 10 cyclic atoms, and comprising 1, 2, 3, 4, or 5, preferably 1, 2, or 3 heteroatoms independently selected from N, O and S. In addition, 5- to 10-membered heteroaryl may be benzo-fused in each case. In particular, heteroaryl is selected from thienyl, furyl, pyrrolyl, oxazolyl, thiazolyl, imidazolyl, pyrazolyl, isoxazolyl, isothiazolyl, oxadiazolyl, triazolyl, thiadiazolyl and the like, and benzo derivatives thereof, such as benzofuranyl, benzothienyl, benzothiazolyl, benzoxazolyl, benzoisoxazolyl, benzimidazolyl, benzotriazolyl, indazolyl, indolyl, and isoindolyl; or pyridyl, pyridazinyl, pyrimidinyl, pyrazinyl, triazinyl and the like, and benzo derivatives thereof, such as quinolyl, quinazolinyl, and isoquinolyl; or azocinyl, indolizinyl, purinyl and the like, and benzo derivatives thereof; or cinnolinyl, phthalazinyl, quinazolinyl, quinoxalinyl, naphthyridinyl, pteridinyl, carbazolyl, acridinyl, phenazinyl, phenothiazinyl, phenoxazinyl, and the like. The term "5-to 6-membered heteroaryl" refers to an aromatic ring system having 5 or 6 cyclic atoms, and comprising 1, 2, or 3, preferably 1 or 2 heteroatoms independently selected from N, O and S. When heteroaryl is described as being optionally substituted with an R group, this means that the heteroaryl is optionally substituted with one or more R groups.

The term "therapeutically effective amount" refers to an amount of a compound provided in the present application that (i) treats a particular disease, condition, or disorder, (ii) alleviates, improves or eliminates one or more symptoms of the particular disease, condition, or disorder, or (iii) delays the onset of one or more symptoms of the particular disease, condition, or disorder described herein. The amount of the compound, that constitutes a "therapeutically effective amount", provided in the present application varies depending on the compound, the state and severity of the disease, the administration regimen, and the age of the mammal to be treated, but can be routinely determined by those skilled in the art based on their own knowledge and the content of the present application. The term "pharmaceutically acceptable" is intended to refer to those compounds, materials, compositions, and/or dosage forms which are, within the scope of sound medical judgment, suitable for use in contact with tissues of human beings and animals without excessive toxicity, irritation, allergic response, or other problems or complications, and commensurate with a reasonable benefit/risk ratio.

The term "pharmaceutically acceptable salt" refers to pharmaceutically acceptable acid addition salts or base addition salts, including salts formed of compounds with inorganic or organic acids, and salts formed of compounds with inorganic or organic bases.

The term "pharmaceutical composition" refers to a mixture of one or more compounds provided in the present application or salts thereof and pharmaceutically acceptable excipients. The pharmaceutical composition is intended to facilitate administration of the compounds provided in the present application to an organism.

The term "pharmaceutically acceptable excipients" refers to those excipients that do not have a significant irritating effect on an organism and that do not impair the biological activity and properties of the active compound. Suitable excipients are well known to those skilled in the art, for example, carbohydrates, waxes, water-soluble and/or water-expandable polymers, hydrophilic or hydrophobic materials, gelatin, oils, solvents, and water.

The word "comprise" or "comprising" should be interpreted in an open, non-exclusive sense, i.e., "including but

not limited to".

The present application further includes isotopically labeled compounds provided in the present application, which are identical to those described herein, but have one or more atoms substituted with an atom having an atomic mass or mass number different from the atomic mass or mass number usually found in nature. Examples of isotopes that can be incorporated into the compounds provided in the present application include isotopes of hydrogen, carbon, nitrogen, oxygen, phosphorus, sulfur, fluorine, iodine, and chlorine, such as $^{2}H$, $^{3}H$, $^{11}C$, $^{13}C$, $^{14}C$, $^{13}N$, $^{15}N$, $^{15}O$, $^{17}O$, $^{18}O$, $^{31}P$, $^{32}P$, $^{35}S$, $^{18}F$, $^{123}I$, $^{125}I$, and $^{36}Cl$.

Some isotopically labeled compounds (e.g., those labeled with $^{3}H$ and $^{14}C$) provided in the present application may be used in compound and/or substrate tissue distribution assay. Tritiated (i.e., $^{3}H$) and carbon-14 (i.e., $^{14}C$) isotopes are particularly preferred for the ease of their preparation and detectability. Positron emission isotopes (e.g., $^{15}O$, $^{13}N$, $^{11}C$, and $^{18}F$) can be used in positron emission tomography (PET) to determine substrate occupancy. Isotopically labeled compounds provided in the present application can generally be prepared by substituting non-isotopically labeled reagents by isotopically labeled reagents through the following procedures similar to those disclosed in the schemes and/or examples below.

The pharmaceutical composition provided in the present application can be prepared by combining the compounds provided in the present application with suitable pharmaceutically acceptable excipients, and can be formulated, for example, into solid, semisolid, liquid, or gaseous formulations such as tablets, pills, capsules, powders, granules, ointments, emulsions, suspensions, suppositories, injections, inhalants, gels, microspheres, and aerosols.

Typical routes of administration for a compound provided in the present application or a pharmaceutically acceptable salt thereof or a pharmaceutical composition thereof include, but are not limited to, oral, rectal, topical, inhalation, parenteral, sublingual, intravaginal, intranasal, intraocular, intraperitoneal, intramuscular, subcutaneous, and intravenous administration.

The pharmaceutical composition provided in the present application may be manufactured by methods well known in the art, such as conventional mixing, dissolution, granulation, emulsification, and lyophilization.

In some embodiments, the pharmaceutical composition is in an oral form. For oral administration, the pharmaceutical composition may be formulated by mixing active compounds with pharmaceutically acceptable excipients well known in the art. These excipients enable the compounds provided in the present application to be formulated into tablets, pills, pastilles, sugar-coated tablets, capsules, liquids, gels, syrups, suspensions, and the like, for oral administration to patients.

Solid oral compositions may be prepared by conventional mixing, filling or tableting methods. For example, solid oral compositions can be obtained by the following method: the active compounds are mixed with solid excipients, the resulting mixture is optionally ground and added with other suitable excipients if required, and then the mixture is processed into granules to obtain cores of tablets or sugar-coated tablets. Suitable excipients include, but are not limited to: binders, diluents, disintegrants, lubricants, glidants, sweeteners or flavoring agents, and the like.

The pharmaceutical composition may also be suitable for parenteral administration, such as sterile solutions, suspensions, or lyophilized products in suitable unit dosage forms.

## DETAILED DESCRIPTION

The present invention is further described below with reference to specific examples, and the advantages and features of the present invention will become more apparent with the description. Experimental procedures without specified conditions in the examples are conducted according to conventional conditions or conditions recommended by the manufacturer. Reagents or instruments without specified manufacturers used herein are conventional products that are commercially available.

The examples herein are exemplary only, and do not limit the scope of the present application in any way. It will be understood by those skilled in the art that various changes or substitutions in form and details may be made to the technical solutions of the present application without departing from the spirit and scope of the present application, and that these changes and substitutions shall all fall within the protection scope of the present application.

Structures of the compounds are determined by nuclear magnetic resonance (NMR) and/or mass spectrometry (MS). NMR shifts are measured in $10^{-6}$ (ppm). Solvents for NMR assay include deuterated dimethyl sulfoxide, deuterated chloroform, deuterated methanol, and the like, with tetramethylsilane (TMS) as the internal standard. "$IC_{50}$" refers to the half inhibitory concentration, which is the concentration at which half of the maximal inhibition effect is achieved.

Acronyms:

DCM: dichloromethane; $BBr_3$: boron tribromide; DMF: *N*,*N*-dimethylformamide; DMSO: dimethyl sulfoxide; LAH: lithium aluminum hydride; TsCl: p-toluenesulfonyl chloride; $Pd(dppf)Cl_2 \cdot CH_2Cl_2$: 1,1'-bis(diphenylphosphino)ferrocene dichloropalladium (II) dichloromethane complex; 1,4-dioxane: dioxane; DIEA: *N,N*-diisopropylethylamine; THF:

tetrahydrofuran; Imidazole: imidazole; TBSCl: *tert*-butyl dimethyl silyl chloride; DIPEA: *N,N*-diisopropylethylamine; HATU: 2-(7-azabenzotriazole)-*N,N,N',N'*-tetramethyluronium hexafluorophosphate; Iodomethane: iodomethane; DIBAL-H: diisopropylaluminum hydride; MeCN: acetonitrile; NBS: N-bromosuccinimide; AIBN: azobisisobutyronitrile; TEMPO: 2,2,6,6-tetramethylpiperidine oxide; Pyridine: pyridine; Toluene: toluene; XPhos Pd $G_2$: chloro(2-dicyclohexylphosphino-2',4',6'-triisopropyl-1,1'-biphenyl)[2-(2'-amino-1,1'-biphenyl)]palladium (II); $Pd_2(dba)_3$: tris(dibenzylideneacetone)dipalladium; Triphosgene: triphosgene; Xantphos: 4,5-bis(diphenylphosphino)-9,9-dimethylxanthene; MTBE: methyl *tert*-butyl ether; $(Bpin)_2$: bis(pinacolato)diboron; TEA: triethylamine; $PCy_3$ or TCHP: tricyclohexylphosphine; NMP: *N*-methyl pyrrolidone; TBAHS: tetrabutylammonium hydrogen sulfate; HFIP: hexafluoroisopropanol; vinyl-Bpin: vinylboronic acid pinacol cyclic ester; CDI: carbonyldiimidazole; *m*-CBPA: *m*-chloroperoxybenzoic acid; NMO: *N*-methylmorpholine oxide; DHP: 3,4-dihydro-2H-pyran; PTSA: *p*-toluenesulfonic acid; and TFA: trifluoroacetic acid

**Example 1: Preparation of 2-(2-isopropylpyridin-3-yl)-8-(4-(1-methyl-4-(trifluoromethyl)-1H-imidazol-2-yl)benzyl)-7,8-dihydro-6H-pyrimido[5,4-b][1,4]oxazine (compound 1)**

The synthesis route is as follows:

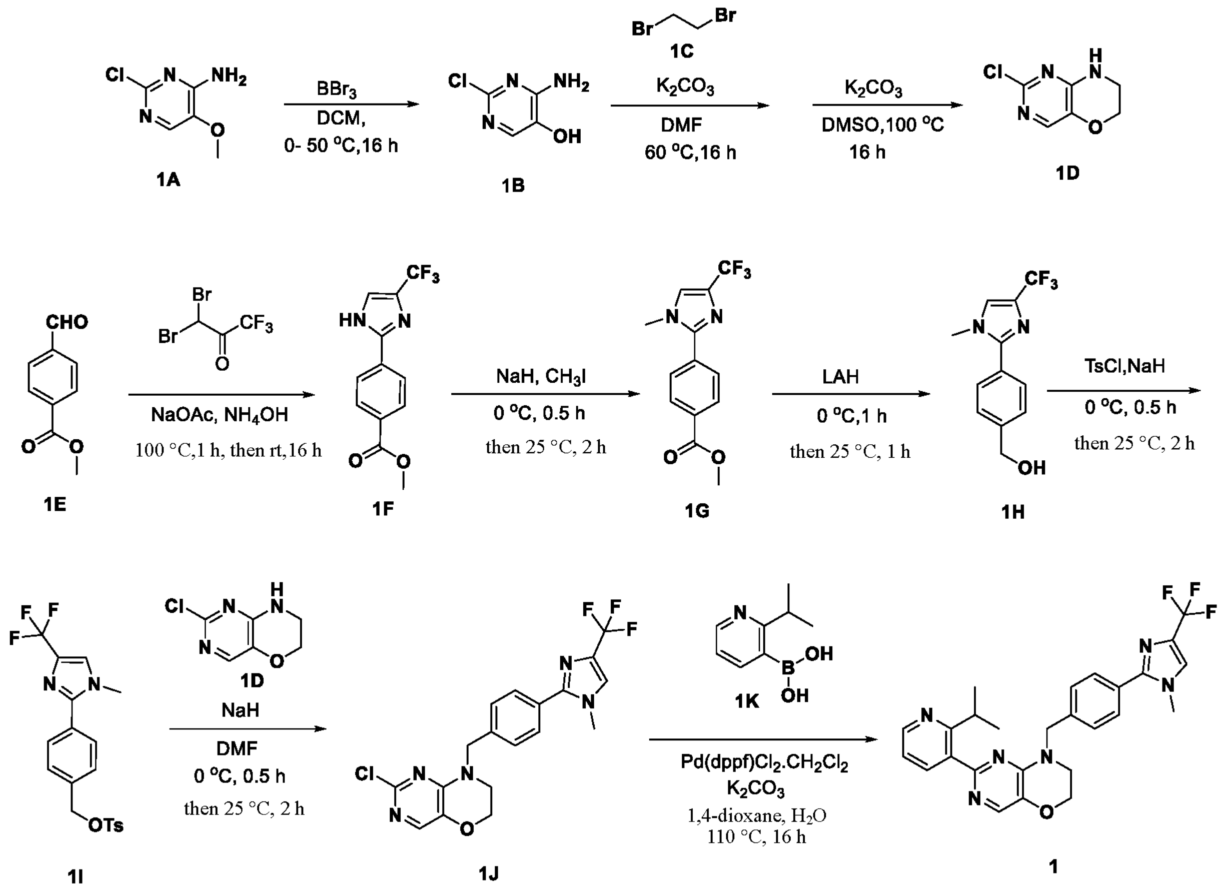


**Step 1: 2-chloro-4-amino-5-hydroxypyrimidine (1B)**

2-chloro-4-amino-5-methoxypyrimidine (1.0 g, 6.3 mmol) was dissolved in dichloromethane (20 mL), and boron tribromide (6.3 mL, 13 mmol, 2.0 eq) was added at 0 °C. The resulting mixture was heated at 50 °C for 16 h. After quenching with methanol (20 mL) at 0 °C, the resulting reaction solution was distilled under vacuum to remove the solvent. The resulting residue was purified by silica gel column chromatography (petroleum ether:ethyl acetate = 5:1-1:1) to obtain a white solid title compound **1B** (0.90 g, yield: 69%). m/z (ESI): $146[M+H]^+$.

**Step 2: 2-chloro-7,8-dihydro-6H-pyrimido[5,4-b][1,4]oxazine (1D)**

The compound **1B** (0.76 g, 5.2 mmol) was added to *N,N*-dimethylformamide (10 mL) at room temperature, followed by potassium carbonate (1.4 g, 10 mmol, 2.0 eq), 1,2-dibromoethane (1.9 g, 10 mmol, 2.0 eq). The resulting

mixture was stirred at 60 °C for 16 h, and then the reaction solution was added to ice water (30 mL). The resulting mixture was extracted with ethyl acetate (20 mL × 3), and the organic phases were combined, then washed with saturated brine (50 mL), dried over anhydrous sodium sulfate, filtered, and distilled under vacuum to remove the solvent. Then the resulting residue was slurried in petroleum ether containing 10% ethyl acetate and filtered to obtain a solid (0.54 g, m/z (ESI): 252$[M+H]^+$).

The resulting solid (0.54 g, 2.1 mmol) was added to dimethyl sulfoxide (5 mL) at room temperature, followed by potassium carbonate (0.59 g, 4.2 mmol, 2.0 eq). After the resulting mixture was stirred at 100 °C for 16 h, the reaction solution was added to ice water (30 mL), and extracted with ethyl acetate (20 mL × 3). The organic phases were combined, then washed with saturated brine (50 mL), dried over anhydrous sodium sulfate, filtered, and distilled under vacuum to remove the solvent. The resulting residue was purified by silica gel column chromatography (petroleum ether: ethyl acetate = 5:1-1:1) to obtain a white solid title compound **1D** (0.23 g, yield: 51%). m/z (ESI): 172$[M+H]^+$.

**Step 3: methyl 4-(4-(trifluoromethyl)-1H-imidazol-2-yl)benzoate (1F)**

At room temperature, 3,3-dibromo-1,1,1-trifluoroacetone (9.9 g, 37 mmol, 1.2 eq) and sodium acetate (3.0 g, 37 mmol, 1.2 eq) were added to water, and the resulting mixture was heated at 100 °C under stirring for reaction for 1 h. The reaction solution was cooled to room temperature, and slowly added dropwise to a methanol (100 mL) solution containing methyl 4-formylbenzoate (5.0 g, 30.46 mmol, 1.0 eq). Then aqueous ammonia (35 mL) was added to the reaction solution for reaction at room temperature under stirring for 16 h. The resulting mixture was distilled under vacuum to remove the solvent, and then the resulting residue was added to water (100 mL) and extracted with ethyl acetate (100 mL × 3). The resulting organic phases were combined, then washed with saturated brine (50 mL), and distilled under vacuum to remove the solvent. The resulting residue was purified by silica gel column chromatography (petroleum ether: ethyl acetate = 5:1-1:1) to obtain a solid title compound **1F** (5.0 g, yield: 49%, purity: 80%). m/z (ESI): 271$[M+H]^+$.

**Step 4: methyl 4-(1-methyl-4-(trifluoromethyl)-1H-imidazol-2-yl) benzoate (1G)**

The compound **1F** (3.0 g, 11 mmol) was dissolved in tetrahydrofuran (30 mL), and sodium hydride (60%, 0.67 g, 17 mmol, 1.5 eq) was added in portions at 0 °C. After the reaction solution was stirred at 0 °C for 0.5 h, iodomethane (3.2 g, 22 mmol, 2.0 eq) was added, and the resulting mixture was restored slowly to room temperature for reaction for 2 h. The reaction solution was added to ice water (30 mL) and extracted with ethyl acetate (20 mL × 3). The organic phases were combined, then washed with saturated brine (50 mL), dried over anhydrous sodium sulfate, filtered, and distilled under vacuum to remove the solvent to obtain a solid title compound **1G** (3.0 g, yield: 81%). LC-MS: m/z (ESI): 285$[M+H]^+$.

**Step 5: 4-(1-methyl-4-(trifluoromethyl)-1H-imidazol-2-yl)benzyl alcohol (1H)**

The compound **1G** (3.0 g, 11 mmol) was added to tetrahydrofuran (60 mL), and lithium aluminum hydride (1.3 g, 33 mmol, 3.0 eq) was added in portions at 0 °C. The resulting mixture was stirred at 0 °C for reaction for 1 h, and then restored slowly to room temperature for reaction for 1 h. At 0 °C, 1.3 mL of water, 1.3 mL of 15% aqueous NaOH solution, and 3.8 mL of water were sequentially added to the reaction solution. The resulting mixture was stirred at room temperature for 1 h, and then filtered through diatomite, and the filter cake was rinsed with dichloromethane. The resulting filtrates were combined and distilled under vacuum to remove the solvent to obtain an oily title compound **1H** (3.0 g, yield: 90%). m/z (ESI): 257$[M+H]^+$.

**Step 6: 4-(1-methyl-4-(trifluoromethyl)-1H-imidazol-2-yl)benzyl 4-methylbenzenesulfonate (1I)**

The compound **1H** (2.1 g, 8.3 mmol) was added to tetrahydrofuran (20 mL), and sodium hydride (60%, 0.49 g, 12 mmol, 1.5 eq) was added at 0 °C. The resulting reaction solution was stirred at 0 °C for reaction for 0.5 h, and then *p*-toluenesulfonyl chloride (1.9 g, 9.9 mmol, 1.2 eq) was added. The reaction solution was restored slowly to room temperature, and kept under stirring for reaction for 2 h. The reaction solution was added to ice water (50 mL) and extracted with ethyl acetate (50 mL × 3). The resulting organic phases were combined, then washed with saturated brine (50 mL), and distilled under vacuum to remove the solvent. The resulting residue was purified by silica gel column chromatography (petroleum ether:ethyl acetate = 5:1-1:1) to obtain a solid title compound **11** (0.60 g, yield: 18%). m/z (ESI): 411$[M+H]^+$.

**Step 7: 2-chloro-8-(4-(1-methyl-4-(trifluoromethyl)-1H-imidazol-2-yl)benzyl)-7,8-dihydro-6H-pyrimido[5,4-b][1,4]oxazine (1J)**

The compound **1D** (0.10 g, 0.58 mmol) was added to N,N-dimethylformamide (2 mL), and NaH (60%, 35 mg, 0.87 mmol, 1.5 eq) was added at 0 °C. The resulting mixture was stirred at 0 °C for 0.5 h, and then the compound **1I** (0.19 g, 0.46 mmol, 0.8 eq) was added. The reaction solution was restored slowly to room temperature, and kept under stirring for reaction for 2 h. The reaction solution was added to ice water (10 mL) and extracted with ethyl acetate (10 mL × 3). The organic phases were combined, then washed with saturated brine (10 mL), dried over anhydrous sodium sulfate, filtered, and distilled under vacuum to remove the solvent to obtain a solid title compound **1J** (0.13 g, yield: 53%). m/z (ESI): 410[M+H]$^+$.

**Step 8: 2-(2-isopropylpyridin-3-yl)-8-(4-(1-methyl-4-(trifluoromethyl)-1H-imidazol-2-yl)benzyl)-7,8-dihydro-6H-pyrimido[5,4-b][1,4]oxazine (compound 1)**

The compound **1J** (0.12 g, 0.26 mmol), a compound **1K** (0.21 g, 1.3 mmol, 5.0 eq), potassium carbonate (0.18 g, 1.3 mmol, 5.0 eq), and 1,1'-bis(diphenylphosphino)ferrocene dichloropalladium (II) dichloromethane complex (21 mg, 25.7 μmol, 0.1 eq) were added to a mixed solution of dioxane (4 mL) and water (1 mL). The reaction solution was stirred under a nitrogen atmosphere at 110 °C for 16 h. After cooling, the reaction solution was filtered through diatomite, then the filter cake was rinsed with ethyl acetate, and the resulting filtrates were combined and distilled under vacuum to remove the solvent to obtain a crude compound, which was purified by preparative chromatography (Waters Xbridge C18, 10-95% aqueous acetonitrile solution) to obtain a solid title compound 1 (45 mg, yield: 35%).

m/z (ESI): 495[M+H]$^+$
$^1$H NMR (400 MHz, DMSO-$d_6$): $\delta$ 8.53 (dd, *J*= 4.8 Hz, 1.6 Hz, 1H), 7.99 (s, 1H) , 7.93 (s, 1H), 7.88 (dd, *J*= 7.6 Hz, 2.0 Hz, 1H), 7.69 (d, *J*= 8.4 Hz, 2H), 7.41 (d, *J*= 8.0 Hz, 2H), 7.25 (dd, *J*= 8.0 Hz, 4.8 Hz, 1H), 4.95 (s, 2H), 4.30 (t, *J*= 4.0 Hz, 2H), 3.75 (s, 3H), 3.66-3.76(m,1H), 3.61(t, *J*= 4.0 Hz, 2H), 1.07 (d, *J* =8.0 Hz, 6H).

**Example 2: Preparation of 2-(4-cyclopropyl-6-methoxypyrimidin-5-yl)-8-(4-(1-methyl-4-(trifluoromethyl)-1H-imidazol-2-yl)benzyl)-7,8-dihydro-6H-pyrimido [5,4-b] [1,4] oxazine (compound 2)**

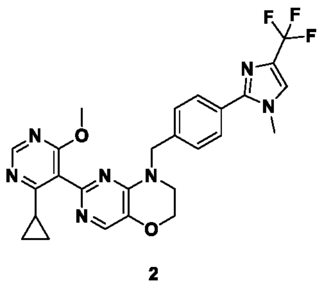

**2**

A compound **2K** was used instead of the compound **1K** to prepare the compound **2** by using a method similar to that in Example 1.

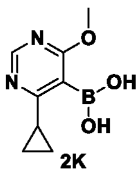

**2K**

m/z (ESI): 524[M+H]$^+$
$^1$H NMR (400 MHz, DMSO-$d_6$): $\delta$ 8.59 (s, 1H), 7.95 (s, 1H), 7.93 (s, 1H), 7.68 (d, *J* = 8.0 Hz, 2H), 7.43 (d, *J*= 8.0 Hz, 2H), 4.86 (s, 2H), 4.28 (m, 2H), 3.84 (s, 3H), 3.76 (s, 3H), 3.59 (m, 2H), 1.77-1.72 (m, 1H), 0.97 (m, 2H), 0.83 (m, 2H).

**Example 3: Preparation of 7-(4-cyclopropyl-6-methoxypyrimidin-5-yl)-1-(4-(1-methyl-4-(trifluoromethyl)-1H-imidazol-2-yl)benzyl)-2,3-dihydro-1H-pyrido [3,4-b] [1,4] oxazine (compound 3)**

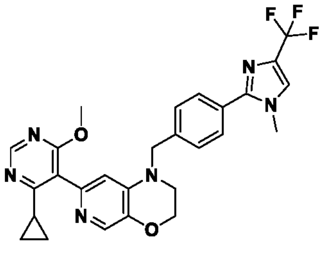

3

A compound **3A** was used instead of a compound **1A** to prepare the compound **3** by using a method similar to that in Example **2.**

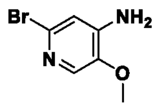

3A

m/z (ESI): 523[M+H]$^+$

$^1$H NMR (400 MHz, DMSO-$d_6$): $\delta$ 8.54(s, 1H), 7.93(s, 1H), 7.91(s, 1H), 7.69(d, $J$ = 8.4 Hz, 2H), 7.42(d, $J$ = 8.4 Hz, 2H), 6.79(s, 1H), 4.67(s, 2H), 4.30(m, 2H), 3.76(s, 3H), 3.73(s, 3H), 3.57 (t, $J$ = 3.8 Hz, 2H), 1.79-1.76(m, 1H), 0.93-0.91(m, 2H), 0.78-0.76(m, 2H).

**Example 4: Preparation of 2-(2-isopropylpyridin-3-yl)-8-(4-(1-methyl-4-(trifluoromethyl)-1H-imidazol-2-yl)benzyl)-7,8-dihydropteridin-6(5H)-one (compound 4)**

The synthesis route is as follows:

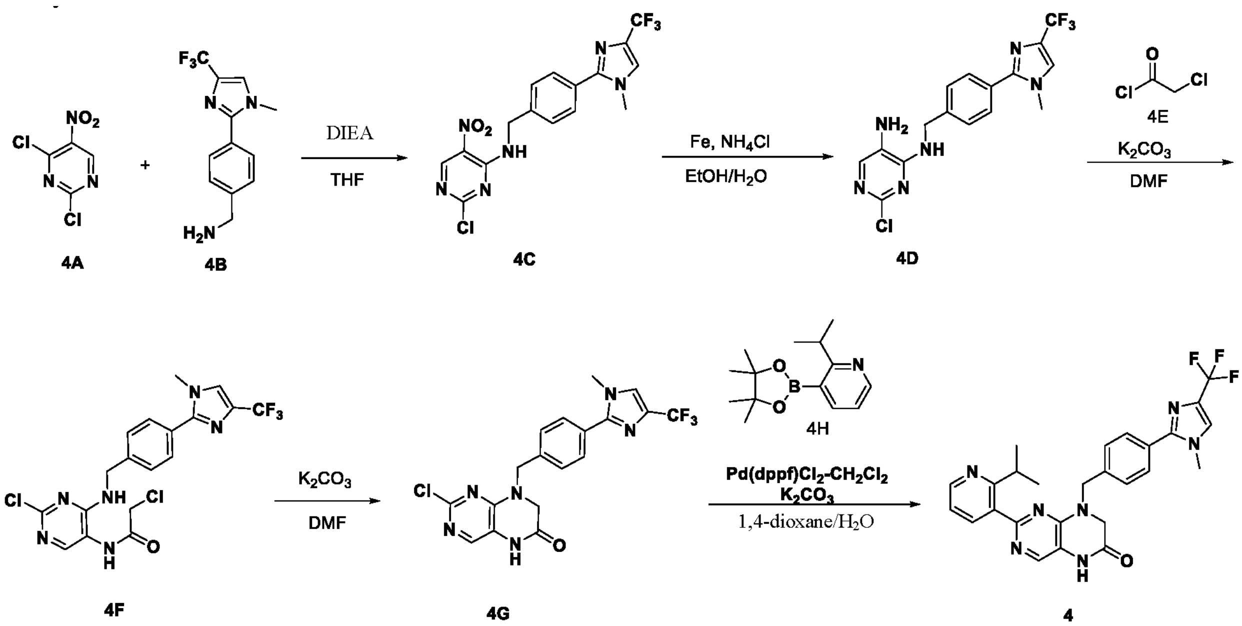

4A 4B 4C 4D 4F 4G 4

**Step 1: 2-chloro-N (4-(1-methyl-4-(trifluoromethyl)-1H-imidazol-2-yl)benzyl)-5-nitropyrimidin-4-amine (4C)**

At room temperature, 2,4-dichloro-5-nitropyrimidine (0.71 g, 3.7 mmol) and *N,N*-diisopropylethylamine (1.0 g, 7.8 mmol, 2.0 eq) were dissolved in tetrahydrofuran (20 mL), and the reaction solution was cooled to 0 °C and added with a compound **4B** (0.93 g, 3.7 mmol, 1.0 eq). The resulting mixture was stirred at 0 °C for reaction for 0.5 h, and restored slowly to room temperature for reaction for 2 h. The reaction solution was added to ice water (50 mL) and extracted with ethyl acetate (50 mL × 3). The organic phases were combined, then back-washed once with saturated brine (50 mL), and distilled under vacuum to remove the solvent, and then the resulting residue was purified by silica gel column chromatography (petroleum ether:ethyl acetate = 5:1-3:1) to obtain a yellow solid **4C** (0.90 g, yield: 60%). m/z (ESI): 413 $[M+H]^+$.

**Step 2: 2-chloro-*N*$^4$-(4-(1-methyl-4-(trifluoromethyl)-1H-imidazol-2-yl)benzyl)pyrimidine-4,5-diamine (4D)**

At room temperature, the compound **4C** (0.5 g, 1.2 mmol) and reduced iron powder (0.68 g, 12 mmol, 10 eq) were added to water (5 mL) and ethanol (5 mL), followed by ammonium chloride (0.65 g, 12 mmol, 10 eq). The resulting mixture was stirred at 80 °C for reaction for 2 h. The reaction solution was added with 50 mL of ethyl acetate for dilution and filtered while hot, and the filter cake was rinsed with ethyl acetate. After the organic phases were distilled under vacuum to remove the solvent, the resulting residue was purified by silica gel column chromatography (petroleum ether:ethyl acetate = 1:1) to obtain a tawny solid **intermediate 4D** (0.35 g, yield: 76%). m/z (ESI): 383$[M+H]^+$.

**Step 3: 2-chloro-*N*-(2-chloro-4-((4-(1-methyl-4-(trifluoromethyl)-1H-imidazol-2-yl)benzyl)amino)pyrimidin-5-yl)acetamide (4F)**

The compound **4D** (0.5 g, 1.3 mmol) was dissolved in anhydrous N,N-dimethylformamide (10 mL) at room temperature, the resulting solution was cooled to 0 °C, and chloroacetyl chloride (0.15 g, 1.3 mmol, 1 eq) was added slowly, followed by potassium carbonate (0.36 g, 2.61 mmol, 2 eq). After reaction at 0 °C for 2 h, the resulting mixture was added with water (30 mL) for quenching and extracted with ethyl acetate (20 mL × 3). The resulting organic phases were combined, then washed with saturated brine (50 mL), dried over anhydrous sodium sulfate, and filtered. After the organic phases were distilled under vacuum to remove the solvent, the resulting residue was purified by silica gel column chromatography (petroleum ether:ethyl acetate = 5:1-1:1) to obtain a white solid **4F** (0.4 g, yield: 67%). m/z (ESI): 459$[M+H]^+$.

**Step 4: 2-chloro-8-(4-(1-methyl-4-(trifluoromethyl)-1H-imidazol-2-yl)benzyl)-7,8-dihydropteridin-6(5H)-one (4G)**

The compound **4F** (0.3 g, 0.65 mmol) was dissolved in anhydrous *N,N*-dimethylformamide (10 mL) at room temperature, and then potassium carbonate (0.18 g, 1.3 mmol, 2 eq) was added. The resulting mixture was heated to 50 °C and stirred for reaction for 2 h, then water (30 mL) was added to quench the reaction, and the reaction solution was extracted with ethyl acetate (20 mL × 3). The resulting organic phases were combined, then washed with saturated brine (50 mL), dried over anhydrous sodium sulfate, filtered, and distilled under vacuum to remove the solvent, and the resulting residue was purified by silica gel column chromatography (petroleum ether:ethyl acetate = 5:1-1:1) to obtain a white solid **4G** (0.12 g, yield: 44%). m/z (ESI): 423 $[M+H]^+$.

**Step 5: 2-(2-isopropylpyridin-3-yl)-8-(4-(1-methyl-4-(trifluoromethyl)-1H-imidazol-2-yl)benzyl)-7,8-dihydropteridin-6(5H)-one (4)**

The compound **4G** (50 mg, 0.12 mmol), a compound **4H** (98 mg, 0.59 mmol, 5.0 eq), potassium carbonate (82 mg, 0.59 mmol, 5.0 eq), and 1,1'-bis(diphenylphosphino)ferrocene dichloropalladium (II) dichloromethane complex (19 mg, 24 μmol, 0.20 eq) were added to a mixed solution of dioxane (2 mL) and water (0.5 mL) at room temperature. Under the protection of nitrogen, the resulting mixture was allowed to react at 100 °C for 4 h. After cooling, the reaction solution was filtered through diatomite, then the filter cake was rinsed with ethyl acetate, and the resulting filtrates were combined and distilled under vacuum to remove the solvent to obtain a crude compound, which was purified by preparative chromatography (Waters Xbridge C18, 10-85% aqueous acetonitrile solution) to obtain a solid title compound **4** (18 mg, yield: 30%).

m/z (ESI): 508$[M+H]^+$

$^1$H NMR (400 MHz, DMSO-$d_6$): $\delta$ 10.85(brs, 1H), 8.54(dd, *J* = 4.8 Hz, 1.6 Hz, 1H), 7.92-7.89(m, 3H),7.69(d, *J* = 8.4 Hz, 2H), 7.46(d, *J* = 8.4 Hz, 2H), 7.25(dd, *J* = 8.0 Hz, 4.8 Hz, 1H), 4.90(s, 2H), 4.15(s, 2H), 3.77(s, 3H), 3.73-3.68(m,1H), 1.08 (d, *J*= 6.8 Hz, 6H).

**Example 5: Preparation of 2-(4-cyclopropyl-6-methoxypyrimidin-5-yl)-8-(4-(1-methyl-4-(trifluoromethyl)-1H-imidazol-2-yl)benzyl)-7,8-dihydropteridin-6(SH)-one (compound 5)**

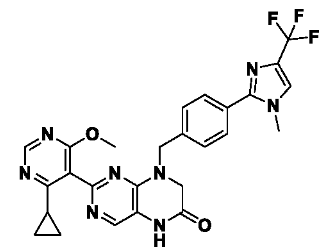

**5**

The compound **2K** was used instead of the compound **4H** to prepare the compound **5** by using a method similar to that in Example **4.**

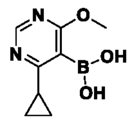

**2K**

m/z (ESI): 537[M+H]$^+$

$^1$H NMR (400 MHz, DMSO-$d_6$): $\delta$ 10.85(brs, 1H), 8.60 (s, 1H), 7.93(s, 1H), 7.87(s, 1H), 7.67(d, $J$ = 8.4 Hz, 2H), 7.48(d, $J$ = 8.0 Hz, 2H), 4.80(s, 2H), 4.13(s, 2H), 3.85(s, 3H), 3.77(s, 3H), 1.81-1.75(m, 1H), 1.00-0.98(m, 2H), 0.86-0.83(m, 2H)

**Example 6: Preparation of 2-(4-cyclopropyl-6-methoxypyrimidin-5-yl)-8-(4-(1-methyl-4-(trifluoromethyl)-1H-imidazol-2-yl)benzyl)-6H-pyrimido[5,4-b][1,4]oxazin-7(8H)-one (compound 6)**

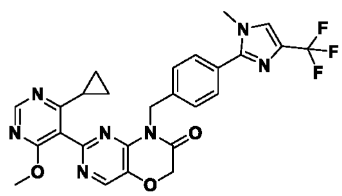

**6**

A compound **6C** was used instead of a compound **1C** to prepare the compound **6** by using a method similar to that in Example **2.**

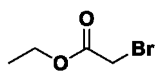

**6C**

m/z (ESI): 538[M+H]$^+$

$^1$H NMR (400 MHz, DMSO-$d_6$): $\delta$ 8.64(s, 1H), 8.53(s, 1H), 7.92(s, 1H), 7.64(d, $J$ = 8.3 Hz, 2H), 7.46(d, $J$ = 8.3 Hz, 2H), 5.24(s, 2H), 5.05(s, 2H), 3.81(s, 3H), 3.75(s, 3H), 1.68(m, 1H), 0.99(m, 2H), 0.77(m, 2H).

**Example 7: Preparation of 8-(4-(6-oxa-4-azaspiro[2.4]hept-4-en-5-yl)benzyl)-2-(4-cyclopropyl-6-methoxypyrimidin-5-yl)-7,8-dihydro-6H-pyrimido[5,4-b][1,4]oxazine (compound 7)**

The synthesis route is as follows:

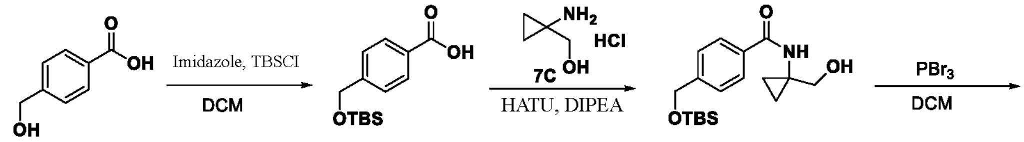


7A 7B 7D

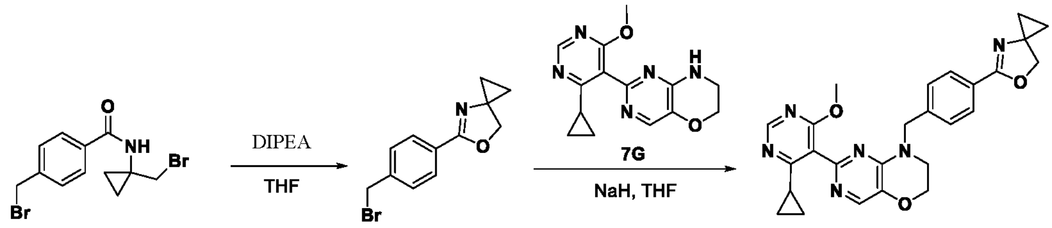


7E 7F 7

**Step 1: 4-(((*tert*-butyldimethylsilyl)oxy)methyl)benzoic acid (7B)**

A compound **7A** (5.0 g, 33 mmol), imidazole (11 g, 0.16 mol), and *tert*-butyldimethylsilyl chloride (4.1 g, 49 mmol) were mixed with dichloromethane (50 mL), and the mixture was stirred overnight at room temperature. After distillation under vacuum to remove the solvent, the resulting residue was purified by silica gel column chromatography (petroleum ether:ethyl acetate = 1:1), and concentrated under vacuum to obtain a colorless liquid **7B** (6.0 g, 23 mmol, yield: 69%). m/z (ESI): 265 $[M+H]^+$.

**Step 2: 4-(((*tert*-butyldimethylsilyl)oxy)methyl)-*N*-(1-(hydroxymethyl)cyclopropyl)benzamide (7D)**

To *N*,*N*-dimethylformamide (25 mL), (1-aminocyclopropyl)methanol hydrochloride (0.83 g, 6.7 mmol), an intermediate **7B** (1.5 g, 5.6 mmol), N,N-diisopropylethylamine (2.2 g, 17 mmol, 2.9 mL), and 2-(7-azabenzotriazole)-*N*,*N*,*N*',*N*'-tetramethyluronium hexafluorophosphate (3.2 g, 8.4 mmol) were added, and the resulting mixture was stirred overnight at room temperature. Water (100 mL) and ethyl acetate (100 mL) were added to the reaction solution for liquid separation after stirring, and the organic phase was washed three times with saturated brine (100 mL × 3), dried over anhydrous sodium sulfate, and filtered. After distillation under vacuum to remove the solvent, the resulting residue was purified by silica gel column chromatography (petroleum ether:ethyl acetate = 1:10-1:1) to obtain a white solid intermediate **7D** (0.5 g, 1.5 mmol, yield: 27%). m/z (ESI): 334 $[M+H]^+$.

**Step 3: 4-(bromomethyl)-*N*-(1-(bromomethyl)cyclopropyl)benzamide (7E)**

The intermediate **7D** (0.5 g, 1.5 mmol) was added to dichloromethane (2 mL), and phosphine tribromide (0.60 g, 2.2 mmol) was added dropwise at 0 °C. The mixture was stirred at room temperature for 2 h. After distillation under vacuum to remove the solvent, the resulting residue was purified by silica gel column chromatography (petroleum ether:ethyl acetate = 1:10-1:1) to obtain a yellow liquid intermediate **7E** (0.2 g, 0.58 mmol, yield: 39%). m/z (ESI): 348 $[M+H]^+$.

**Step 4: 5-(4-(bromomethyl)phenyl)-6-oxa-4-azaspiro[2.4]hept-4-ene (7F)**

The intermediate **7E** (0.30 g, 0.86 mmol) and DIPEA (0.56 g, 4.3 mmol) were added to THF (5 mL). The reaction solution was heated to 60 °C and stirred for 48 h. Ethyl acetate (50 mL) and water (50 mL) were added for extraction and liquid separation, and the organic phase was dried over anhydrous sodium sulfate, filtered, and concentrated under vacuum to obtain a colorless transparent liquid intermediate **7F** (0.20 g, 0.75 mmol, yield: 87%). m/z (ESI): 266, 268 $[M+H]^+$.

**Step 5: 8-(4-(6-oxa-4-azaspiro[2.4]hept-4-en-5-yl)benzyl)-2-(4-cyclopropyl-6-methoxypyrimidin-5-yl)-7,8-dihydro-6H-pyrimido[5,4-b][1,4]oxazine (7)**

A compound **7G** (40 mg, 0.14 mmol) was added to tetrahydrofuran (2 mL), and sodium hydride (10 mg, 0.42 mmol) was added at 0 °C, then the resulting mixture was stirred for 5 min and heated to room temperature, and the intermediate **7F** (37 mg, 139.03 μmol) was added to the reaction solution. The resulting mixture was stirred at 60 °C for 1 h. Water (10 mL) was added to the reaction system, and the resulting mixture was extracted three times with ethyl acetate (10 mL × 3). The resulting organic phases were combined and concentrated under vacuum, and the resulting crude product was prepared and lyophilized to obtain a white solid title compound 7 (2.8 mg, 5.95 μmol, yield: 4.28%).

m/z (ESI): 471[M+H]$^+$.

$^1$H NMR (400 MHz, DMSO-$d_6$): $\delta$ 8.58(s, 1H), 7.95(s, 1H), 7.78(d, $J$ = 8.1 Hz, 2H), 7.37(d, $J$ = 8.1 Hz, 2H), 4.84(s, 2H), 4.40(s, 2H), 4.27(s, 2H), 3.82(s, 3H), 3.56(s, 2H), 1.76-1.69(m, 1H), 1.07(m, 2H), 0.96(m, 2H), 0.85(m, 2H), 0.79(m, 2H).

**Example 8: Preparation of 2-(4-cyclopropyl-6-methoxypyrimidin-5-yl)-8-(4-(5,5-dimethyl-4,5-dihydrooxazol-2-yl)benzyl)-7,8-dihydro-6H-pyrimido[5,4-b][1,4]oxazine (compound 8)**

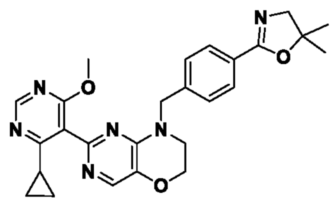

**8**

A compound **8C** was used instead of a compound **7C** to prepare the compound **8** by using a method similar to that in Example **7.**

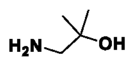

**8C**

m/z (ESI): 473[M+H]$^+$

$^1$H NMR (400 MHz, DMSO-$d_6$) $\delta$ 8.58(s, 1H), 7.94(s, 1H), 7.78(d, $J$ = 7.9 Hz, 2H), 7.37(d, $J$ = 8.0 Hz, 2H), 4.83(s, 2H), 4.26(t, $J$= 4.4 Hz, 2H), 3.83(s, 3H), 3.67(s, 2H), 3.55(t, $J$= 4.6 Hz, 2H), 1.71(m, 1H), 1.41(s, 6H), 0.96(m, 2H), 0.80(m, 2H).

**Example 9: Preparation of 2-(4-cyclopropyl-6-methoxypyrimidin-5-yl)-8-(2-fluoro-4-(1-methyl-4-(trifluoromethyl)-1H-imidazol-2-yl)benzyl)-7,8-dihydro-6H-pyrimido[5,4-b][1,4]oxazine (compound 9)**

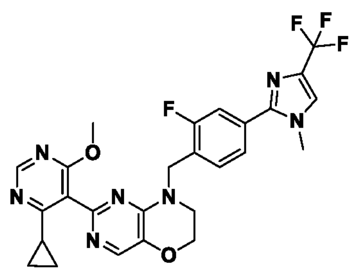

**9**

A compound **9E** was used instead of a compound **1E** to prepare the compound **9** by using a method similar to

that in Example **2.**

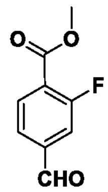

**9E**

m/z (ESI): 542 [M+H]+
$^1$H NMR (400 MHz, Chloroform-*d*) $\delta$8.61(s, 1H), 8.01(s, 1H), 7.52(m, 1H), 7.41(dd, *J* = 10.4, 1.7 Hz, 1H), 7.37-7.31(m, 2H), 4.97(s, 2H), 4.26(t, *J* = 4.5 Hz, 2H), 3.94(s, 3H), 3.78(s, 3H), 3.58(t, *J* = 4.6 Hz, 2H), 1.83(m, 1H), 1.18(m, 2H), 0.88(m, 2H).

**Example 10: Preparation of 2-(4-cyclopropyl-6-methoxypyrimidin-5-yl)-8-(2,6-difluoro-4-(1-methyl-4-(trifluoromethyl)-1H-imidazol-2-yl)benzyl)-7,8-dihydro-6H-pyrimido[5,4-b][1,4]oxazine (compound 10)**

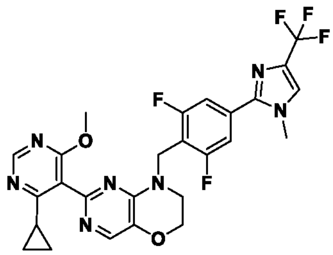

**10**

A compound **10E** was used instead of the compound **1E** to prepare the compound **10** by using a method similar to that in Example **2.**

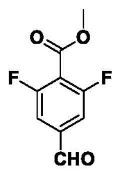

**10E**

m/z (ESI): 542 [M+H]+
$^1$H NMR (400 MHz, Chloroform-*d*) $\delta$ 8.64(s, 1H), 8.09(s, 1H), 7.35(s, 1H), 7.28(s, 2H), 5.11(s, 2H), 4.30-4.19(m, 2H), 3.98(s, 3H), 3.83(s, 3H), 3.63-3.50(m, 2H), 1.91(m, 1H), 1.24(m, 2H), 0.94(m, 2H).

**Example 11: Preparation of 2-(4-cyclopropyl-6-methoxypyrimidin-5-yl)-5-methyl-8-(4-(1-methyl-4-(trifluoromethyl)-1H-imidazol-2-yl)benzyl)-7,8-dihydropteridin-6(5H)-one (compound 11)**

The synthesis route is as follows:

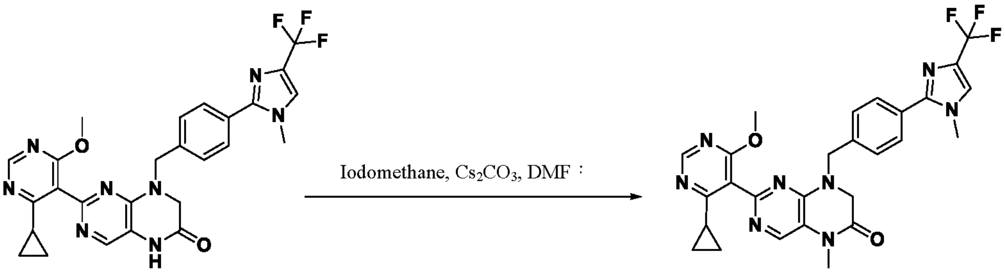


5 11

The compound **5** (7.5 mg, 14 μmol), cesium carbonate (9.1 mg, 28 μmol), and DMF (0.50 mL) were added to a reaction flask. The mixture was cooled to 0 °C, and iodomethane (2.0 mg, 14 μmol, 0.87 μL) was added dropwise for reaction for 30 min. To the resulting mixture, 0.2 mL of water was added, and the resulting mixture was purified by preparative chromatography (Waters Xbridge C18, 5-95% acetonitrile/water mobile phase) to obtain a white solid compound **11** (7.0 mg, 12.71 μmol, yield: 90.96%).

m/z (ESI): 551 $[M+H]^+$

$^1H$ NMR (400 MHz, Chloroform-*d*) δ 8.62(s, 1H), 8.14(s, 1H), 7.93(s, 1H), 7.67(d, *J* = 8.2 Hz, 2H), 7.48(d, *J* = 8.2 Hz, 2H), 4.83(s, 2H), 4.23(s, 2H), 3.85(s, 3H), 3.76(s, 3H), 3.30(s, 3H), 1.79(m, 1H), 1.00(m, 2H), 0.84(m, 2H).

**Example 12: Preparation of 2-(4-cyclopropyl-6-methoxypyrimidin-5-yl)-8-((6-(3,3-dimethylpyrrolidin-1-yl)pyridin-3-yl)methyl)-7,8-dihydro-6H-pyrimido[5,4-b][1,4]oxazine (compound 14)**

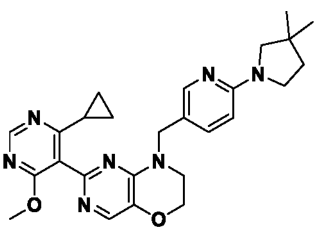

14

The synthesis route is as follows:

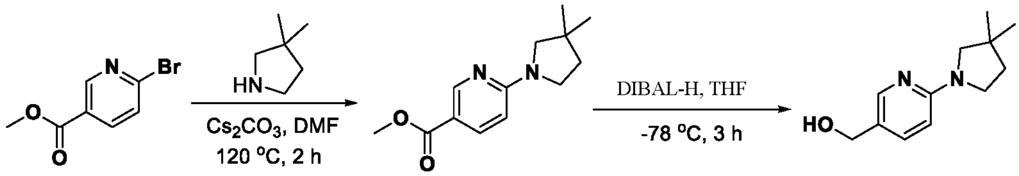


14A 14B 14C

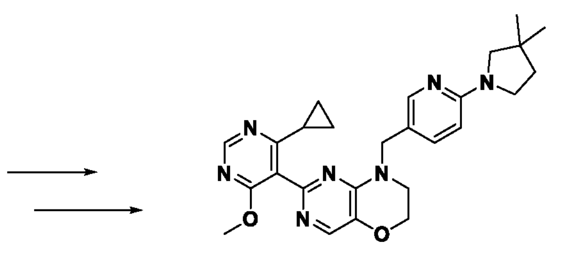

14

**Step 1: Synthesis of methyl 6-(3,3-dimethylpyrrolidin-1-yl)nicotinate (14B)**

In *N,N*-dimethylformamide (3.0 mL), 3,3-dimethylpyrrolidine (0.18 g, 1.8 mmol), methyl 6-bromonicotinate (0.30 g, 1.4 mmol), and cesium carbonate (1.4 g, 4.2 mmol) were dissolved, the resulting mixture was allowed to react at 120 °C for 2 h, after the reaction solution was cooled to room temperature, water (10 mL) was added to the reaction system, and the resulting mixture was extracted three times with ethyl acetate (20 mL × 3). The organic phases were combined, then washed with saturated brine (10 mL), dried over anhydrous sodium sulfate, filtered, and distilled under vacuum to remove the solvent to obtain a crude product **14B** (0.30 mg, yield: 92%). m/z (ESI): 235.1 $[M+H]^+$.

**Step 2: Synthesis of (6-(3,3-dimethylpyrrolidin-1-yl)pyridin-3-yl)methanol (14C)**

The compound **14B** (0.27 mg, 1.2 mmol) was dissolved in anhydrous tetrahydrofuran (2 mL), and the reaction solution was placed at -78 °C, and then diisopropylaluminum hydride (1.5 M, 2.3 mL) was added dropwise. After the addition, the reaction solution was allowed to react at the temperature for another 3 h, then water (10 mL) was added dropwise to quench the reaction, and a saturated aqueous sodium potassium tartrate solution (5.0 mL) was added. The resulting mixture was stirred at room temperature for 30 min, then extracted three times with ethyl acetate (10 mL × 3), and the organic phases were combined, then washed with saturated brine (10 mL), dried over anhydrous sodium sulfate, filtered, and distilled under vacuum to remove the solvent to obtain a crude product 14C (0.23 g, yield: 98%). m/z (ESI): 207.1 $[M+H]^+$.

$^1H$ NMR (400 MHz, DMSO-$d_6$): δ 7.97 (d, *J* = 2.1 Hz, 1H), 7.44 (dd, *J* = 8.6, 2.3 Hz, 1H), 6.38 (d, *J* = 8.6 Hz, 1H), 4.94 (s, 1H), 4.33 (s, 2H), 3.45 (d, *J* = 6.9 Hz, 2H), 3.15 (s, 2H), 1.75 (t, *J* = 7.0 Hz, 2H), 1.10 (s, 6H).

The compound **14C** was used instead of the compound **1H,** and the compound **2K** was used instead of the compound **1K** to prepare the compound **14** by using a method similar to that in Example 1. m/z (ESI): 474.2 $[M+H]^+$.

$^1H$ NMR (400 MHz, $CDCl_3$-*d*) δ 8.61 (s, 1H), 8.10-8.04 (m, 1H), 7.96 (s, 1H), 7.49 (d, *J* = 8.4 Hz, 1H), 6.28 (d, *J* = 8.7 Hz, 1H), 4.70 (s, 2H), 4.23-4.16 (m, 2H), 3.97 (s, 3H), 3.53 (t, *J* = 6.7 Hz, 2H), 3.50-3.44 (m, 2H), 3.21 (s, 2H), 1.86 (m, 1H), 1.80 (t, *J* = 7.0 Hz, 2H), 1.22-1.19 (m, 2H), 1.14 (s, 6H), 0.93 (m, 2H).

**Example 13: Preparation of 2-(4-cyclopropyl-6-methoxypyrimidin-5-yl)-6-methyl-8-(4-(1-methyl-4-(trifluoromethyl)-1H-imidazol-2-yl)benzyl)-7,8-dihydro-6H-pyrimido[5,4-b][1,4]oxazine (compound 15)**

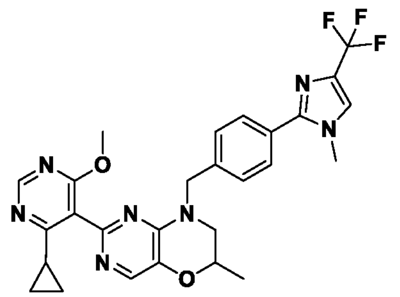

**15**

The synthesis route is as follows:

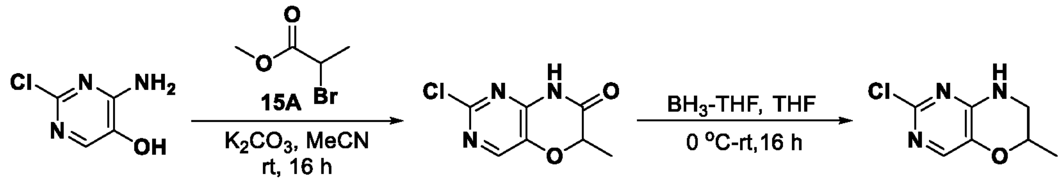


1B 15B 15C

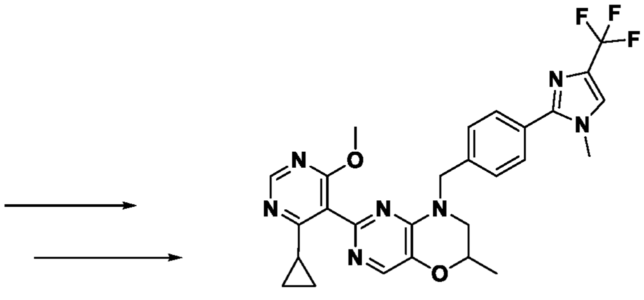

15

**Step 1: 2-chloro-6-methyl-6H-pyrimido[5,4-b][1,4]oxazin-7(8H)-one (15B)**

An intermediate **1B** (500 mg, 3.4 mmol), methyl 2-bromopropionate (0.57 g, 3.4 mmol), and potassium carbonate (712 mg, 5.2 mmol) were dissolved in acetonitrile (5 mL), and the resulting mixture was allowed to react at room temperature for 16 h. Water (10 mL) was then added to the reaction solution, and the resulting mixture was extracted three times with ethyl acetate (20 mL × 3). The resulting organic phases were combined, then washed with saturated brine (20 mL), dried over anhydrous sodium sulfate, and filtered. After the organic phases were distilled under vacuum to remove the solvent, the resulting residue was purified by silica gel column chromatography (petroleum ether:ethyl acetate = 5:1-1:1) to obtain an **intermediate 15B** (200 mg, yield: 29%). m/z (ESI): 200 $[M+H]^+$.

**Step 2: 2-chloro-6-methyl-7,8-dihydro-6H-pyrimido[5,4-b][1,4]oxazine (15C)**

In an ice bath, the intermediate **15B** (200 mg, 1.0 mmol) was dissolved in anhydrous tetrahydrofuran (5 mL), to which a borane-tetrahydrofuran complex (2 mL, 1 mol/L, 2.0 eq) was added, and after the addition, the reaction solution was kept at room temperature for reaction for 16 h. Then methanol (5 mL) was added to the reaction system to quench the reaction. After distillation under vacuum to remove the solvent, water (10 mL) was added, and the resulting mixture was extracted three times with ethyl acetate (20 mL × 3). The resulting organic phases were combined, then washed with saturated brine (20 mL), dried over anhydrous sodium sulfate, filtered, and distilled under vacuum to remove the solvent, and the resulting residue was purified by silica gel column chromatography (petroleum ether:ethyl acetate = 5:1-1:1) to obtain a white solid **intermediate 15C** (100 mg, yield: 54%). m/z (ESI): 186 $[M+H]^+$.

The compound **15C** was used instead of the compound **1D** to prepare the compound **15** by using a method similar to that in Example **2.**

m/z (ESI): 538 $[M+H]^+$.

$^1$H NMR (400 MHz, DMSO-$d_6$): δ 8.64 (s, 1H), 7.96 (s, 1H), 7.93 (s, 1H), 7.67 (d, $J$ = 8.4 Hz, 2H), 7.43 (d, $J$ = 8.0 Hz, 2H), 5.00 (d, $J$ = 15.2 Hz, 1H), 4.70 (d, $J$ = 15.6 Hz, 1H), 4.35-4.30 (m, 1H), 3.84 (s, 3H), 3.76 (s, 3H), 3.61 (dd, $J$ = 12.8 Hz, 2.4 Hz, 1H), 3.30-3.27 (m, 1H), 1.78-1.73 (m, 1H), 1.32 (d, $J$ = 6.4 Hz, 3H), 0.99-0.96 (m, 2H), 0.85-0.82 (m, 2H).

**Example 14: Preparation of 2-(4-cyclopropyl-6-methoxypyrimidin-5-yl)-8-(((5-(1-methyl-4-(trifluoromethyl)-1H-imidazol-2-yl)pyridin-2-yl)methyl)-7,8-dihydro-6H-pyrimido [5,4-b][1,4]oxazine (compound 16)**

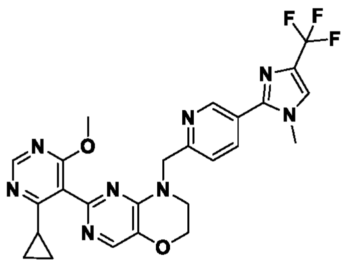

16

A compound **16E** was used instead of the compound **1E** to prepare the compound **16** by using a method similar to that in Example **2.**

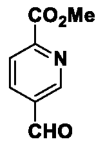

16E

m/z (ESI): 525.1 [M+H]$^+$.

$^1$H NMR (400 MHz, DMSO-$d_6$): 6 8.83 (dd, *J* = 2.3, 0.9 Hz, 1H), 8.56 (s, 1H), 8.10 (dd, *J* = 8.2, 2.3 Hz, 1H), 8.00 (d, *J* = 1.4 Hz, 1H), 7.96 (s, 1H), 7.43 (d, *J* = 8.2 Hz, 1H), 4.96 (s, 2H), 4.34 (t, *J* = 4.5 Hz, 2H), 3.78 (d, *J* = 5.4 Hz, 6H), 3.74 (t, *J* = 4.7 Hz, 2H), 1.66 (m, 1H), 0.91 (m, 2H), 0.71 (m, 2H).

**Example 15: Preparation of 2-(4-cyclopropyl-6-methoxypyrimidin-5-yl)-8-((6-(1-methyl-4-(trifluoromethyl)-1H-imidazol-2-yl)pyridin-3-yl)methyl)-7,8-dihydro-6H-pyrimido [5,4-b][1,4]oxazine (compound 17**

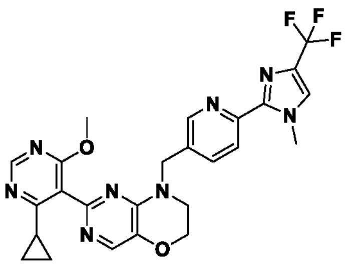

17

A compound **17E** was used instead of the compound **1E** to prepare the compound **17** by using a method similar to that in Example **2.**

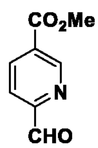

17E

m/z (ESI): 525 [M+H]$^+$.

$^1$H NMR (400 MHz, DMSO-$d_6$): δ 8.68 (s, 1H), 8.61 (s, 1H), 8.06 (d, *J* = 8.2 Hz, 1H), 7.99 (s, 1H), 7.97 (s, 1H), 7.89 (d, *J* = 8.2 Hz, 1H), 4.88 (s, 2H), 4.31 (d, *J* = 4.2 Hz, 2H), 4.09 (s, 3H), 3.87 (s, 3H), 3.68-3.63 (m, 2H), 2.06-1.95 (m, 1H), 1.04-0.97 (m, 2H), 0.91-0.80 (m, 2H).

**Example 16: Preparation of 2-(4-cyclopropyl-6-methoxypyrimidin-5-yl)-8-(2,3-difluoro-4-(1-methyl-4-(trifluoromethyl)-1H-imidazol-2-yl)benzyl)-7,8-dihydro-6H-pyrimido[5,4-b][1,4]oxazine (compound 18)**

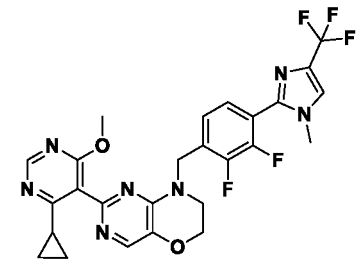

**18**

The synthesis route is as follows:

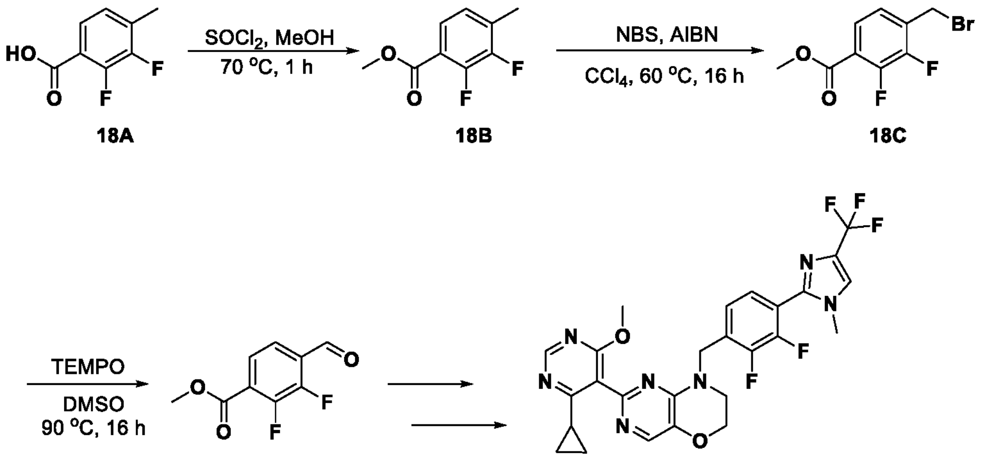


**18A** **18B** **18C**

**18D** **18**

**Step 1: Synthesis of methyl 2,3-difluoro-4-methylbenzoate (18B)**

2,3-difluoro-4-methylbenzoic acid **18A** (4.0 g, 23 mmol, 1.0 eq) was dissolved in methanol (50 mL), to which thionyl chloride (5.5 g, 46 mmol, 2.0 eq) was slowly added, and then the resulting mixture was heated to 70 °C for reaction for 1 h. After the reaction solution was cooled to room temperature and distilled under vacuum to remove the solvent, the resulting residue was purified by silica gel column chromatography (petroleum ether:ethyl acetate = 40:1) to obtain a colorless oily intermediate **18B** (4.0 g, yield: 93%).

**Step 2: Synthesis of methyl 2,3-difluoro-4-bromomethylbenzoate (18C)**

The intermediate **18B** (4 g, 21 mmol, 1.0 eq) was dissolved in carbon tetrachloride (40 mL), then N-bromosuccinimide (5.7 g, 32 mol, 1.5 eq) and azobisisobutyronitrile (706 mg, 4.3 mmol, 0.2 eq) were added, and the resulting mixture was allowed to react at 60 °C for 16 h. After the reaction solution was cooled to room temperature and distilled under vacuum to remove the solvent, the resulting residue was purified by silica gel column chromatography (petroleum ether:ethyl acetate = 100:1-50:1) to obtain a colorless oily intermediate **18C** (2.8 g, yield: 49%).

**Step 3: Synthesis of methyl 2,3-difluoro-4-formylbenzoate (18D)**

The intermediate **18C** (2.8 g, 11 mmol, 1.0 eq) was dissolved in dimethyl sulfoxide (40 mL), then 2,2,6,6-tetramethylpiperidine oxide (1.8 g, 12 mol, 1.1 eq) was added, and the resulting mixture was heated to 90 °C for reaction for 16 h. To the reaction system, 100 mL of water was added, and then the resulting mixture was extracted three times with ethyl acetate (60 mL × 3). The organic phases were combined, then washed with saturated brine (60 mL), dried over anhydrous sodium sulfate, filtered, and distilled under vacuum to remove the solvent, and the resulting residue was

purified by silica gel column chromatography (petroleum ether:ethyl acetate = 5:1-2:1) to obtain a colorless oily intermediate **18D** (0.87 g, yield: 40%).

The compound **18D** was used instead of the compound **1E** to prepare the compound **18** by using a method similar to that in Example **2.**

m/z (ESI): 560 $[M+H]^+$.

$^1H$ NMR (400 MHz, DMSO-$d_6$) δ: 8.59 (s, 1H), 8.04 (s, 1H), 7.95 (s ,1H), 7.60-7.55 (m, 1H), 7.35 (d, *J* = 8.0 Hz, 1H), 4.83 (s, 2H), 4.29 (t, *J* = 6.0 Hz, 2H), 3.79 (s, 3H), 3.62-3.60 (m, 5H), 1.74-1.69 (m, 1H), 0.99-0.95 (m, 2H), 0.85-0.77 (m, 2H).

**Example 17: Preparation of 8-(4-(2H-tetrazol-5-yl)benzyl)-2-(4-cyclopropyl-6-methoxypyrimidin-5-yl)-7,8-dihydro-6H-pyrimido[5,4-b][1,4]oxazine (compound 19)**

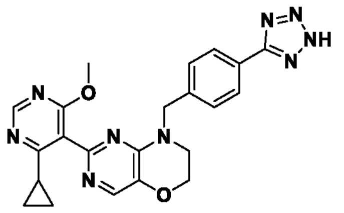

**19**

The synthesis route is as follows:

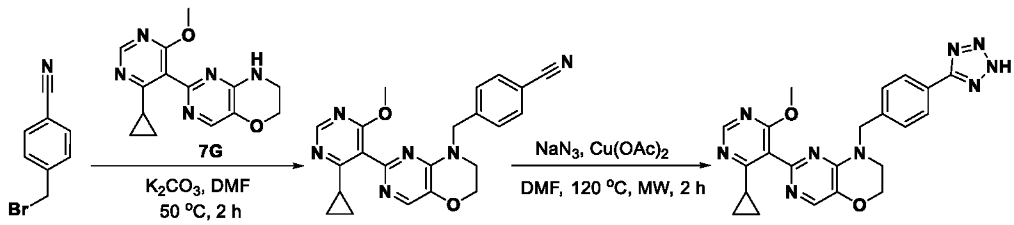


**19A** **19B** **19**

**Step 1: Synthesis of 4-((2-(4-cyclopropyl-6-methoxypyrimidin-5-yl)-7,8-dihydro-6H-pyrimido[5,4-b] [1,4]oxazin-8-yl)methyl)benzonitrile (19B)**

An intermediate **7G** (150 mg, 526 μmol) was dissolved in *N,N*-dimethylformamide (3 mL), then potassium carbonate (513.91 mg, 1.58 mmol) and 4-cyanobenzyl bromide (206.14 mg, 1.05 mmol) were added, and the resulting mixture was allowed to react at 50 °C for 2 h. After the reaction solution was cooled to room temperature, water (10 mL) was added to the reaction system, and then the resulting mixture was extracted three times with ethyl acetate (10 mL × 3). The organic phases were combined, then washed with saturated brine (10 mL), dried over anhydrous sodium sulfate, filtered, and distilled under vacuum to remove the solvent, and the resulting residue was purified by silica gel column chromatography to obtain an intermediate **19B** (177 mg, yield: 84%). m/z (ESI): 401.1 $[M+H]^+$.

$^1H$ NMR (400 MHz, DMSO-$d_6$): δ 8.58 (s, 1H), 7.96 (s, 1H), 7.81 (d, *J* = 8.3 Hz, 2H), 7.48 (d, *J* = 8.2 Hz, 2H), 4.86 (s, 2H), 4.29 (t, *J* = 4.4 Hz, 2H), 3.81 (s, 3H), 3.59 (t, *J* = 4.4 Hz, 2H), 1.76-1.62 (m, 1H), 0.95 (m, 2H), 0.78 (m, 2H).

**Step 2: 8-(4-(2H-tetrazol-5-yl)benzyl)-2-(4-cyclopropyl-6-methoxypyrimidin-5-yl)-7,8-dihydro-6H-pyrimido[5,4-b][1,4]oxazine (19)**

The intermediate **19B** (160 mg, 400 μmol), sodium azide (52 mg, 800 μmol), and copper acetate (7.3 mg, 40 μmol) were dissolved in *N,N*-dimethylformamide (1 mL), and the resulting mixture was subjected to microwave reaction at 120 °C for 2 h. After the reaction solution was cooled to room temperature, water (10 mL) was added to the reaction system, and then the resulting mixture was extracted three times with ethyl acetate (10 mL × 3). The organic phases were combined, then washed with saturated brine (10 mL), dried over anhydrous sodium sulfate, filtered, and distilled under vacuum to remove the solvent, and the resulting residue was purified by preparative chromatography (Waters Xbridge C18, 10-75% aqueous acetonitrile solution) to obtain a solid title compound **19** (50 mg, yield: 28%). m/z (ESI):

444.2 [M+H]+.

$^1$H NMR (400 MHz, DMSO-$d_6$): δ 8.59 (s, 1H), 7.97 (d, *J* = 8.2 Hz, 2H), 7.95 (s, 1H), 7.46 (d, *J* = 8.2 Hz, 2H), 4.85 (s, 2H), 4.28 (t, *J* = 4.3 Hz, 2H), 3.84 (s, 3H), 3.59 (t, *J* = 4.4 Hz, 2H), 1.75 (m, 1H), 0.97 (m, 2H), 0.82 (m, 2H).

**Example 18: Preparation of 8-(4-(2-methyl-tetrazol-5-yl)benzyl)-2-(4-cyclopropyl-6-methoxypyrimidin-5-yl)-7,8-dihydro-6H-pyrimido[5,4-b][1,4]oxazine (compound 20)**

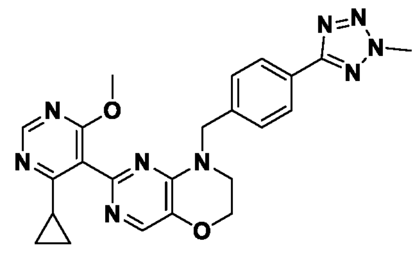

**20**

The synthesis route is as follows:

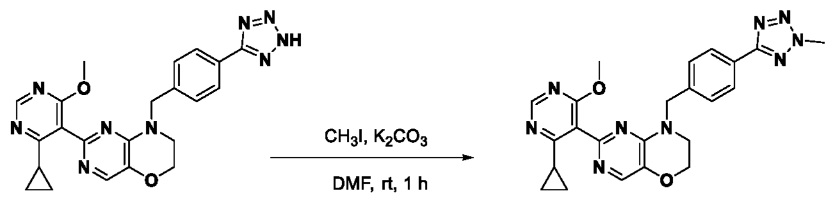


**19** **20**

**Step 1: Synthesis of 8-(4-(2-methyl-tetrazol-5-yl)benzyl)-2-(4-cyclopropyl-6-methoxypyrimidin-5-yl)-7,8-dihydro-6H-pyrimido[5,4-b][1,4]oxazine (20)**

The compound 19 (45 mg, 101 μmol) and potassium carbonate (28 mg, 0.20 mmol) were dissolved in *N,N*-dimethylformamide (1 mL), then iodomethane (14 mg, 0.10 mmol, 6.3 μL) was added, and the resulting mixture was allowed to react at room temperature for 1 h. Water (10 mL) was added to the reaction system, and then the resulting mixture was extracted three times with ethyl acetate (20 mL × 3). The organic phases were combined, then washed with saturated brine (10 mL), dried over anhydrous sodium sulfate, filtered, and distilled under vacuum to remove the solvent, and the resulting residue was purified by preparative chromatography (Waters Xbridge C18, 10-75% aqueous acetonitrile solution) to obtain a solid title compound 20 (4 mg, yield: 8%). m/z (ESI): 458.2 [M+H]+. $^1$H NMR (400 MHz, DMSO-$d_6$): δ 8.58 (s, 1H), 8.01 (d, *J* = 8.2 Hz, 2H), 7.96 (s, 1H), 7.47 (d, *J* = 8.2 Hz, 2H), 4.87 (s, 2H), 4.42 (s, 3H), 4.34-4.24 (m, 2H), 3.83 (s, 3H), 3.65-3.56 (m, 2H), 1.74 (m, 1H), 0.96 (m, 2H), 0.79 (m, 2H).

**Example 19: Preparation of 2-(4-cyclopropyl-6-methoxypyrimidin-5-yl)-6-methyl-8-(4-(1-methyl-4-(trifluoromethyl)-1H-imidazol-2-yl)benzyl)-6H-pyrimido [5,4-b] [1,4] oxazin-7(8H)-one (compound 21)**

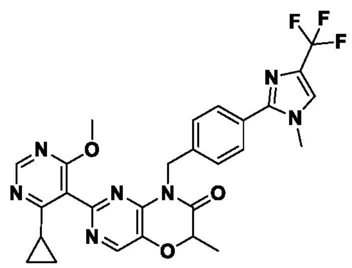

**21**

The compound **15B** was used instead of the compound **1D** to prepare the compound **21** by using a method similar to that in Example **2.**

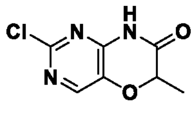

**15B**

m/z (ESI): 552.1 $[M+H]^+$.

$^1$H NMR (400 MHz, DMSO-$d_6$): δ 8.64 (s, 1H), 8.56 (s, 1H), 7.92 (s, 1H), 7.64 (d, *J* = 8.0 Hz, 2H), 7.43 (d, *J* = 8.0 Hz, 2H), 5.24-5.21 (m, 3H), 3.81 (s, 3H), 3.75 (s, 3H), 1.73-1.67 (m, 1H), 1.59 (d, *J* = 6.8 Hz, 3H), 0.99-0.97 (m, 2H), 0.79-0.76 (m, 2H).

**Example 20: Preparation of 2-(4-cyclopropyl-6-methoxypyrimidin-5-yl)-6,6-dimethyl-8-(4-(1-methyl-4-(trifluoromethyl)-1H-imidazol-2-yl)benzyl)-6H-pyrimido [5,4-b] [1,4] oxazin-7(8H)-one (compound 22)**

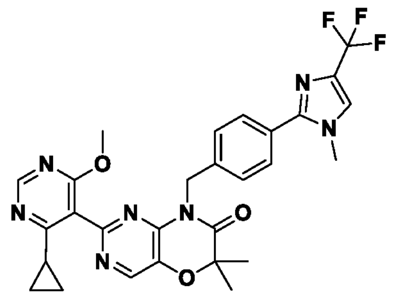

**22**

The synthesis route is as follows:

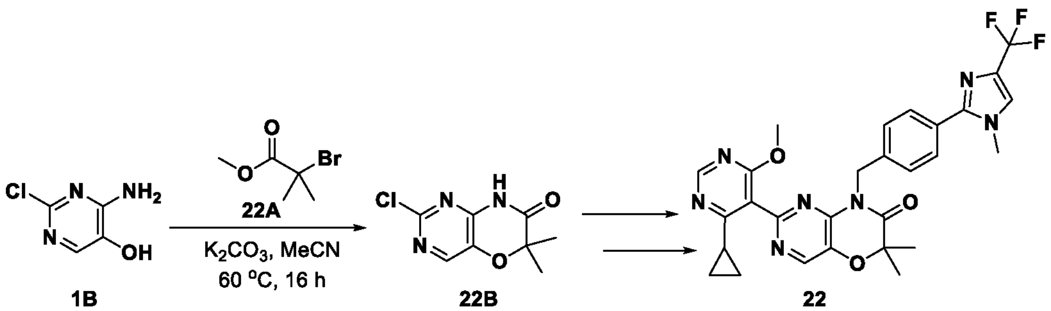


**1B** **22B** **22**

**Step 1: 2-chloro-6,6-dimethyl-6H-pyrimido[5,4-b][1,4]oxazin-7(8H)-one (22B)**

An intermediate **1B** (500 mg, 3.4 mmol), methyl 2-bromoisobutyrate (0.62 g, 3.4 mmol), and potassium carbonate (0.71 g, 5.1 mmol) were dissolved in acetonitrile (5.0 mL), and the reaction solution was placed at 60 °C for reaction for 16 h. Water (10 mL) was then added to the reaction solution, and the resulting mixture was extracted three times with ethyl acetate (20 mL × 3). The resulting organic phases were combined, then washed with saturated brine (20 mL), dried over anhydrous sodium sulfate, and filtered. After the organic phases were distilled under vacuum to remove the solvent, the resulting residue was purified by silica gel column chromatography (petroleum ether:ethyl acetate = 5:1-1:1) to obtain an intermediate **22B** (0.20 g, yield: 27%). m/z (ESI): 214 $[M+H]^+$.

The compound **22B** was used instead of the compound **1D** to prepare the compound **22** by using a method similar to that in Example **2.** m/z (ESI): 566.5 $[M+H]^+$.

$^1$H NMR (400 MHz, DMSO-$d_6$): δ 8.64 (s, 1H), 8.57 (s, 1H), 7.92 (s, 1H), 7.65 (d, *J* = 8.0 Hz, 2H), 7.40 (d, *J* = 8.0 Hz, 2H), 5.24 (s, 2H), 3.82 (s, 3H), 3.75 (s, 3H), 1.72-1.78 (m, 1H), 1.59 (s, 6H), 1.0-0.97 (m, 2H), 0.80-0.77 (m, 2H).

**Example 21: Preparation of 2-(4-cyclopropyl-6-methoxypyrimidin-5-yl)-7-methyl-8-(4-(1-methyl-4-(trifluoromethyl)-1H-imidazol-2-yl)benzyl)-7,8-dihydro-6H-pyrimido[5,4-b][1,4]oxazine (compound 23)**

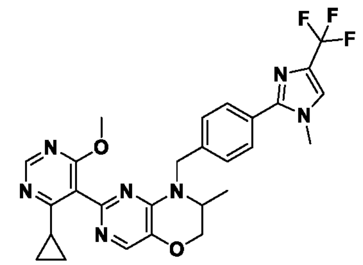

**23**

A compound **23C** was used instead of the compound **1C** to prepare the compound **23** by using a method similar to that in Example **2.**

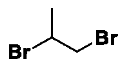

**23C**

m/z (ESI): 538 $[M+H]^+$.

$^1$H NMR (400 MHz, DMSO-$d_6$): δ 8.58 (s, 1H), 7.99 (s, 1H), 7.93 (s ,1H), 7.66 (d, *J* = 8.0 Hz, 2H), 7.44 (d, *J* = 8.0 Hz, 2H), 5.14 (d, *J* = 16.0 Hz, 1H), 4.65 (d, *J* = 16.0 Hz, 1H), 4.18-4.14 (m,1H), 4.10-4.07 (m,1H), 3.86-3.79 (m, 4H), 3.76(s, 3H), 1.80-1.73 (m, 1H), 1.23 (d, *J* = 8.0 Hz, 3H), 0.98-0.94 (m, 2H), 0.93-0.75 (m, 2H).

**Example 22: Preparation of 2-(4-cyclopropyl-6-methoxypyrimidin-5-yl)-5-cyclopropyl-8-(4-(1-methyl-4-(trifluoromethyl)-1H-imidazol-2-yl)benzyl)-7,8-dihydropteridin-6(5H)-one (compound 24)**

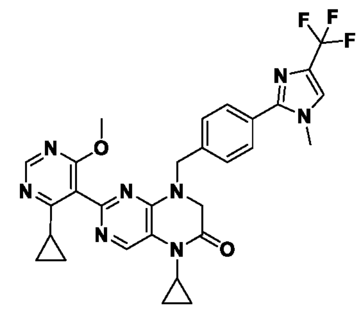

**24**

The synthesis route is as follows:

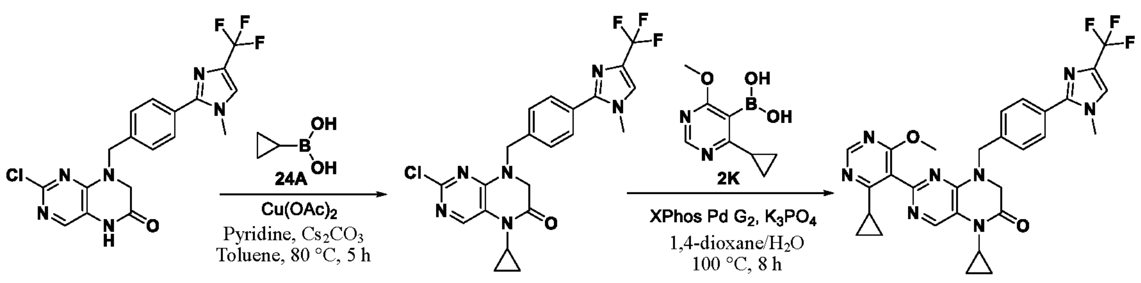


**4G** **24B** **24**

**Step 1: 2-chloro-5-cyclopropyl-8-(4-(1-methyl-4-(trifluoromethyl)-1H-imidazol-2-yl)benzyl)-7,8-dihydropteridin-6(5H)-one (24B)**

Cyclopropylboronic acid (61 mg, 710 μmol), an intermediate **4G** (0.15 g, 0.35 mmol), pyridine (28 mg, 0.36 mmol, 29 μL), copper acetate (64 mg, 0.35 mmol), and cesium carbonate (58 mg, 0.18 mmol) were dissolved in toluene (5.0 mL) for reaction at 80 °C for 5 h. After the reaction solution was cooled to room temperature, water (10 mL) was added to the reaction system, and then the resulting mixture was extracted three times with ethyl acetate (20 mL × 3). The organic phases were combined, then washed with saturated brine (10 mL), dried over anhydrous sodium sulfate, filtered, and distilled under vacuum to remove the solvent, and the resulting residue was purified by preparative chromatography (Waters Xbridge C18, 10-90% aqueous acetonitrile solution) to obtain an intermediate compound **24B** (24 mg, yield: 15%). m/z (ESI): 463.1 $[M+H]^+$.

**Step 2: 2-(4-cyclopropyl-6-methoxypyrimidin-5-yl)-5-cyclopropyl-8-(4-(1-methyl-4-(trifluoromethyl)-1H-imidazol-2-yl)benzyl)-7,8-dihydropteridin-6(SH)-one (24)**

An intermediate **2K** (21 mg, 0.11 mmol), an intermediate **24B** (23 mg, 50 μmol), chloro(2-dicyclohexylphosphino-2',4',6'-triisopropyl-1,1'-biphenyl)[2-(2'-amino-1,1'-biphenyl)]palladium (II) (7.8 mg, 9.9 μmol), and potassium phosphate (32 mg, 0.15 mmol) were added to a mixed solution of 1,4-dioxane (1.0 mL) and water (0.010 mL), and after air therein was replaced by nitrogen, the resulting mixture was allowed to react under the protection of nitrogen at 100 °C for 8 h. After cooling to room temperature, the reaction solution was filtered through diatomite, then the filter cake was rinsed with ethyl acetate, and the resulting filtrates were combined and distilled under vacuum to remove the solvent to obtain a crude compound, which was purified by preparative chromatography (Waters Xbridge C18, 10-90% aqueous acetonitrile solution) to obtain a solid title compound **24** (5 mg, yield: 17%).

m/z (ESI): 577.2 $[M+H]^+$.

$^1H$ NMR (400 MHz, $CDCl_3$): δ 8.64 (s, 1H), 8.49 (s, 1H), 7.61 (d, *J* = 7.4 Hz, 2H), 7.45 (d, *J* = 7.4 Hz, 2H), 7.32 (s, 1H), 4.89 (s, 2H), 4.08 (s, 2H), 3.98 (s, 3H), 3.77 (s, 3H), 2.70 (s, 1H), 1.87 (s, 1H), 1.29-1.21 (m, 4H), 0.91 (s, 4H).

**Example 23: Preparation of 2-(4-cyclopropyl-6-methoxypyrimidin-5-yl)-5-(2,2,2-trifluoroethyl)-8-(4-(1-methyl-4-(trifluoromethyl)-1H-imidazol-2-yl)benzyl)-7,8-dihydropteridin-6(5H)-one (compound 25)**

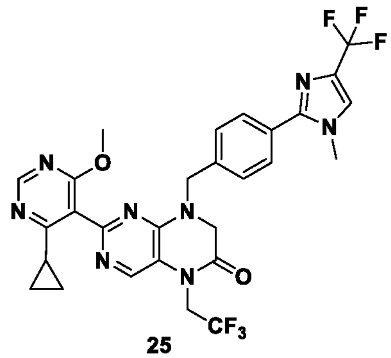

25

The synthesis route is as follows:

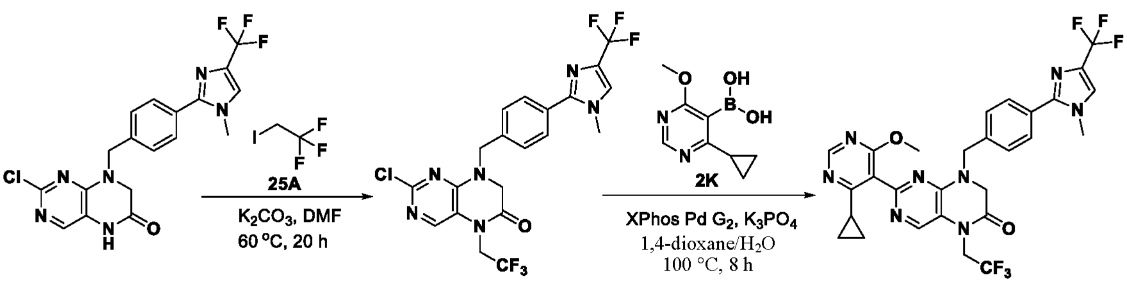


4G 25B 25

**Step 1: Synthesis of 2-chloro-8-(4-(1-methyl-4-(trifluoromethyl)-1H-imidazol-2-yl)benzyl)-5-(2,2,2-trifluoroethyl)-7,8-dihydropteridin-6(5H)-one (25B)**

The intermediate **4G** (0.10 g, 0.24 mmol), 1,1,1-trifluoro-2-iodoethane (75 mg, 0.36 mmol), and potassium carbonate (65 mg, 0.47 mmol) were dissolved in *N,N*-dimethylformamide (2.0 mL), and the resulting mixture was allowed to react at 60 °C for 20 h. After the reaction solution was cooled to room temperature, water (10 mL) was added to the reaction system, and then the resulting mixture was extracted three times with ethyl acetate (10 mL × 3). The organic phases were combined, then washed with saturated brine (10 mL), dried over anhydrous sodium sulfate, filtered, and distilled under vacuum to remove the solvent, and the resulting residue was purified by silica gel column chromatography to obtain an intermediate **25B** (42 mg, yield: 35%). m/z (ESI): 505.1 $[M+H]^+$.

$^1H$ NMR (400 MHz, $CDCl_3$): δ 7.86 (s, 1H), 7.67 (d, *J* = 8.2 Hz, 2H), 7.46 (d, *J* = 8.2 Hz, 2H), 7.32 (s, 1H), 4.91 (s, 2H), 4.55 (q, *J* = 8.3 Hz, 2H), 4.16 (s, 2H), 3.79 (s, 3H).

The compound **25B** was used instead of the compound **24B** to prepare the compound **25** by using a method similar to that in Example **22.**

m/z (ESI): 619.2 $[M+H]^+$.

$^1H$ NMR (400 MHz, $CDCl_3$): δ 8.64 (s, 1H), 8.20 (s, 1H), 7.63 (d, *J* = 7.3 Hz, 2H), 7.47 (d, *J* = 7.4 Hz, 2H), 7.33 (s, 1H), 4.95 (s, 2H), 4.69-4.57 (m, 2H), 4.22 (s, 2H), 3.97 (s, 3H), 3.78 (s, 3H), 1.85 (s, 1H), 1.25 (m, 2H), 0.93 (m, 2H).

**Example 24: Preparation of 2-(4-cyclopropyl-6-methoxypyrimidin-5-yl)-5,7-dimethyl-8-(4-(1-methyl-4-(trifluoromethyl)-1H-imidazol-2-yl)benzyl)-7,8-dihydropteridin-6(5H)-one (compound 26)**

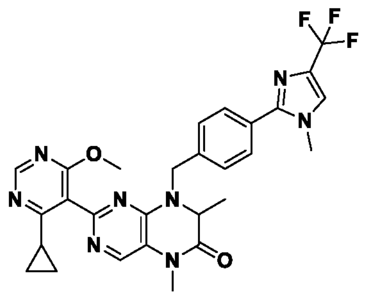

**26**

The synthesis route is as follows:

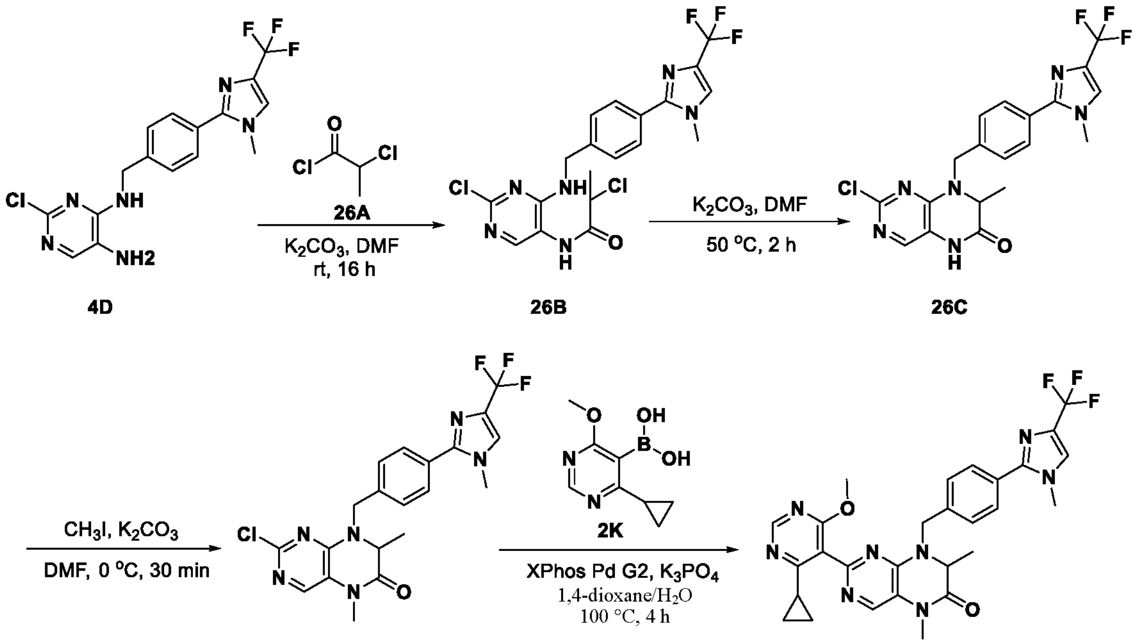


4D 26B 26C 26D 26

**Step 1: Synthesis of 2-chloro-N (2-chloro-4-((4-(1-methyl-4-(trifluoromethyl)-1H-imidazol-2-yl)benzyl)amino)pyrimidin-5-yl)propionamide (26B)**

In an ice bath, the compound **4D** (0.36 g, 0.93 mmol) was dissolved in anhydrous *N,N*-dimethylformamide (5.0 mL), then 2-chloropropionyl chloride (0.14 g, 1.1 mmol) was slowly added to the solution, followed by potassium carbonate (0.26 g, 1.9 mmol), and the resulting mixture was allowed to react at room temperature for 16 h. Ice water (10 mL) was added to quench the reaction, and the reaction solution was extracted with ethyl acetate (20 mL × 3). The resulting organic phases were combined, then washed with saturated brine (10 mL), dried over anhydrous sodium sulfate, filtered, and distilled under vacuum to remove the solvent, and the resulting residue was purified by silica gel column chromatography (petroleum ether:ethyl acetate = 5:1-1:1) to obtain an intermediate **26B** (0.42 g, yield: 96%). m/z (ESI): 473.0 $[M+H]^+$.

$^1H$ NMR (400 MHz, DMSO-$d_6$): δ 9.72 (s, 1H), 7.95 (s, 1H), 7.93 (s, 1H), 7.69 (d, *J* = 8.2 Hz, 2H), 7.47 (d, *J* = 8.2 Hz, 2H), 4.73-4.62 (m, 3H), 3.78 (s, 3H), 1.65 (d, *J* = 6.7 Hz, 3H).

**Step 2: 2-chloro-7-methyl-8-(4-(1-methyl-4-(trifluoromethyl)-1H-imidazol-2-yl)benzyl)-7,8-dihydropteridin-6(5H)-one (26C)**

The compound **26B** (0.42 g, 0.90 mmol) was dissolved in anhydrous *N,N*-dimethylformamide (10 mL) at room temperature, and then potassium carbonate (0.25 g, 1.8 mmol) was added. The resulting mixture was heated to 50 °C and stirred for reaction for 2 h, then water (20 mL) was added to quench the reaction, and the resulting reaction solution was extracted three times with ethyl acetate (40 mL × 3). The resulting organic phases were combined, then washed with saturated brine (50 mL), dried over anhydrous sodium sulfate, and filtered. After the organic phases were distilled under vacuum to remove the solvent, the resulting residue was purified by silica gel column chromatography (petroleum ether:ethyl acetate = 5:1-1:1) to obtain an intermediate **26C** (0.30 g, yield: 78%). m/z (ESI): 437.1 $[M+H]^+$.

$^1H$ NMR (400 MHz, DMSO-$d_6$) δ 10.89 (s, 1H), 7.94 (s, 1H), 7.71 (d, *J* = 8.1 Hz, 2H), 7.66 (s, 1H), 7.49 (d, *J* = 8.1 Hz, 2H), 5.14 (d, *J* = 15.7 Hz, 1H), 4.58 (d, *J* = 15.7 Hz, 1H), 4.19 (q, *J* = 6.5 Hz, 1H), 3.78 (s, 3H), 1.35 (d, *J* = 6.8 Hz, 3H).

**Step 3: 2-chloro-5,7-dimethyl-8-(4-(1-methyl-4-(trifluoromethyl)-1H-imidazol-2-yl)benzyl)-7,8-dihydropteridin-6(5H)-one (26D)**

In an ice bath, the compound **26C** (0.30 g, 0.69 mmol) and potassium carbonate (0.45 g, 1.4 mmol) were dissolved in *N,N*-dimethylformamide (5.0 mL), and iodomethane (97 mg, 0.69 mmol, 43 μL) was added dropwise for reaction for 30 min. Water (10 mL) was then added to the reaction solution, and the resulting mixture was extracted three times with ethyl acetate (20 mL × 3). The resulting organic phases were combined, then washed with saturated brine (20 mL), dried over anhydrous sodium sulfate, filtered, and distilled under vacuum to remove the solvent, and the

resulting residue was purified by silica gel column chromatography (petroleum ether:ethyl acetate = 5:1-1:1) to obtain an intermediate **26D** (0.28 g, yield: 91%). m/z (ESI): 451.0 $[M+H]^+$.

$^1H$ NMR (400 MHz, DMSO-$d_6$): δ 7.97 (s, 1H), 7.94-7.92 (m, 1H), 7.70 (d, *J* = 8.3 Hz, 2H), 7.50 (d, *J* = 8.3 Hz, 2H), 5.17 (d, *J* = 15.7 Hz, 1H), 4.58 (d, *J* = 15.7 Hz, 1H), 4.32 (q, *J* = 6.8 Hz, 1H), 3.78 (s, 3H), 3.26 (s, 3H), 1.34 (d, *J* = 6.8 Hz, 3H).

**Step 4: 2-(4-cyclopropyl-6-methoxypyrimidin-5-yl)-5,7-dimethyl-8-(4-(1-methyl-4-(trifluoromethyl)-1H-imidazol-2-yl)benzyl)-7,8-dihydropteridin-6(SH)-one (26)**

The intermediate **2K** (31 mg, 0.14 mmol), the intermediate **26D** (40 mg, 89 μmol), XPhos Pd G2 (14 mg, 18 μmol), and potassium phosphate (57 mg, 0.27 mmol) were added to a mixed solution of 1,4-dioxane (1 mL) and water (0.01 mL), and after air therein was replaced by nitrogen, the resulting mixture was allowed to react under the protection of nitrogen at 100 °C for 4 h. After cooling to room temperature, the reaction solution was filtered through diatomite, then the filter cake was rinsed with ethyl acetate, and the resulting filtrates were combined and distilled under vacuum to remove the solvent to obtain a crude compound, which was purified by preparative chromatography (Waters Xbridge C18, 10-90% aqueous acetonitrile solution) to obtain a solid title compound 26 (16 mg, yield: 32%). m/z (ESI): 565.3 $[M+H]^+$.

$^1H$ NMR (400 MHz, DMSO-$d_6$): δ 8.60 (s, 1H), 8.20 (s, 1H), 7.93 (s, 1H), 7.66 (d, *J* = 8.3 Hz, 2H), 7.48 (d, *J* = 8.2 Hz, 2H), 5.14 (d, *J* = 15.8 Hz, 1H), 4.63 (d, *J* = 15.8 Hz, 1H), 4.40 (q, *J* = 6.8 Hz, 1H), 3.83 (s, 3H), 3.76 (s, 3H), 3.33 (s, 3H), 1.75 (m, 1H), 1.35 (d, *J* = 6.8 Hz, 3H), 1.01-0.93 (m, 2H), 0.88-0.74 (m, 2H).

**Example 25: Preparation of 7-(4-cyclopropyl-6-methoxypyrimidin-5-yl)-1-(4-(1-methyl-4-(trifluoromethyl)-1H-imidazol-2-yl)benzyl)-3,4-dihydropyrimido[4,5-d]pyrimidin-2(1H)-one (compound 27)**

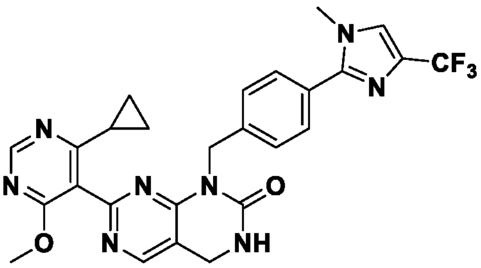

**27**

The synthesis route is as follows:

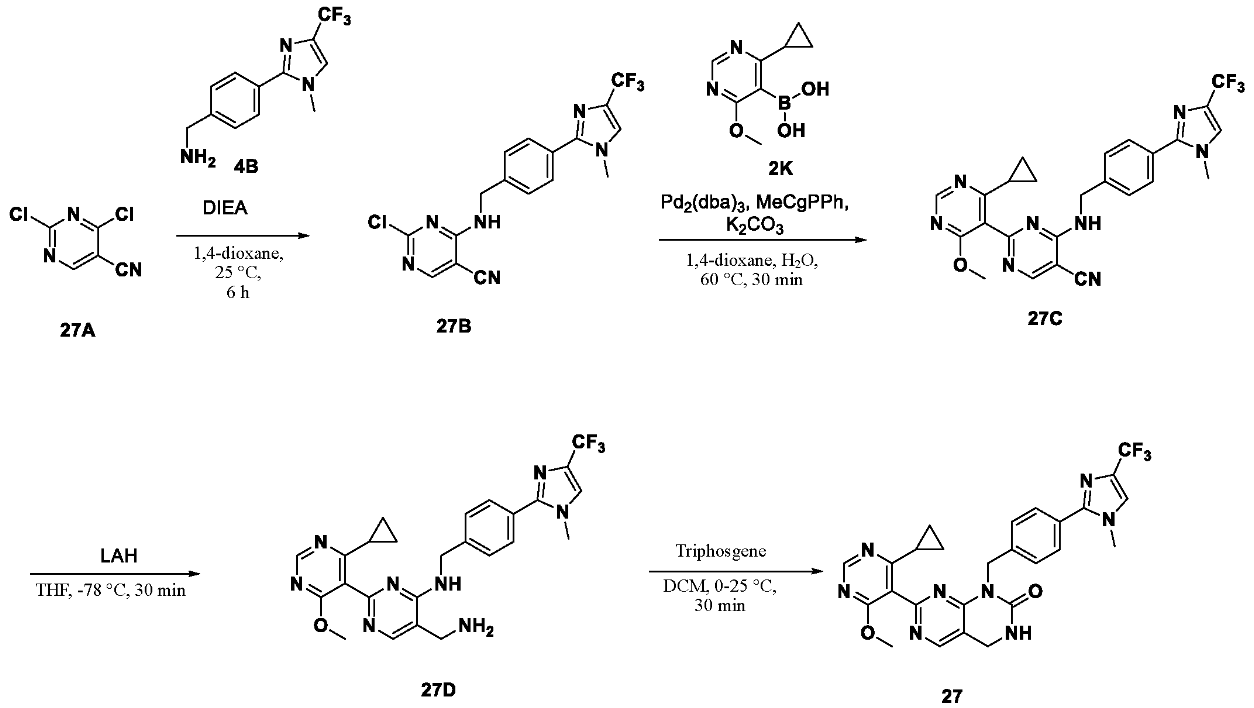


27A 27B 27C 27D 27

**Step 1: 2-chloro-4-((4-(1-methyl-4-(trifluoromethyl)-1H-imidazol-2-yl)benzyl)amino)pyrimidine-5-carbonitrile (27B)**

A compound **27A** (500 mg, 2.87 mmol, 1.0 eq) and the compound **4B** (732 mg, 2.87 mmol, 1.0 eq.) were dissolved in 1,4-dioxane (10 mL) at room temperature, and *N,N*-diisopropylethylamine (556 mg, 4.31 mmol, 1.5 eq.) was added. The resulting mixture was allowed to react at 25 °C for 16 h and concentrated under vacuum to obtain a residue. The residue was purified by silica gel column chromatography to obtain an intermediate **27B** (350 mg, yield: 31%). m/z (ESI): 392.8 $[M+H]^+$

**Step 2: 4'-cyclopropyl-6'-methoxy-4-((4-(1-methyl-4-(trifluoromethyl)-1H-imidazol-2-yl)benzyl)amino)-[2,5'-bipyrimidine]-5-carbonitrile (27C)**

The compound **27B** (330 mg, 0.84 mmol, 1.0 eq.) and the compound **2K** (215 mg, 1.1 mmol, 1.3 eq.) were dissolved in 1,4-dioxane (3 mL), and then tris(dibenzylideneacetone)dipalladium (46 mg, 0.08 mmol, 0.1 eq.), 1,3,5,7-tetramethyl-6-phenyl-2,4,8-trioxa-6-phosphaadamantane (47 mg, 0.16 mmol, 0.02 eq.), potassium carbonate (348 mg, 2.52 mmol, 3.0 eq.) and water (0.5 mL) were added. The resulting mixture was allowed to react at 60 °C for 30 min under an argon atmosphere. The reaction solution was concentrated under vacuum to obtain a brown residue, and the residue was purified by silica gel column chromatography to obtain an intermediate **27C** (310 mg, yield: 73%). m/z (ESI): 506.6 $[M+H]^+$

**Step 3: 5-(aminomethyl)-4'-cyclopropyl-6'-methoxy-N (4-(1-methyl-4-(trifluoromethyl)-1H-imidazol-2-yl)benzyl)-[2,5'-bipyrimidin]-4-amine (27D)**

The compound **27C** (310 mg, 0.61 mmol, 1.0 eq.) was dissolved in anhydrous tetrahydrofuran (3 mL) at room temperature. Lithium aluminum hydride (30 mg, 0.79 mmol, 1.3 eq.) was added slowly at - 78 °C, then the resulting mixture was held at this temperature for 30 min, and aqueous sodium hydroxide was added to quench the reaction. The reaction solution was extracted with ethyl acetate (30 mL × 3), and the organic phases were combined, then washed with saturated brine, dried over anhydrous sodium sulfate, filtered, and concentrated under vacuum to obtain a residue. The resulting residue was purified by silica gel column chromatography to obtain an intermediate **27D** (115 mg, yield: 37%). m/z (ESI): 510.6 $[M+H]^+$

**Step 4: 7-(4-cyclopropyl-6-methoxypyrimidin-5-yl)-1-(4-(1-methyl-4-(trifluoromethyl)-1H-imidazol-2-yl)benzyl)-3,4-dihydropyrimido[4,5-d]pyrimidin-2(1H)-one (27)**

The compound **27D** (0.11 g, 0.22 mmol, 1.0 eq) was dissolved in dichloromethane (5 mL) at room temperature, and in an ice bath, triphosgene (24 mg, 0.08 mmol, 0.35 eq.) was added. The resulting mixture was heated to room temperature for reaction for 30 min, then the reaction solution was concentrated under vacuum to obtain a residue, and the resulting residue was purified by silica gel column chromatography to obtain a compound **27** (60 mg, yield: 51%). m/z (ESI): 536.7 $[M+H]^+$.

**Example 26: Preparation of 2(4-cyclopropyl-6-methoxypyrimidin-5-yl)-8-((4-(1-(difluoromethyl)-4-(trifluoromethyl)-1H-imidazol-2-yl)phenyl)methyl)-5-methyl-7,8-dihydropteridin-6(5H)-one (compound 28)**

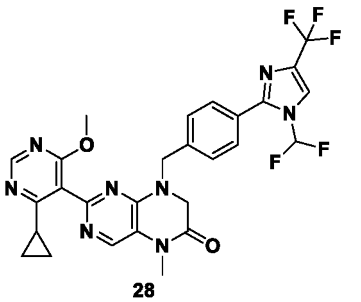

**28**

The synthesis route is as follows:

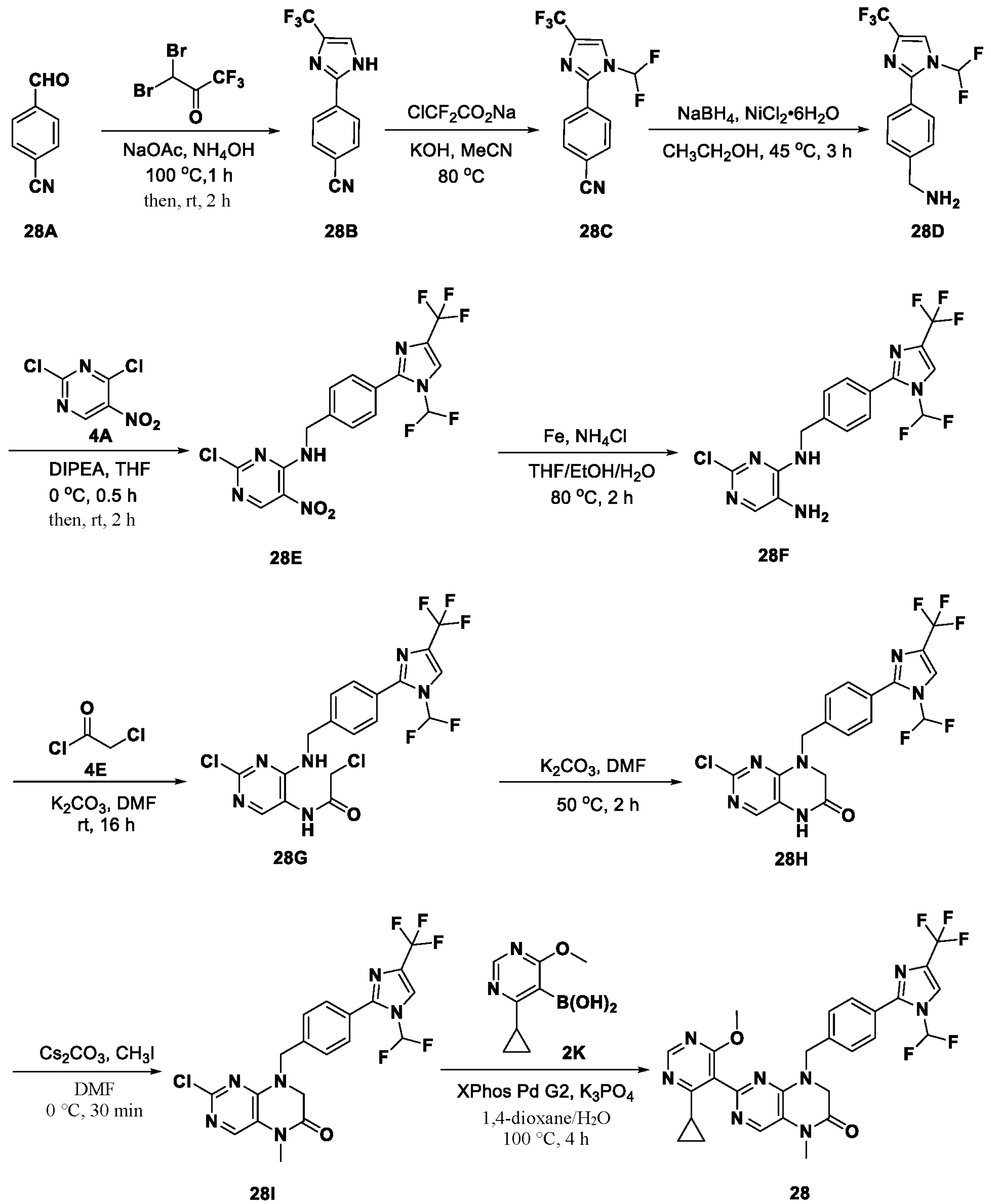


28A 28B 28C 28D

28E 28F

28G 28H

28I 28

**Step 1: Synthesis of 4-(4-(trifluoromethyl)-1H-imidazol-2-yl)benzonitrile (28B)**

At room temperature, 3,3-dibromo-1,1,1-trifluoroacetone (9.9 g, 37 mmol, 1.2 eq) and sodium acetate (3.0 g, 37 mmol, 1.2 eq) were dissolved in water, and the resulting mixture was allowed to react in a 100 °C oil bath for 1 h. The reaction solution was cooled to room temperature, and added slowly dropwise to a solution of 4-cyanobenzaldehyde (2.0 g, 15 mmol, 1.0 eq) in methanol (20 mL), and after ammonia (6 mL) was added, the reaction solution was stirred at room temperature for 16 h. After the reaction solution was concentrated, the resulting residue was poured into water (50 mL) and extracted with ethyl acetate (50 mL × 3). The resulting organic phases were combined, then washed with

saturated brine (50 mL), and distilled under vacuum to remove the solvent. The resulting residue was purified by silica gel column chromatography to obtain an intermediate **28B** (2.6 g, yield: 72%). m/z (ESI): 238 $[M+H]^+$.

**Step 2: Synthesis of 4-(1-(difluoromethyl)-4-(trifluoromethyl)-1H-imidazol-2-yl)benzonitrile (28C)**

The intermediate **28B** (1.0 g, 4.2 mmol), sodium difluorochloroacetate (3.3 g, 21 mmol, 5.0 eq), and potassium hydroxide (1.4 g, 25 mmol, 6.0 eq) were dissolved in anhydrous acetonitrile (20 mL), and the resulting mixture was heated to 80 °C for reaction. After LC-MS showed that the reaction was complete, the solvent was spin-dried, then water (20 mL) was added, and the resulting mixture was extracted with ethyl acetate (50 mL × 3). The resulting organic phases were combined, then washed with saturated brine (50 mL), and distilled under vacuum to remove the solvent. The resulting residue was purified by silica gel column chromatography to obtain an intermediate **28C** (0.81 g, yield: 67%). m/z (ESI): 288 $[M+H]^+$.

**Step 3: Synthesis of (4-(1-(difluoromethyl)-4-(trifluoromethyl)-1H-imidazol-2-yl)phenyl)methylamine (28D)**

The intermediate **28C** (0.81 g, 2.8 mmol) and nickel chloride hexahydrate (0.80 g, 3.4 mmol, 7.5 eq) were dissolved in ethanol, two drops of water were added, then sodium borohydride (0.80 g, 21 mmol, 1.2 eq) was added in portions, and the resulting mixture was heated to 45 °C for reaction for 3 h. After cooling to room temperature, the reaction was quenched with 3 M HCl solution (6.7 mL), then the pH was adjusted to basic with aqueous ammonia, and the resulting reaction solution was extracted with dichloromethane (20 mL × 3). The resulting organic phases were combined, then washed with saturated brine (20 mL), and distilled under vacuum to remove the solvent, and the resulting residue was purified by silica gel column chromatography (dichloromethane:methanol = 4:1) to obtain an intermediate **28D** (0.41 g, yield: 50%). m/z (ESI): 292 $[M+H]^+$.

**Step 4: 2-chloro-*N*-(4-(1-(difluoromethyl)-4-(trifluoromethyl)-1H-imidazol-2-yl)benzyl)-5-nitropyrimidin-4-amine (28E)**

In an ice bath, 2,4-dichloro-5-nitropyrimidine (0.36 g, 1.9 mmol) and N,N-diisopropylethylamine (0.48 g, 3.7 mmol, 2.0 eq) were dissolved in tetrahydrofuran (15 mL), then the **28D** (0.54 g, 1.9 mmol, 1.0 eq) was added, and the resulting mixture was stirred at 0 °C for reaction for 0.5 h and then slowly restored to room temperature for reaction for 2 h. The reaction solution was poured into ice water (50 mL), and the resulting mixture was extracted with ethyl acetate (50 mL × 3). The organic phases were combined, then washed with saturated brine (50 mL), and distilled under vacuum to remove the solvent, and the resulting residue was purified by silica gel column chromatography (petroleum ether:ethyl acetate = 4:1) to obtain a yellow solid intermediate **28E** (0.36 g, yield: 43%). m/z (ESI): 449 $[M+H]^+$.

**Step 5: 2-chloro-*N*$^4$-(4-(1-(difluoromethyl)-4-(trifluoromethyl)-1H-imidazol-2-yl)benzyl)pyrimidine-4,5-diamine (28F)**

At room temperature, the compound **28E** (0.36 g, 0.80 mmol) and reduced iron powder (0.45 g, 8.0 mmol, 10 eq) were added to a mixed solvent of water (5.0 mL), tetrahydrofuran (5.0 mL), and ethanol (5.0 mL), then ammonium chloride (0.43 g, 8 mmol, 10 eq) was added, and the resulting solution was stirred at 80 °C for reaction for 2 h. The reaction solution was added with ethyl acetate (50 mL) for dilution and filtered while hot, and the filter cake was rinsed with ethyl acetate. After the organic phases were distilled under vacuum to remove the solvent, the resulting residue was purified by silica gel column chromatography (petroleum ether:ethyl acetate = 2:1) to obtain a tawny solid intermediate **28F** (0.30 g, yield: 89%). m/z (ESI): 419 $[M+H]^+$.

**Step 6: 2-chloro-N(2-chloro-4-((4-(1-(difluoromethyl)-4-(trifluoromethyl)-1H-imidazol-2-yl)benzyl)amino)pyrimidin-5-yl)acetamide (28G)**

The compound **28F** (0.30 g, 0.72 mmol) was dissolved in anhydrous *N,N*-dimethylformamide (10 mL) at room temperature, then the resulting solution was cooled to 0 °C, and chloroacetyl chloride (97 mg, 0.86 mmol, 68 μL, 1.2 eq) was added slowly, followed by potassium carbonate (0.20 g, 1.4 mmol, 2.0 eq). The resulting mixture was heated slowly to room temperature for reaction for 16 h, then water (30 mL) was added to quench the reaction, and the reaction solution was extracted with ethyl acetate (20 mL × 3). The resulting organic phases were combined, then washed with saturated brine (50 mL), dried over anhydrous sodium sulfate, filtered, and distilled under vacuum to remove the solvent, and the resulting residue was purified by silica gel column chromatography (petroleum ether:ethyl acetate = 2:1-1:1) to obtain an intermediate **28G** (0.30 g, yield: 85%). m/z (ESI): 495 $[M+H]^+$.

**Step 7: 2-chloro-8-(4-(1-(difluoromethyl)-4-(trifluoromethyl)-1H-imidazol-2-yl)benzyl)-7,8-dihydropteridin-6(5H)-one (28H)**

The compound **28G** (0.30 g, 0.61 mmol) was dissolved in anhydrous *N,N*-dimethylformamide (10 mL) at room temperature, and potassium carbonate (0.17 mg, 1.2 mmol, 2.0 eq) was added. The resulting mixture was heated to 50 °C and stirred for reaction for 2 h, then water (30 mL) was added to quench the reaction, and the resulting reaction solution was extracted with ethyl acetate (20 mL × 3). The resulting organic phases were combined, then washed with saturated brine (50 mL), dried over anhydrous sodium sulfate, and filtered. After the organic phases were distilled under vacuum to remove the solvent, the resulting residue was purified by silica gel column chromatography (petroleum ether:ethyl acetate = 2:1-1:1) to obtain a yellow solid intermediate **28H** (0.21 mg, yield: 73%). m/z (ESI): 459 $[M+H]^+$.

**Step 8: 2-chloro-8-(4-(1-(difluoromethyl)-4-(trifluoromethyl)-1H-imidazol-2-yl)benzyl)-5-methyl-7,8-dihydropteridin-6(5H)-one (28I)**

The intermediate **28H** (0.10 g, 0.22 mmol) and cesium carbonate (0.14 g, 0.44 mmol) were added to *N,N*-dimethylformamide (5 mL), the resulting mixture was cooled to 0 °C, then iodomethane (31 mg, 0.22 mmol, 13.6 μL) was added dropwise, and after the addition, the mixture was allowed to react under this reaction condition for 30 min. The reaction solution was poured into water, and extracted three times with ethyl acetate (20 mL × 3). The resulting organic phases were combined, then washed with saturated brine (20 mL), dried over anhydrous sodium sulfate, filtered, and distilled under vacuum to remove the solvent, and the resulting residue was purified by silica gel column chromatography (petroleum ether:ethyl acetate = 2:1-1:1) to obtain a brown solid **intermediate 28I** (97 mg, yield: 94%). m/z (ESI): 473 $[M+H]^+$.

**Step 9: 2-(4-cyclopropyl-6-methoxypyrimidin-5-yl)-8-((4-(1-(difluoromethyl)-4-(trifluoromethyl)-1H-imidazol-2-yl)phenyl)methyl)-5-methyl-7,8-dihydropteridin-6(5H)-one (28)**

The intermediate **28I** (40 mg, 85 μmol), the intermediate **2K** (25 mg, 0.13 mmol), XPhos Pd G2 (13 mg, 17 μmol), and potassium phosphate (55 mg, 0.25 mmol) were added to a mixed solution of 1,4-dioxane (1 mL) and $H_2O$ (0.01 mL), and after air therein was replaced by nitrogen, the resulting mixture was allowed to react under the protection of nitrogen at 100 °C for 4 h. After cooling to room temperature, the reaction solution was filtered through diatomite, then the filter cake was rinsed with ethyl acetate, and the resulting filtrates were combined and distilled under vacuum to remove the solvent to obtain a crude compound, which was purified by preparative chromatography (Waters Xbridge C18, 10-90% aqueous acetonitrile solution) to obtain a solid title compound **28** (18 mg, yield: 36%).

m/z (ESI): 587 $[M+H]^+$.

$^1H$ NMR (400 MHz, DMSO-$d_6$): δ 8.62 (s, 1H), 8.52 (t, *J* = 1.6 Hz, 1H), 8.15 (s, 1H), 7.76 (s, 1H), 7.61 (d, *J* = 8.2 Hz, 2H), 7.55 (d, *J* = 8.0 Hz, 2H), 4.86 (s, 2H), 4.23 (s, 2H), 3.85 (s, 3H), 3.31 (s, 3H), 1.79 (m, 1H), 1.03-0.98 (m, 2H), 0.85 (m, 2H).

**Example 27: Preparation of 7-(4-cyclopropyl-6-methoxypyrimidin-5-yl)-3-methyl-1-(4-(1-methyl-4-(trifluoromethyl)-1H-imidazol-2-yl)benzyl)-3,4-dihydropyrimido[4,5-d]pyrimidin-2(1H)-one (compound 29)**

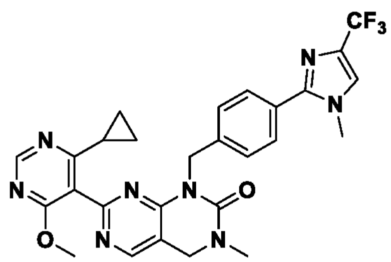

**29**

The synthesis route is as follows:

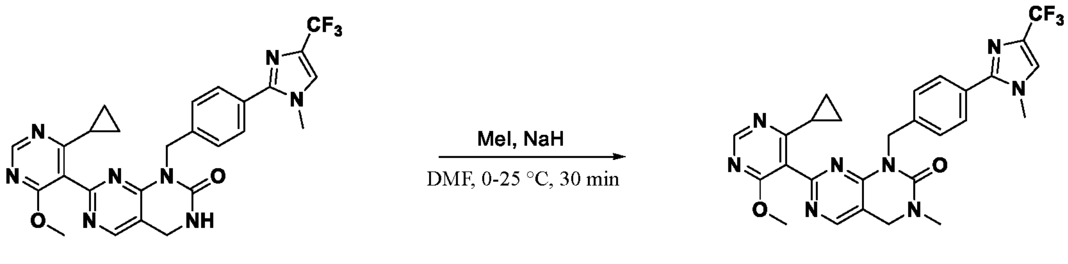


27 29

**Step 1: 7-(4-cyclopropyl-6-methoxypyrimidin-5-yl)-3-methyl-1-(4-(1-methyl-4-(trifluoromethyl)-1H-imidazol-2-yl)benzyl)-3,4-dihydropyrimido [4,5-d] pyrimidin-2(1H)-one (29)**

The compound **27** (20 mg, 0.04 mmol, 1.0 eq.) was dissolved in anhydrous *N,N*-dimethylformamide (3 mL) at 0 °C, and then sodium hydride was added (2.4 mg, 0.06 mmol, 1.5 eq.). After stirring at room temperature for 10 min, iodomethane (6 mg, 0.04 mmol, 1.0 eq.) was added, then the resulting mixture was stirred for 30 min, and an aqueous ammonium chloride solution was added to quench the reaction. The reaction solution was extracted with ethyl acetate (30 mL × 3), and the organic phases were combined, then washed with saturated brine, dried over anhydrous sodium sulfate, filtered, and concentrated under vacuum to obtain a residue. The resulting residue was purified by C18 column chromatography (eluted with 5-95% aqueous acetonitrile solution) to obtain a product **29** (8 mg, yield: 36%).

m/z (ESI): 550.7 $[M+H]^+$
1H NMR (400 MHz, DMSO-d6) δ 8.63 (s, 1H), 8.56 (s, 1H), 7.92 (s, 1H), 7.61 (d, J = 8.3 Hz, 2H), 7.38 (d, J = 8.1 Hz, 2H), 5.18 (s, 2H), 4.62 (s, 2H), 3.80 (s, 3H), 3.74 (s, 3H), 2.98 (s, 3H), 1.68-1.60 (m, 1H), 0.99-0.94 (m, 2H), 0.77-0.72 (m, 2H).

**Example 28: Preparation of 2-(4-cyclopropyl-6-methoxypyrimidin-5-yl)-8-((1-(1-methyl-4-(trifluoromethyl)-1H-imidazol-2-yl)piperidin-4-yl)methyl)-7,8-dihydro-6H-pyrimido[5,4-b][1,4]oxazine (compound 30)**

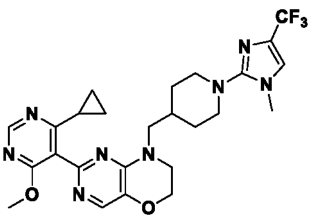

30

The synthesis route is as follows:

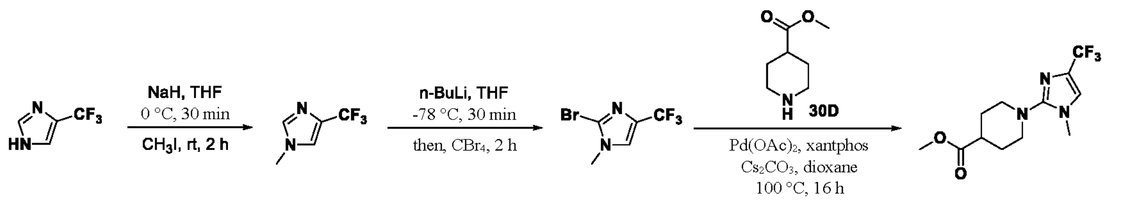


30A 30B 30C , 30E

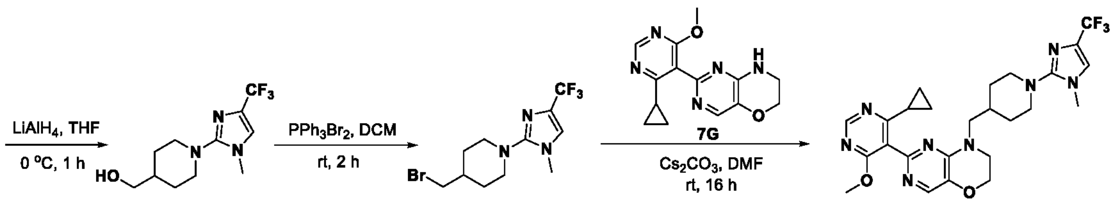


30F 30G 30

**Step 1: Synthesis of 1-methyl-4-(trifluoromethyl)-1H-imidazole (30B)**

4-(trifluoromethyl)-1H-imidazole (5.0 g, 37 mmol) was dissolved in anhydrous tetrahydrofuran (50 mL), and in an ice bath, sodium hydride (content: 60%) (1.6 g, 68 mmol) was added. The resulting mixture was allowed to react at the temperature for 30 min, then iodomethane (5.2 g, 37 mmol) was added, and the mixture was allowed to react at room temperature for 2 h. The reaction solution was poured into a saturated aqueous ammonium chloride solution (20 mL), and the resulting solution was extracted with ethyl acetate (20 mL × 3). The resulting organic phases were combined, then washed with saturated brine (20 mL), dried over anhydrous sodium sulfate, filtered, and distilled under vacuum to remove the solvent, and the resulting residue was purified by silica gel column chromatography (petroleum ether:ethyl acetate = 2:1-1:1) to obtain a pale yellow liquid **30B** (4.58 g, yield 83%). MS m/z (ESI): 151.0 $[M+H]^+$.

**Step 2: Synthesis of 2-bromo-1-methyl-4-(trifluoromethyl)-1H-imidazole (30C)**

The compound **30B** (2.0 g, 13.32 mmol) was dissolved in tetrahydrofuran (20 mL), and cooled to - 78 °C under the protection of nitrogen, and then 2.5 M solution of *n*-butyllithium in *n*-hexane (13 mmol, 5.4 mL) was added dropwise. After the addition, the resulting mixture was allowed to react at the temperature for 30 min, then a solution of carbon tetrabromide (6.6 g, 20 mmol) in tetrahydrofuran was added, and after the addition, the resulting mixture was allowed to react at the temperature for 2 h. The reaction solution was poured into a saturated aqueous ammonium chloride solution (20 mL), and the resulting solution was extracted with ethyl acetate (20 mL × 3). The resulting organic phases were combined, then washed with saturated brine (50 mL), dried over anhydrous sodium sulfate, filtered, and distilled under vacuum to remove the solvent, and the resulting residue was purified by silica gel column chromatography (petroleum ether:ethyl acetate = 10:1-6:1) to obtain a pale yellow liquid **30C** (1.76 g, yield: 57%).

$^1$H NMR (400 MHz, $CDCl_3$): δ 7.31 (s, 1H), 3.68 (s, 3H).

**Step 3: Synthesis of methyl 1-(1-methyl-4-(trifluoromethyl)-1H-imidazol-2-yl)piperidine-4-carboxylate (30E)**

The compound **30C** (0.20 g, 0.87 mmol), a compound **30D** (0.15 g, 1.1 mmol), cesium carbonate (0.85 g, 2.6 mmol), palladium acetate (20 mg, 87 μmol), and Xantphos (0.10 g, 0.17 mmol) were dissolved in 1,4-dioxane (10 mL), and after air therein was replaced by nitrogen, the resulting mixture was allowed to react under the protection of nitrogen at 100 °C for 16 h. After cooling to room temperature, the reaction solution was filtered through diatomite to remove insoluble solids, then the filter cake was rinsed with ethyl acetate, the filtrate was concentrated, and the resulting residue was purified by silica gel column chromatography (petroleum ether: ethyl acetate = 10:1) to obtain a white solid **30E** (0.12 g, yield: 48%).

$^1$H NMR (400 MHz, $CDCl_3$): δ 7.01 (s, 1H), 3.71 (s, 3H), 3.51 (s, 3H), 3.26 (dt, *J* = 12.3, 3.1 Hz, 2H), 2.94 (td, *J* = 12.1, 2.6 Hz, 2H), 2.47 (tt, *J* = 11.3, 4.0 Hz, 1H), 2.03 (dd, *J* = 13.3, 3.2 Hz, 2H), 1.88 (td, *J* = 13.3, 12.4, 3.8 Hz, 2H).

**Step 4: Synthesis of (1-(1-methyl-4-(trifluoromethyl)-1H-imidazol-2-yl)piperidin-4-yl)methanol (30F)**

The compound **30E** (0.13 g, 0.44 mmol) was dissolved in anhydrous tetrahydrofuran (1.2 mL), lithium aluminum hydride (18 mg, 0.48 mmol) was added in portions at 0 °C, and after the addition, the resulting mixture was allowed to react at the temperature for 1 h. Ice water was added dropwise to the reaction solution to quench the reaction, then the

resulting solution was filtered, the filtrate was concentrated, and the resulting residue was purified by silica gel column chromatography (petroleum ether:ethyl acetate = 2:1) to obtain a pale yellow liquid **30F** (60 mg, yield: 94%). MS m/z (ESI): 264.1 $[M+H]^+$.

**Step 5: Synthesis of 4-(bromomethyl)-1-(1-methyl-4-(trifluoromethyl)-1H-imidazol-2-yl)piperidine (30G)**

The compound **30F** (35 mg, 0.13 mmol) was dissolved in anhydrous dichloromethane (0.5 mL), and a solution of triphenylphosphine dibromide (76 mg, 0.17 mmol) in dichloromethane was added at 0 °C. After the addition, the resulting solution was gradually heated to room temperature and was then allowed to react at room temperature for 2 h. The reaction solution was added with water for quenching and extracted with dichloromethane (20 mL × 3), and the resulting organic phases were combined, then washed with saturated brine (10 mL), dried over anhydrous sodium sulfate, and filtered. After the organic phases were distilled under vacuum to remove the solvent, the resulting residue was purified by silica gel column chromatography (petroleum ether:ethyl acetate = 5:1) to obtain a colorless transparent liquid **30G** (35 mg, yield: 80%). MS m/z (ESI): 326.0/328.0 $[M+H]^+$.

**Step 6: Synthesis of 2-(4-cyclopropyl-6-methoxypyrimidin-5-yl)-8-((1-(1-methyl-4-(trifluoromethyl)-1H-imidazol-2-yl)piperidin-4-yl)methyl)-7,8-dihydro-6H-pyrimido[5,4-b][1,4]oxazine (30)**

The compound **7G** (31 mg, 0.11 mmol), the compound **30G** (35 mg, 0.11 mmol) were dissolved in *N,N*-dimethylformamide (1.0 mL), and then cesium carbonate (70 mg, 0.21 mmol) was added for reaction at room temperature for 16 h. To the reaction solution, a saturated aqueous ammonium chloride solution (10 mL) was added, then the resulting solution was extracted with ethyl acetate (10 mL × 3), and the resulting organic phases were combined, then washed with saturated brine (20 mL), dried over anhydrous sodium sulfate, and filtered. After the organic phases were distilled under vacuum to remove the solvent, the resulting residue was purified by preparative chromatography (Waters Xbridge C18, 10-90% aqueous acetonitrile solution) to obtain a white solid **30** (6.5 mg, yield: 21%). MS m/z (ESI): 531.2 $[M+H]^+$.

$^1$H NMR (400 MHz, DMSO-$d_6$): δ 8.60 (s, 1H), 7.86 (s, 1H), 7.52 (s, 1H), 4.25 (t, *J* = 4.2 Hz, 2H), 3.82 (s, 3H), 3.66-3.60 (m, 2H), 3.52 (d, *J* = 7.3 Hz, 2H), 3.46 (s, 3H), 3.23 (m, 2H), 2.71-2.66 (m, 2H), 1.92-1.83 (m, 1H), 1.79-1.76 (m, 1H), 1.67-1.64 (m, 2H), 1.39-1.30 (m, 2H), 1.02-0.90 (m, 2H), 0.88-0.85 (m, 2H).

**Example 29: Preparation of 2-(4-cyclopropyl-6-methoxypyrimidin-5-yl)-8-((5-(1-methyl-4-(trifluoromethyl)-1H-imidazol-2-yl)pyrimidin-2-yl)methyl)-7,8-dihydro-6H-pyrimido[5,4-b][1,4]oxazine (compound 31)**

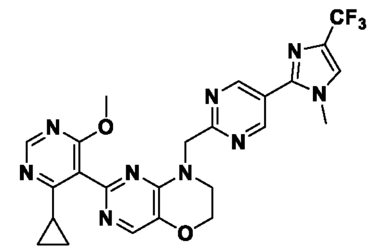

**31**

The synthesis route is as follows:

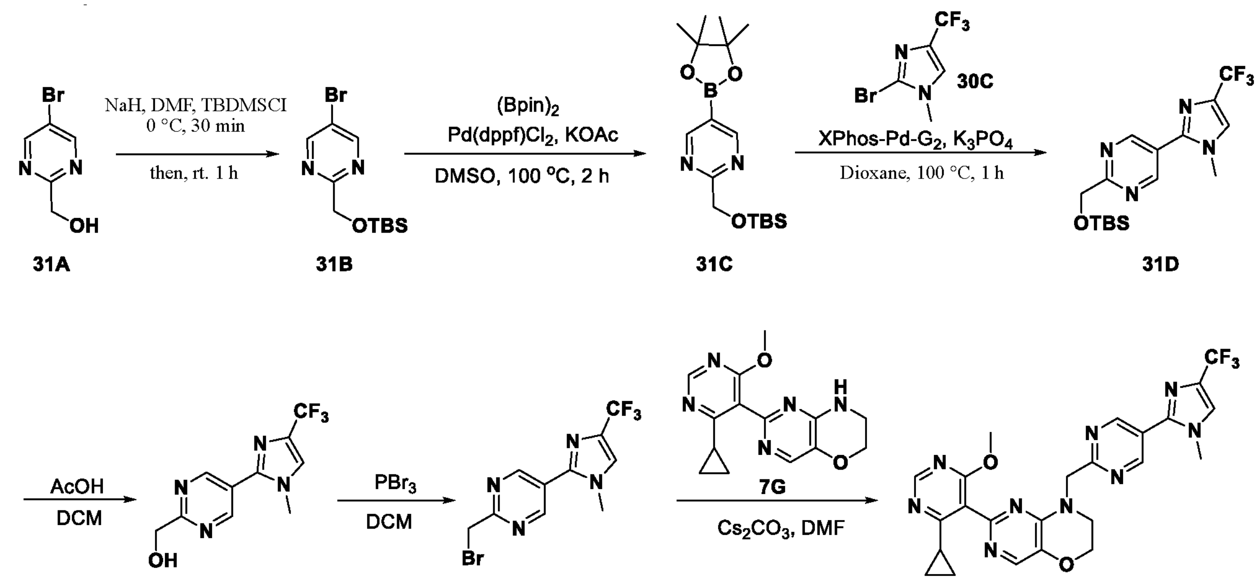


31A 31B 31C 31D

31E 31F 31

**Step 1: Synthesis of 5-bromo-2-(((*tert*-butyldimethylsilyl)oxy)methyl)pyrimidine (31B)**

(5-bromopyrimidin-2-yl)methanol (0.50 g, 2.7 mmol) was dissolved in *N,N*-dimethylformamide (6.0 mL), and sodium hydride (0.16 g, 6.6 mmol) was added in an ice bath, then the resulting solution was stirred at the temperature for 30 min, and tert-butyldimethylchlorosilane (0.60 g, 4.0 mmol) was added for reaction at room temperature for 1 h. To the reaction solution, a saturated aqueous ammonium chloride solution (10 mL) was added, then the resulting solution was extracted with MTBE (10 mL × 3), and the resulting organic phases were combined, then washed with saturated brine (20 mL), dried over anhydrous sodium sulfate, and filtered. After the organic phases were distilled under vacuum to remove the solvent, the resulting residue was purified by silica gel column chromatography (petroleum ether:ethyl acetate = 10:1) to obtain a colorless transparent liquid **31B** (0.62 g, yield: 77%).

$^1H$ NMR (400 MHz, $CDCl_3$): δ 8.64 (s, 2H), 4.75 (s, 2H), 0.80 (s, 9H), 0.00 (s, 6H).

**Step 2: Synthesis of 2-(((*tert*-butyldimethylsilyl)oxy)methyl)-5-(4,4,5,5-tetramethyl-1,3,2-dioxaborolan-2-yl)pyrimidine (31C)**

The compound **31B** (0.60 g, 2.0 mmol), bis(pinacolato)diboron (1.5 g, 5.9 mmol), potassium acetate (0.58 g, 5.9 mmol), and 1,1'-bis(diphenylphosphino)ferrocene dichloropalladium (0.14 g, 0.20 mmol) were dissolved in dimethyl sulfoxide (5.0 mL) for reaction under the protection of nitrogen at 100 °C for 2 h.The reaction solution was filtered, and the filter cake was washed with ethyl acetate. Then a saturated aqueous ammonium chloride solution (10 mL) was added to the filtrate. The resulting solution was extracted with ethyl acetate (10 mL × 3), and the resulting organic phases were combined, then washed three times with saturated brine (10 mL × 3), dried over anhydrous sodium sulfate, and filtered. After the organic phases were distilled under vacuum to remove the solvent, the resulting residue was purified by column chromatography (petroleum ether:ethyl acetate = 20:1-5:1) to obtain a white solid **31C** (0.70 g, yield: 99%). MS m/z (ESI): 269.1 $[M+H]^+$.

**Step 3: 2-(((*tert*-butyldimethylsilyl)oxy)methyl)-5-(1-methyl-4-(trifluoromethyl)-1H-imidazol-2-yl)pyrimidine (31D)**

The compound **31C** (0.61 g, 1.7 mmol), the compound **30C** (0.40 g, 1.7 mmol), potassium phosphate (1.1 g, 5.2 mmol), and XPhos-Pd-$G_2$ (0.14 g, 0.17 mmol) were dissolved in 1,4-dioxane (2.0 mL), and water (0.5 mL) was added. After air therein was replaced by nitrogen, the resulting mixture was allowed to react under the protection of nitrogen at 100 °C for 1 h. The reaction solution was filtered, the filtrate was concentrated to dryness, and the resulting residue was purified by silica gel column chromatography (petroleum ether:ethyl acetate = 10:1-8:1) to obtain a pale yellow liquid **31D** (0.54 g, yield: 89%). MS m/z (ESI): 373.2 $[M+H]^+$.

**Step 4: (5-(1-methyl-4-(trifluoromethyl)-1H-imidazol-2-yl)pyrimidin-2-yl)methanol (31E)**

The compound **31D** (0.30 g, 0.80 mmol) was dissolved in dichloromethane (5.0 mL), acetic acid (0.15 g, 2.4 mmol) was added dropwise at 0 °C, and after the addition, the resulting solution was allowed to react at room temperature for 1 h. The reaction solution was filtered, and the filter cake was washed with ethyl acetate and dried to obtain a white solid **31E** (0.18 g, yield: 84%). MS m/z (ESI): 259.1 $[M+H]^+$.

**Step 5: 2-(bromomethyl)-5-(1-methyl-4-(trifluoromethyl)-1H-imidazol-2-yl)pyrimidine (31F)**

The compound **31E** (50 mg, 0.19 mmol) was dissolved in anhydrous dichloromethane (1.0 mL), then phosphorus tribromide (0.10 mg, 0.39 mmol) was added in an ice bath, and the resulting solution was allowed to react at room temperature for 2 h. The reaction solution was concentrated, added with a sodium bicarbonate solution to adjust the pH to 9, and then extracted with ethyl acetate (10 mL × 3), and the resulting organic phases were combined, then washed with saturated brine (20 mL), dried over anhydrous sodium sulfate, and filtered. After the organic phases were distilled under vacuum to remove the solvent, the resulting residue was purified by silica gel column chromatography (petroleum ether:ethyl acetate = 3:1) to obtain a white solid **31F** (20 mg, yield: 32%). MS m/z (ESI): 321.0/323.0 $[M+H]^+$.

**Step 6: Synthesis of 2-(4-cyclopropyl-6-methoxypyrimidin-5-yl)-8-((5-(1-methyl-4-(trifluoromethyl)-1H-imidazol-2-yl)pyrimidin-2-yl)methyl)-7,8-dihydro-6H-pyrimido[5,4-b][1,4]oxazine (31)**

A compound **7G** (21 mg, 74 μmol) and the compound **31F** (21 mg, 74 μmol) were dissolved in anhydrous *N,N*-dimethylformamide (0.5 mL) then $Cs_2CO_3$ (41 mg, 0.12 mmol) was added, and the resulting solution was allowed to react at 65 °C for 2 h. To the reaction solution, a saturated aqueous ammonium chloride solution (10 mL) was added, and the resulting solution was extracted with ethyl acetate (10 mL × 3). The resulting organic phases were combined, then washed with saturated brine (20 mL), dried over anhydrous sodium sulfate, and filtered. After the organic phases were distilled under vacuum to remove the solvent, the resulting residue was purified by preparative chromatography to obtain a white solid **31** (7.6 mg, yield: 23%).

MS m/z (ESI): 526.2 $[M+H]^+$.

$^1H$ NMR (400 MHz, DMSO-$d_6$): δ 9.16 (s, 2H), 8.60 (s, 1H), 8.12 (s, 1H), 8.02 (s, 1H), 5.15 (m, 2H), 4.45 (m, 2H), 3.89 (s, 5H), 3.78 (s, 3H), 1.59 (m, 1H), 0.92 (m, 2H), 0.67 (m, 2H).

**Example 30: Preparation of 2-(4-cyclopropyl-6-methoxypyrimidin-5-yl)-8-(3-fluoro-4-(1-methyl-4-(trifluoromethyl)-1H-imidazol-2-yl)benzyl)-7,8-dihydro-6H-pyrimido[5,4-b][1,4]oxazine (compound 32)**

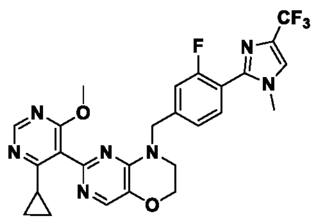

32

A compound **32E** was used instead of methyl 4-formylbenzoate in step **3,** and a compound **2K** was used instead of the compound **1K** to prepare the compound **32** by using a method similar to that in Example **1.**

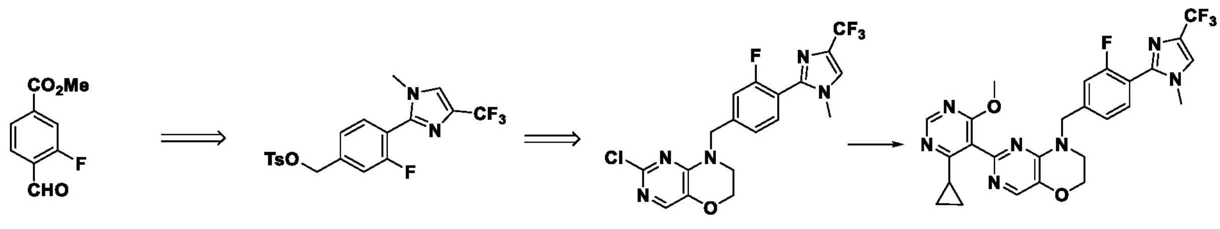

32E 32I 32J 32

m/z (ESI): 542.1 $[M+H]^+$.

$^1H$ NMR (400 MHz, $CDCl_3$): δ 8.61 (s, 1H), 8.05 (s, 1H), 7.59 (t, *J* = 7.6 Hz, 1H), 7.36 (s, 1H), 7.23-7.17 (m, 2H), 4.93 (s, 2H), 4.30-4.23 (m, 2H), 3.96 (s, 3H), 3.65 (s, 3H), 3.57-3.50 (m, 2H), 1.84 (m, 1H), 1.20 (m, 2H), 0.90 (m, 2H).

**Example 31: Preparation of 2-(4-cyclopropyl-6-methoxypyrimidin-5-yl)-5-ethyl-8-(4-(1-methyl-4-(trifluoromethyl)-1H-imidazol-2-yl)benzyl)-7,8-dihydropteridin-6(5H)-one (compound 33)**

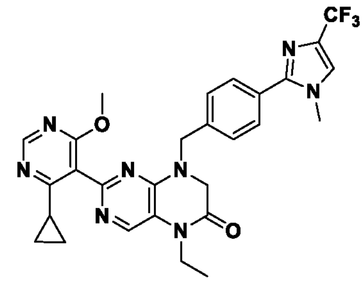

33

The synthesis route is as follows:

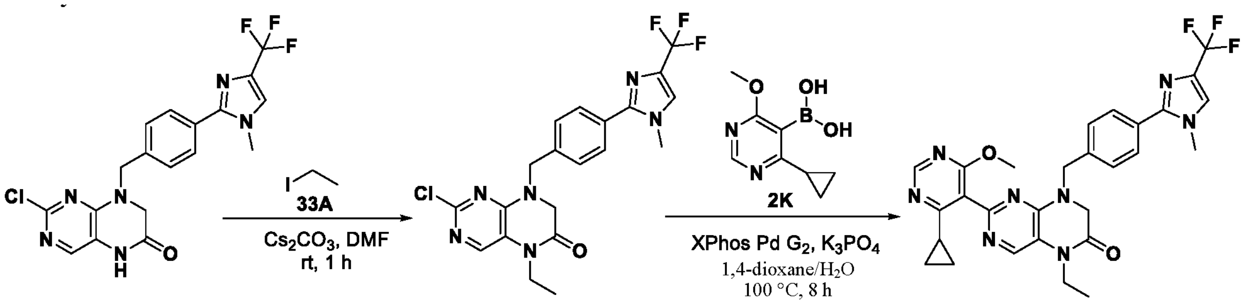


4G 33B 33

**Step 1: Synthesis of 2-chloro-8-(4-(1-methyl-4-(trifluoromethyl)-1H-imidazol-2-yl)benzyl)-5-ethyl-7,8-dihydropteridin-6(5H)-one (33B)**

An intermediate **4G** (66 mg, 156 μmol), iodoethane (75 mg, 0.36 mmol, 35 μL), and $Cs_2CO_3$ (0.15 g, 0.47 mmol) were dissolved in *N,N*-dimethylformamide (1.0 mL), and the resulting solution was allowed to react at room temperature for 1 h. To the reaction solution, a saturated aqueous ammonium chloride solution (10 mL) was added, and the resulting solution was extracted with ethyl acetate (10 mL × 3). The resulting organic phases were combined, then washed with saturated brine (20 mL), dried over anhydrous sodium sulfate, filtered, and distilled under vacuum to remove the solvent, and the resulting residue was purified by silica gel column chromatography to obtain an intermediate **33B** (50 mg, yield: 71%).

$^1H$ NMR (400 MHz, $CDCl_3$): δ 7.75 (s, 1H), 7.65 (d, *J* = 7.8 Hz, 2H), 7.45 (d, *J* = 7.7 Hz, 2H), 7.32 (s, 1H), 4.87 (s, 2H), 4.08 (s, 2H), 3.94 (q, *J* = 7.3 Hz, 2H), 3.78 (s, 3H), 1.30-1.22 (m, 3H).

The compound **33B** was used instead of the compound **24B** to prepare the compound **33** by using a method similar to that in Example **22.**

m/z (ESI): 565.2 $[M+H]^+$.

$^1H$ NMR (400 MHz, DMSO-$d_6$): δ 8.60 (s, 1H), 8.18 (s, 1H), 7.91 (s, 1H), 7.66 (d, *J* = 7.8 Hz, 2H), 7.47 (d, *J* = 7.9 Hz, 2H), 4.82 (s, 2H), 4.21 (s, 2H), 3.95 (d, *J* = 7.4 Hz, 2H), 3.85 (s, 3H), 3.75 (s, 3H), 1.80 (m, 1H), 1.18 (t, *J* = 7.1 Hz, 3H), 0.99 (m, 2H), 0.84 (m, 2H).

**Example 32: Preparation of 2-(4-cyclopropyl-6-methoxypyrimidin-5-yl)-5-ethyl-8-(4-(1-ethyl-4-(trifluoromethyl)-1H-imidazol-2-yl)benzyl)-7,8-dihydropteridin-6(5H)-one (compound 34)**

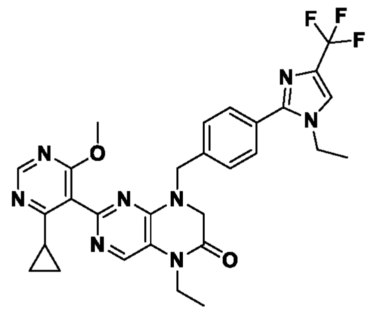

**34**

Iodoethane and sodium hydride were used instead of sodium difluorochloroacetate and potassium hydroxide in step **2,** and iodoethane was used instead of iodomethane in step **8** to prepare the compound **34** by using a method similar to that in Example **26.**

LC-MS: m/z (ESI): 579.2 $[M+H]^+$.

$^1H$ NMR (400 MHz, DMSO-$d_6$): δ 8.61 (s, 1H), 8.20 (s, 1H), 8.02 (s, 1H), 7.59 (d, *J* = 8.3 Hz, 2H), 7.49 (d, *J* = 8.2 Hz, 2H), 4.83 (s, 2H), 4.22 (s, 2H), 4.07 (q, *J* = 7.3 Hz, 2H), 3.96 (q, *J* = 7.0 Hz, 2H), 3.86 (s, 3H), 1.81 (m, 1H), 1.31 (t, *J* = 7.2 Hz, 3H), 1.19 (t, *J* = 7.0 Hz, 3H), 1.03-0.98 (m, 2H), 0.84 (m, 2H).

**Example 33: Preparation of 7-(4-cyclopropyl-6-methoxypyrimidin-5-yl)-3-methyl-1-(4-(1-methyl-4-(trifluoromethyl)-1H-imidazol-2-yl)benzyl)pyrimido[4,5-d]pyrimidine-2,4(1H,3H)-dione (compound 35)**

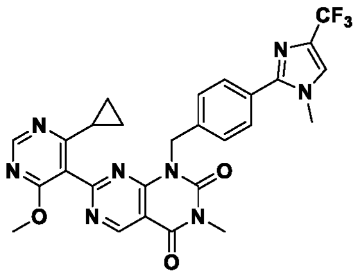

**35**

The synthesis route is as follows:

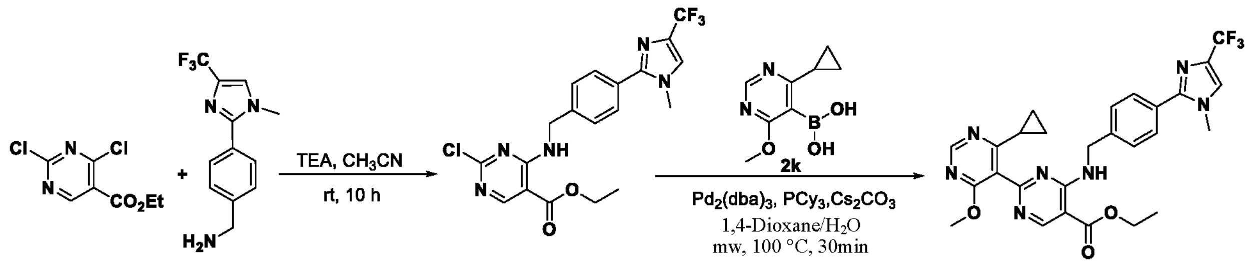


35A 4B 35B 35C

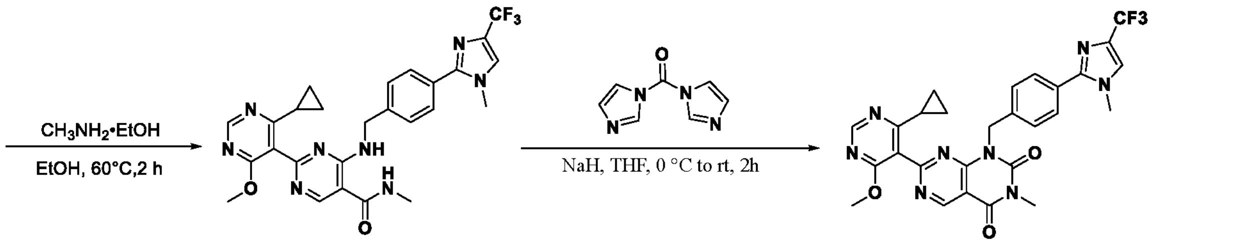


35D 35

**Step 1: Synthesis of ethyl 2-chloro-4-((4-(1-methyl-4-(trifluoromethyl)-1H-imidazol-2-yl)benzyl)amino)pyrimidine-5-carboxylate (35B)**

Ethyl 2,4-dichloropyrimidine-5-carboxylate (1.1 g, 5.0 mmol, 1.0 eq) was dissolved in acetonitrile (10 mL) at room temperature, then (4-(1-methyl-4-(trifluoromethyl)-1H-imidazol-2-yl)phenyl)methylamine **4B** (1.3 g, 5.0 mmol, 1.0 eq) and triethylamine (1.5 g, 15 mmol, 3 eq) were sequentially added for reaction at room temperature for 10 h, then water (30 mL) was added to the reaction system, and the resulting solution was extracted three times with ethyl acetate (20 mL × 3). The organic phases were combined, then washed with saturated brine (20 mL), dried over anhydrous sodium sulfate, filtered, and distilled under vacuum to remove the solvent, and the resulting residue was purified by column chromatography to obtain an intermediate **35B** (1.6 g, yield: 74%).

**Step 2: Synthesis of ethyl 4'-cyclopropyl-6'-methoxy-4-((4-(1-methyl-4-(trifluoromethyl)-1H-imidazol-2-yl)benzyl)amino)-[2,5'-bipyrimidine]-5-carboxylate (35C)**

The compound **35B** (0.40 g, 0.91 mmol) and (4-cyclopropyl-6-methoxypyrimidin-5-yl)boronic acid **2K** (0.18 g, 0.91 mmol) were dissolved in dioxane (3.0 mL) at room temperature. Then 0.5 mL of water was added, followed by tris(dibenzylideneacetone)dipalladium (83 mg, 0.091 mmol, 0.1 eq), tricyclohexylphosphine (51 mg, 0.18 mmol, 0.2 eq), and cesium carbonate (0.38 g, 2.7 mmol, 3.0 eq). After the resulting mixture was subjected to microwave reaction under a nitrogen atmosphere at 100 °C for 30 min, water (5 mL) was added dropwise to the reaction solution to quench the reaction, and then the resulting solution was extracted three times with ethyl acetate (20 mL × 3). The organic phases were combined, then washed with saturated brine (10 mL), dried over anhydrous sodium sulfate, filtered, and distilled under vacuum to remove the solvent, and the resulting residue was purified by column chromatography to obtain an intermediate **35C** (0.32 g, yield: 64%).

**Step 3: Synthesis of 4'-cyclopropyl-6'-methoxy-*N*-methyl-4-((4-(1-methyl-4-(trifluoromethyl)-1H-imidazol-2-yl)benzyl)amino)-[2,5'-bipyrimidine]-5-carboxamide (35D)**

The compound **35C** (0.32 g, 0.58 mmol) was dissolved in ethanol (10 mL) at room temperature, then a solution of methylamine in ethanol (2.0 mL) was added, and the resulting mixture was heated to 60 °C for reaction for 2 h. The reaction solution was distilled under vacuum to remove ethanol, then a saturated aqueous ammonium chloride solution was added, and the resulting solution was extracted three times with ethyl acetate (20 mL × 3). The organic phases were combined, then washed with saturated brine (10 mL), dried over anhydrous sodium sulfate, filtered, and distilled under vacuum to remove the solvent, and the resulting residue was purified by column chromatography to obtain an intermediate **35D** (0.13 g, yield: 41%). m/z (ESI): 539.2 $[M+H]^+$.

**Step 4: Synthesis of 7-(4-cyclopropyl-6-methoxypyrimidin-5-yl)-3-methyl-1-(4-(1-methyl-4-(trifluoromethyl)-1H-imidazol-2-yl)benzyl)pyrimido[4,5-d]pyrimidine-2,4(1H,3H)-dione (35)**

The compound **35D** (40 mg, 0.074 mmol) was dissolved in anhydrous tetrahydrofuran (5 mL), sodium hydride (4.6 mg, 1.2 mmol, 2.0 eq) was slowly added in an ice bath, after the addition, the resulting solution was allowed to react at the temperature for 30 min, and then N,N'-carbonyldiimidazole (33 mg, 0.22 mmol, 3 eq) was added. After reaction at 0 °C for 30 min, the resulting solution was gradually heated to room temperature for reaction for 2 h, and then ice water (5 mL) was added to the reaction system to quench the reaction. After the reaction solution was concentrated to remove tetrahydrofuran, the remaining solution was extracted three times with ethyl acetate (20 mL × 3). The organic phases were combined, then washed with saturated brine (10 mL), dried over anhydrous sodium sulfate, filtered, and distilled under vacuum to remove the solvent, and the resulting residue was purified by preparative chromatography (Waters Xbridge C18, 10-90% aqueous acetonitrile solution) to obtain a white solid title compound **35** (11 mg, yield: 27%). m/z (ESI): 565.2 $[M+H]^+$.

$^1H$ NMR (400 MHz, DMSO-$d_6$) δ 9.36 (s, 1H), 8.69 (s, 1H), 7.93 (d, *J* = 1.3 Hz, 1H), 7.67-7.61 (m, 2H), 7.50 (d, *J* = 8.2 Hz, 2H), 5.43 (s, 2H), 3.83 (s, 3H), 3.74 (s, 3H), 1.75-1.68 (m, 1H), 1.01 (p, *J* = 3.6 Hz, 2H), 0.77 (dq, *J* = 6.8, 3.4 Hz, 2H).

**Example 34: Preparation of 7-(4-cyclopropyl-6-methoxypyrimidin-5-yl)-3-methyl-1-(4-(1-methyl-4-(trifluoromethyl)-1H-imidazol-2-yl)benzyl)-2,3-dihydropyrimido[4,5-d]pyrimidin-4(1H)-one (compound 36)**

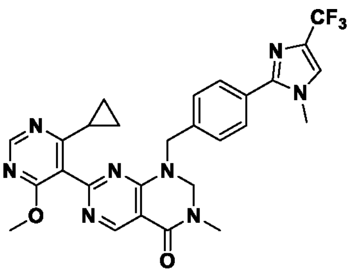

**36**

The synthesis route is as follows:

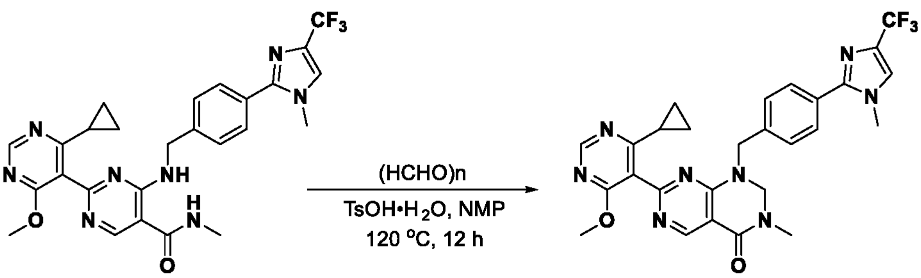


**35D** **36**

**Step 1: Synthesis of 7-(4-cyclopropyl-6-methoxypyrimidin-5-yl)-3-methyl-1-(4-(1-methyl-4-(trifluoromethyl)-1H-imidazol-2-yl)benzyl)-2,3-dihydropyrimido [4,5-d] pyrimidin-4(1H)-one (36)**

The compound **35D** (20 mg, 0.037 mmol) was dissolved in N-methylpyrrolidinone (2 mL), *p*-toluenesulfonic acid monohydrate (70 mg, 0.37 mmol, 10 eq) was added at room temperature, then paraformaldehyde (32 mg, 1.5 mmol, 40 eq, Aladdin, Cat. No.: C104188) was added, and the resulting solution was allowed to react at 120 °C for 12 h. After cooling to room temperature, water (5 mL) was added to the reaction system to quench the reaction, and the solution was extracted three times with ethyl acetate (10 mL × 3). The organic phases were combined, then washed with saturated brine (10 mL), dried over anhydrous sodium sulfate, filtered, and distilled under vacuum to remove the solvent, and the resulting residue was purified by preparative chromatography (Waters Xbridge C18, 10-90% aqueous acetonitrile solution) to obtain a white solid title compound **36** (2.3 mg, yield: 11%). m/z (ESI): 551.2 $[M+H]^+$.

**Example 35: Preparation of 2-(4-cyclopropyl-6-methoxypyrimidin-5-yl)-8-(4-(1-methyl-4-(trifluoromethyl)-1H-imidazol-2-yl)benzyl)-6H-pyrimido[5,4-b] [1,4]thiazin-7(8H)-one (compound 38)**

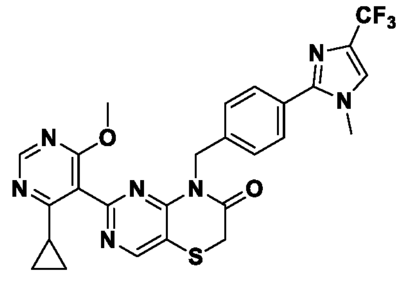

38

The synthesis route is as follows:

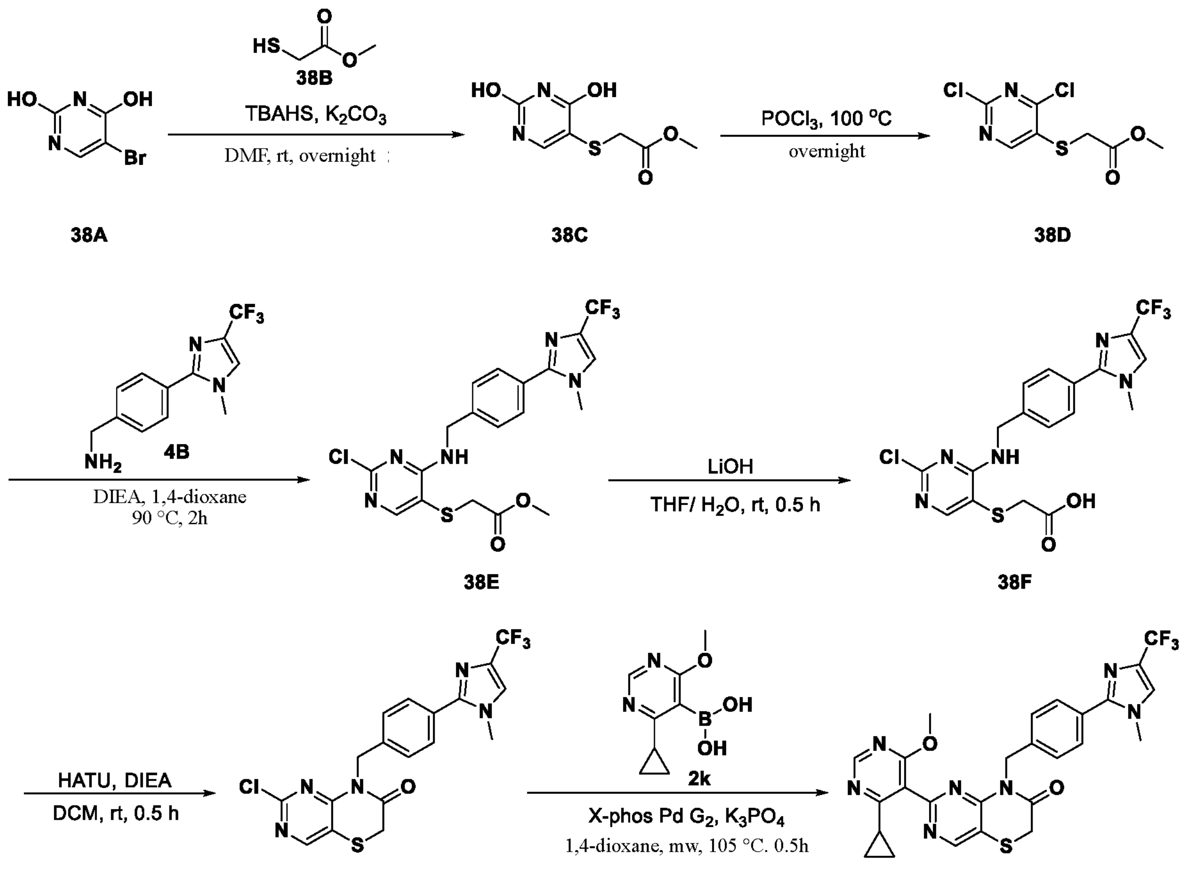


38A 38C 38D

38E 38F

38G 38

**Step 1: Synthesis of methyl 2-((2,4-dihydroxypyrimidin-5-yl)thio)acetate (38C)**

A compound **38A** (3.0 g, 16 mmol, 1.0 eq) and methyl thioglycolate (1.7 g, 16 mmol, 1.0 eq) were dissolved in *N,N*-dimethylformamide (40 mL) at room temperature, and then tetrabutylammonium hydrogen sulfate (1.3 g, 3.9 mmol, 0.25 eq) and potassium carbonate (4.8 g, 35 mmol, 2.2 eq) were sequentially added. The resulting solution was allowed to react overnight at room temperature, then the reaction solution was filtered, and the filtrate was concentrated to obtain a yellow oily substance **38C** (3.4 g, yield: 99%). m/z (ESI): 217.1 $[M+H]^+$.

**Step 2: methyl 2-((2,4-dichloropyrimidin-5-yl)thio)acetate (38D)**

The compound **38C** (3.4 g, 16 mmol) was added to phosphorus oxychloride (20 mL) at room temperature, then the reaction solution was heated to 100 °C, stirred overnight, and concentrated under vacuum to remove phosphorus oxychloride to obtain an oily residue, and ethyl acetate was added. The organic phases were washed with water and saturated brine respectively, dried over anhydrous sodium sulfate, filtered, concentrated under vacuum and purified by silica gel column chromatography to obtain an intermediate **38D** (2.8 g, yield: 70%). m/z (ESI): 253.1 $[M+H]^+$.

**Step 3: methyl 2-((2-chloro-4-((4-(1-methyl-4-(trifluoromethyl)-1H-imidazol-2-yl)benzyl)amino)pyrimidin-5-yl)thio)acetate (38E)**

The compound **38D** (0.20 g, 0.79 mmol, 1.0 eq) and a compound **4B** (0.20 g, 0.79 mmol, 1.0 eq) were dissolved in 1,4-dioxane at room temperature, and then *N,N*-diisopropylethylamine (0.20 g, 1.6 mmol, 2.0 eq) was added. After reaction at 90 °C for 2 h, the resulting solution was concentrated under vacuum and purified by silica gel column chromatography to obtain an intermediate **38E** (110 mg, yield: 29%). m/z (ESI): 471.9 $[M+H]^+$.

**Step 4: 2-((2-chloro-4-((4-(1-methyl-4-(trifluoromethyl)-1H-imidazol-2-yl)benzyl)amino)pyrimidin-5-yl)thio)acetic acid (38F)**

The compound **38E** (0.11 g, 0.23 mmol, 1.0 eq) was dissolved in tetrahydrofuran (3 mL) at room temperature, and then an aqueous solution of lithium hydroxide (8.3 mg, 0.35 mmol, 1.5 eq) was added. After stirring at room temperature for 30 min, 2 M hydrochloric acid solution was added to adjust the mixture to neutral. The resulting solution was extracted with ethyl acetate, and the organic phases were combined, then washed with saturated brine, dried over anhydrous sodium sulfate, filtered, and concentrated under vacuum to obtain an intermediate **38F** (0.10 g, yield: 95%). m/z (ESI): 457.8 $[M+H]^+$.

**Step 5: 2-chloro-8-(4-(1-methyl-4-(trifluoromethyl)-1H-imidazol-2-yl)benzyl)-6H-pyrimido[5,4-b][1,4]thiazin-7(8H)-one (38G)**

The compound **38F** (95 mg, 0.21 mmol, 1.0 eq) was dissolved in dichloromethane (5 mL) at room temperature, then *N,N*-diisopropylethylamine (54 mg, 0.42 mmol, 2.0 eq) and *N,N,N',N'*-tetramethyl-O-(7-azabenzotriazol-1-yl)uronium hexafluorophosphate (95 mg, 0.25 mmol, 1.2 eq) were added for reaction at room temperature for 30 min, then water was added to the reaction system, and the resulting solution was extracted with dichloromethane. The organic phases were combined, then washed with saturated brine, dried over anhydrous sodium sulfate, filtered, and concentrated under vacuum to obtain a crude product, and the crude product was purified by silica gel column chromatography to obtain **38G** (70 mg, 76%). m/z (ESI): 440.1 $[M+H]^+$.

**Step 6: 2-(4-cyclopropyl-6-methoxypyrimidin-5-yl)-8-(4-(1-methyl-4-(trifluoromethyl)-1H-imidazol-2-yl)benzyl)-6H-pyrimido[5,4-b] [1,4]thiazin-7(8H)-one (38)**

The compound **38G** (30 mg, 0.070 mmol, 1.0 eq) and a compound **2k** (18 mg, 0.09 mmol, 1.3 eq) were dissolved in 1,4-dioxane (2 mL) and water (0.5 mL) at room temperature, and then chloro(2-dicyclohexylphosphino-2',4',6'-triisopropyl-1,1'-biphenyl)[2-(2'-amino-1,1'-biphenyl)]palladium (5.5 mg, 0.01mmol, 0.1 eq) and potassium phosphate (45 mg, 0.21 mmol, 3.0 eq) were added. The resulting solution was allowed to react in a microwave reactor under a nitrogen atmosphere at 105 °C for 30 min, then the reaction solution was concentrated, and the concentrate was purified by silica gel column chromatography to obtain a compound **38** (7 mg, yield: 18%). m/z (ESI): 554.1 $[M+H]^+$.

**Example 36: Preparation of 2-(4-cyclopropyl-6-methoxypyrimidin-5-yl)-5-methyl-8-(1-(4-(1-methyl-4-(trifluoromethyl)-1H-imidazol-2-yl)phenyl)cyclopropyl)-7,8-dihydropteridin-6(5H)-one (compound 39)**

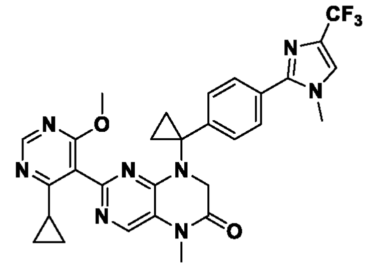

39

The synthesis route is as follows:

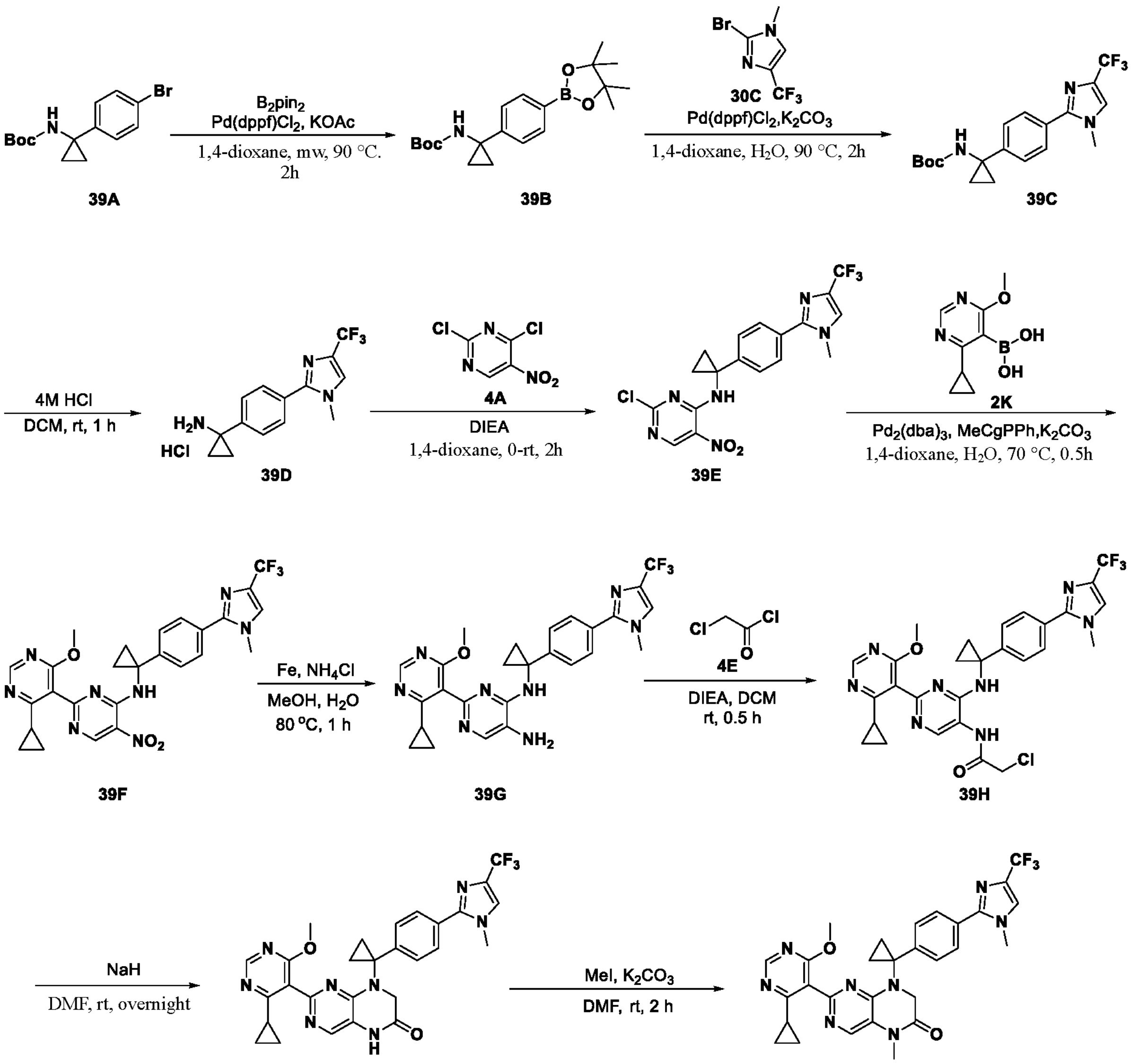


39I

39

**Step 1: Synthesis of *tert*-butyl (1-(4-(4,4,5,5-tetramethyl-1,3,2-dioxaborolan-2-yl)phenyl)cyclopropyl)carbamate (39B)**

A compound **39A** (1.0 g, 3.2 mmol, 1.0 eq) and bis(pinacolato)diboron (1.2g, 4.8 mmol, 1.5 eq) were dissolved in 1,4-dioxane (20 mL) at room temperature, and then [1,1'-bis(diphenylphosphino)ferrocene]dichloropalladium (0.23 g, 0.32 mmol, 0.1 eq) and potassium acetate (0.94 g, 9.6 mmol, 3.0 eq) were added. The resulting solution was allowed to react under a nitrogen atmosphere at 90 °C for 2 h, then the reaction solution was concentrated to obtain a residue,

and the residue was purified by silica gel column chromatography to obtain an intermediate **39B** (1.1 g, yield: 96%). m/z (ESI): 360.3 $[M+H]^+$.

**Step 2: Synthesis of *tert*-butyl (1-(4-(1-methyl-4-trifluoromethyl-1H-imidazol-2-yl)phenyl)cyclopropyl)carbamate (39C)**

The compound **39B** (0.94 g, 2.6 mmol, 1.2 eq) and a compound **30C** (0.50g, 2.18 mmol, 1.0 eq) were dissolved in 1,4-dioxane (10 mL) and water (1 mL) at room temperature, and then [1,1'-bis(diphenylphosphino)ferrocene]dichloropalladium (0.16 g, 0.22 mmol, 0.1 eq) and potassium carbonate (903 mg, 6.54 mmol, 3.0 eq) were added. The resulting solution was allowed to react under a nitrogen atmosphere at 90 °C for 2 h, then the reaction solution was concentrated, and the concentrate was purified by silica gel column chromatography to obtain a product **39C** (0.8 g, yield: 96%). m/z (ESI): 382.2 $[M+H]^+$.

**Step 3: Synthesis of 1-(4-(1-methyl-4-(trifluoromethyl)-1H-imidazol-2-yl)phenyl)cyclopropane-1-amine hydrochloride (39D)**

The compound **39C** (0.40 g, 1.1 mmol, 1.0 eq) was dissolved in dichloromethane (5 mL) at room temperature, and then 4M hydrochloric acid 1,4-dioxane solution (0.8 mL, 3.0 eq) was added. The resulting solution was allowed to react at room temperature for 1h, then the reaction solution was concentrated to obtain **39D** (0.34 g, yield: 100%). m/z (ESI): 282.3 $[M+H]^+$.

**Step 4: 2-chloro-*N*-(1-(4-(1-methyl-4-(trifluoromethyl)-1H-imidazol-2-yl)phenyl)cyclopropyl)-5-nitropyrimidin-4-amine (39E)**

The compound **39D** (0.12 g, 0.38 mmol, 1.0 eq) and a compound **4A** (74 mg, 0.38 mmol, 1.0 eq) were dissolved in 1,4-dioxane (5 mL) at 0 °C, and then *N,N*-diisopropylethylamine (98 mg, 0.76 mmol, 2.0 eq) was added. The resulting solution was heated to room temperature for reaction for 2 h, then the reaction solution was concentrated under vacuum to obtain a residue, and the residue was purified by silica gel column chromatography to obtain an intermediate **39E** (120 mg, yield: 72%). m/z (ESI): 439.1 $[M+H]^+$.

**Step 5: 4'-cyclopropyl-6'-methoxy-*N*-(1-(4-(1-methyl-4-(trifluoromethyl)-1H-imidazol-2-yl)phenyl)cyclopropyl)-5-nitro-[2,5'-bipyrimidin]-4-amine (39F)**

The compound 39E (0.10 g, 0.23 mmol, 1.0 eq) and a compound **2K** (59 mg, 0.30 mmol, 1.5 eq) were dissolved in 1,4-dioxane (2.5 mL) and water (0.5 mL) at room temperature, and then tris(dibenzylideneacetone)dipalladium (18 mg, 0.02 mmol, 0.1 eq), 1,3,5,7-tetramethyl-6-phenyl-2,4,8-trioxa-6-phosphaadamantane (15 mg, 0.05 mmol, 0.2 eq), and potassium carbonate (95 mg, 0.69 mmol, 3.0 e.) were added. The resulting solution was allowed to react under a nitrogen atmosphere at 70 °C for 30 min, then the reaction solution was concentrated, and the concentrate was purified by silica gel column chromatography to obtain **39F** (103 mg, yield: 81%). m/z (ESI): 553.1 $[M+H]^+$.

**Step 6: 4'-cyclopropyl-6'-methoxy-*N⁴*-(1-(4-(1-methyl-4-(trifluoromethyl)-1H-imidazol-2-yl)phenyl)cyclopropyl)-[2,5'-bipyrimidine]-4,5-diamine (39G)**

The compound **39F** (0.10 g, 0.18 mmol, 1.0 eq) was dissolved in methanol (5 mL) at room temperature, and then iron powder (50 mg, 0.90 mmol, 5.0 eq), ammonium chloride (100 mg, 1.80 mmol, 10.0 eq), and water were sequentially added. The resulting solution was allowed to react at 80 °C for 1 h, then the reaction solution was cooled to room temperature and filtered, and the filtrate was concentrated to obtain a crude intermediate **39G** (95 mg, yield: 99%). m/z (ESI): 523.1 $[M+H]^+$.

**Step 7: 2-chloro-*N*-(4'-cyclopropyl-6'-methoxy-4-((1-(4-(1-methyl-4-(trifluoromethyl)-1H-imidazol-2-yl)phenyl)cyclopropyl)amino)-[2,5'-bipyrimidin]-5-yl)acetamide (39H)**

In an ice bath, the compound **39G** (90 mg, 0.17 mmol, 1.0 eq) was dissolved in dichloromethane (3 mL), and then *N,N*-diisopropylethylamine (44 mg, 0.34 mol, 2.0 eq) and chloroacetyl chloride (19 mg, 0.17 mmol, 1.0 eq) were added. The resulting solution was allowed to react at room temperature for 30 min, then the reaction solution was concentrated, and the concentrate was purified by silica gel column chromatography to obtain **39H** (50 mg, yield: 49%). m/z (ESI): 599.1 $[M+H]^+$.

**Step 8: 2-(4-cyclopropyl-6-methoxypyrimidin-5-yl)-8-(1-(4-(1-methyl-4-(trifluoromethyl)-1H-imidazol-2-yl)phenyl)cyclopropyl)-7,8-dihydropteridin-6(5H)-one (391)**

In an ice bath, the compound **39H** (45 mg, 0.08 mmol, 1.0 eq) was dissolved in *N,N*-dimethylformamide (2 mL), and then sodium hydride (4.0 mg, 0.10 mmol, 1.1 eq) was slowly added. The resulting solution was allowed to react overnight at room temperature, quenched with water, and extracted with ethyl acetate. The organic phases were combined, then washed with saturated brine, dried over anhydrous sodium sulfate, filtered, and concentrated under vacuum to obtain a residue, and the residue was purified by silica gel column chromatography to obtain **39I** (32 mg, yield: 71%). m/z (ESI): 563.2 $[M+H]^+$.

**Step 9: 2-(4-cyclopropyl-6-methoxypyrimidin-5-yl)-5-methyl-8-(1-(4-(1-methyl-4-(trifluoromethyl)-1H-imidazol-2-yl)phenyl)cyclopropyl)-7,8-dihydropteridin-6(5H)-one (39)**

The compound **39I** (25 mg, 0.040 mmol, 1.0 eq) was dissolved in *N,N*-dimethylformamide at room temperature, then iodomethane (6.0 mg, 0.040 mmol, 1.0 eq) and potassium carbonate (11 mg, 0.080 mmol, 2.0 eq) were added for reaction at room temperature for 2 h, water was added, and the resulting solution was extracted with ethyl acetate. The organic phases were combined, then washed with saturated brine, dried over anhydrous sodium sulfate, filtered, and concentrated under vacuum to obtain a residue, and the residue was purified by silica gel column chromatography to obtain a compound **39** (13 mg, yield: 56%). m/z (ESI): 577.1 $[M+H]^+$.

**Example 37: Preparation of 2-(4-cyclopropyl-6-methoxypyrimidin-5-yl)-5-methyl-8-(4-(1-ethyl-4-(trifluoromethyl)-1H-imidazol-2-yl)benzyl)-7,8-dihydropteridin-6(5H)-one (compound 40)**

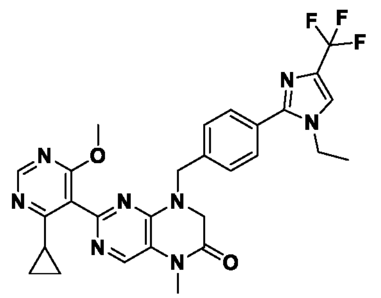

**40**

Iodoethane and sodium hydride were used instead of sodium difluorochloroacetate and potassium hydroxide in step **2** to prepare the compound **40** by using a method similar to that in Example **26.** LC-MS: m/z (ESI): 565.2 $[M+H]^+$.

$^1H$ NMR (400 MHz, DMSO-$d_6$): δ 8.61 (s, 1H), 8.14 (s, 1H), 8.02 (s, 1H), 7.59 (d, *J* = 8.3 Hz, 2H), 7.49 (d, *J* = 8.2 Hz, 2H), 4.84 (s, 2H), 4.23 (s, 2H), 4.07 (q, *J* = 7.3 Hz, 2H), 3.85 (s, 3H), 3.31 (s, 3H), 1.81-1.77 (m, 1H), 1.31 (t, *J* = 7.2 Hz, 3H), 1.02-0.98 (m, 2H), 0.86-0.82 (m, 2H).

**Example 38: Preparation of 2-(4-cyclopropyl-6-methoxypyrimidin-5-yl)-5-methyl-8-(4-(1-isopropyl-4-(trifluoromethyl)-1H-imidazol-2-yl)benzyl)-7,8-dihydropteridin-6(5H)-one (compound 41)**

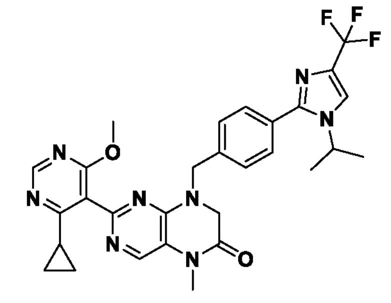

41

2-iodopropane and sodium hydride were used instead of sodium difluorochloroacetate and potassium hydroxide in step **2** to prepare the compound **41** by using a method similar to that in Example **26.** LC-MS: m/z (ESI): 579.2 $[M+H]^+$.

$^1$H NMR (400 MHz, DMSO-$d_6$): δ 8.61 (s, 1H), 8.16 (s, 1H), 8.14 (s, 1H), 7.53-7.48 (m, 4H), 4.83 (s, 2H), 4.43-4.38 (m" 1H), 4.24 (s, 2H), 3.84 (s, 3H), 3.30 (s, 3H), 1.81-1.76 (m, 1H), 1.33 (d, *J* = 8.0 Hz, 6H), 1.02-0.98 (m, 2H), 0.86-0.82 (m, 2H).

**Example 39: Preparation of 2-(4-cyclopropyl-6-methoxypyrimidin-5-yl)-10-(4-(1-methyl-4-(trifluoromethyl)-1H-imidazol-2-yl)benzyl)-8,9,9a,10-tetrahydropyrimido[4,5-d]pyrrolo[1,2-a]pyrimidin-5(7H)-one (compound 42)**

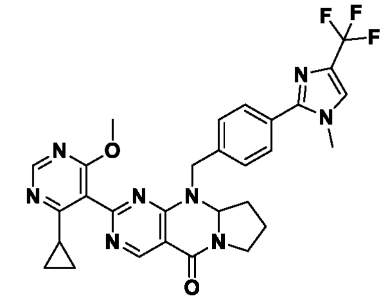

42

The synthesis route is as follows:

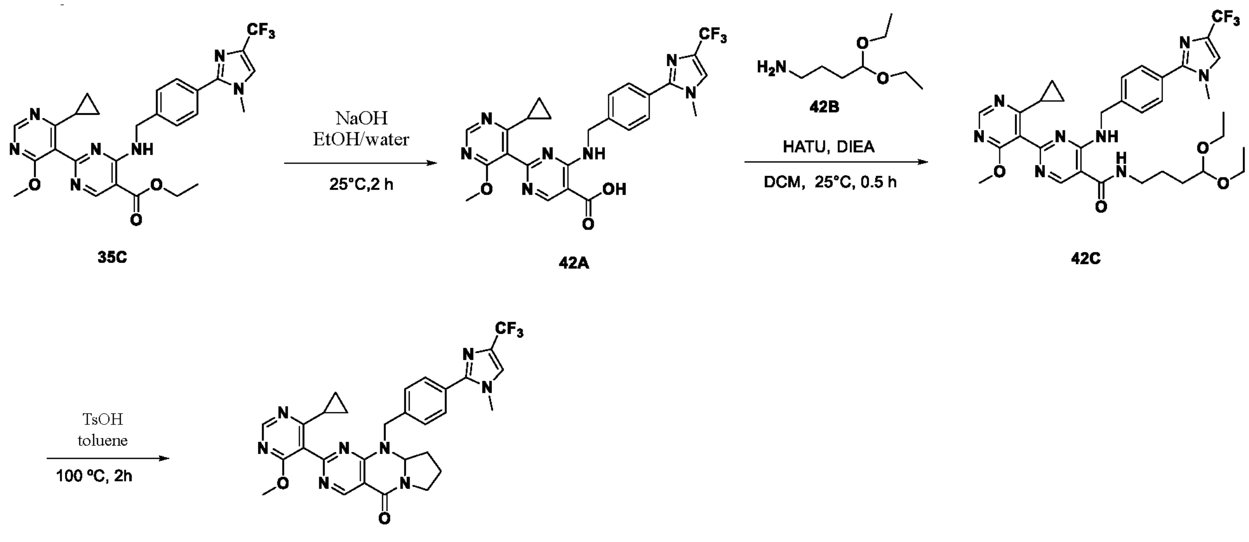


35C

42A

42C

42

**Step 1: 4'-cyclopropyl-6'-methoxy-4-((4-(1-methyl-4-(trifluoromethyl)-1H-imidazol-2-yl)benzyl)amino)-[2,5'-bipyrimidine]-5-carboxylic acid (42A)**

The compound **35C** (0.55 g, 1 mmol, 1.0 eq) was dissolved in ethanol (5 mL) at room temperature, and then 2 M aqueous sodium hydroxide solution (3 mL) was added. The resulting solution was allowed to react under a nitrogen atmosphere at 25 °C for 2 h, then 0.5 M hydrochloric acid solution (15 mL) was added for acidification, and the resulting solution was extracted with ethyl acetate (20 mL × 3). The resulting organic phases were combined, then washed with saturated brine, dried over anhydrous sodium sulfate, filtered, and concentrated under vacuum to obtain a crude intermediate **42A** (0.53 g, yield: 100%). m/z (ESI): 526.3 $[M+H]^+$.

**Step 2: 4'-cyclopropyl-N (4,4-diethoxybutyl)-6'-methoxy-4-((4-(1-methyl-4-(trifluoromethyl)-1H-imidazol-2-yl)benzyl)amino)-[2,5'-bipyrimidine]-5-carboxamide (42C)**

The compound **42A** (52 mg, 0.10 mmol, 1.0 eq), a compound **42B** (20 mg, 0.13 mmol, 1.3 eq), and 2-(7-azabenzotriazoly)-*N*,*N*,*N'*,*N'*-tetramethyluronium hexafluorophosphate (50 mg, 0.13 mmol, 1.3 eq) were mixed in dichloromethane (5 mL) at room temperature, and then *N,N*-diisopropylethylamine (0.33 g, 0.26 mmol, 2.6 eq) was added. The resulting mixture was allowed to react under a nitrogen atmosphere at 25 °C for 0.5 h. The reaction solution was concentrated, and the concentrate was purified by reversed-phase C18 silica gel column chromatography (with 5-65% aqueous acetonitrile solution as the elution phase) to obtain a product **42C** (42 mg, yield: 63%). m/z (ESI): 669.4 $[M+H]^+$.

**Step 3: 2-(4-cyclopropyl-6-methoxypyrimidin-5-yl)-10-(4-(1-methyl-4-(trifluoromethyl)-1H-imidazol-2-yl)benzyl)-8,9,9a,10-tetrahydropyrimido[4,5-d]pyrrolo[1,2-a]pyrimidin-5(7H)-one (42)**

The compound **42C** (42 mg, 0.63 mmol, 1.0 eq) was dissolved in toluene (5 mL) at room temperature, and then *p*-toluenesulfonic acid monohydrate (19 mg, 0.10 mmol, 1.6 eq) was added. After stirring at 100 °C for reaction for 2 h, the reaction solution was concentrated. The resulting crude mixture was purified by reversed-phase C18 silica gel column chromatography (with 5-65% aqueous acetonitrile solution as the elution phase) to obtain a compound **42** (25 mg, yield: 69%). m/z (ESI): 577.3 $[M+H]^+$. $^1H$ NMR (400 MHz, DMSO-$d_6$) δ 8.76 (s, 1H), 8.63 (s, 1H), 7.94 (d, *J* = 1.4 Hz, 1H), 7.70-7.63 (m, 2H), 7.46 (d, *J* = 8.2 Hz, 2H), 5.25 (dd, *J* = 9.1, 4.7 Hz, 1H), 5.04 (d, *J* = 16.3 Hz, 1H), 4.83 (d, *J* = 16.3 Hz, 1H), 3.85 (s, 3H), 3.76 (s, 3H), 3.61-3.49 (m, 1H), 2.42 (dt, *J* = 11.4, 5.3 Hz, 1H), 2.01-1.94 (m, 2H), 1.88-1.72 (m, 1H), 1.02-0.95 (m, 2H), 0.83 (dd, *J* = 8.1, 3.2 Hz, 2H).

**Example 40: Preparation of 2-(4-cyclopropyl-6-methoxypyrimidin-5-yl)-5-methyl-8-(4-(5-methyl-3-(trifluoromethyl)-1H-pyrazol-1-yl)benzyl)-7,8-dihydropteridin-6(SH)-one (compound 43)**

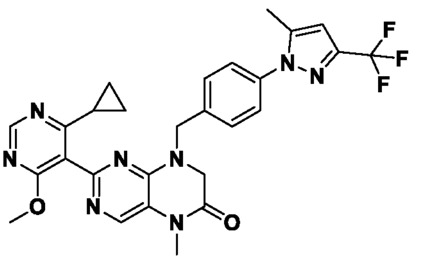

**43**

The synthesis route is as follows:

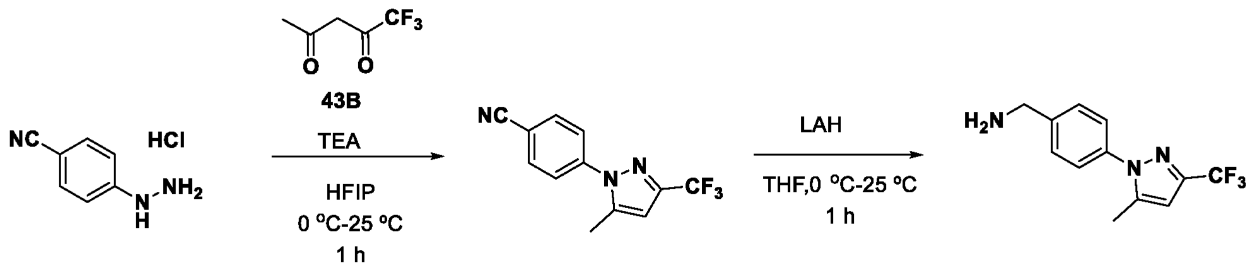


43A 43C 43D

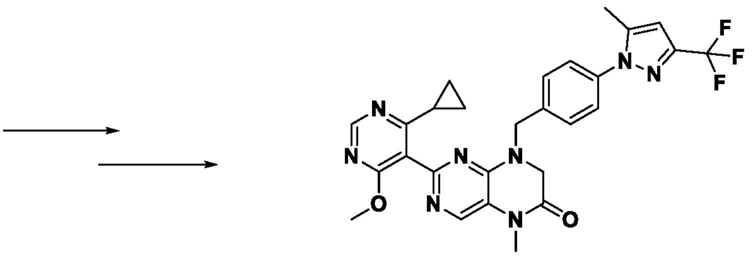

43

**Step 1: 4-(5-methyl-3-(trifluoromethyl)-1H-pyrazol-1-yl)benzonitrile (43C)**

To hexafluoroisopropanol (5 mL), 4-hydrazinobenzonitrile hydrochloride (1.0 g, 5.9 mmol) and 1,1,1-trifluoropentane-2,4-dione (0.91 g, 5.9 mmol, 1.0 eq) were added, and triethylamine (1.2 g, 11.8 mmol, 1.6 mL, 2.0 eq) was added slowly at 0 °C. The resulting solution was allowed to react at room temperature for 1 h, then the reaction solution was added to water (20 mL), and the resulting mixture was extracted with dichloromethane (20 mL × 3). The organic phases were combined, dried over anhydrous sodium sulfate, filtered, and distilled under vacuum to obtain a crude product. The crude product was purified by silica gel column chromatography (with 5% ethyl acetate in petroleum ether as the elution phase) to obtain a white solid **43C** (1.0 g, 4.0 mmol, yield: 68%). m/z (ESI): 252.5 $[M+H]^+$.

**Step 2: (4-(5-methyl-3-(trifluoromethyl)-1H-pyrazol-1-yl)phenyl)methylamine (43D)**

The compound **43C** (1.0 g, 4.0 mmol) was added to tetrahydrofuran (20 mL) at 0 °C, and lithium aluminum hydride (0.30 g, 8.0 mmol, 2 eq) was added slowly in portions under stirring. The reaction solution was slowly restored to room temperature and stirred for reaction for 1 h, then 0.3 mL of water, 0.3 mL of 15% aqueous sodium hydroxide solution, and 0.9 mL of water were added slowly to quench the reaction, and the resulting solution was stirred at room temperature for another 1 h. The reaction solution was filtered and distilled under vacuum to obtain a pale yellow oily substance **43D** (0.85 g, 3.3 mmol, yield: 84%).

In subsequent steps, the compound **43D** was used instead of the compound **28D** in step 4 to prepare the compound **43** by using a method similar to that in Example **26.**

m/z (ESI): 551.5 $[M+H]^+$.

$^1$H NMR (400 MHz, DMSO-$d_6$): δ 8.61 (s, 1H), 8.16 (s, 1H), 8.14 (s, 1H), 7.53-7.48 (m, 4H), 4.83 (s, 2H), 4.43-4.38 (m" 1H), 4.24 (s, 2H), 3.84 (s, 3H), 3.30 (s, 3H), 1.81-1.76 (m, 1H), 1.33 (d, *J* = 8.0 Hz, 6H), 1.02-0.98 (m, 2H), 0.86-0.82 (m, 2H).

**Example 41: Preparation of 2-(4-cyclopropyl-6-methoxypyrimidin-5-yl)-5-methyl-8-(3-fluoro-4-(5-methyl-3-(trifluoromethyl)-1H-pyrazol-1-yl)benzyl)-7,8-dihydropteridin-6(SH)-one (compound 44)**

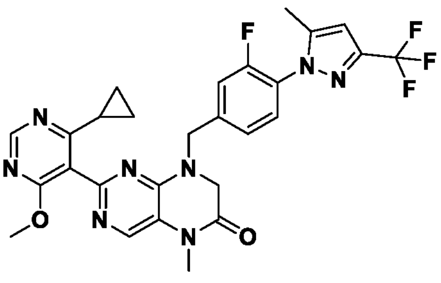

**44**

3-fluoro-4-hydrazinobenzonitrile was used instead of 4-hydrazinobenzonitrile hydrochloride in step 1 to prepare the compound **44** by using a method similar to that in Example **40.**

m/z (ESI): 569.2 $[M+H]^+$.

$^1H$ NMR (400 MHz, DMSO-$d_6$): δ 8.61 (s, 1H), 8.15 (s, 1H), 7.61 (t, *J* = 8.0 Hz ,1H) , 7.51 (dd, *J* = 4.0 Hz, 8.0 Hz ,1H), 7.38 (dd, *J* =4.0 Hz,8.0 Hz ,1H), 6.78 (s, 1H), 4.85 (s, 2H), 4.28(s, 2H), 3.86 (s, 3H), 3.83 (s, 3H), 2.19(s,3H), 1.02-0.98 (m, 1H), 1.01-0.98 (m, 2H), 0.85-0.81 (m, 2H).

**Example 42: Preparation of 2-(4-cyclopropyl-6-methoxypyrimidin-5-yl)-5-methyl-8-((2-(trifluoromethyl)-6,7-dihydro-5H-benzo[c]imidazo[1,2-a]azepin-9-yl)methyl)-7,8-dihydropteridin-6(5H)-one (compound 45)**

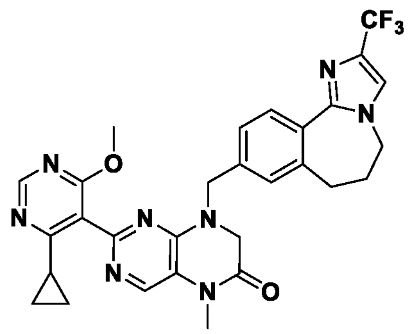

**45**

The synthesis route is as follows:

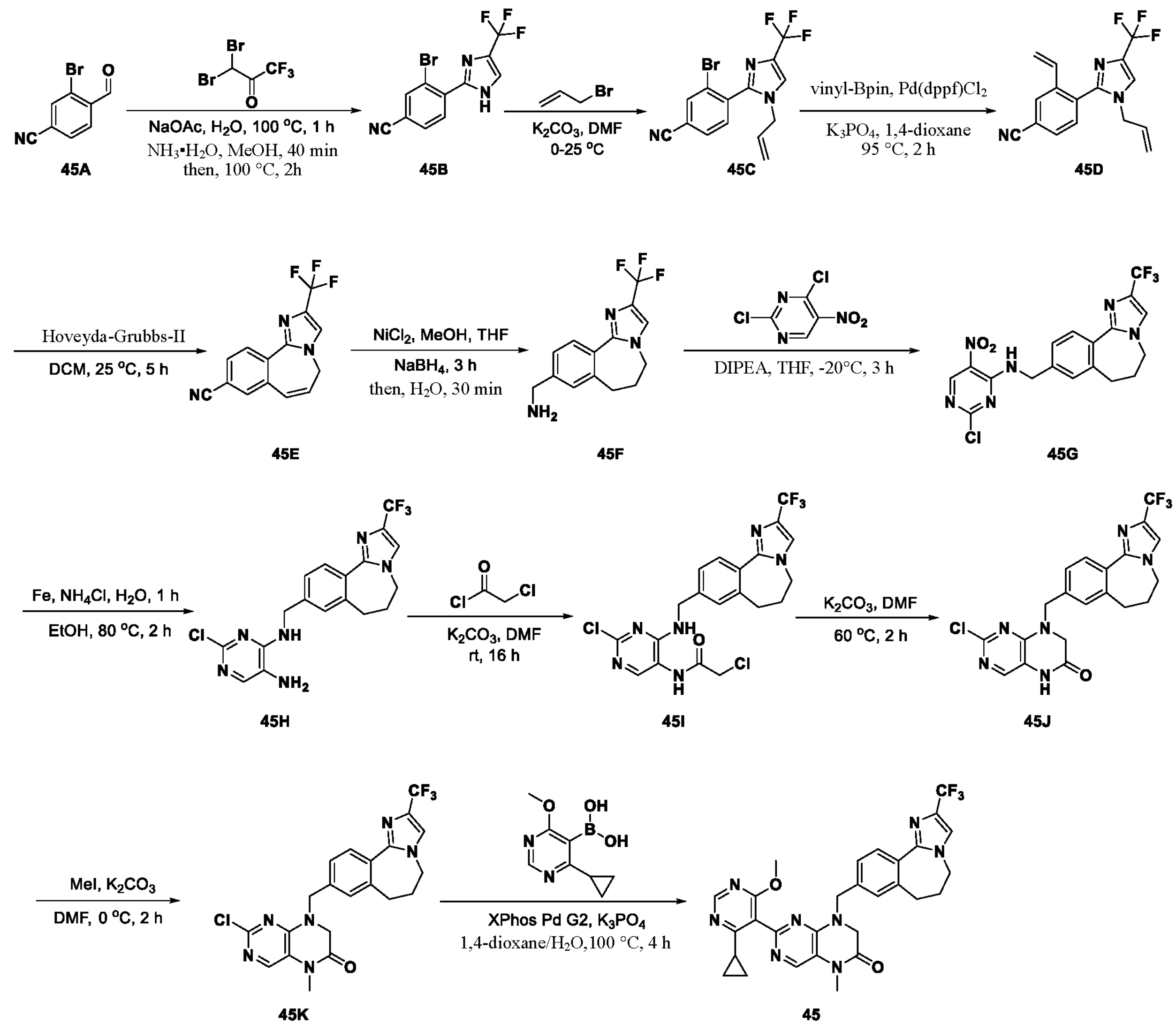


**Step 1: 3-bromo-4-(4-(trifluoromethyl)-1H-imidazol-2-yl)benzonitrile (45B)**

1,1-dibromo-3,3,3-trifluoroacetone (2.6 g, 9.5 mmol, 1.3 mL) and sodium acetate (0.78 g, 9.5 mmol) were dissolved in water (3.0 mL), and the mixture was stirred at 100 °C for 1 h and then cooled to room temperature. Then 2-bromo-4-cyanobenzaldehyde (1.0 g, 4.8 mmol) was dissolved in methanol (32 mL) and aqueous ammonia (7.0 mL), and the mixture was added slowly to the reaction solution described above. The resulting solution was then stirred at room temperature for 40 min, and heated to 100 °C for reflux reaction for 2 h. The mixture was then cooled to room temperature, and concentrated under vacuum to remove most of methanol, and the precipitated solid was filtered, washed three times with water, and dried to obtain a crude **intermediate 45B** (1.7 g), which was used directly in the next step. MS m/z (ESI): 315.9 $[M+H]^+$.

**Step 2: 4-(1-allyl-4-(trifluoromethyl)-1H-imidazol-2-yl)-3-bromobenzonitrile (45C)**

Allyl bromide (0.14 g, 1.1 mmol, 98 μL) was slowly added to a solution of compound **45B** (0.36 g, 1.1 mmol) and potassium carbonate (0.31 g, 2.3 mmol) in *N,N*-dimethylformamide (0.91 mL), and the resulting solution was allowed to react overnight at room temperature. After the reaction, the mixture was poured into water, and then extracted three times with ethyl acetate (10 mL × 3). The organic phases were combined and concentrated under vacuum, and the concentrate was isolated by column chromatography to obtain an **intermediate 45C** (0.30 g, 0.84 mmol, yield: 74%).

MS m/z (ESI): 355.9 $[M+H-Boc]^+$;
$^1$H NMR (400 MHz, DMSO-$d_6$) δ 8.41 (d, *J* = 1.6 Hz, 1H), 8.04 (d, *J* = 1.3 Hz, 1H), 8.02 (dd, *J* = 7.9, 1.6 Hz, 1H), 7.73 (d, *J* = 7.9 Hz, 1H), 5.85 (ddt, *J* = 17.1, 10.3, 5.6 Hz, 1H), 5.13 (dq, *J* = 10.3, 1.4 Hz, 1H), 4.91 (dq, *J* = 17.2,

1.5 Hz, 1H), 4.49 (dt, *J* = 5.6, 1.6 Hz, 2H).

**Step 3: 4-(1-allyl-4-(trifluoromethyl)-1H-imidazol-2-yl)-3-vinylbenzonitrile (45D)**

The **intermediate 45C** (0.80 g, 2.3 mmol), vinylboronic acid pinacol cyclic ester (0.42 g, 2.7 mmol, 0.46 mL), Pd(dppf)$Cl_2$ (0.33 g, 0.45 mmol), and potassium phosphate (1.4 g, 6.7 mmol) were dissolved in dioxane (5.0 mL), and the mixture was allowed to react under the protection of nitrogen at 95 °C for 2 h under microwave conditions. The reaction solution was then poured into a mixed system of ethyl acetate and water, and the resulting solution was extracted three times with ethyl acetate (30 mL × 3). The organic phases were combined and spin-dried, and the resulting residue was purified by column chromatography to obtain a product **45D** (0.30 g, 0.99 mmol, yield: 44%).

$^1$H NMR (400 MHz, $CDCl_3$) δ 7.94 (d, *J* = 1.5 Hz, 1H), 7.63 (dd, *J* = 7.8, 1.6 Hz, 1H), 7.51 (d, *J* = 8.0 Hz, 1H), 7.44-7.37 (m, 1H), 6.51 (dd, *J* = 17.5, 11.0 Hz, 1H), 5.85-5.71 (m, 2H), 5.44 (d, *J* = 11.0 Hz, 1H), 5.27 (d, *J* = 10.2 Hz, 1H), 5.08 (d, *J* = 17.0 Hz, 1H), 4.32 (d, *J* = 5.7 Hz, 2H).

**Step 4: 2-(trifluoromethyl)-5H-benzo[c]imidazo[1,2-a]azepine-9-carbonitrile (45E)**

The second-generation Hoveyda-Grubbs catalyst (CAS NO.: 301224-40-8, 43 mg, 69 μmol) was added to a solution of **intermediate 45D** (0.21 g, 0.69 mmol) in dichloromethane (5.0 mL). The mixture was stirred at room temperature for 5 h, then a mixed solution of dichloromethane and water was added to the mixture, and the resulting mixture was extracted three times with dichloromethane (10 mL × 3). The organic phases were combined and concentrated, and the concentrate was isolated by column chromatography to obtain a product, **intermediate 45E** (0.15 g, 0.55 mmol, yield: 79%). MS m/z (ESI): 276.1 $[M+H]^+$.

**Step 5: (2-(trifluoromethyl)-6,7-dihydro-5H-benzo[c]imidazo[1,2-a]azepin-9-yl)methylamine (45F)**

Nickel dichloride (23 mg, 95 μmol) was added to a solution of intermediate **45E** (0.13 g, 0.47 mmol) in methanol (5.0 mL) and tetrahydrofuran (5.0 mL) at 0 °C. Then sodium borohydride (72 mg, 1.9 mmol) was added to the reaction solution in portions for reaction at room temperature for 3 h. To the reaction solution, 1.0 mL of water was added, and the resulting solution was stirred for half an hour to quench excessive sodium borohydride. The mixture was spin-dried and dissolved in tetrahydrofuran, then the resulting solution was filtered, and the filtrate was spin-dried to obtain a crude intermediate **45F** (0.13 g, 0.46 mmol, yield: 98%), which was used directly in the next step.

**Step 6: 2-chloro-5-nitro-*N*-((2-(trifluoromethyl)-6,7-dihydro-5H-benzo[c]imidazo[1,2-a]azepin-9-yl)methyl)pyrimidin-4-amine (45G)**

Diisopropylethylamine (0.14 g, 1.1 mmol, 0.18 mL) was added to a solution of 2,4-dichloro-5-nitropyrimidine (81 mg, 0.42 mmol) in tetrahydrofuran (5.0 mL) at -20 °C, then a solution of **intermediate 45F** (0.12 g, 0.42 mmol) in tetrahydrofuran (2.0 mL) was added slowly dropwise, and after the addition, the resulting solution was allowed to react at -20 °C for 3 h. The mixture was poured into a mixed solvent of ethyl acetate/water, then the resulting solution was extracted three times with ethyl acetate (10 mL × 3), and the organic phases were combined, spin-dried and purified by column chromatography to obtain an **intermediate 45G** (0.13 g, 0.30 mmol, yield: 71%).

$^1$H NMR (400 MHz, $CDCl_3$) δ 9.09 (s, 1H), 7.84 (d, *J* = 7.9 Hz, 1H), 7.37 (d, *J* = 1.5 Hz, 2H), 4.88 (d, *J* = 5.8 Hz, 2H), 3.97 (t, *J* = 6.9 Hz, 2H), 2.78 (t, *J* = 7.1 Hz, 2H), 2.38 (m, 2H).

**Step 7: 2-chloro-$N^4$-((2-(trifluoromethyl)-6,7-dihydro-5H-benzo[c]imidazo[1,2-a]azepin-9-yl)methyl)pyrimidine-4,5-diamine (45H)**

Reduced iron powder (51 mg, 0.91 mmol) and ammonium chloride (49 mg, 0.91 mmol) were dissolved in water (2.0 mL), and the resulting solution was allowed to react at 100 °C for 1 h. Then, a solution of **intermediate 45G** (80 mg, 0.18 mmol) in ethanol (2.0 mL) was added for reaction at 80 °C for 2 h, the reaction solution was diluted with ethyl acetate and filtered while hot, the filter cake was rinsed with ethyl acetate, then the organic phases were combined and spin-dried, and the resulting residue was purified by column chromatography to obtain an **intermediate 45H** (55 mg, 0.13 mmol, yield: 74%).

MS m/z (ESI): 409.1 $[M+H]^+$.

**Step 8: 2-chloro-*N*-(2-chloro-4-(((2-(trifluoromethyl)-6,7-dihydro-5H-benzo[c]imidazo[1,2-a]azepin-9-yl)methyl)amino)pyrimidin-5-yl)acetamide (45I)**

Chloroacetyl chloride (55 mg, 0.49 mmol), the **intermediate 45H** (0.20 g, 0.49 mmol), and potassium carbonate (0.20 mg, 1.5 mmol, 0.27 mL) were dissolved in anhydrous *N,N*-dimethylformamide (5.0 mL) at room temperature for reaction for 16 h, then a mixed solvent of water and ethyl acetate was added to the reaction system, and the resulting solution was extracted three times with ethyl acetate (10 mL × 3). The organic phases were combined and spin-dried, and the resulting residue was purified by column chromatography to obtain an **intermediate 45I** (0.18 g, 0.36 mmol, yield: 74%).

**Step 9: 2-chloro-8-((2-(trifluoromethyl)-6,7-dihydro-5H-benzo[c]imidazo[1,2-a]azepin-9-yl)methyl)-7,8-dihydropteridin-6(5H)-one (45J)**

Potassium carbonate (0.17 g, 1.2 mmol) and the **intermediate 45I** (0.20 g, 0.41 mmol) were dissolved in anhydrous *N,N*-dimethylformamide (20 mL) at room temperature. The resulting solution was heated to 60 °C for reaction for 2 h, then water and ethyl acetate were added to the reaction solution, and the mixture was extracted three times with ethyl acetate (60 mL × 3). The organic phases were combined and spin-dried, and the resulting residue was purified by column chromatography to obtain an **intermediate 45J** (0.14 g, 0.30 mmol, yield: 73%).

**Step 10: 2-chloro-5-methyl-8-((2-(trifluoromethyl)-6,7-dihydro-5H-benzo[c]imidazo[1,2-a]azepin-9-yl)methyl)-7,8-dihydropteridin-6(5H)-one (45K)**

A solution of iodomethane (47 mg, 0.33 mmol) in *N,N*-dimethylformamide (0.5 mL) was added dropwise to a solution of **intermediate 45J** (0.14 g, 0.30 mmol) and potassium carbonate (0.12 g, 0.90 mmol) in *N,N*-dimethylformamide (1.0 mL). The mixture was allowed to react at 0 °C for 2 h. After the reaction, the mixture was poured into ice water, a solid was precipitated and filtered, and the filter cake was washed with water and dried to obtain an **intermediate 45K** (0.13 g, 0.28 mmol, yield: 93%).

**Step 11: 2-(4-cyclopropyl-6-methoxypyrimidin-5-yl)-5-methyl-8-((2-(trifluoromethyl)-6,7-dihydro-5H-benzo [c] imidazo [1,2-a]azapine-9-yl)methyl)-7,8-dihydropteridin-6(5H)-one (45)**

The **intermediate 45K** (42 mg, 0.09 mmol), (4-cyclopropyl-6-methoxypyrimidin-5-yl)boronic acid (35 mg, 0.18 mmol), XPhos Pd G2 (14 mg, 0.02 mmol), and potassium phosphate (58 mg, 0.27 mmol) were added to a mixed solution of water (0.01 mL) and dioxane (1.0 mL), then the resulting solution was allowed to react at 100 °C for 4 h under the protection of nitrogen, and a crude product from concentration under vacuum was purified by preparative chromatography (Waters Xbridge C18, 5-95% acetonitrile/water mobile phase) to obtain a compound **45** (18 mg, 0.03 mmol, yield: 34%).

MS m/z (ESI): 577.1 $[M+H]^+$;
$^1$H NMR (400 MHz, DMSO-$d_6$) δ 8.61 (s, 1H), 8.14 (s, 1H), 7.97 (d, *J* = 1.4 Hz, 1H), 7.63 (d, *J* = 7.9 Hz, 1H), 7.36-7.30 (m, 2H), 4.81 (s, 2H), 4.23 (s, 2H), 3.98 (t, *J* = 6.7 Hz, 2H), 3.84 (s, 3H), 3.31 (s, 3H), 2.64 (t, *J* = 7.0 Hz, 2H), 2.24 (t, *J* = 6.9 Hz, 2H), 1.82-1.74 (m, 1H), 1.02-0.96 (m, 2H), 0.85-0.77 (m, 2H).

**Example 43: Preparation of 2-(4-cyclopropyl-6-methoxypyrimidin-5-yl)-5-ethyl-8-((2-(trifluoromethyl)-6,7-dihydro-5H-benzo[c]imidazo[1,2-a]azepin-9-yl)methyl)-7,8-dihydropteridin-6(5H)-one (compound 46)**

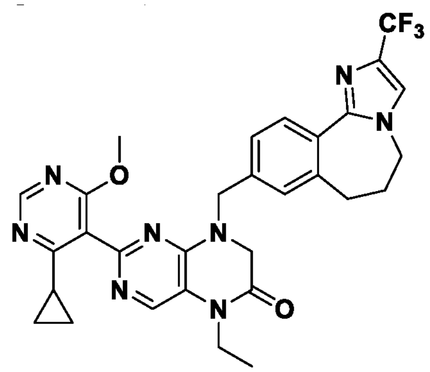

**46**

Iodoethane was used instead of iodomethane to prepare the compound **46** by using a synthesis route similar to that for the compound **45.**

MS m/z (ESI): 591.2 $[M+H]^+$;

$^1$H NMR (400 MHz, DMSO-$d_6$) δ 8.62 (s, 1H), 8.20 (s, 1H), 7.98 (d, *J* = 1.3 Hz, 1H), 7.63 (d, *J* = 8.2 Hz, 1H), 7.37-7.32 (m, 2H), 4.80 (s, 2H), 4.22 (s, 2H), 4.05-3.89 (m, 4H), 3.85 (s, 3H), 2.64 (t, *J* = 7.0 Hz, 2H), 2.29-2.18 (m, 2H), 1.85-1.73 (m, 1H), 1.19 (t, *J* = 7.0 Hz, 3H), 1.01-0.93 (m, 2H), 0.87-0.77 (m, 2H).

**Example 44: Preparation of 2-(4-cyclopropyl-6-methoxypyrimidin-5-yl)-5-isopropyl-8-((2-(trifluoromethyl)-6,7-dihydro-5H-benzo[c]imidazo[1,2-a]azepin-9-yl)methyl)-7,8-dihydropteridin-6(5H)-one (compound 47)**

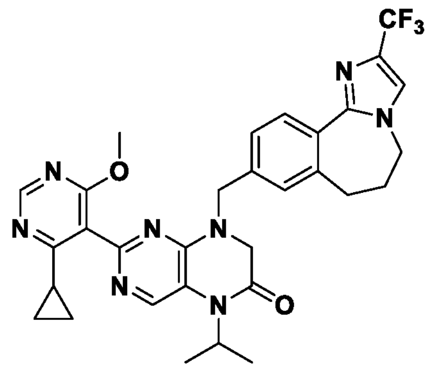

**47**

2-iodopropane was used instead of iodomethane to prepare the compound **47** by using a synthesis route similar to that for the compound **45.**

MS m/z (ESI): 605.3 $[M+H]^+$.

$^1$H NMR (400 MHz, DMSO-$d_6$) δ 8.62 (s, 1H), 8.35 (s, 1H), 7.98 (s, 1H), 7.63 (d, *J* = 7.7 Hz, 1H), 7.33 (d, *J* = 8.2 Hz, 2H), 4.79 (s, 2H), 4.12 (s, 2H), 3.98 (t, *J* = 6.7 Hz, 2H), 3.85 (s, 3H), 3.30 (s, 1H), 2.67 (s, 2H), 2.24 (t, *J* = 6.8 Hz, 2H), 1.80 (d, *J* = 8.0 Hz, 1H), 1.48 (d, *J* = 6.9 Hz, 6H), 1.00 (m, 2H), 0.83 (m, 2H).

**Example 45: Preparation of 5-cyclopropyl-2-(4-cyclopropyl-6-methoxypyrimidin-5-yl)-8-((2-(trifluoromethyl)-6,7-dihydro-5H-benzo[c]imidazo[1,2-a]azepin-9-yl)methyl)-7,8-dihydropteridin-6-(5H)-one (compound 48)**

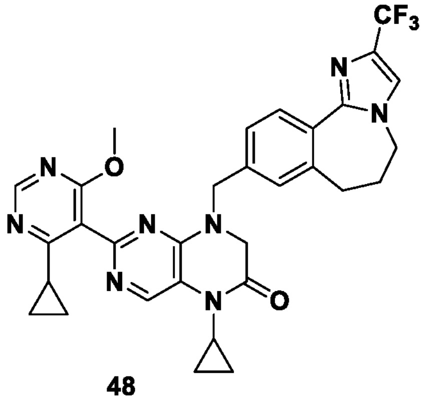

48

The synthesis route is as follows:

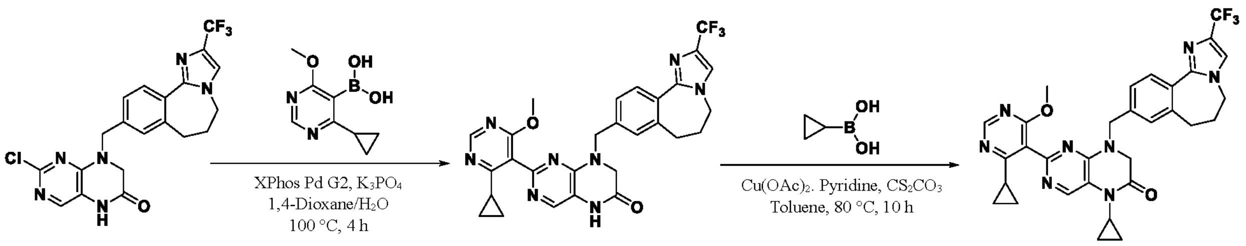


45J 48A 48

**Step 1: 2-(4-cyclopropyl-6-methoxypyrimidin-5-yl)-8-((2-(trifluoromethyl)-6,7-dihydro-5H-benzo [c] imidazo [1,2-a]azepin-9-yl)methyl)-7,8-dihydropteridin-6(5H)-one (48A)**

The **intermediate 45J** (50 mg, 0.11 mmol), (4-cyclopropyl-6-methoxypyrimidin-5-yl)boronic acid (32 mg,0.17 mmol), XPhos Pd G2 (18 mg, 0.022 mmol), and potassium phosphate (71 mg, 0.33 mmol) were added to a mixed solution of water (0.02 mL) and dioxane (2 mL), and the resulting solution was allowed to react at 100 °C for 4 h under the protection of nitrogen. The reaction solution was distilled under vacuum, and the resulting crude product was purified by preparative chromatography (Waters Xbridge C18, 10-90% aqueous acetonitrile solution) to obtain a compound **48A** (20 mg, 0.036 mmol, yield: 32%).

**Step 2: 5-cyclopropyl-2-(4-cyclopropyl-6-methoxypyrimidin-5-yl)-8-((2-(trifluoromethyl)-6,7-dihydro-5H-benzo[c] imidazo [1,2-a]azepin-9-yl)methyl)-7,8-dihydropteridin-6(5H)-one (48)**

The **intermediate 48A** (20 mg, 0.036 mmol), cyclopropylboronic acid (6.1 mg, 0.071 mmol), pyridine (2.8 mg, 0.036 mmol), copper acetate (6.5 mg, 0.036 mmol), and cesium carbonate (5.8 mg, 0.018mmol) were added to toluene (5.0 mL) for reaction at 80 °C for 10 h. The reaction solution was distilled under vacuum, and the resulting crude product was purified by preparative chromatography (Waters Xbridge C18, 10-90% aqueous acetonitrile solution) to obtain a compound **48** (8.0 mg, yield: 37%).

MS m/z (ESI): 603.1 $[M+H]^+$;
$^1$H NMR (400 MHz, DMSO-$d_6$) δ 8.62 (s, 1H), 8.38 (s, 1H), 7.98 (d, *J* = 1.4 Hz, 1H), 7.63 (d, *J* = 7.8 Hz, 1H), 7.31-7.37 (m, 2H), 4.78 (s, 2H), 4.14 (s, 2H), 3.98 (t, *J* = 6.8 Hz, 2H), 3.85 (s, 3H), 2.74-2.69 (m, 1H), 2.64 (t, *J* = 7.1 Hz, 2H), 2.20-2.28 (m, 2H), 1.84-1.76 (m, 1H), 1.17-1.12 (m, 2H), 1.03-0.98 (m, 2H), 0.86-0.80 (m, 2H), 0.78-0.73 (m, 2H).

**Example 46: Preparation of 7-(4-cyclopropyl-6-methoxypyrimidin-5-yl)-4,4-dimethyl-1-(3-fluoro-4-(1-methyl-4-(trifluoromethyl)-1H-imidazol-2-yl)benzyl)-1,4-dihydro-2H-pyrimido[4,5-d][1,3]oxazin-2-one (compound 49)**

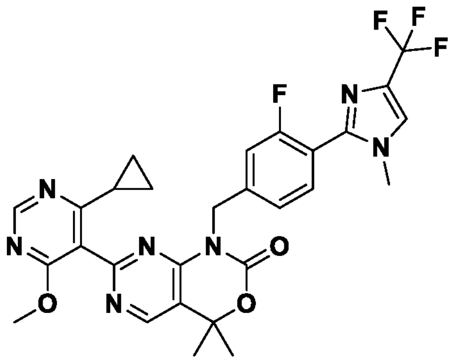

49

The synthesis route is as follows:

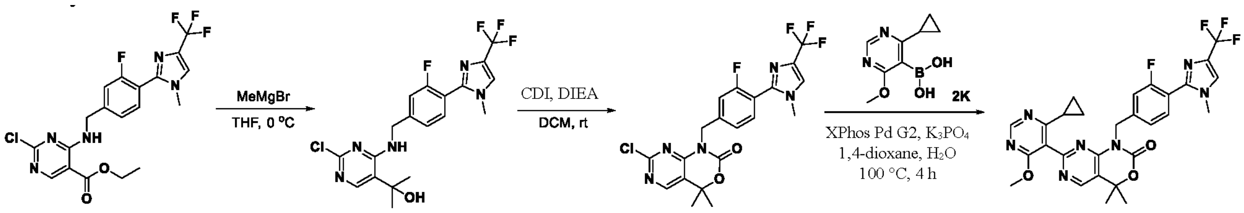


49A 49B 49C 49

**Step 1: 2-(2-chloro-4-((3-fluoro-4-(1-methyl-4-(trifluoromethyl)-1H-imidazol-2-yl)benzyl)amino)pyrimidin-5-yl)propan-2-ol (49B)**

3-fluoro-4-(1-methyl-4-(trifluoromethyl)-1H-imidazol-2-yl)benzylamine was used instead of the compound **4B** to prepare an **intermediate 49A** by using synthesis steps similar to those for the compound **35B.**

The **intermediate 49A** (0.40 g, 0.87 mmol) was dissolved in tetrahydrofuran (10 mL). Then methyl magnesium bromide (3.0 M, 1.75 mL) was added in portions in an ice bath for reaction overnight in the ice bath until the reaction was monitored to be complete by LC-MS. Water (10 mL) was added to quench the reaction, then the resulting solution was extracted with ethyl acetate (10 mL × 3), the organic phases were combined, dried over anhydrous sodium sulfate, and filtered, and the resulting residue was purified by column chromatography to obtain an **intermediate 49B** (0.29 g, 0.66 mmol, yield: 75%).

LC-MS: m/z (ESI): 444.1 $[M+H]^+$;

$^1H$ NMR (400 MHz, DMSO-$d_6$) δ 8.29 (m, 1H), 8.02-7.99 (m, 1H), 7.93 (s, 1H), 7.56 (m, 1H), 7.37-7.30 (m, 2H), 5.87 (s, 1H), 4.73 (m, 2H), 3.60 (s, 3H), 1.52 (s, 6H).

**Step 2: 7-chloro-1-(3-fluoro-4-(1-methyl-4-(trifluoromethyl)-1H-imidazol-2-yl)benzyl)-4,4-dimethyl-1,4-dihydro-2H-pyrimido[4,5-d][1,3]oxazin-2-one (49C)**

Carbonyldiimidazole (0.18 g, 1.2 mmol) was dissolved in dichloromethane (2.0 mL), then diisopropylethylamine (0.16 g, 1.2 mmol, 0.21 mL) and the **intermediate 49B** (0.18 g, 40.41 mmol) were added for reaction overnight at room temperature, then an ammonium chloride solution (10 mL) was added to quench the reaction, and the resulting solution was extracted three times with dichloromethane (10 mL × 3). The resulting residue was purified by column chromatography to obtain an **intermediate 49C** (0.19 g, 0.40 mmol, yield: 99%).

LC-MS: m/z (ESI): 470.0 $[M+H]^+$;

$^1H$ NMR (400 MHz, DMSO-$d_6$) δ 8.62 (s, 1H), 8.02-7.98 (m, 1H), 7.56 (m, 1H), 7.42-7.36 (m, 1H), 7.30 (m, 1H), 5.20 (s, 2H), 3.60 (s, 3H), 1.73 (s, 6H).

**Step 3: 7-(4-cyclopropyl-6-methoxypyrimidin-5-yl)-4,4-dimethyl-1-(3-fluoro-4-(1-methyl-4-(trifluoromethyl)-1H-imidazol-2-yl)benzyl)-1,4-dihydro-2H-pyrimido [4,5-d] [1,3] oxazin-2-one (49)**

The **intermediate 49C** (41 mg, 0.21 mmol), (4-cyclopropyl-6-methoxypyrimidin-5-yl)boronic acid (50 mg, 0.11

mmol), XPhos Pd G2 (17 mg, 0.021 mmol), and potassium phosphate (68 mg, 0.32 mmol) were added to a mixed solution of water (0.1 mL) and dioxane (2.0 mL), then the resulting solution was allowed to react at 100 °C for 4 h under the protection of nitrogen, and a crude product from concentration was purified by preparative chromatography (Waters Xbridge C18, 5-95% acetonitrile/water mobile phase) to obtain a compound **49** (12 mg, 0.021 mmol, yield: 19%).

LC-MS: m/z (ESI): 584.1 $[M+H]^+$;
$^1$H NMR (400 MHz, DMSO-$d_6$) δ 8.84 (s, 1H), 8.66 (s, 1H), 8.01-7.98 (m, 1H), 7.54 m, 1H), 7.34 (m, 1H), 7.27 (m, 1H), 5.23 (s, 2H), 3.82 (s, 3H), 3.59-3.57 (m, 3H), 1.79 (s, 6H), 1.74 (m, 1H), 1.00 (m, 2H), 0.81 (m, 2H).

**Example 47: Preparation of 7-(4-cyclopropyl-6-methoxypyrimidin-5-yl)-4,4-dimethyl-1-(4-(1-methyl-4-(trifluoromethyl)-1H-imidazol-2-yl)benzyl)-1,4-dihydro-2H-pyrimido[4,5-d][1,3]oxazin-2-one (compound 50)**

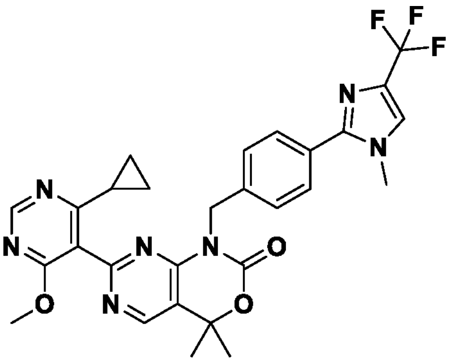

**50**

The compound **35B** was used instead of the compound **49A** to prepare the compound **50** by using synthesis steps similar to those for the compound **49.**

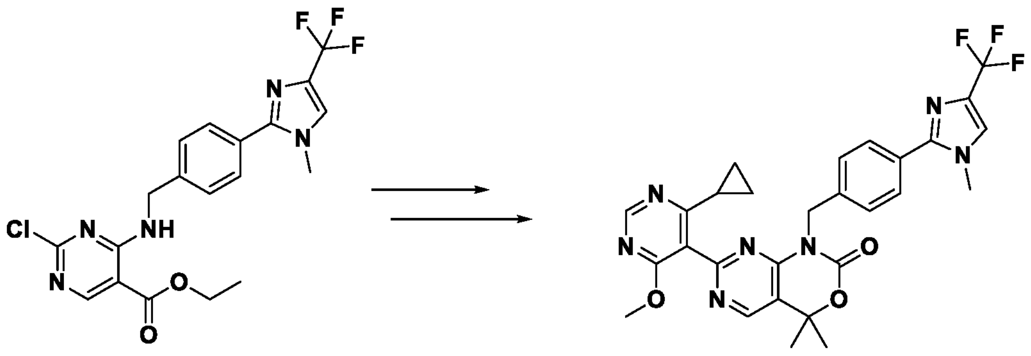

**35B** **50**

LC-MS: m/z (ESI): 554 $[M+H]^+$;
$^1$H NMR (400 MHz, DMSO-$d_6$) δ 8.84 (s, 1H), 8.66 (s, 1H), 7.92 (d, *J* = 1.3 Hz, 1H), 7.66 (d, *J* = 8.3 Hz, 2H), 7.41 (d, *J* = 8.3 Hz, 2H), 5.24 (s, 2H), 3.82 (s, 3H), 3.75 (s, 3H), 1.78 (s, 6H), 1.74-1.70 (m, 1H), 1.00 (dq, *J* = 6.1, 3.5 Hz, 2H), 0.79 (dt, *J* = 8.2, 3.3 Hz, 2H).

**Example 48: Preparation of 7'-(4-cyclopropyl-6-methoxypyrimidin-5-yl)-1'-(3-fluoro-4-(1-methyl-4-(trifluoromethyl)-1H-imidazol-2-yl)benzyl)spiro[cyclopropane-1,4'-pyrimido[4,5-d][1,3]oxazine]-2'(1'H)-one (compound 51)**

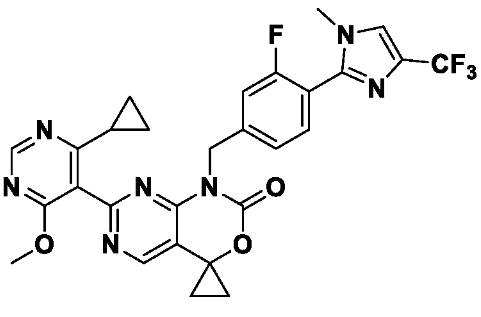

**51**

The synthesis route is as follows:

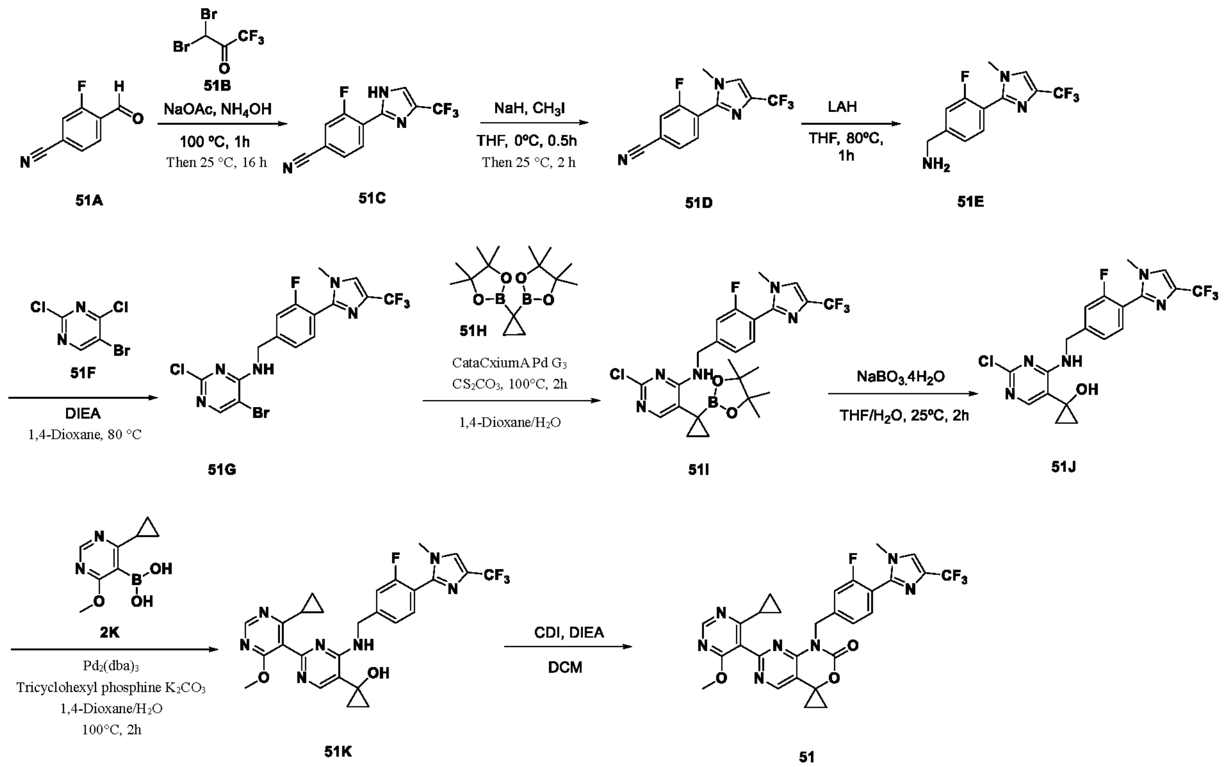


**Step 1: 3-fluoro-4-(4-(trifluoromethyl)-1H-imidazol-2-yl)benzonitrile (51C)**

At room temperature, 3,3-dibromo-1,1,1-trifluoroacetone (9.9 g, 37 mmol, 1.2 eq) and sodium acetate (3.0 g, 37 mmol, 1.2 eq) were added to water, and the resulting mixture was heated at 100 °C under stirring for reaction for 1 h. The reaction solution was cooled to room temperature, and slowly added dropwise to a solution of 2-fluoro-4-cyanobenzaldehyde (4.5 g, 30.2 mmol, 1.0 eq) in methanol (100 mL).Then aqueous ammonia (35 mL) was added to the reaction solution for reaction at room temperature under stirring for 16 h. The resulting mixture was distilled under vacuum to remove the solvent, and then the resulting residue was added to water (100 mL) and extracted with ethyl acetate (100 mL × 3). The resulting organic phases were combined, then washed with saturated brine (50 mL), and distilled under vacuum to remove the solvent. The resulting residue was purified by silica gel column chromatography (petroleum ether:ethyl acetate = 5:1-1:1) to obtain a solid title compound **51C** (5.0 g, yield: 65%). m/z (ESI): 256 $[M+H]^+$.

**Step 2: 3-fluoro-4-(1-methyl-4-(trifluoromethyl)-1H-imidazol-2-yl)benzonitrile (51D)**

The compound 51C (2.8 g, 11 mmol) was dissolved in tetrahydrofuran (30 mL), and sodium hydride (content: 60%, 0.67 g, 17 mmol, 1.5 eq) was added in portions at 0 °C. After the reaction solution was stirred at 0 °C for 0.5 h, iodomethane (3.2 g, 22 mmol, 2.0 eq) was added, and the resulting mixture was restored slowly to room temperature

for reaction for 2 h. The reaction solution was added to ice water (30 mL) and extracted with ethyl acetate (20 mL × 3). The organic phases were combined, then washed with saturated brine (50 mL), dried over anhydrous sodium sulfate, filtered, and distilled under vacuum to remove the solvent to obtain a solid title compound **51D** (3.0 g, yield: 81%). LC-MS: m/z (ESI): 270[M+H]$^+$.

**Step 3: 3-fluoro-4-(1-methyl-4-(trifluoromethyl)-1H-imidazol-2-yl)benzylamine (51E)**

The compound **51D** (2.9 g, 11 mmol) was added to tetrahydrofuran (60 mL), and lithium aluminum hydride (1.3 g, 33 mmol, 3.0 eq) was added in portions at 0 °C. The resulting mixture was stirred at 80°C for reaction for 1 h. At 0°C, 1.3 mL of water, 1.3 mL of 15% aqueous NaOH solution, and 3.8 mL of water were sequentially added to the reaction solution. The resulting mixture was stirred at room temperature for 1 h, and then filtered through diatomite, and the filter cake was rinsed with dichloromethane. The resulting filtrates were combined and distilled under vacuum to remove the solvent to obtain an oily title compound **51E** (3.0 g, yield: 100%). m/z (ESI): 274[M+H]$^+$.

**Step 4: 5-bromo-2-chloro-N (3-fluoro-4-(1-methyl-4-(trifluoromethyl)-1H-imidazol-2-yl)benzyl)pyrimidin-4-amine (51G)**

A compound **51F** (0.45 g, 2.0 mmol), diisopropylethylamine (0.52 g, 4.0 mmol), and the compound **51E** (0.55 g, 2.0 mmol) were mixed with 1,4-dioxane (5 mL), and the mixture was heated in an oil bath to 80 °C and stirred for 2 h. After distillation under vacuum to remove the solvent, the resulting residue was purified by silica gel column chromatography (petroleum ether:ethyl acetate = 3:1), and concentrated under vacuum to obtain a white solid **51G** (0.77 g, yield: 83%). m/z (ESI): 464 [M+H]$^+$.

**Step 5: 2-chloro-*N*-(3-fluoro-4-(1-methyl-4-(trifluoromethyl)-1H-imidazol-2-yl)benzyl)-5-(1-(4,4,5,5-tetramethyl-1,3,2-dioxaborolan-2-yl)cyclopropyl)pyrimidin-4-amine (511)**

The compound **51G** (464 mg, 1.0 mmol), a compound **51H** (0.89 g, 3.0 mmol), methanesulfonic acid[*n*-butyldi(1-adamantyl)phosphine](2-amino-1,1'-biphenyl-2-yl)palladium (II) (36.5 mg, 0.05 mmol), and cesium carbonate (1.63 g, 5.0 mmol) were added to 1,4-dioxane (5 mL) and water (1 mL), and the resulting mixture was heated in an oil bath to 100 °C and stirred for 2 h. Ethyl acetate (100 mL) was added for extraction and liquid separation, then the organic phase was washed three times with saturated brine (100 mL × 3), dried over anhydrous sodium sulfate, filtered, and distilled under vacuum to remove the solvent, and the resulting residue was purified by silica gel column chromatography (petroleum ether:ethyl acetate = 1:10-1:1) to obtain a colorless liquid **51I** (0.18 g, yield: 32%). m/z (ESI): 552 [M+H]$^+$.

**Step 6: 1-(2-chloro-4-((3-fluoro-4-(1-methyl-4-(trifluoromethyl)-1H-imidazol-2-yl)benzyl)amino)pyrimidin-5-yl)cyclopropan-1-ol (51J)**

The intermediate **51I** (0.18 g, 0.32 mmol) was added to tetrahydrofuran (5 mL) and water (5 mL), and then sodium perborate (0.49 g, 3.2 mmol) was added. The mixture was stirred at room temperature for 2 h. After distillation under vacuum to remove the solvent, the resulting residue was purified by silica gel column chromatography (petroleum ether:ethyl acetate = 1:5) to obtain a pale yellow liquid intermediate **51J** (74.6 mg, yield: 53%). m/z (ESI): 442 [M+H]$^+$.

**Step 7: 1-(4'-cyclopropyl-4-((3-fluoro-4-(1-methyl-4-(trifluoromethyl)-1H-imidazol-2-yl)benzyl)amino)-6'-methoxy-[2,5'-bipyrimidin]-5-yl)cyclopropan-1-ol (51K)**

The intermediate **51J** (74.6 mg, 0.17 mmol), a compound **2K** (39 mg, 0.2 mmol), tris(dibenzylideneacetone)dipalladium (15.5 mg, 0.017 mmol), tricyclohexylphosphine (9.5 mg, 0.034 mmol), and potassium carbonate (70.38 mg, 0.51mmol) were added to 1,4-dioxane (5 mL) and water (1 mL). The reaction solution was heated to 100°C and stirred for 2 h. Water (50 mL) was added for extraction and liquid separation, and the organic phase was washed three times with saturated brine (100 mL × 3), dried over anhydrous sodium sulfate, and filtered. After distillation under vacuum to remove the solvent, the resulting residue was purified by silica gel column chromatography (petroleum ether:ethyl acetate = 1:10-1:1) to obtain a white solid intermediate **51K** (28 mg, 0.05 mmol, yield: 30%). m/z (ESI): 556 [M+H]$^+$.

**Step 8: 7'-(4-cyclopropyl-6-methoxypyrimidin-5-yl)-1'-(3-fluoro-4-(1-methyl-4-(trifluoromethyl)-1H-imidazol-2-yl)benzyl)spiro [cyclopropane-1,4'-pyrimido [4,5-d][1,3]oxazine]-2'(1'H)-one (51)**

The compound **51K** (28 mg, 0.05 mmol) was added to dichloromethane (2 mL), and then carbonyldiimidazole (81 mg, 0.5 mmol) and diisopropylethylamine (26 mg, 0.2 mmol) were added and stirred at room temperature for 2 h.

The reaction solution was concentrated under vacuum, and the resulting crude product was purified by preparative chromatography (Waters Xbridge C18, 5-95% acetonitrile/water mobile phase) to obtain a white solid title compound **51** (3.6 mg, 6.2 μmol, yield: 12.4%).

LC-MS: m/z (ESI): 582[M+H]+.

$^1$H NMR (400 MHz, DMSO-$d_6$) δ 8.65 (s, 1H), 8.50 (s, 1H), 7.99 (s, 1H), 7.54 (t, *J* = 7.8 Hz, 1H), 7.37 (d, *J* = 11.1 Hz, 1H), 7.30 (d, *J* = 7.9 Hz, 1H), 5.25 (s, 2H), 3.81 (s, 3H), 3.61-3.56 (m, 3H), 1.73 (dq, *J* = 8.2, 4.7, 4.1 Hz, 1H), 1.59-1.53 (m, 2H), 1.53-1.46 (m, 2H), 1.06-0.97 (m, 2H), 0.80 (dt, *J* = 7.9, 3.5 Hz, 2H).

**Example 49: Preparation of 7'-(4-cyclopropyl-6-methoxypyrimidin-5-yl)-1'-(4-(1-methyl-4-(trifluoromethyl)-1H-imidazol-2-yl)benzyl)spiro [cyclopropane-1,4'-pyrimido [4,5-d][1,3]oxazine]-2'(1'H)-one (compound 52)**

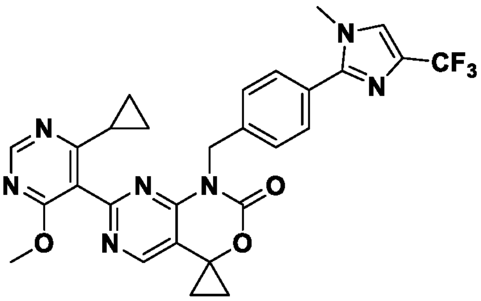

**52**

The compound **4B** was used instead of the compound **51E** to prepare the compound **52** by using synthesis steps similar to those for the compound **51.**

LC-MS: m/z (ESI): 564[M+H]+
$^1$H NMR (400 MHz, DMSO-$d_6$) δ 8.66 (s, 1H), 8.51 (s, 1H), 7.93 (s, 1H), 7.66 (d, *J* = 7.4 Hz, 2H), 7.44 (d, *J* = 7.9 Hz, 2H), 5.25 (s, 2H), 3.81 (s, 3H), 1.72 (s, 1H), 1.53 (d, *J* = 11.4 Hz, 4H), 0.99 (s, 2H), 0.78 (s, 2H).

**Example 50: Preparation of 7'-(4-cyclopropyl-6-methoxypyrimidin-5-yl)-1'-(4-(5-methyl-3-(trifluoromethyl)-1H-pyrazol-1-yl)benzyl)spiro [cyclopropane-1,4'-pyrimido [4,5-d][1,3]oxazine]-2'(1'H)-one (compound 53)**

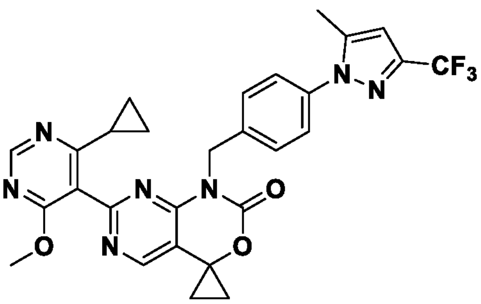

**53**

The compound **43D** was used instead of the compound **51E** to prepare the compound **53** by using synthesis steps similar to those for the compound **51.**

LC-MS: m/z (ESI): 564[M+H]+
$^1$H NMR (400 MHz, DMSO-$d_6$) δ 8.65 (s, 1H), 8.51 (s, 1H), 7.59-7.42 (m, 4H), 6.75 (s, 1H), 5.26 (s, 2H), 3.81 (s, 3H), 2.32 (s, 3H), 1.71 (tt, *J* = 8.2, 4.5 Hz, 1H), 1.62-1.53 (m, 2H), 1.53-1.44 (m, 2H), 0.99 (dq, *J* = 6.3, 3.8 Hz, 2H), 0.79 (dt, *J* = 8.2, 3.3 Hz, 2H).

**Example 51: Preparation of 7'-(4-cyclopropyl-6-methoxypyrimidin-5-yl)-1'-(4-(5-methyl-3-(trifluoromethyl)-1H-pyrazol-1-yl)benzyl)spiro [oxetane-3,4'-pyrimido [4,5-d] [1,3] oxazine]-2'(1'H)-one (compound 54)**

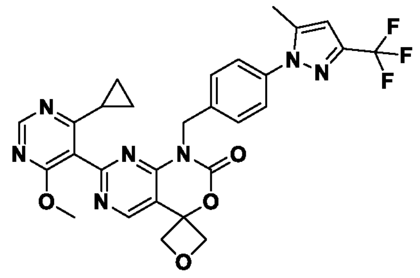

**54**

The synthesis route and specific synthesis steps are as follows:

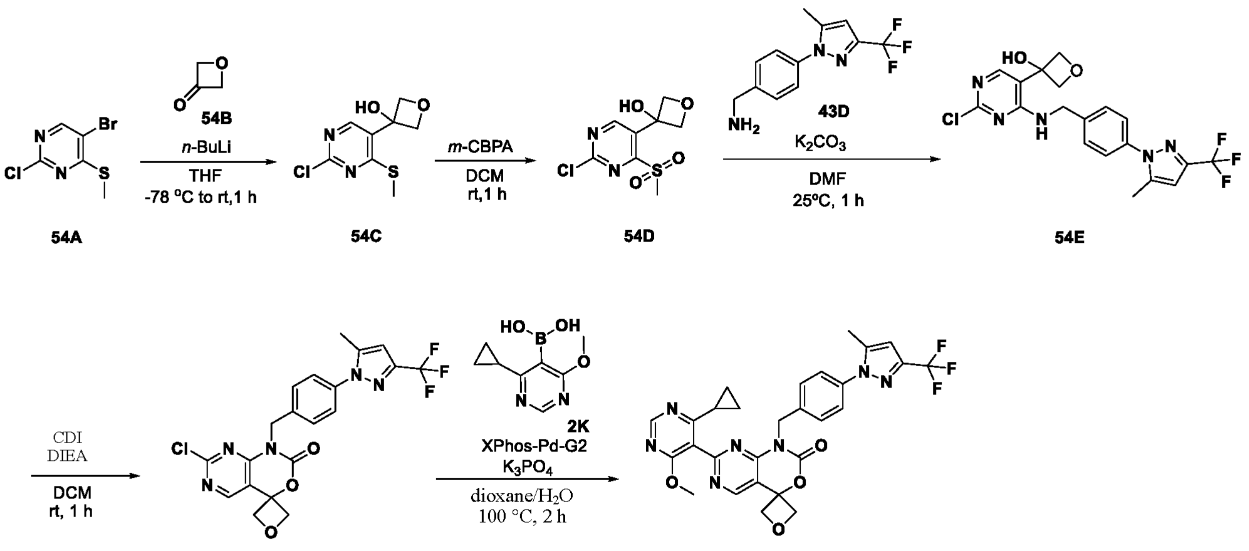


**Step 1: 3-(2-chloro-4-(methylthio)pyrimidin-5-yl)oxetan-3-ol (54C)**

A compound **54A** (0.50 g, 2.1 mmol) and oxetanone **54B** (0.15 g, 2.1 mmol, 1.0 eq.) were added to tetrahydrofuran (10 mL). Under the protection of nitrogen, the resulting mixture was cooled in a dry ice-ethanol bath to -78 °C, then 1.6 M n-butyllithium solution (1.3 mL, 2.2 mmol, 1.05 eq.) was slowly added dropwise and stirred at -78 °C for half an hour, and the resulting solution was restored slowly to room temperature for reaction at room temperature for half an hour. In an ice water bath, 1 M aqueous dilute hydrochloric acid solution (10 mL) was added to the reaction solution dropwise to quench the reaction, then the mixture was kept stand and layered, the water phase was extracted three times with 20 mL of ethyl acetate, and the organic phase was dried over anhydrous sodium sulfate, filtered, and concentrated under vacuum to obtain a crude product. The crude product was purified by silica gel column chromatography (petroleum ether:ethyl acetate = 5:1-1:1) to obtain **54C** (0.19 g, yield: 39%). m/z (ESI): 233[M+H]$^+$.

**Step 2: 3-(2-chloro-4-(methylsulfonyl)pyrimidin-5-yl)oxetan-3-ol (54D)**

The compound **54C** (0.15 g, 644.6 μmol) was added to dichloromethane (10 mL), then *m*-chloroperoxybenzoic acid (0.22 g, 1.3 mmol, 2.0 eq.) was added, and the resulting mixture was stirred at room temperature for reaction for 1 h. To the reaction solution, 1 mL of 20% aqueous sodium thiosulfate solution was added to quench the reaction, then 10 mL of water was added to the reaction solution, and the organic phase was separated, dried over anhydrous sodium sulfate, filtered, and concentrated under vacuum to obtain a crude product **54D** (0.15 g, yield: 88%). m/z (ESI): 265[M+H]$^+$.

**Step 3: 1-(2-chloro-4-((4-(5-methyl-3-(trifluoromethyl)-1H-pyrazol-1-yl)benzyl)amino)pyrimidin-5-yl)oxetan-3-ol (54E)**

The compound **54D** (0.15 g, 570.9 μmol), the compound **43D** (0.15 g, 570.9 μmol, 1.0 eq.), and potassium carbonate (0.16 g, 1.1 mmol, 2.0 eq.) were added to *N,N*-dimethylformamide (5 mL), and the resulting mixture was stirred at room temperature for reaction for 1 h. The reaction solution was poured into 30 mL of water, then the resulting solution was extracted three times with 20 mL of ethyl acetate, and the organic phase was dried over anhydrous sodium sulfate, filtered, and concentrated under vacuum to obtain a crude product. The crude product was purified by silica gel column chromatography (petroleum ether:ethyl acetate =1:1-1:5) to obtain **54E** (0.18 g, yield: 72%). m/z (ESI): 438$[M+H]^+$.

**Step 4: 7'-chloro-1'-(4-(5-methyl-3-(trifluoromethyl)-1H-pyrazol-1-yl)benzyl)spiro[oxetane-3,4'-pyrimido[4,5-d][1,3]oxazine]-2'(1'H)-one (54F)**

The compound **54E** (0.18 g, 409.3 μmol), *N,N'*-carbonyldiimidazole (0.18 g, 1.2 mmol, 3.0 eq.), and *N,N'*-diisopropylethylamine (0.16 g, 1.2 mmol, 213.9 μL, 3.0 eq.) were added to dichloromethane (10 mL), and then the resulting mixture was stirred at room temperature for reaction for 1 h. The reaction solution was poured into 30 mL of water, then the resulting solution was extracted with ethyl acetate (20 mL × 3), and the organic phase was dried over anhydrous sodium sulfate, filtered, and concentrated under vacuum to obtain a crude product. The crude product was purified by silica gel column chromatography (petroleum ether:ethyl acetate =1:1-1:5) to obtain **54F** (60 mg, yield: 32%). m/z (ESI): 466$[M+H]^+$.

**Step 5: 7'-(4-cyclopropyl-6-methoxypyrimidin-5-yl)-1'-(4-(5-methyl-3-(trifluoromethyl)-1H-pyrazol-1-yl)benzyl)spiro[oxetane-3,4'-pyrimido[4,5-d][1,3]oxazine]-2'(1'H)-one (54)**

The compound **54F** (50 mg, 107.3 μmol), (4-cyclopropyl-6-methoxypyrimidin-5-yl)boronic acid **2K** (42 mg, 214.7 μmol, 2.0 eq.), Xphos Pd G2 (16.5 mg, 21.5 μmol, 0.2 eq.), and potassium phosphate (68.3 mg, 322.0 μmol, 3.0 eq.) were added to a mixed solvent of water (0.1 mL) and 1,4-dioxane (2 mL) for reaction at 100 °C for 2 h under the protection of nitrogen. The resulting mixture was filtered through a 13 mm 0.45 μM syringe filter and concentrated under vacuum to obtain a crude product. The resulting crude product was purified by preparative chromatography (Waters Xbridge C18, 10-70% aqueous acetonitrile solution) to obtain a white solid **54** (12 mg, yield: 19%).

LC-MS: m/z (ESI): 580.1 $[M+H]^+$.

$^1H$ NMR (400 MHz, DMSO-$d_6$) δ 9.15 (s, 1H), 8.67 (s, 1H), 7.49 (s, 4H), 6.74 (s, 1H), 5.18 (s, 2H), 5.01 (d, *J* = 8.1 Hz, 2H), 4.95 (d, *J* = 8.0 Hz, 2H), 3.82 (s, 3H), 2.31 (s, 3H), 1.72 (m, 1H), 1.01 (m, 2H), 0.80 (m, 2H).

**Example 52: Preparation of 7'-(4-cyclopropyl-6-methoxypyrimidin-5-yl)-1'-(4-(5-methyl-3-(trifluoromethyl)-1H-pyrazol-1-yl)benzyl)spiro[cyclobutane-1,4'-pyrimido[4,5-d] [1,3]oxazin]-2'(1'H)-one (compound 55)**

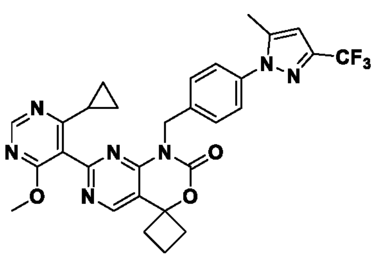

**55**

**Cyclobutanone** was used instead of **oxetanone 54B** to prepare the compound **55** by using synthesis steps similar to those for the compound **54.**

LC-MS: m/z (ESI): 578.2 $[M+H]^+$.

$^1H$ NMR (400 MHz, DMSO-$d_6$) δ 9.05 (s, 1H), 8.66 (s, 1H), 7.50 (d, *J* = 8.6 Hz, 2H), 7.43 (d, *J* = 8.5 Hz, 2H), 6.74 (s, 1H), 5.24 (s, 2H), 3.82 (s, 3H), 2.77-2.63 (m, 4H), 2.31 (s, 3H), 2.15-1.95 (m, 2H), 1.73 (m, 1H), 1.00 (m, 2H), 0.79 (m, 2H).

**Example 53: Preparation of 7-(4-cyclopropyloxypyrimidin-5-yl)-1-(3-fluoro-4-(1-methyl-4-(trifluoromethyl)-1H-imidazol-2-yl)benzyl)-4,4-dimethyl-1,4-dihydro-2H-pyrimido [4,5-d][1,3]oxazin-2-one (compound 56)**

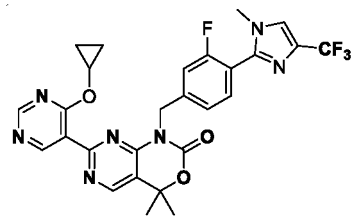

**56**

In the last step, the compound **56A** was used instead of the compound **2K** to prepare the compound **56** by using synthesis steps similar to those for the compound **49.**

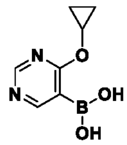

**56A**

LC-MS: m/z (ESI): 570.1 $[M+H]^+$.
$^1$H NMR (400 MHz, DMSO-$d_6$) δ 8.76 (s, 1H), 8.62 (s, 1H), 8.45 (s, 1H), 7.99 (d, *J* = 1.3 Hz, 1H), 7.57-7.47 (m, 2H), 7.38 (m, 1H), 6.09-5.94 (m, 1H), 5.29 (s, 2H), 5.24 (m, 1H), 5.18 (m, 1H), 4.61 (m, 2H), 3.58 (s, 3H), 1.74 (s, 6H).

**Example 54: Preparation of 7-(4-cyclopropyl-6-(difluoromethoxy)pyrimidin-5-yl)-1-(3-fluoro-4-(1-methyl-4-(trifluoromethyl)-1H-imidazol-2-yl)benzyl)-4,4-dimethyl-1,4-dihydro-2H-pyrimido[4,5-d][1,3]oxazin-2-one (compound 57)**

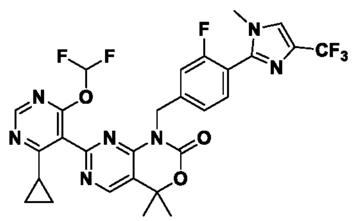

**57**

In the last step, the compound **57A** was used instead of the compound **2K** to prepare the compound **57** by using synthesis steps similar to those for the compound **49.**

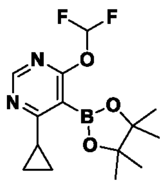

**57A**

LC-MS: m/z (ESI): 602.2 $[M+H]^+$.
$^1$H NMR (400 MHz, DMSO-$d_6$) δ 8.90 (s, 1H), 8.79 (s, 1H), 7.81 (m, 1H), 7.48 (m, 4H), 6.75 (s, 1H), 5.26 (s,

2H), 2.31 (s, 3H), 1.87 (m, 1H), 1.80 (s, 6H), 1.06 (m, 2H), 0.88 (m, 2H).

**Example 55: Preparation of 7-(4-cyclopropyl-6-methoxypyrimidin-5-yl)-1-(3-fluoro-4-(1-methyl-4-(trifluoromethyl)-1H-imidazol-2-yl)benzyl)-4-(hydroxymethyl)-4-methyl-1,4-dihydro-2H-pyrimido[4,5-d][1,3]oxazin-2-one (compound 58)**

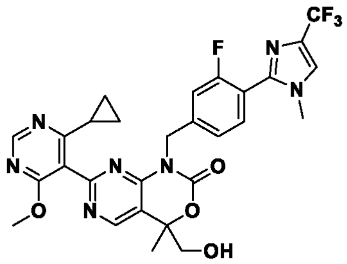

**58**

The synthesis route and specific synthesis steps are as follows:

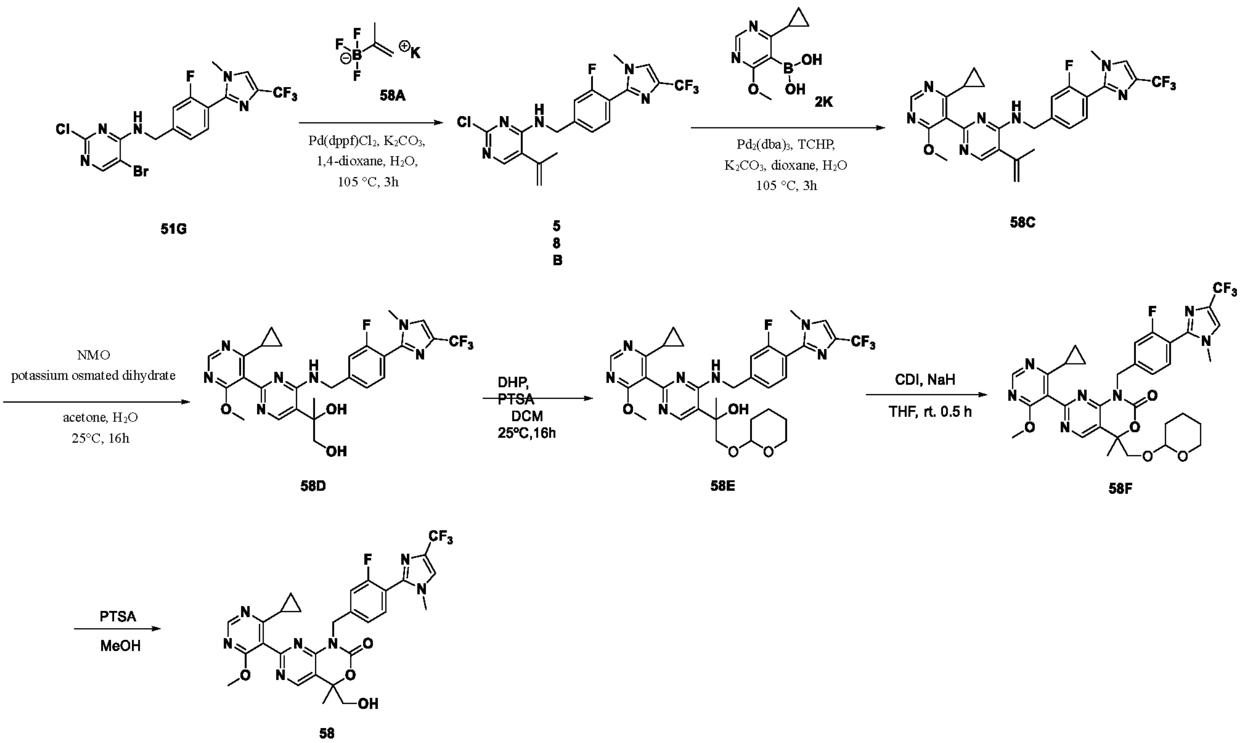


**Step 1: 2-chloro-*N*-(3-fluoro-4-(1-methyl-4-(trifluoromethyl)-1H-imidazol-2-yl)benzyl)-5-(prop-1-en-2-yl)pyrimidin-4-amine (58B)**

The compound **51G** (0.30 g, 0.65 mmol) and potassium isopropenyltrifluoroborate (96 mg, 0.65 mmol) were dissolved in 1,4-dioxane (10 mL) and water (1 mL) at room temperature, and then [1,1'-bis(diphenylphosphino)ferrocene]dichloropalladium (51 mg, 0.07 mmol) and potassium carbonate (269 mg, 1.95 mmol) were added. The resulting mixture was stirred under a nitrogen atmosphere at 105 °C for reaction for 3 h. The reaction solution was concentrated, and the resulting crude product was purified by silica gel column chromatography (petroleum ether:ethyl acetate = 1:1-1:5) to obtain a compound **58B** (0.19 g, 0.46 mmol, yield: 70%). m/z (ESI): 426.2 [M+H]$^+$.

**Step 2: 4'-cyclopropyl-N (3-fluoro-4-(1-methyl-4-(trifluoromethyl)-1H-imidazol-2-yl)benzyl)-6'-methoxy-5-(prop-1-en-2-yl)-[2,5'-bipyrimidin]-4-amine (58C)**

The compound **58B** (0.19 g, 0.46 mmol) and the compound **2K** (0.19 g, 0.69 mmol) were dissolved in 1,4-dioxane (10 mL) and water (1 mL) at room temperature, and then tris(dibenzylideneacetone)dipalladium (46 mg, 0.05 mmol), tricyclohexylphosphorus (28 mg, 0.1 mmol), and potassium carbonate (190 mg, 1.38 mmol) were added. The resulting mixture was stirred under a nitrogen atmosphere at 105 °C for reaction for 3 h. The reaction solution was concentrated, and the resulting crude product was purified by silica gel column chromatography (petroleum ether:ethyl acetate = 1:1-1:5) to obtain a compound **58C** (0.17 g, 0.32 mmol, yield: 70%). m/z (ESI): 540.1 $[M+H]^+$.

**Step 3: 2-(4'-cyclopropyl-4-((3-fluoro-4-(1-methyl-4-(trifluoromethyl)-1H-imidazol-2-yl)benzyl)amino)-6'-methoxy-[2,5'-bipyrimidin]-5-yl)propane-1,2-diol (58D)**

*N*-methylmorpholine oxide (112 mg, 0.96 mmol) and potassium osmate dihydrate (9 mg, 0.03 mmol) were added to a solution of compound **58C** (0.17 g, 0.32 mmol) in acetone (5 mL) and water (2 mL) at room temperature. The resulting mixture was stirred at room temperature for reaction for 16 h. The reaction solution was concentrated, and the resulting crude product was purified by silica gel column chromatography (methanol:dichloromethane = 1:100-5:100) to obtain a compound **58D** (0.12 g, 0.21 mmol, yield: 65%). m/z (ESI): 573.8 $[M+H]^+$.

**Step 4: 2-(4'-cyclopropyl-4-((3-fluoro-4-(1-methyl-4-(trifluoromethyl)-1H-imidazol-2-yl)benzyl)amino)-6'-methoxy-[2,5'-bipyrimidin]-5-yl)-1-((tetrahydro-2H-pyran-2-yl)oxy)propan-2-ol (58E)**

At room temperature, 3,4-dihydro-2H-pyran (53 mg, 0.63 mmol) and p-toluenesulfonic acid were sequentially added to a solution of compound **58D** (121 mg, 0.21 mmol) in dichloromethane, and the resulting solution was stirred at room temperature for reaction for 16 h. The reaction solution was concentrated, and the resulting crude product was purified by silica gel column chromatography (methanol:dichloromethane = 0:100-5:100) to obtain a compound **58E** (65 mg, 0.10 mmol, yield: 50%). m/z (ESI): 658.3 $[M+H]^+$.

**Step 5: 7-(4-cyclopropyl-6-methoxypyrimidin-5-yl)-1-(3-fluoro-4-(1-methyl-4-(trifluoromethyl)-1H-imidazol-2-yl)benzyl)-4-methyl-4-(((tetrahydro-2H-pyran-2-yl)oxy)methyl)-1,4-dihydro-2H-pyrimido [4,5-d] [1,3] oxazin-2-one (58F)**

Sodium hydride (12 mg, 0.3 mmol, 60%, oily substance) was added to a solution of compound **58E** (65 mg, 0.10 mmol) and *N,N'*-carbonyldiimidazole (24 mg, 0.15 mmol) in tetrahydrofuran (3 mL) at room temperature. The resulting solution was stirred at room temperature for reaction for 30 min, and quenched with methanol. The reaction solution was concentrated, and the resulting crude product was purified by silica gel column chromatography (methanol:dichloromethane = 0:100-5:100) to obtain a compound **58F** (51 mg, 0.07 mmol, yield: 75%). m/z (ESI): 684.3 $[M+H]^+$.

**Step 6: 7-(4-cyclopropyl-6-methoxypyrimidin-5-yl)-1-(3-fluoro-4-(1-methyl-4-(trifluoromethyl)-1H-imidazol-2-yl)benzyl)-4-(hydroxymethyl)-4-methyl-1,4-dihydro-2H-pyrimido[4,5-d] [1,3]oxazin-2-one (58)**

At room temperature, p-toluenesulfonic acid was added to a solution of compound **58F** (48 mg, 0.07 mmol) in methanol (10 mL). The resulting solution was stirred at room temperature for reaction for 30 min. The reaction solution was concentrated, and the resulting crude product was purified by preparative chromatography (Waters Xbridge C18, 10-70% aqueous acetonitrile solution) to obtain a compound **58** (20 mg, 0.03 mmol, yield: 43%).

m/z (ESI): 600.2 $[M+H]^+$
$^1$H NMR (400 MHz, DMSO-$d_6$) δ 8.78 (s, 1H), 8.65 (s, 1H), 7.99 (s, 1H), 7.51 (t, *J* = 7.7 Hz, 1H), 7.37-7.27 (m, 2H), 5.70 (t, *J* = 5.4 Hz, 1H), 5.32-5.16 (m, 2H), 3.80 (s, 3H), 3.73 (h, *J* = 5.5 Hz, 2H), 3.58 (d, *J* = 1.6 Hz, 3H), 1.74 (s, 3H), 1.73-1.69 (m, 1H), 1.00-0.98 (m, 2H), 0.82-0.71 (m, 2H).

**Example 56: Preparation of 7-(4-cyclopropyl-6-methoxypyrimidin-5-yl)-4-ethynyl-1-(3-fluoro-4-(1-methyl-4-(trifluoromethyl)-1H-imidazol-2-yl)benzyl)-4-methyl-1,4-dihydro-2H-pyrimido[4,5-d][1,3]oxazin-2-one (compound 59)**

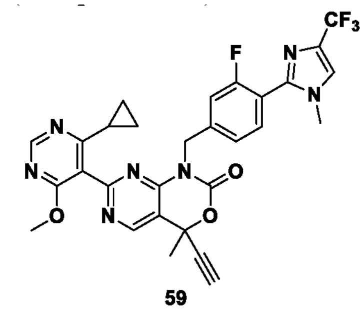

59

The synthesis route and specific synthesis steps are as follows:

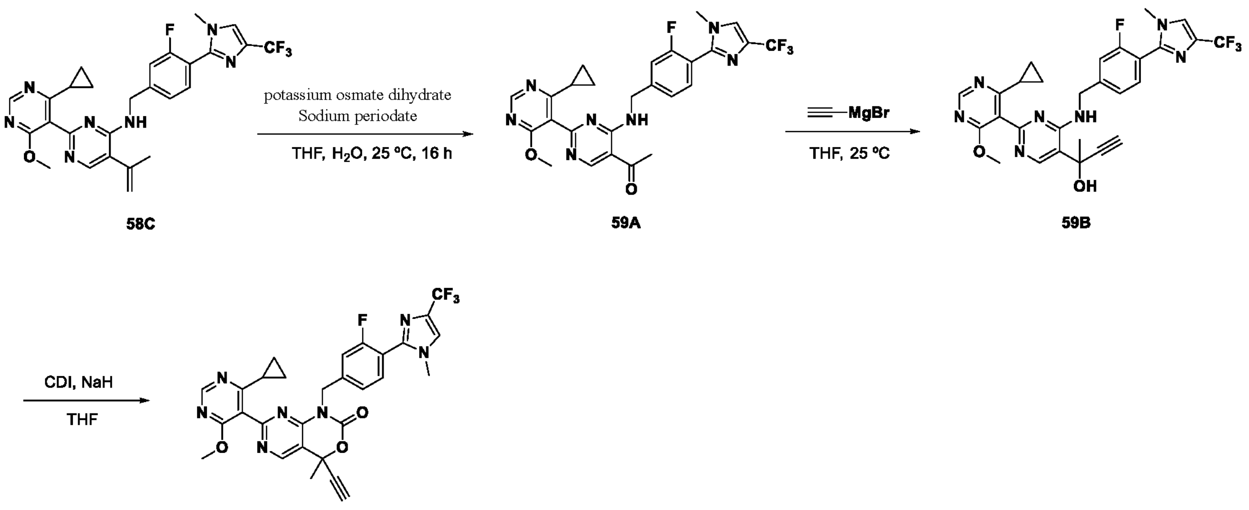


58C 59A 59B

59

**Step 1: 1-(4'-cyclopropyl-4-((3-fluoro-4-(1-methyl-4-(trifluoromethyl)-1H-imidazol-2-yl)benzyl)amino)-6'-methoxy-[2,5'-bipyrimidin]-5-yl)ethan-1-one (59A)**

Sodium periodate (58 mg, 0.27 mmol) and potassium osmate dihydrate (3 mg, 0.01 mmol) were added to a solution of compound **58C** (50 mg, 0.09 mmol) in tetrahydrofuran (3 mL) and water (1 mL) at room temperature. The resulting solution was stirred at room temperature for reaction for 16 h. The reaction solution was filtered and concentrated under vacuum, and the concentrate was purified by column chromatography to obtain **59A** (33 mg, 0.06 mmol, yield: 70%). m/z (ESI): 542.2 $[M+H]^+$.

**Step 2: 2-(4'-cyclopropyl-4-((3-fluoro-4-(1-methyl-4-(trifluoromethyl)-1H-imidazol-2-yl)benzyl)amino)-6'-methoxy-[2,5'-bipyrimidin]-5-yl)but-3-yn-2-ol (59B)**

Ethynylmagnesium bromide (1.0 mL, 0.5 mmol, 0.5 M THF solution) was added dropwise to a solution of compound **59A** (33 mg, 0.06 mmol) in tetrahydrofuran (3 mL) at room temperature, and the resulting mixture was stirred for reaction for 3 h. The reaction solution was quenched with 1 mL of methanol and concentrated under vacuum, and the resulting crude product was purified by preparative chromatography (Waters Xbridge C18, 10-70% aqueous acetonitrile solution) to obtain a compound **59B** (17 mg, 0.03 mmol, yield: 50%). m/z (ESI): 568.2 $[M+H]^+$.

**Step 3: 7-(4-cyclopropyl-6-methoxypyrimidin-5-yl)-4-ethynyl-1-(3-fluoro-4-(1-methyl-4-(trifluoromethyl)-1H-imidazol-2-yl)benzyl)-4-methyl-1,4-dihydro-2H-pyrimido[4,5-d] [1,3]oxazin-2-one (59)**

Sodium hydride (6 mg, 0.15 mmol, 60%, oily substance) was added to a solution of compound **59B** (17 mg, 0.03 mmol) and *N,N'*-carbonyldiimidazole (10 mg, 0.06 mmol) in tetrahydrofuran (3 mL) at room temperature. The resulting solution was stirred at room temperature for reaction for 30 min, and quenched with methanol. The reaction solution was concentrated under vacuum, and the resulting crude product was purified by preparative chromatography (Waters Xbridge C18, 10-70% aqueous acetonitrile solution) to obtain a compound **59** (11 mg, 0.02 mmol, yield: 75%).
m/z (ESI): 594.3 $[M+H]^+$.

$^1H$ NMR (400 MHz, DMSO-$d_6$) δ 9.00 (s, 1H), 8.67 (s, 1H), 8.00 (s, 1H), 7.55 (t, $J$ = 7.8 Hz, 1H), 7.36-7.28 (m, 2H), 5.36-5.26 (m, 2H), 4.25 (s, 1H), 3.81 (s, 3H), 3.62-3.55 (m, 3H), 2.15 (s, 3H), 1.76-1.71 (m, 1H), 1.06-0.96 (m, 2H), 0.82 (s, 2H).

**Example 57: Preparation of 2-(4-cyclopropyl-6-methoxypyrimidin-5-yl)-8-(4-(1-methyl-4-(trifluoromethyl)-1H-imidazol-2-yl)benzyl)pteridin-7(8H)-one (compound 60)**

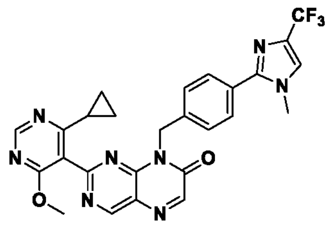

**60**

The synthesis route and specific synthesis steps are as follows:

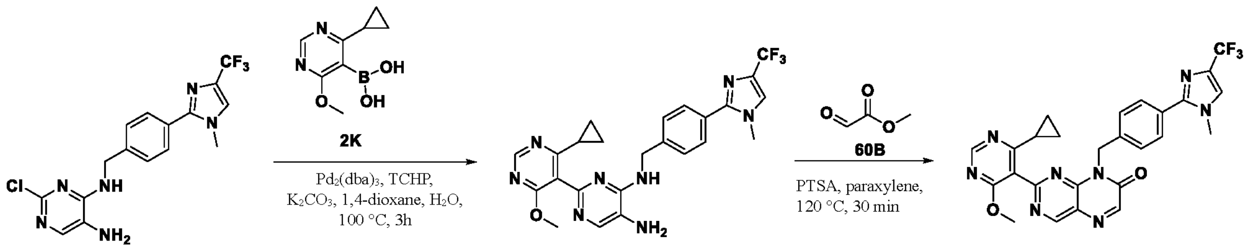


**4D** **60A** **60**

**Step 1: 4'-cyclopropyl-6'-methoxy-*N*$^4$-(4-(1-methyl-4-(trifluoromethyl)-1H-imidazol-2-yl)benzyl)-[2,5'-bipyrimidine]-4,5-diamine (60A)**

The compound **4D** (100 mg, 0.26 mmol) and the compound **2K** (108 mg, 0.39 mmol) were dissolved in 1,4-dioxane (10 mL) and water (1 mL) at room temperature, and then tris(dibenzylideneacetone)dipalladium (27 mg, 0.03 mmol), tricyclohexylphosphine (17 mg, 0.06 mmol), and potassium carbonate (108 mg, 0.78 mmol) were added. The resulting reaction solution was stirred under a nitrogen atmosphere at 100 °C for reaction for 3 h. After the reaction solution was concentrated under vacuum, the resulting crude product was purified by reversed-phase C18 column chromatography (with 5-70% aqueous acetonitrile solution as the elution phase) to obtain a compound **60A** (40 mg, 0.08 mmol, yield: 30%). m/z (ESI): 497.1 $[M+H]^+$.

**Step 2: 2-(4-cyclopropyl-6-methoxypyrimidin-5-yl)-8-(4-(1-methyl-4-(trifluoromethyl)-1H-imidazol-2-yl)benzyl)pteridin-7(8H)-one (60)**

A compound **60B** (141 mg, 0.8 mmol, 50% toluene solution) and p-toluenesulfonic acid (0.7 mg, 0.004 mmol) were added to a solution of compound **60A** (40 mg, 0.08 mmol) in *p*-xylene (3 mL) at room temperature. The resulting reaction solution was heated by microwave at 120 °C for 30 min, and concentrated under vacuum, and then the resulting crude product was purified by preparative chromatography (Waters Xbridge C18, 10-70% aqueous acetonitrile solution) to obtain a compound **60** (15 mg, 0.03 mmol, yield: 38%).

m/z (ESI): 535.1 $[M+H]^+$.

$^1H$ NMR (400 MHz, DMSO-$d_6$) δ 9.37 (s, 1H), 8.70 (s, 1H), 8.48 (s, 1H), 7.92 (s, 1H), 7.67-7.61 (m, 2H), 7.49-7.47 (m, 2H), 5.49 (s, 2H), 3.84 (s, 3H), 3.74 (s, 3H), 1.79-1.73 (m, 1H), 1.06-0.99 (m, 2H), 0.80-0.76 (m, 2H).

**Example 58: Preparation of 2-(4-cyclopropyl-6-methoxypyrimidin-5-yl)-6-methyl-8-(4-(1-methyl-4-(trifluoromethyl)-1H-imidazol-2-yl)benzyl)pteridin-7(8H)-one (compound 61)**

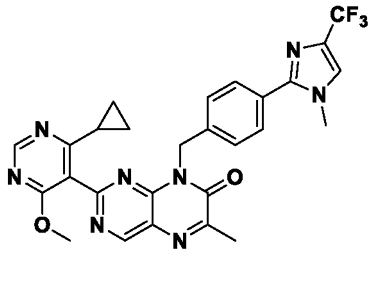

**61**

In the last step, the compound **61A** was used instead of the compound **60B** to prepare the compound **61** by using synthesis steps similar to those for the compound **60.**

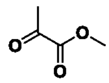

**61A**

m/z (ESI): 549.2 [M+H]+.

**Example 59**

The starting material **A** in the table below was used instead of the compound **1E** to prepare compounds **62-64** by using synthesis steps similar to those for the compound **2.**

| No. | Starting material **A** | Structure of compound | m/z [M+H] | $^1$HNMR |
|---|---|---|---|---|
| **62** | 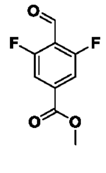 | 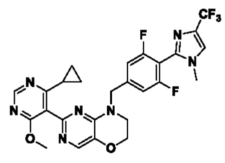 62 | 560.2 | $^1$H NMR (400 MHz, DMSO-*d6*) $\delta$ 8.62(s, 1H), 8.08(s, 1H), 8.00(s, 1H), 7.29(d, *J* = 12.0, 2H), 4.89(s, 2H), 4.34(t, *J* = 4.0 Hz, 2H), 3.85(s, 3H), 3.67 (t, *J* = 4.0 Hz, 2H), 3.59 (s, 3H), 1.80-1.72 (m, 1H), 1.03-0.97(m, 2H), 0.90-0.82(m, 2H). |
| **63** | 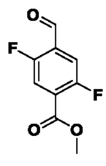 | 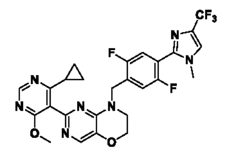 63 | 560.2 | $^1$H NMR (400 MHz, DMSO-$d_6$) $\delta$ 8.59 (s, 1H), 8.02 (s, 1H), 7.97 (s, 1H), 7.52 (dd, *J* = 9.6, 5.8 Hz, 1H), 7.36 (dd, *J* = 10.0, 5.9 Hz, 1H), 4.87 (s, 2H), 4.33 (t, *J* = 4.6 Hz, 2H), 3.81 (s, 3H), 3.67 (t, *J* = 4.5 Hz, 2H), 3.62 (s, 3H), 1.74 (m, 1H), 0.98 (m, 2H), 0.82 (m, 2H). |
| **64** | 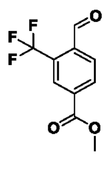 | 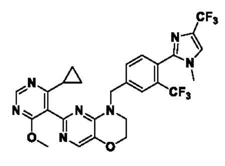 64 | 592.2 | $^1$H NMR (400 MHz, DMSO-$d_6$) 6 8.59 (s, 1H), 7.98 (m, 2H), 7.81 (s, 1H), 7.73-7.68 (m, 1H), 7.64 (d, *J* = 7.9 Hz, 1H), 4.96 (s, 2H), 4.31 (t, *J* = 4.5 Hz, 2H), 3.81 (s, 3H), 3.64 (t, *J* = 4.5 Hz, 2H), 3.44 (s, 3H), 1.72 (m, 1H), 0.97 (m, 2H), 0.86-0.77 (m, 2H). |

**Example 60: Preparation of 2-(4-cyclopropyl-6-methoxypyrimidin-5-yl)-5-methyl-8-(3-fluoro-4-(1-methyl-4-(trifluoromethyl)-1H-imidazol-2-yl)benzyl)-7,8-dihydropteridin-6(SH)-one (compound 65)**

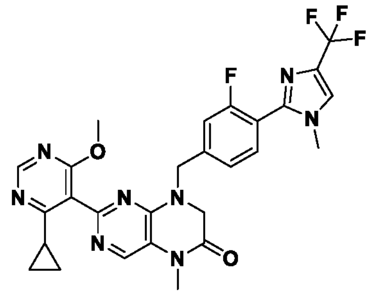

**65**

The compound **51E** was used instead of the compound **28D** in step **4** to prepare the compound **65** by using a method similar to that in Example **26.**

LC-MS: m/z (ESI): 569.2 $[M+H]^+$.

$^1H$ NMR (400 MHz, DMSO-$d_6$) δ 8.62 (s, 1H), 8.15 (s, 1H), 8.00 (s, 1H), 7.56 (t, *J* = 7.7 Hz, 1H), 7.40 (dd, *J* = 11.2, 1.5 Hz, 1H), 7.34 (dd, *J* = 7.9, 1.6 Hz, 1H), 4.84 (s, 2H), 4.26 (s, 2H), 3.84 (s, 3H), 3.59 (s, 3H), 3.31 (s, 3H), 1.78 (m, 1H), 1.00 (m, 2H), 0.84 (m, 2H).

**Example 61: Preparation of 2-(4-cyclopropyl-6-methoxypyrimidin-5-yl)-5-methyl-8-(3,5-difluoro-4-(1-methyl-4-(trifluoromethyl)-1H-imidazol-2-yl)benzyl)-7,8-dihydropteridin-6(SH)-one (compound 66)**

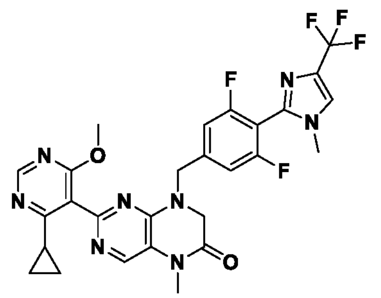

**66**

The compound **66A** was used instead of the compound **51A** in step **1** to prepare the compound **66B** by using a method similar to that for the compound **51E.** The compound **66B** was then used instead of the compound **28D** in step **4** to prepare the compound **66** by using a method similar to that in Example **26.**

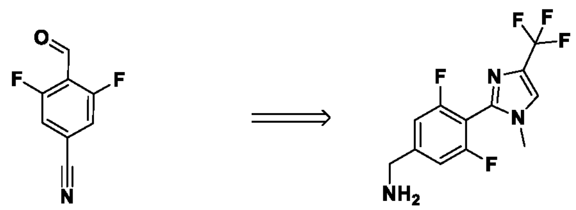

**66A** **66B**

LC-MS: m/z (ESI): 587.2 $[M+H]^+$.

$^1H$ NMR (400 MHz, DMSO-$d_6$) δ 8.62 (s, 1H), 8.16 (s, 1H), 8.06 (s, 1H), 7.34 (d, *J* = 8.5 Hz, 2H), 4.84 (s, 2H), 4.29 (s, 2H), 3.83 (s, 3H), 3.56 (s, 3H), 3.31 (s, 3H), 1.77 (m, 1H), 1.01 (m, 2H), 0.84 (m, 2H).

**Example 62: Preparation of 2-(4-cyclopropyl-6-methoxypyrimidin-5-yl)-5-methyl-8-(3-fluoro-4-(1-isopropyl-4-(trifluoromethyl)-1H-imidazol-2-yl)benzyl)-7,8-dihydropteridin-6(SH)-one (compound 67)**

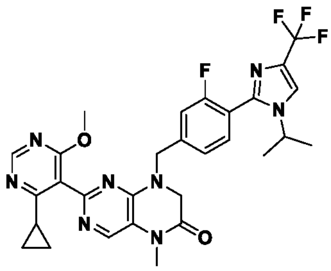

67

2-iodopropane was used instead of iodomethane in step **2** to prepare the compound **67A** by using a method similar to that for the compound **51E.** The compound **67A** was then used instead of the compound **28D** in step **4** to prepare the compound **67** by using a method similar to that in Example **26.**

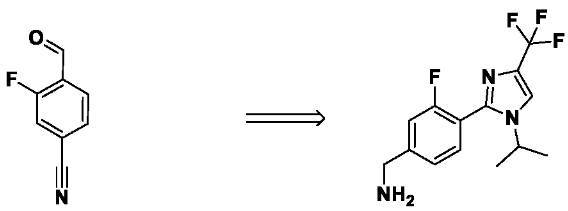

66A 67A

LC-MS: m/z (ESI): 597.2 $[M+H]^+$.

$^1$H NMR (400 MHz, DMSO-d6) δ 8.61 (s, 1H), 8.24 (s, 1H), 8.15 (s, 1H), 7.51 (t, *J* = 7.7 Hz, 1H), 7.44-7.29 (m, 2H), 4.84 (s, 2H), 4.27 (s, 2H), 4.12 (m, 1H), 3.84 (s, 3H), 3.31 (s, 3H), 1.78 (m, 1H), 1.35 (d, *J* = 6.6 Hz, 6H), 1.03-0.96 (m, 2H), 0.82 (m, 2H).

**Example 63: Preparation of 2-(4-cyclopropyl-6-methoxypyrimidin-5-yl)-5-methyl-8-(3-fluoro-4-(1-ethyl-4-(trifluoromethyl)-1H-imidazol-2-yl)benzyl)-7,8-dihydropteridin-6(SH)-one (compound 68)**

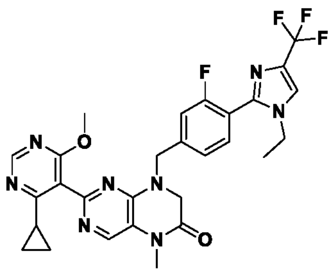

68

Iodoethane was used instead of iodomethane in step **2** to prepare the compound **68A** by using a method similar to that for the compound **51E.** The compound **68A** was then used instead of the compound **28D** in step **4** to prepare the compound **68** by using a method similar to that in Example **26.**

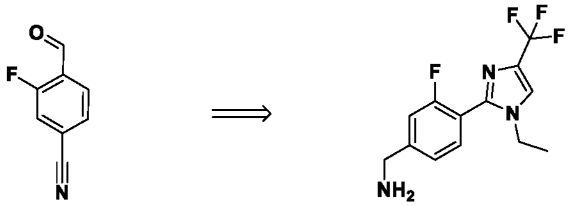

66A 68A

LC-MS: m/z (ESI): 583.2 [M+H]+.

$^1$H NMR (400 MHz, DMSO-$d_6$) δ 8.55 (s, 1H), 8.08 (s, 1H), 8.03 (s, 1H), 7.53-7.42 (m, 1H), 7.33 (d, *J* = 10.9 Hz, 1H), 7.27 (d, *J* = 8.0 Hz, 1H), 4.78 (s, 2H), 4.20 (s, 2H), 3.86-3.68 (m, 5H), 3.18-2.97 (m, 3H), 1.71 (m, 1H), 1.19 (t, *J* = 7.3 Hz, 3H), 0.93 (m, 2H), 0.82-0.70 (m, 2H).

**Example 64: Preparation of 2-(4-cyclopropyl-6-methoxypyrimidin-5-yl)-8-(3-fluoro-4-(1-methyl-4-(trifluoromethyl)-1H-imidazol-2-yl)benzyl)-5,7-dimethyl-7,8-dihydropteridin-6(SH)-one (compound 70)**

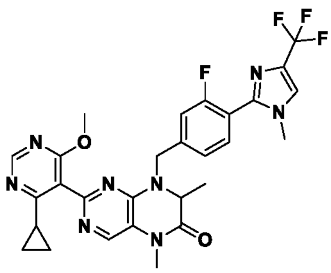

**70**

The compound **51E** was used instead of the compound **28D** in step **4,** and the compound **70A** was used instead of chloroacetyl chloride in step **6** to prepare the compound **70** by using a method similar to that in Example **26.**

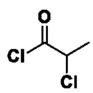

**70A**

LC-MS: m/z (ESI): 583.2 [M+H].

$^1$H NMR (400 MHz, DMSO-$d_6$) δ 8.60 (s, 1H), 8.20 (s, 1H), 7.99 (s, 1H), 7.54 (t, *J* = 7.8 Hz, 1H), 7.42-7.37 (m, 1H), 7.33 (dd, *J* = 8.6, 0.9 Hz, 1H), 5.08 (d, *J* = 17.0 Hz, 1H), 4.70 (d, *J* = 16.3 Hz, 1H), 4.46 (q, *J* = 6.8, 6.3 Hz, 1H), 3.82 (s, 3H), 3.58 (s, 3H), 3.27 (s, 3H), 1.78-1.71 (m, 1H), 1.36 (d, *J* = 6.8 Hz, 3H), 0.98 (m, 2H), 0.87-0.76 (m, 2H).

**Example 65: Preparation of 2-(4-cyclopropyl-6-methoxypyrimidin-5-yl)-8-(3-fluoro-4-(1-methyl-4-(trifluoromethyl)-1H-imidazol-2-yl)benzyl)-5-cyclopropyl-7,8-dihydropteridin-6(5H)-one (compound 71)**

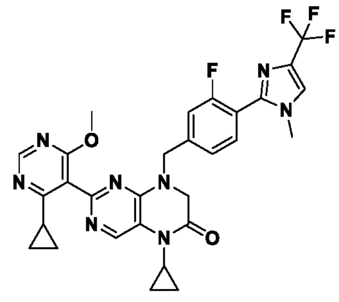

**71**

The compound **51E** was used instead of the compound **28D** in step **4** to prepare the compound **71A** by using a method similar to that in step **4** to step 7 of Example **26.** The compound **71A** was then used instead of the compound **45J** to prepare the compound **71** by using a method similar to that in Example **45.**

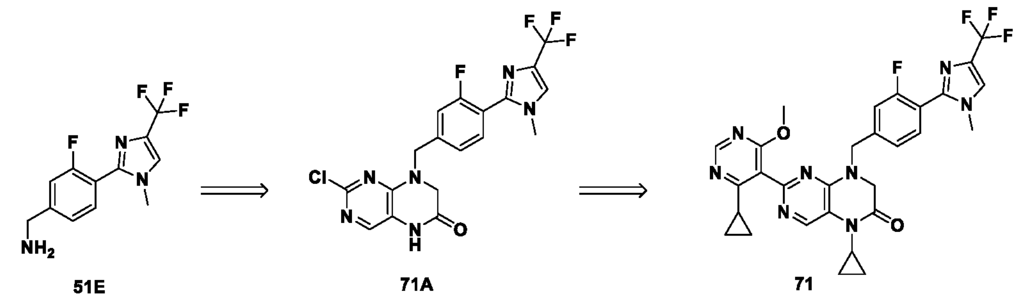

**51E** **71A** **71**

LC-MS: m/z (ESI): 595.2 $[M+H]^+$.

$^1H$ NMR (400 MHz, DMSO-$d_6$) δ 8.68 (s, 1H), 8.45 (s, 1H), 8.06 (s, 1H), 7.62 (t, *J* = 7.7 Hz, 1H), 7.46 (d, *J* = 11.1 Hz, 1H), 7.40 (dd, *J* = 7.9, 1.2 Hz, 1H), 4.88 (s, 2H), 4.24 (s, 2H), 3.91 (s, 3H), 3.67 (s, 3H), 2.76 (m, 1H), 1.86 (m, 1H), 1.21 (m, 2H), 1.07 (m, 2H), 0.91 (m, 2H), 0.83 (m, 2H).

**Example 66: Preparation of 2-(4-cyclopropyl-6-methoxypyrimidin-5-yl)-8-(4-(5-methyl-3-(trifluoromethyl)-1H-pyrazol-1-yl)benzyl)-5-cyclopropyl-7,8-dihydropteridin-6(SH)-one (compound 72)**

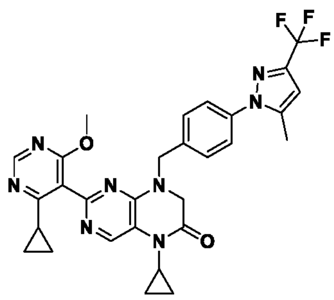

**72**

The compound **43D** was used instead of the compound **28D** in step **4** to prepare the compound **72A** by using a method similar to that in step **4** to step **7** in Example **26.** The compound **72A** was then used instead of the compound **45J** to prepare the compound **72** by using a method similar to that in Example **45.**

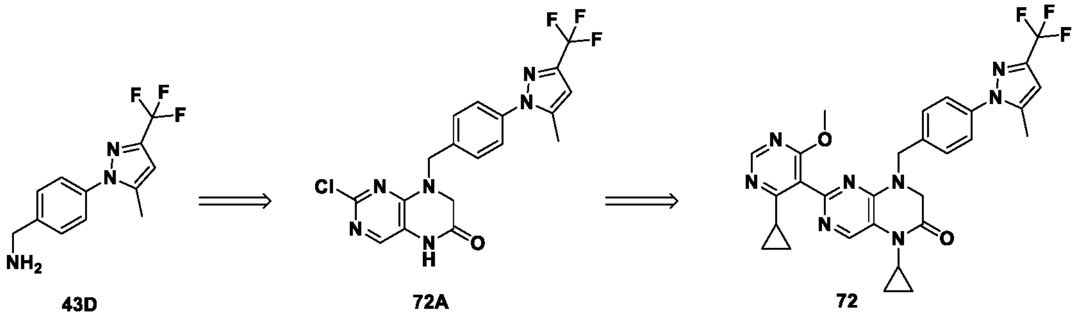

43D 72A 72

LC-MS: m/z (ESI): 577.2 [M+H].

$^1$H NMR (400 MHz, DMSO-$d_6$) δ 8.62 (s, 1H), 8.38 (s, 1H), 7.53 (m, 4H), 6.76 (s, 1H), 4.81 (s, 2H), 4.15 (s, 2H), 3.85 (s, 3H), 2.71-2.66(m, 1H) ,2.32 (s, 3H), 1.83-1.75 (m, 1H),1. 15-1. 13 (m, 2H), 1.01-0.98 (m, 2H), 0.87-0.83 (m, 2H). 0.77-0.75 (m, 2H).

**Example 67: Preparation of 2-(4-cyclopropyl-6-methoxypyrimidin-5-yl)-8-(3-fluoro-4-(1-ethyl-4-(trifluoromethyl)-1H-imidazol-2-yl)benzyl)-5-cyclopropyl-7,8-dihydropteridin-6(5H)-one (compound 73)**

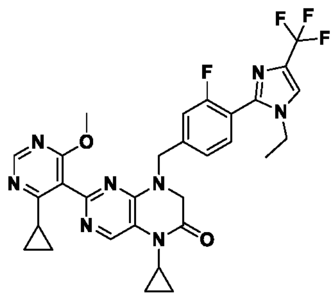

73

The compound **68A** was used instead of the compound **28D** in step **4** to prepare the compound **73B** by using a method similar to that in step **4** to step **7** of Example **26.** The compound **73B** was then used instead of the compound **45J** to prepare the compound **73** by using a method similar to that in Example **45.**

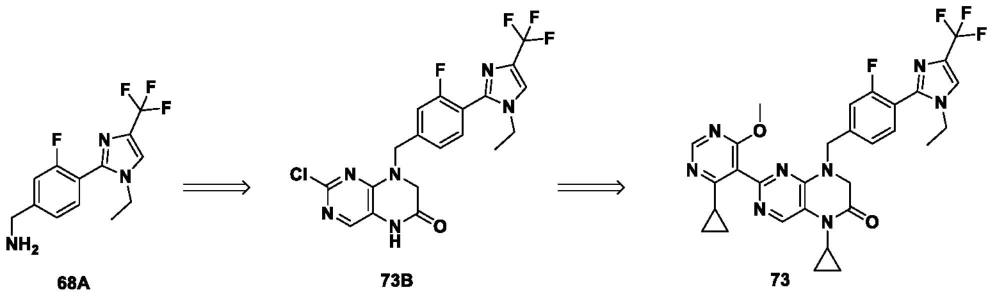

68A 73B 73

LC-MS: m/z (ESI): 609.2 [M+H]$^+$.

$^1$H NMR (400 MHz, DMSO-$d_6$) δ 8.62 (s, 1H), 8.38 (s, 1H), 8.11 (s, 1H), 7.53 (t, *J* = 7.7 Hz, 1H), 7.39 (d, *J* = 10.9 Hz, 1H), 7.33 (dd, *J* = 7.9, 1.6 Hz, 1H), 4.81 (s, 2H), 4.17 (s, 2H), 3.90-3.81 (m, 5H), 2.69 (m, 1H), 1.79 (m, 1H), 1.26 (t, *J* = 7.2 Hz, 3H), 1.15 (m, 2H), 1.00 (m, 2H), 0.84 (m, 2H), 0.76 (m, 2H).

**Example 68: Preparation of 7-(4-cyclopropyl-6-methoxypyrimidin-5-yl)-4,4-dimethyl-1-(4-(5-methyl-3-(trifluoromethyl)-1H-pyrazol-1-yl)benzyl)-1,4-dihydro-2H-pyrimido[4,5-d][1,3]oxazin-2-one (compound 74)**

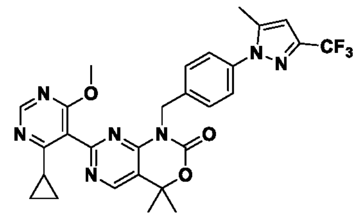

74

The compound **43D** was used instead of the compound **4B** to prepare the compound **74A** by using synthesis steps similar to those for the compound **35B.** The compound **74A** was then used instead of the compound **49A** to prepare the compound **74** by using synthesis steps similar to those for the compound **49.**

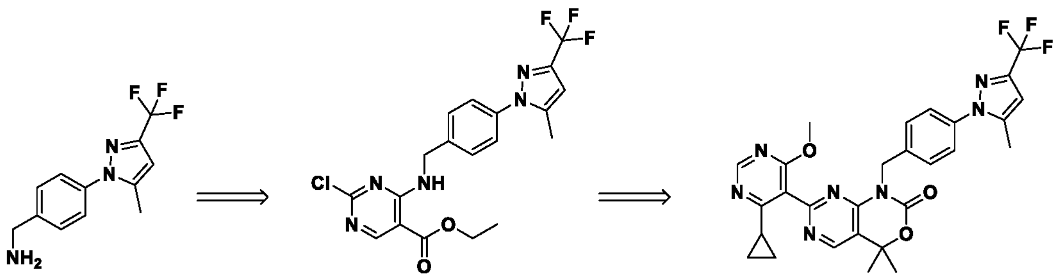

43D 74A 74

LC-MS: m/z (ESI): 566.2 [M+H].

$^1$H NMR (400 MHz, DMSO-$d_6$) δ 8.84 (s, 1H), 8.66 (s, 1H),7.52 (d, *J* = 8.0 Hz, 2H), 7.45 (d, *J* = 8.0 Hz, 2H), 6.75 (s, 1H), 5.25 (s, 2H), 3.82 (s, 3H), 2.31 (s, 3H), 1.78 (s, 6H), 1.75-1.69 (m, 1H), 1.00-0.98(m, 2H), 0.98-0.78(m, 2H).

**Example 69: Preparation of 7-(4-cyclopropyl-6-difluoromethoxypyrimidin-5-yl)-4,4-dimethyl-1-(4-(5-methyl-3-(trifluoromethyl)-1H-pyrazol-1-yl)benzyl)-1,4-dihydro-2H-pyrimido[4,5-d][1,3]oxazin-2-one (compound 75)**

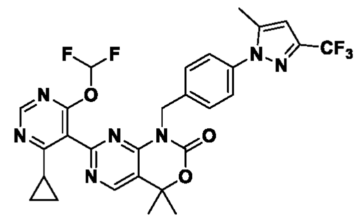

75

The compound **74A** was used instead of the compound **49A,** and the compound **57A** was used instead of the compound **2K** to prepare the compound **75** by using synthesis steps similar to those for the compound **49.**

LC-MS: m/z (ESI): 602.2 [M+H].

$^1$H NMR (400 MHz, DMSO-$d_6$) δ 8.90 (s, 1H), 8.79 (s, 1H), 7.81 (m, 1H), 7.48 (m, 4H), 6.75 (s, 1H), 5.26 (s, 2H), 2.31 (s, 3H), 1.87 (m, 1H), 1.80 (s, 6H), 1.06 (m, 2H), 0.88 (m, 2H).

**Example 70: Preparation of 7-(4-cyclopropyl-6-methoxypyrimidin-5-yl)-4,4-dimethyl-1-(3-fluoro-4-(1-ethyl-4-(trifluoromethyl)-1H-imidazol-2-yl)benzyl)-1,4-dihydro-2H-pyrimido[4,5-d][1,3]oxazin-2-one (compound 76)**

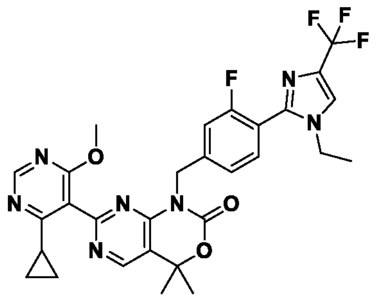

76

The compound **68A** was used instead of the compound **4B** to prepare the compound **76A** by using synthesis steps similar to those for the compound **35B.** The compound **76A** was then used instead of the compound **49A** to prepare the compound **76** by using synthesis steps similar to those for the compound **49.**

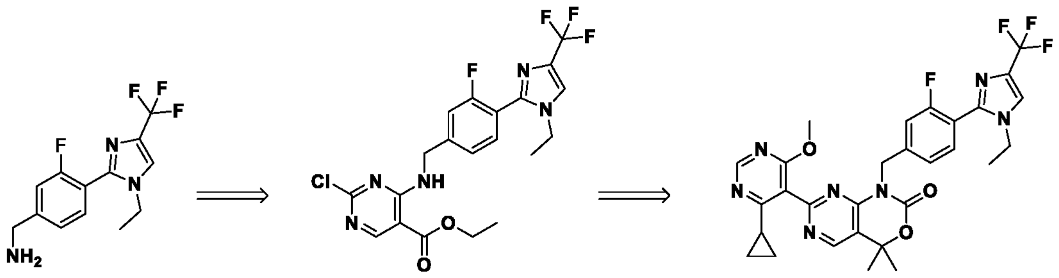

68A 76A 76

LC-MS: m/z (ESI): 598.2 [M+H].

$^1$H NMR (400 MHz, DMSO-$d_6$) δ 8.84 (s, 1H), 8.66 (s, 1H), 8.01 (s, 1H), 7.54 (m, 1H), 7.34 (m, 1H), 7.27 (m, 1H), 5.23 (s, 2H), 3.86-3.58 (m, 2H), 3.59 (s, 3H), 1.79 (s, 6H), 1.74 (m, 1H), 1.22 (t, $J$ = 7.2 Hz, 3H), 1.00 (m, 2H), 0.81 (m, 2H).

**Example 71: Preparation of 2-(4-cyclopropyl-6-methoxypyrimidin-5-yl)-8-(3-fluoro-4-(1-cyclopropyl-4-(trifluoromethyl)-1H-imidazol-2-yl)benzyl)-5-cyclopropyl-7,8-dihydropteridin-6(5H)-one (compound 77)**

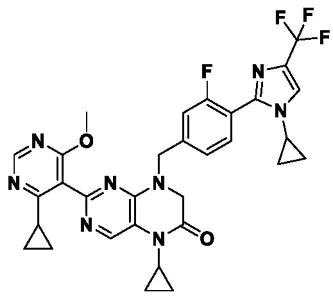

77

Iodocyclopropane was used instead of iodomethane in step **2** to prepare the compound **77A** by using a method similar to that in step **1** to step **3** of Example **48.** The compound **77A** was then used instead of the compound **28D** in step **4** to prepare the compound **77B** by using a method similar to that in step **4** to step **7** in Example **26.** The compound **77B** was then used instead of the compound **45J** to prepare the compound 77 by using a method similar to that in Example **45.**

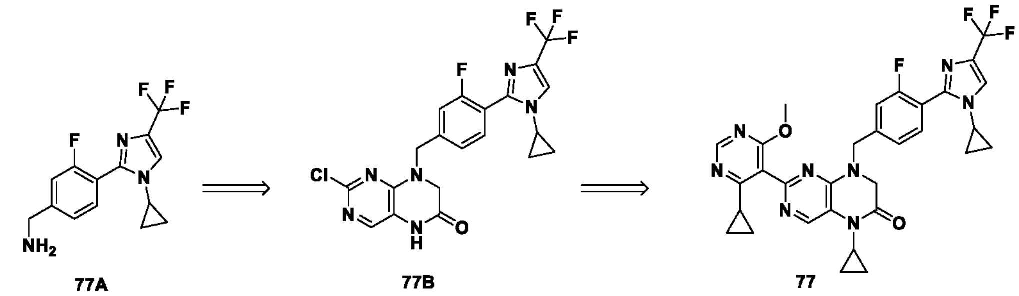

77A 77B 77

LC-MS: m/z (ESI): 621.2 $[M+H]^+$.

$^1H$ NMR (400 MHz, DMSO-$d_6$) δ 8.62 (s, 1H), 8.39 (s, 1H), 8.00 (s, 1H), 7.59 (t, *J* = 7.7 Hz, 1H), 7.38 (d, *J* = 11.0 Hz, 1H), 7.33 (d, *J* = 7.9 Hz, 1H), 4.82 (s, 2H), 4.16 (s, 2H), 3.84 (s, 3H), 3.49- 3.41 (m, 1H), 2.68-2.73 (m, 1H), 2.04-1.95 (m, 1H), 0.93-0.69 (m, 12H).

**Example 72: Preparation of 2-(4-cyclopropyl-6-methoxypyrimidin-5-yl)-8-(4-(1-methyl-4-(trifluoromethyl)-1H-imidazol-2-yl)benzyl)-5-isopropyl-7,8-dihydropteridin-6(5H)-one (compound 78)**

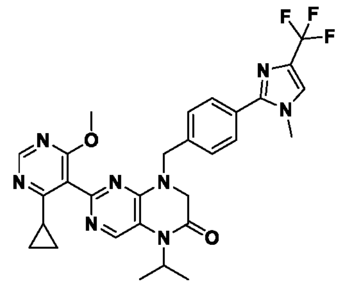

**78**

Iodomethane and sodium hydride were used instead of sodium difluorochloroacetate and potassium hydroxide in step **2,** and 2-iodopropane was used instead of iodomethane in step **8** to prepare the compound **78** by using a method similar to that in Example **26.**

LC-MS: m/z (ESI): 579.2 $[M+H]^+$.

$^1H$ NMR (400 MHz, Chloroform-*d*) δ 8.63 (s, 1H), 8.28 (s, 1H), 7.94 (s, 1H),7.61 (d, *J* = 8.1 Hz, 2H), 7.46 (d, *J* = 8.1 Hz, 2H), 4.90 (m, 3H), 4.02 (s, 2H), 3.97 (s, 3H), 3.77 (s, 3H), 1.87 (m, 1H), 1.59 (m, 6H), 1.26-1.19 (m, 2H), 0.92 (m, 2H).

**Example 73: Preparation of 2-(4-cyclopropyl-6-methoxypyrimidin-5-yl)-5-methyl-8-((7-methyl-2-(trifluoromethyl)-6,7-dihydro-5H-benzo[c]imidazo[1,2-a]azepin-9-yl)methyl)-7,8-dihydropteridin-6(5H)-one (compound 79)**

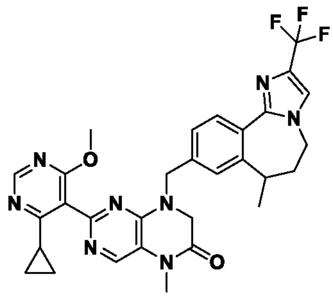

**79**

Isopropenylboronic acid pinacol ester was used instead of vinylboronic acid pinacol cyclic ester in step **3** to prepare the compound **79** by using a method similar to that in Example **42.**
LC-MS: m/z (ESI): 591.2 $[M+H]^+$.
$^1H$ NMR (400 MHz, DMSO-$d_6$) δ 8.62 (s, 1H), 8.14 (s, 1H), 7.98 (s, 1H), 7.59 (d, *J* = 7.7 Hz, 1H), 7.40-7.31 (m, 2H), 4.83 (s, 2H), 4.25 (s, 2H), 4.21-4.13 (m, 1H), 3.84 (s, 3H), 3.68 (s, 1H), 3.31 (s, 3H), 2.76 (m, 1H), 1.78 (m, 2H), 1.45 (s, 1H), 1.12 (d, *J* = 6.9 Hz, 3H), 1.00 (m, 2H), 0.84 (m, 2H).

Example 74: Preparation of 7-(4-cyclopropyl-6-methoxypyrimidin-5-yl)-1-(4-(1-methyl-4-**(trifluoromethyl)-1H-imidazol-2-yl)benzyl)-1,4-dihydro-2H-pyrimido [4,5-d] [1,3] oxazin-2-one (compound 80)**

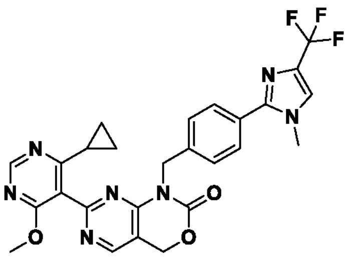

**80**

The synthesis route is as follows:

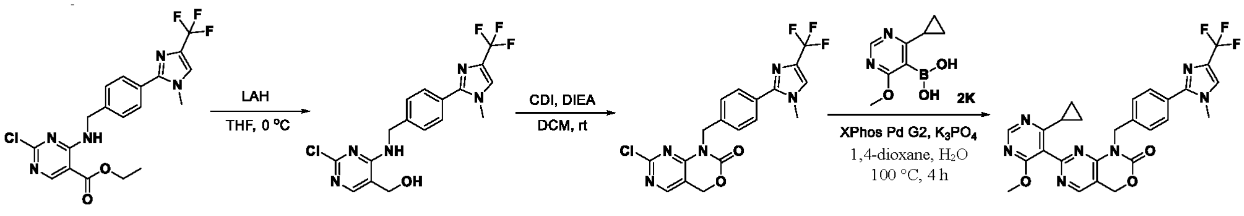


**Step 1: (2-chloro-4-((4-(1-methyl-4-(trifluoromethyl)-1H-imidazol-2-yl)benzyl)amino)pyrimidin-5-yl)methanol (80A)**

The **intermediate 35B** (0.44 g, 1.0 mmol) was dissolved in tetrahydrofuran (10 mL), after the resulting solution was cooled to -78 °C, lithium aluminum hydride (1.0 mL, 1.0 mmol, 1 M tetrahydrofuran solution) was added dropwise under stirring, and the resulting reaction solution was slowly heated to 0 °C for reaction for half an hour. Water (10 mL) was added to quench the reaction, then the resulting solution was extracted with ethyl acetate (10 mL×3), the organic phases were combined, dried over anhydrous sodium sulfate, and filtered, and the resulting residue was purified by column chromatography (with petroleum ether containing 20-100% ethyl acetate as the elution phase) to obtain a compound **80A** (0.16 g, 0.4 mmol, yield: 40%). MS m/z (ESI): 398.1 $[M+H]^+$; The compound **80A** was used instead of the compound **49B** to prepare the compound **80** by using a synthesis method similar to that in Example **46.**
MS m/z (ESI): 538.2 $[M+H]^+$.
$^1H$ NMR (400 MHz, DMSO-d6) δ 8.71 (s, 1H), 8.65 (s, 1H), 7.92 (s, 1H), 7.66-7.63 (m, 2H), 7.44 (d, *J*= 8.2 Hz, 2H), 5.58 (m, 2H), 5.21 (s, 2H), 3.81 (s, 3H), 3.75 (s, 3H), 1.68 (m, 1H), 0.99 (m, 2H), 0.78 (m, 2H).

**Example 75: Preparation of 7-(4-cyclopropyl-6-methoxypyrimidin-5-yl)-4-methyl-1-(4-(1-methyl-4-(trifluoromethyl)-1H-imidazol-2-yl)benzyl)-1,4-dihydro-2H-pyrimido [4,5-d][1,3]oxazin-2-one (compound 81)**

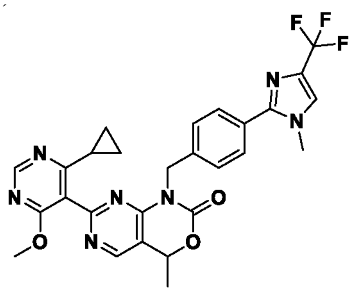

81

The synthesis route is as follows:

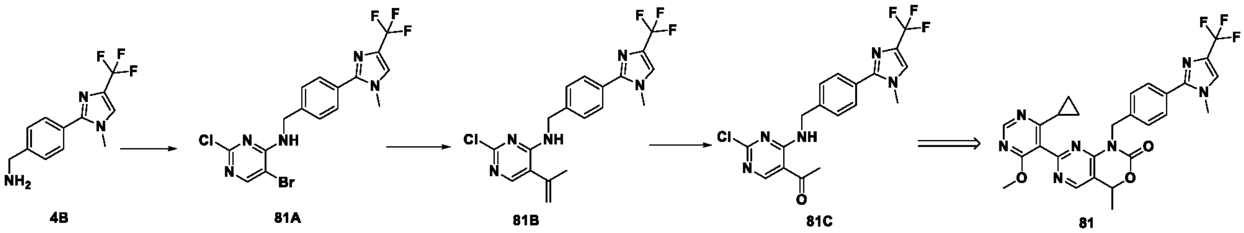

4B 81A 81B 81C 81

The compound **4B** was used instead of the compound **51E** to prepare a compound **81A** by using a synthesis method similar to that for the compound **51G** in Example **48.** The compound **81A** was used instead of the compound **51G** to prepare a compound **81B** by using a synthesis method similar to that for the compound **58C** in Example **55.** The compound **81B** was then used instead of the compound **58C** to prepare a compound **81C** by using a synthesis method similar to that for the compound **59A** in Example **56.** The compound **81C** was used instead of the compound **35B** to prepare the compound **81** by using a synthesis route and a synthesis method similar to those in Example **74.**

MS m/z (ESI): 552.2 $[M+H]^+$.

$^1H$ NMR (400 MHz, DMSO-d6) δ 8.75 (s, 1H), 8.66 (s, 1H), 7.93 (s, 1H), 7.64 (d, *J* = 8.2 Hz, 2H), 7.42 (d, *J* = 8.1 Hz, 2H), 5.86 (m, 1H), 5.21 (s, 2H), 3.81 (s, 3H), 3.75 (s, 3H), 1.75 (d, *J* = 6.5 Hz, 3H), 1.68 (m, 1H), 0.98 (m, 2H), 0.78 (m, 2H).

**Example 76: Preparation of 2-(4-cyclopropyl-6-methoxypyrimidin-5-yl)-5-methyl-8-((2-(trifluoromethyl)-6,7-dihydro-5H-benzo[c]imidazo[1,2-a]azepin-9-yl)methyl)-7,8-dihydropteridine-6(5H)-thione (compound 82)**

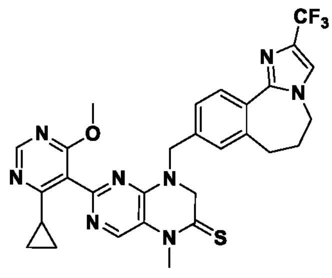

82

The synthesis route is as follows:

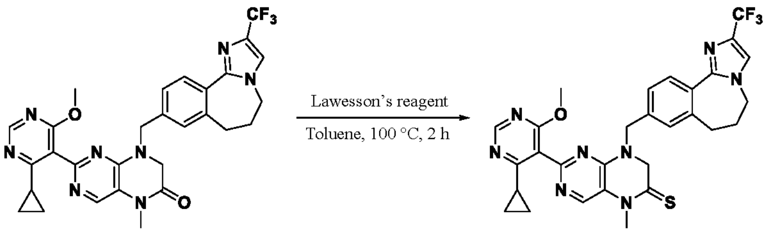


45 82

Lawesson's reagent 2,4-bis(*p*-methoxyphenyl)-1,3-dithia-diphosphetane-2,4 sulfide (14.0 mg, 34.7 μmol) was added to a solution of compound **45** (10.0 mg, 17.3 μmol) in toluene (1 mL). The resulting mixture was stirred at 100 °C for reaction for 2 h and then concentrated under vacuum. The resulting crude was purified by preparative chromatography (Waters Xbridge C18, eluted with 20-80% aqueous acetonitrile solution) to obtain a compound 82 (3.0 mg, yield: 29%).

MS m/z (ESI): 593.1 $[M+H]^+$.

$^1H$ NMR (400 MHz, $CDCl_3$) δ 8.68 (s, 1H), 8.30 (s, 1H), 7.82 (d, *J* = 7.9 Hz, 1H), 7.52 (s, 1H), 7.38 (d, *J* = 1.2 Hz, 1H), 7.00 (s, 1H), 4.94 (s, 2H), 4.68 (s, 2H), 4.03 (s, 3H), 4.00 - 3.90 (m, 5H), 2.78 - 2.70 (m, 2H), 2.42 - 2.33 (m, 2H), 1.91 (s, 1H), 1.31 (s, 2H), 0.96 (s, 2H).

**Example 77: Preparation of 2-(4-cyclopropyl-6-methoxypyrimidin-5-yl)-8-(3-fluoro-4-(1-methyl-4-(trifluoromethyl)-1H-imidazol-2-yl)benzyl)-5-methyl-7,8-dihydropteridine-6(SH)-thione (compound 83)**

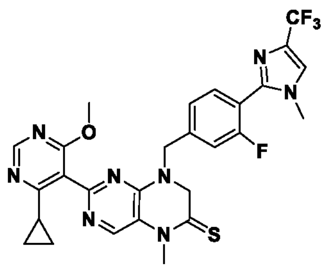

83

The synthesis route is as follows:

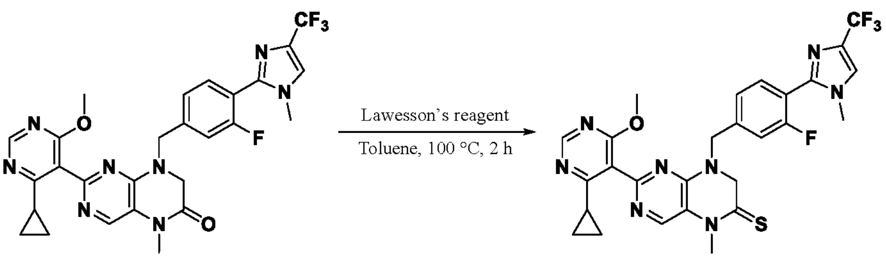


65 83

Lawesson's reagent (20.9 mg, 51.8 μmol) was added to a solution of compound **65** (14.7 mg, 25.9 μmol) in toluene (1 mL). The resulting mixture was stirred at 100 °C for reaction for 2 h and then concentrated under vacuum. The resulting crude product was purified by preparative chromatography (Waters Xbridge C18, eluted with 20-80% aqueous acetonitrile solution) to obtain a compound **83** (5.0 mg, yield: 33%).

MS m/z (ESI): 585.1 $[M+H]^+$.

$^1H$ NMR (400 MHz, $CDCl_3$) δ 8.57 (s, 1H), 8.31 (s, 1H), 7.76 (s, 1H), 7.54 (t, J = 7.5 Hz, 1H), 7.38 (d, J = 9.1 Hz, 2H), 4.94 (s, 2H), 4.69 (s, 2H), 3.93 (s, 6H), 3.65 (s, 3H), 1.84 (m, 1H), 1.14 - 1.11 (m, 2H), 0.92 - 0.89 (m, 2H).

**Example 78: Preparation of 7-(4-cyclopropyl-6-methoxypyrimidin-5-yl)-1-(3-fluoro-4-(1-methyl-4-(trifluoromethyl)-1H-imidazol-2-yl)benzyl)-2,4-dimethyl-1,4-dihydropyrimido [5,4-e][1,2,4]triazin-3(2H)-one (compound 84)**

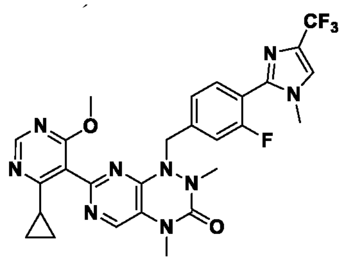

84

The synthesis route is as follows:

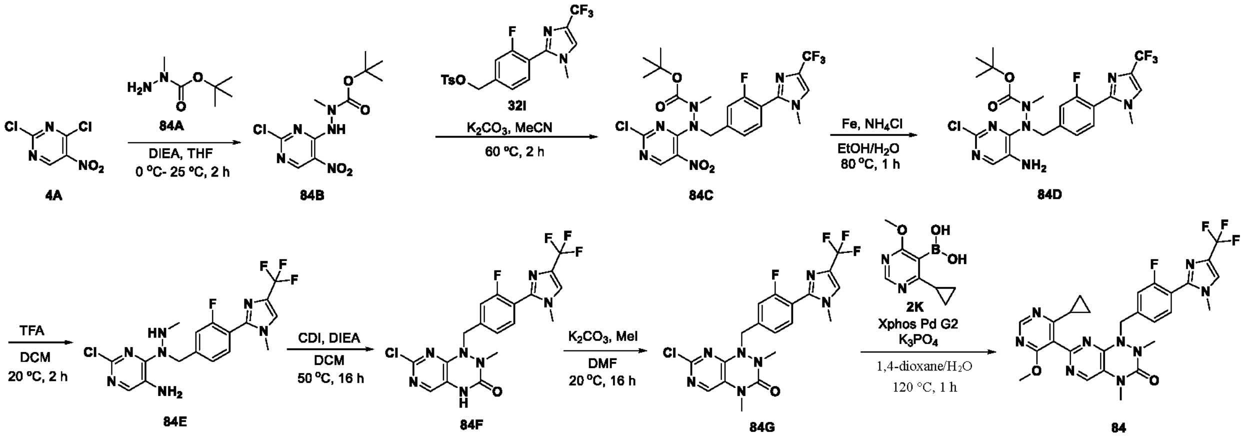


**Step 1: 2-(2-chloro-5-nitropyrimidin-4-yl)-1-methylhydrazine-1-carboxylic acid *tert-butyl* ester (84B)**

The compound **4A** (0.35 g, 1.8 mmol) and *N,N*-diisopropylethylamine (0.47 g, 3.6 mmol) were dissolved in tetrahydrofuran (20 mL), and a compound **84A** (0.26 g, 1.8mmol) was added slowly at 0 °C. The resulting mixture was slowly heated to room temperature and stirred for reaction for 2 h. The resulting reaction solution was distilled under vacuum to remove the solvent. The resulting residue was purified by silica gel column chromatography (petroleum ether:ethyl acetate = 4:1-2:1) to obtain a compound **84B** (0.42 g, yield: 77%). m/z (ESI): 304.1 $[M+H]^+$.

**Step 2: 2-(2-chloro-5-nitropyrimidin-4-yl)-2-(3-fluoro-4-(1-methyl-4-(trifluoromethyl)-1H-imidazol-2-yl)benzyl)-1-methylhydrazine-1-carboxylic acid *tert-butyl* ester (84C)**

The compound **84B** (0.42 g, 1.4 mmol) and potassium carbonate (0.38 g, 2.8 mmol) were dissolved in acetonitrile (5 mL), and a compound **32I** (0.64g, 1.5 mmol) was added. The resulting mixture was stirred at 60°C for reaction for 2 h. The resulting reaction solution was distilled under vacuum to remove the solvent. The resulting residue was purified by silica gel column chromatography (petroleum ether:ethyl acetate = 4:1-1:1) to obtain a compound **84C** (0.28 g, yield: 36%). m/z (ESI): 560.1 $[M+H]^+$.

**Step 3: 2-(5-amino-2-chloropyrimidin-4-yl)-2-(3-fluoro-4-(1-methyl-4-(trifluoromethyl)-1H-imidazol-2-yl)benzyl)-1-methylhydrazine-1-carboxylic acid *tert-butyl* ester (84D)**

The compound **84C** (80 mg, 142.9 μmol), iron powder (56 mg, 1.0 mmol), and ammonium chloride (22.1 mg, 428.7 μmol) were mixed in water (1 mL) and ethanol (2 mL). The resulting mixture was stirred at 80°C for reaction for 1 h. The reaction solution was concentrated under vacuum, and 20 mL of ethyl acetate and 2 g of anhydrous magnesium sulfate were added to the residue. The resulting mixture was stirred for 10 min, and filtered, and the filtrate was concentrated to obtain a crude compound **84D** (70 mg, crude product). m/z (ESI): 530.2 $[M+H]^+$.

**Step 4: 2-chloro-4-(1-(3-fluoro-4-(1-methyl-4-(trifluoromethyl)-1H-imidazol-2-yl)benzyl)-2-methylhydrazino)pyrimidin-5-amine (84E)**

The compound **84D** (70 mg, 125 μmol) was dissolved in 2 mL of dichloromethane, and 0.5 mL of trifluoroacetic acid was added at 0 °C. The reaction solution was stirred at 20 °C for reaction for 2 h, then the resulting solution was concentrated under vacuum, and 10 mL of ethyl acetate was added to the resulting residue, and the resulting mixture was then concentrated under vacuum again to obtain a crude compound **84E** (72 mg, crude product). LC-MS: m/z (ESI): 430.1$[M+H]^+$.

**Step 5: 7-chloro-1-(3-fluoro-4-(1-methyl-4-(trifluoromethyl)-1H-imidazol-2-yl)benzyl)-2-methyl-1,4-dihydropyrimido[5,4-e][1,2,4]triazin-3(2H)-one (84F)**

The crude compound **84E** (72.0 mg, about 125 μmol) and *N,N*-diisopropylethylamine (129 mg, 1.0 mmol) were dissolved in 3 mL of dichloromethane, and *N'N*-carbonyldiimidazole (37.7 mg, 0.23 mmol) was added. The resulting mixture was stirred at 50 °C for reaction for 16 h. The resulting crude product was concentrated under vacuum, and the concentrate was purified by reversed-phase C18 column chromatography (with 5-95% aqueous acetonitrile solution as the elution phase) to obtain a compound **84F** (40 mg, yield: 70%). m/z (ESI): 456.1 $[M+H]^+$.

**Step 6: 7-chloro-1-(3-fluoro-4-(1-methyl-4-(trifluoromethyl)-1H-imidazol-2-yl)benzyl)-2,4-dimethyl-1,4-dihydropyrimido[5,4-e][1,2,4]triazin-3(2H)-one (84G)**

The compound **84F** (40.0 mg, 88 μmol) and potassium carbonate (45.5 mg, 329 μmol) were added to 3 mL of *N,N*-dimethylformamide, and iodomethane (31.1 mg, 219.4 μmol) was added. The resulting mixture was stirred at 20°C for reaction for 16 h. The reaction solution was quenched with water and extracted with ethyl acetate (10 mL × 3), and the organic phases were combined, then washed with saturated brine, dried over anhydrous sodium sulfate, filtered, and concentrated to obtain a compound **84G** (50 mg, crude product). MS m/z (ESI): 470.1 [M+H]+.

**Step 7: 7-(4-cyclopropyl-6-methoxypyrimidin-5-yl)-1-(3-fluoro-4-(1-methyl-4-(trifluoromethyl)-1H-imidazol-2-yl)benzyl)-2,4-dimethyl-1,4-dihydropyrimido[5,4-e][1,2,4]triazin-3(2H)-one (84)**

The crude compound **84G** (50 mg, about 88 μmol), the compound **2K** (18.6 mg, 96 μmol), XPhos Pd G2 (15.1 mg, 19.2 μmol), and potassium phosphate (40.7 mg, 191.6 μmol) were mixed in water (0.4 mL) and 1,4-dioxane (2 mL). The resulting mixture was stirred for microwave reaction at 120 °C for 1 h under the protection of nitrogen. The reaction solution was concentrated, and the resulting residue was purified by preparative chromatography (Waters Xbridge C18, with 10-70% aqueous acetonitrile solution as the elution phase) and lyophilized to obtain a compound **84** (8.0 mg, yield: 16%).

MS m/z (ESI): 579.2 $[M+H]^+$.

$^1H$ NMR (400 MHz, Chloroform-d) δ 8.61 (s, 1H), 7.93 (s, 1H), 7.68-7.64 (m, 2H), 7.47-7.40 (m, 2H), 4.88 (s, 2H), 3.87 (s, 3H), 3.85 (s, 3H), 3.76 (s, 3H), 3.56 (s, 3H), 1.77 (m, 1H), 0.99 (m, 2H), 0.83 (m, 2H).

**Example 79: Preparation of 2'-(4-cyclopropyl-6-methoxypyrimidin-5-yl)-5'-methyl-8'-(4-(1-methyl-4-(trifluoromethyl)-1H-imidazol-2-yl)benzyl)-5',8'-dihydro-6' H-spiro [cyclopropane-1,7'-pteridin]-6'-one (compound 85)**

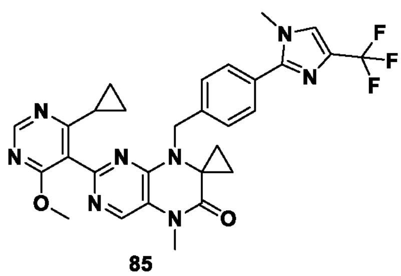

**85**

The synthesis route is as follows:

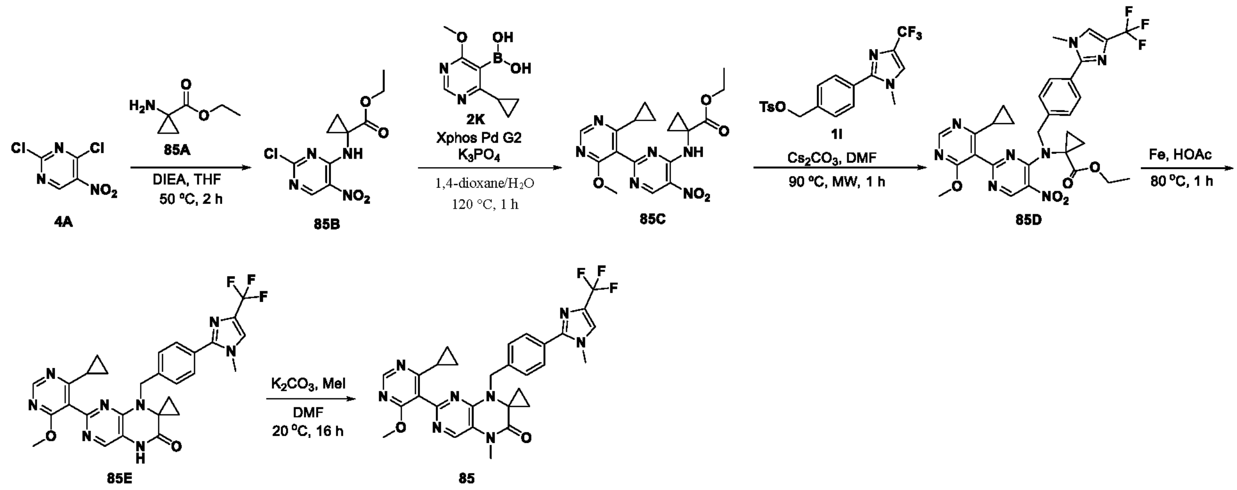


**Step 1: ethyl 1-((2-chloro-5-nitropyrimidin-4-yl)amino)cyclopropane-1-carboxylate (85B)**

The compound **4A** (0.35 g, 1.8 mmol) and *N,N*-diisopropylethylamine (0.47 g, 3.6 mmol) were dissolved in tetrahydrofuran (20 mL), and the compound **85A** (0.26 g, 1.8mmol) was added slowly at 0 °C. The resulting mixture was stirred at 50°C for reaction for 2 h. The resulting reaction solution was distilled under vacuum to remove the solvent. The resulting residue was purified by silica gel column chromatography (petroleum ether:ethyl acetate = 4:1-2:1) to obtain a compound **85B** (0.41 g, yield: 79%). m/z (ESI): 287.0 $[M+H]^+$.

**Step 2: ethyl 1-((4'-cyclopropyl-6'-methoxy-5-nitro-[2,5'-bipyrimidin]-4-yl)amino)cyclopropane-1-carboxylate (85C)**

The compound **85B** (210 mg, 0.73 mmol), the compound **2K** (283 mg, 1.5 mmol), XPhos Pd G2 (60.4 mg, 77 μmol), and potassium phosphate (400 mg, 1.9 mmol) were mixed in water (1 mL) and 1,4-dioxane (10 mL). The resulting mixture was stirred for microwave reaction at 120 °C for 1 h under the protection of nitrogen. The reaction solution was concentrated, and the resulting residue was purified by reversed-phase C18 column chromatography (with 10-70% aqueous acetonitrile solution as the elution phase) and lyophilized to obtain a compound **85C** (130 mg, yield: 44%). m/z (ESI): 401.1 $[M+H]^+$.

**Step 3: ethyl 1-((4'-cyclopropyl-6'-methoxy-5-nitro-[2,5'-bipyrimidin]-4-yl)(4-(1-methyl-4-(trifluoromethyl)-1H-imidazol-2-yl)benzyl)amino)cyclopropane-1-carboxylate (85D)**

The compound **85C** (130 mg, 0.325 mmol), cesium carbonate (212 mg, 0.65 mol), and the compound **1l** (200 mg, 0.49 mmol) were added to *N'N*-dimethylformamide (5 mL). The resulting mixture was stirred at 90 °C for microwave reaction for 1 h. The reaction solution was added to 30 mL of water, and the resulting solution was extracted with ethyl acetate (30 mL × 3). The organic phases were combined, then washed once with 30 mL of saturated brine, dried over anhydrous sodium sulfate, filtered, and concentrated under vacuum, and the resulting crude product was purified by reversed-phase C18 column chromatography (with 10-80% aqueous acetonitrile solution as the elution phase) and lyophilized to obtain a compound **85D** (50 mg, yield: 24%). m/z (ESI): 639.2 $[M+H]^+$.

**Step 4: 2'-(4-cyclopropyl-6-methoxypyrimidin-5-yl)-8'-(4-(1-methyl-4-(trifluoromethyl)-1H-imidazol-2-yl)benzyl)-5',8'-dihydro-6'H-spiro[cyclopropane-1,7'-pteridine]-6'-one (85E)**

The compound **85D** (45 mg, 70.5 μmol) and iron powder (39 mg, 704 μmol) were added to acetic acid (5 mL), and the resulting mixture was stirred at 80 °C for reaction for 1 h. The reaction solution was filtered while hot, the filter cake was washed with ethanol (10 mL × 3), and the filtrates were combined and concentrated under vacuum. To the resulting residue, 10 mL of water was added, and the resulting solution was extracted with ethyl acetate (30 mL × 2). The organic phases were combined, then washed once with 30 mL of saturated brine, dried over anhydrous sodium sulfate, filtered, and concentrated under vacuum, and the resulting crude product was purified by reversed-phase C18 column chromatography (with 10-80% aqueous acetonitrile solution as the elution phase) and lyophilized to obtain a compound **85E** (31 mg, yield: 78%). m/z (ESI): 563.2 $[M+H]^+$.

**Step 5: 2'-(4-cyclopropyl-6-methoxypyrimidin-5-yl)-5'-methyl-8'-(4-(1-methyl-4-(trifluoromethyl)-1H-imidazol-2-yl)benzyl)-5',8'-dihydro-6'H-spiro[cyclopropane-1,7'-pteridine]-6'-one (85)**

The compound **85E(26** mg, 46 μmol) and potassium carbonate (45.5 mg, 329 μmol) were added to *N,N*-dimethylformamide (3 mL), and then iodomethane (31.1 mg, 219.4 μmol) was added. The resulting mixture was stirred at 20°C for reaction for 16 h. The reaction solution was filtered, the filtrate was concentrated, and the resulting residue was purified by preparative chromatography (Waters Xbridge C18, with 10-70% aqueous acetonitrile solution as the elution phase) and lyophilized to obtain a compound **85** (14 mg, yield: 53%).

LC-MS: m/z (ESI): 577.2 $[M+H]^+$.

$^1H$ NMR (400 MHz, DMSO-d6) δ 8.58 (s, 1H), 8.18 (s, 1H), 7.92 (s, 1H), 7.65 (d, J = 8.3 Hz, 2H), 7.40 (d, J = 8.0 Hz, 2H), 4.72 (s, 2H), 3.79 (s, 3H), 3.75 (s, 3H), 3.35 (s, 3H), 1.74 (m, 1H), 1.34 (m, 2H), 1.27 (m, 2H), 0.94 (m, 2H), 0.74 (m, 2H).

**Example 80: Preparation of 2'-(4-cyclopropyl-6-methoxypyrimidin-5-yl)-5'-methyl-8'-(3-fluoro-4-(1-methyl-4-(trifluoromethyl)-1H-imidazol-2-yl)benzyl)-5',8'-dihydro-6'H-spiro[cyclopropane-1,7'-pteridin]-6'-one (compound 86)**

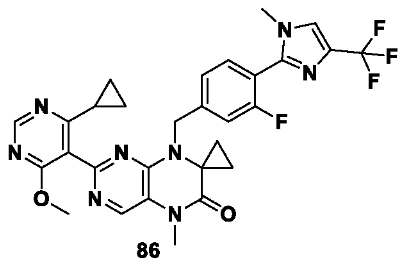

**86**

The compound **32I** was used instead of the compound **1I** in step 3 to prepare the compound **86** by using a route and steps similar to those in Example **79.**

LC-MS: m/z (ESI): 595.2 $[M+H]^+$.

$^1H$ NMR (400 MHz, DMSO-$d_6$) δ 8.59 (s, 1H), 8.18 (s, 1H), 7.99 (s, 1H), 7.54 (t, *J* = 7.8 Hz, 1H), 7.37-7.30 (m, 1H), 7.26 (dd, *J* = 8.0, 1.6 Hz, 1H), 4.72 (s, 2H), 3.79 (s, 3H), 3.58 (s, 3H), 3.34 (s, 3H), 1.74 (m, 1H), 1.37 (m, 2H), 1.28 (m, 2H), 0.95 (m, 2H), 0.77 (m, 2H).

**Example 81: Preparation of 2-(4-cyclopropyl-6-methoxypyrimidin-5-yl)-8-(4-(1-methyl-4-(trifluorom ethyl)- 1H-imidazol-2-yl)benzyl)-5-(2,2,2-trifluoroethyl)-5,6,7,8-tetrahydropteridine (compound 87)**

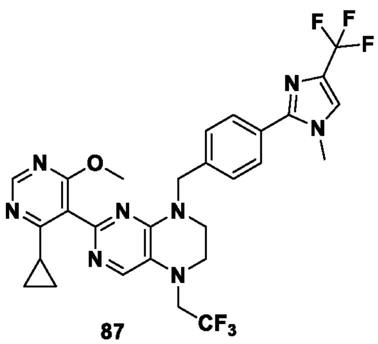

**87**

The synthesis route is as follows:

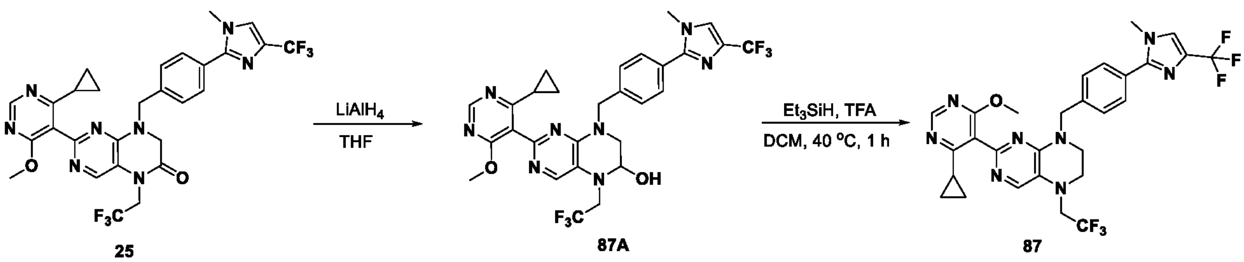

25 87A 87

**Step 1: 2-(4-cyclopropyl-6-methoxypyrimidin-5-yl)-8-(4-(1-methyl-4-(trifluoromethyl)-1H-imidazol-2-yl)benzyl)-5-(2,2,2-trifluoroethyl)-5,6,7,8-tetrahydropteridin-6-ol (87A)**

The compound **25** (25.0 mg, 40.4 μmol) was added to tetrahydrofuran (5 mL) at room temperature, and lithium aluminum hydride (7.7 mg, 202.1 μmol) was added in portions at 0 °C. The resulting mixture was allowed to react at 0 °C for 1 h, and then the reaction solution was restored slowly to room temperature for reaction for 1 h. The reaction solution was cooled to 0 °C and quenched with ethyl acetate, stirred at room temperature for 1 h, and filtered through diatomite, then the filter cake was washed with ethyl acetate, and the organic phases were combined and spin-dried to obtain a crude compound **87A** (10 mg, yield: 40%). m/z (ESI): 621.2 [M+H]$^+$.

**Step 2: 2-(4-cyclopropyl-6-methoxypyrimidin-5-yl)-8-(4-(1-methyl-4-(trifluoromethyl)-1H-imidazol-2-yl)benzyl)-5-(2,2,2-trifluoroethyl)-5,6,7,8-tetrahydropteridine (compound 87)**

The compound **87A** (5.0 mg, 8.1 μmol) was added to dichloromethane (5 mL) at room temperature, and then triethylsilane (1.4 mg, 12.1 μmol) and trifluoroacetic acid (9.2 mg, 80.6 μmol) were added. The reaction solution was heated to 40 °C and stirred for reaction for 1 h, then the reaction solution was concentrated, and the resulting residue was purified by preparative chromatography (Waters Xbridge C18, with 10-70% aqueous acetonitrile solution as the elution phase) to obtain a compound **87** (3 mg, yield: 62%).

LC-MS: m/z (ESI): 605.2 [M+H]$^+$.

$^1$H NMR (400 MHz, DMSO-d6) δ 8.57 (s, 1H), 7.94 - 7.90 (m, 2H), 7.67 (d, *J* = 8.3 Hz, 2H), 7.41 (d, *J* = 8.3 Hz, 2H), 4.86 (s, 2H), 4.25 (q, *J* = 9.6 Hz, 2H), 3.83 (s, 3H), 3.76 (s, 3H), 3.56 - 3.51 (m, 2H), 3.50 - 3.45 (m, 2H), 1.77 (m, 1H), 0.96 (m, 2H), 0.81 (m, 2H).

**Example 82: Preparation of 2-(4-cyclopropyl-6-methoxypyrimidin-5-yl)-6,6-difluoro-8-(4-(1-methyl-4-(trifluoromethyl)-1H-imidazol-2-yl)benzyl)-7,8-dihydro-6H-pyrimido[5,4-b][1,4]oxazine (compound 88)**

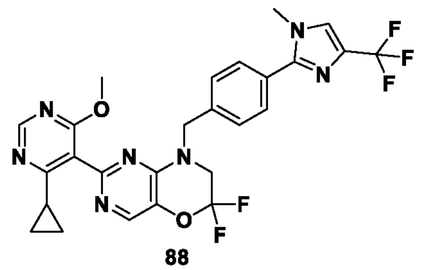
88

The compound **88A** was used instead of 1,2-dibromoethane in step 2, and the compound **2K** was used instead of the compound **1K** in step **8** to prepare the compound **88** by using a route and steps similar to those in Example **1.**

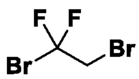
88A

LC-MS: m/z (ESI): 560.5 [M+H]$^+$.

$^1$H NMR (400 MHz, DMSO-*d6*) δ 8.63 (s, 1H), 8.36 (s, 1H), 7.94 (s, 1H), 7.69 (d, *J* = 8.4 Hz, 2H), 7.46 (d, *J* =

8.0 Hz, 2H), 4.90 (s, 2H), 4.19 (t, *J* = 6.6 Hz, 2H), 3.85 (s, 3H), 3.76(s,3H) ,1.74-1.69 (m, 1H), 1.01-0.98 (m, 2H), 0.87-0.82 (m, 2H).

**Test Example 1: *In vitro* activity assay for USP1**

**Experimental instruments:**

| Name of instrument | Manufacturer | Model |
|---|---|---|
| Oscillator | Boxun | BSD-YX3400 |
| Plate reader | PerkinElmer | Envision |
| Centrifugal machine | Eppendorf | Eppendorf Mixmate |
| Compound dilution and sample loading instrument | PerkinElmer | Echo |

**Experimental materials:**

USP1 (Recombinant Human His6-USP1/His6-UAF1 Complex Protein, CF) used in the experiment was purchased from R&D, with Cat. No. of E-568-050. A complex of USP1 with 6 HIS-tags at the N-terminus and UAF1 with 6 HIS-tags at the N-terminus was expressed by a eukaryotic baculovirus expression system. Then the expressed complex was purified by affinity chromatography based on a nickel column to obtain a product with a purity higher than 80% and a concentration of 1 mg/mL, then the product was subpackaged, and stored at -80 °C.

The assay kit (Ub-CHOP2-Reporter Deubiquitination Assay Kit) was purchased from Lifesensors, with Cat. No. of PR1101. The assay kit was subpackaged and stored at -80 °C. The kit contains a ubiquitinated reporter enzyme that, when deubiquitinated by USP1/UAF1, generates activity, so that after a substrate is catalyzed, the substrate is excited by 485 nm laser to produce a 531 nm emitted light signal.

Information on other reagents and consumables required for the experiment is as follows:

| Reagent | Brand | Cat. No. |
|---|---|---|
| CHAPS | Sangon | A600110-0001 |
| 1M Tris-HCl Solution, pH 8.0 | Sangon | B548127 |
| Calcium chloride dihydrate | Sangon | A501331 |
| β-mercaptoethanol | sigmaaldrich | M3148-100ml |
| 96-well plate | Thermofisher | 249952 |
| Black 384-well plate | Perkinelmer | 6007270 |

**Experimental method:**

Each compound to be tested was dissolved with DMSO to 10 mM. The compound and pure DMSO (with a total volume of 50 nL) were loaded to each well of the 384-well plate by using an ECHO instrument to obtain gradient-diluted sample concentrations at different ratios. The enzyme was diluted with a freshly prepared reaction solution (20 mM Tris-HCl (pH 8.0), 2 mM $CaCl_2$, 2 mM β-mercaptoethanol, 0.05% CHAPS, and $ddH_2O$). To each well, 5 μL of diluted enzyme reaction solution was added, then the plate was centrifuged and oscillated to mix the enzyme and the compound, and the mixture was centrifuged again and placed on ice. The kit reporter system and the substrate were diluted with the reaction solution, then 5 μL of diluted liquid was added to each well, and mixed by centrifugation. The mixture was incubated at room temperature for 0.5 h. An Envision plate reader (PerkinElmer, excitation wavelength: 485 nm, emission wavelength: 530 nm) was used to measure the fluorescence signal in each well. The inhibitory activity ($IC_{50}$ values) of the compound on enzyme activity was calculated by using a four-parameter Logistic Model. In the following equation, x represents the concentration of the compound in a logarithmic form; and F(x) represents an effect value (inhibition rate of enzyme activity at the concentration): F(x) =(A+((B-A)/(1+((C/x)^D)))). A, B, C, and D are four parameters. The $IC_{50}$ values were further calculated using Xlfit as the concentration of the compound required for 50% inhibition of enzyme activity in the best-fit curve. The test results are shown in Table 1.

Table 1 *In vitro* inhibitory activity of USP1

| Compound No. | $IC_{50}$ (nM) | Compound No. | $IC_{50}$ (nM) |
|---|---|---|---|
| Compound **1** | 18 | Compound **2** | 19.2 |
| Compound **3** | 21.3 | Compound **4** | 43.3 |
| Compound **5** | 43.7 | Compound **6** | 21.1 |
| Compound **9** | 29.7 | Compound **10** | 106 |
| Compound **11** | 45.4 | Compound **15** | 34 |
| Compound **16** | 123.8 | Compound **21** | 16.7 |
| Compound **22** | 14.3 | Compound **23** | 21.9 |
| Compound **24** | 23.6 | Compound **25** | 27.2 |
| Compound **26** | 41.4 | Compound **28** | 33.7 |
| Compound **29** | 20 | Compound **30** | 44.7 |
| Compound **32** | 10.1 | Compound **33** | 31.3 |
| Compound **34** | 18.9 | Compound **35** | 69.5 |
| Compound **36** | 82.7 | Compound **40** | 26.9 |
| Compound **41** | 19 | Compound **42** | 86.6 |
| Compound **43** | 22.6 | Compound **44** | 13.9 |
| Compound **45** | 19.9 | Compound **47** | 45.9 |
| Compound **48** | 26.1 | Compound **49** | 33.2 |
| Compound **50** | 20.2 | Compound **51** | 13.8 |
| Compound **52** | 23.2 | Compound **53** | 8.9 |
| Compound **54** | 23.2 | Compound **55** | 8.7 |
| Compound **57** | 8.1 | Compound **58** | 36.9 |
| Compound **59** | 13.5 | Compound **60** | 17.9 |
| Compound **61** | 36.3 | Compound **62** | 9.8 |
| Compound **63** | 11.3 | Compound **64** | 22.7 |
| Compound **65** | 28.5 | Compound **66** | 27.2 |
| Compound **67** | 16.8 | Compound **68** | 12.1 |
| Compound **70** | 37.9 | Compound **71** | 71.6 |
| Compound **72** | 38.0 | Compound **73** | 45.2 |
| Compound **74** | 17.3 | Compound **75** | 14.8 |
| Compound **77** | 18.4 | Compound **78** | 96.0 |
| Compound **79** | 95.2 | Compound **80** | 11.7 |
| Compound **81** | 27.2 | Compound **82** | 59.3 |
| Compound **83** | 48.5 | Compound **84** | 69.7 |

**Test Example 2: Assay for inhibition of proliferation of MDA-MB-436 cells by USP1 inhibitor**

Assay based on CellTiter-Glo luminescent cell viability assay system.

**Experimental instruments:**

| Name of instrument | Manufacturer | Model |
|---|---|---|
| Oscillator | Boxun | BSD-YX3400 |
| Plate reader | PerkinElmer | Envision |
| Centrifugal machine | Eppendorf | Eppendorf Mixmate |
| Compound dilution and sample loading instrument | PerkinElmer | Echo |
| Cell incubator | THERMO | THERMOHeracellVIOS 250i |

**Experimental materials:**

The MDA-MB-436 cells used in the experiment were purchased from CoBioer Biosciences Co., Ltd., with Cat. No. of CBP60385. The cells were subcultured in DMEM with 10% FBS, and frozen in liquid nitrogen at a low passage number, and the passage number of the experimental cells did not exceed 15.

The assay kit (CellTiter-Glo® Luminescent Cell Viability Assay) was purchased from Promega, with Cat. No. of G7573, and was stored at -30 °C after subpackage. The kit is a homogeneous assay method for assaying the number of viable cells in a culture based on quantitative determination of ATP. The kit produces a luminescence signal proportional to the amount of ATP present, and the amount of ATP is directly proportional to the number of cells in the culture. Information on other reagents and consumables required for the experiment is as follows:

| Reagent | Brand | Cat. No. |
|---|---|---|
| PBS | Hyclone | SH30256.01 |
| DMEM | Gibco | 11995-065 |
| FBS | Gibco | 10099-141C |
| White 384-well plate | Corning | 3765 |
| Loading slot | Corning | 4877 |
| 10 mL pipette (sterile) | Corning | 4492 |
| ML323 | Selleck | S7529 |

ML323 structure is as follows:

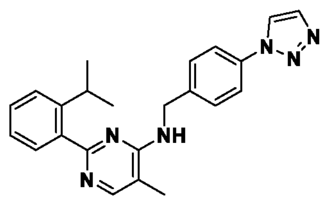

**Experimental method:**

The cultured cells were trypsinized, collected and centrifuged, then resuspended at an adjusted concentration in a culture solution (DMEM + 10% FBS), and cultured overnight on a 384-well plate (400 cells/20 μl/well) in a cell incubator with 5% $CO_2$ at 37 °C. An ECHO instrument was used to added the compound and pure DMSO onto each well of the 384-well plate. The total volume of the compound and DMSO is 100 nL, and the instrument obtains gradient diluted sample concentrations through different ratios. To each well, 30 μL of culture solution was added, and after mixing by centrifugal oscillation and re-centrifugation, the cells were cultured in a cell incubator for 7 days (one column of cells was subjected to CTG assay on the day of dosing). After day 7, 25 μL of CTG assay solution was added to each well, and after mixing by centrifugal oscillation and re-centrifugation, the cells were placed at room temperature away

from light for 10 min. In terms of chemiluminescence signals, an Envision plate reader (PerkinElmer, emission wavelength: 400-700 nm) was used to measure a signal in each well. The chemiluminescence value [RLU]cpd on day 7 was obtained for the treatment group, the chemiluminescence value [RLU]cell on day 7 was obtained for the non-treatment group (given DMSO only), and the chemiluminescence value [RLU]background on day 0 was obtained for the parallel non-treatment group (given DMSO only) by CTG assay on day 0. The inhibition rate of the compound on proliferation was calculated as follows: Inhibition rate (%) = [1 -

([RLU]cpd - [RLU]background)/([RLU]cell - [RLU]background)] × 100%, and the inhibitory activity ($GI_{50}$ values) of the compound on proliferation was calculated by using a four-parameter Logistic Model. In the following equation, x represents the concentration of the compound in a logarithmic form; and F(x) represents an effect value (inhibition rate of proliferation at the concentration): F(x) =(A+((B-A)/(1+((C/x)^D)))). A, B, C, and D are four parameters. The $GI_{50}$ values were further calculated using Xlfit as the concentration of the compound required for 50% inhibition of proliferation in the best-fit curve.

The inhibitory activity of the compounds provided in the present application on proliferation of MDA-MB-436 was determined by the foregoing assay. Measured $GI_{50}$ values are shown in Table 2.

Table 2 Inhibitory activity of compounds on proliferation of MDA-MB-436 cells

| No. | $GI_{50}$ (nM) | No. | $GI_{50}$ (nM) |
|---|---|---|---|
| ML323 | 1824.8 | Compound **1** | 22.9 |
| Compound **2** | 20.9 | Compound **3** | 47.3 |
| Compound **5** | 152.5 | Compound **6** | 420.5 |
| Compound **9** | 50.4 | Compound **10** | 507.1 |
| Compound **11** | 32.8 | Compound **15** | 20.1 |
| Compound **16** | 1084.0 | Compound **21** | 97.5 |
| Compound **22** | 19.9 | Compound **23** | 32.5 |
| Compound **24** | 86.2 | Compound **25** | 84.8 |
| Compound **26** | 41.1 | Compound **28** | 51.1 |
| Compound **29** | 24.4 | Compound **30** | 161.0 |
| Compound **32** | 11.4 | Compound **33** | 53.8 |
| Compound **34** | 39.6 | Compound **35** | 363.9 |
| Compound **36** | 545.9 | Compound **40** | 22.6 |
| Compound **41** | 28.4 | Compound **42** | 474.1 |
| Compound **43** | 32.6 | Compound **44** | 27.5 |
| Compound **45** | 53.2 | Compound **50** | 20.2 |
| Compound **52** | 59.6 | Compound **57** | 19.8 |
| Compound **58** | 81.8 | Compound **61** | 81.4 |
| Compound **62** | 12.3 | Compound **63** | 24.5 |
| Compound **65** | 26.8 | Compound **66** | 29.6 |
| Compound **67** | 17.3 | Compound **68** | 27.0 |
| Compound **70** | 93.1 | Compound **71** | 74.1 |
| Compound **73** | 48.0 | Compound **74** | 30.7 |
| Compound **77** | 48.7 | Compound **78** | 53.1 |
| Compound **79** | 80.2 | Compound **81** | 69.3 |
| Compound **82** | 35.8 | Compound **83** | 23.0 |

### Test Example 3: Assay for inhibition of compounds provided in the present application on CYP enzymes

Typical substrate metabolic responses of five major human CYP subtypes (CYP1A2, CYP2C9, CYP2C19, CYP2D6, CYP3A4) were evaluated using 150-donor pooled human liver microsomes (purchased from Corning, Cat. No.: 452117). The influence of different concentrations of each compound to be tested on metabolic responses of phenacetin (CYP1A2), diclofenac sodium (CYP2C9), S-mephenytoin (CYP2C19), bufuralol hydrochloride (CYP2D6), and midazolam (CYP3A4) was determined by liquid chromatography-mass spectrometry (LC/MS/MS).

The reaction system (200 μL) (100 mmol/L phosphate buffer, pH 7.4, containing DMSO, acetonitrile and methanol at a volume ratio of 0.3%:0.6%:0.1%) of 30 μM phenacetin, 10 μM diclofenac sodium, 35 μM S-mephenytoin, 5 μM bufuralol hydrochloride, 3 μM midazolam, 1 mM reduced nicotinamide adenine dinucleotide phosphate (NADPH), the compound to be tested (at concentrations of 0.1, 0.3, 1, 3, 10, 30 μmol/L, respectively), or positive compound or blank control, with pooled human liver microsomes (0.2 mg/mL) was incubated at 37 °C for 5 min. Then 200 μL of acetonitrile solution containing 3% formic acid and 40 nM internal standard verapamil was added and centrifuged at 4000 rpm for 50 min. The mixture was cooled on ice for 20 min and centrifuged at 4000 rpm for 20 min to precipitate the protein. Then 200 μL of supernatant was analyzed by LC-MS/MS.

The peak area was calculated from the chromatogram.

The residual activity ratio (%) was calculated according to the following equation:

$$\text{Peak area ratio} = \text{metabolite peak area/internal standard peak area}$$

$$\text{Residual activity ratio (\%)} = \text{peak area ratio of compounds-to-be-tested group/peak area ratio of blank group}$$

The half inhibitory concentration ($IC_{50}$) for CYP was calculated by Excel XLfit 5.3.1.3.

The measured half inhibitory concentration ($IC_{50}$) values of the compounds provided in the present application for CYP are shown in Table 3.

Table 3 Half inhibitory concentration ($IC_{50}$) of the compounds provided in the present application for CYP

| Compound No. | CYP1A2 $IC_{50}$ (μM) | CYP2C9 $IC_{50}$ (μM) | CYP2C19 $IC_{50}$ (μM) | CYP2D6 $IC_{50}$ (μM) | CYP3A4 $IC_{50}$ (μM) |
|---|---|---|---|---|---|
| Compound **11** | > 30 | 28.9 | 17.1 | > 30 | > 30 |
| Compound **74** | > 30 | 4.5 | 10.2 | > 30 | > 30 |

### Test Example 4: Caco-2 permeability assay

The apparent permeability coefficient ($P_{app}$) of the analytical drug was determined by liquid chromatography-mass spectrometry (LC-MS/MS) through a Caco-2 cell model.

In this test example, Caco-2 cells were purchased from American Type Culture Collection (ATCC); 4-hydroxyethyl piperazine ethane sulfonic acid (HEPES) was purchased from Beijing Solarbio Science & Technology Co., Ltd.; Hanks' balanced salt solution (HBSS) and non-essential amino acids (NEAA) were purchased from ThermoFisher Scientific; penicillin, streptomycin, and trypsin/EDTA were purchased from Solarbio; fetal bovine serum (FBS) and DMEM medium were purchased from Corning; HTS-96-well Transwell plates and other sterile consumables were purchased from Corning; Millicell resistance measuring system was purchased from Millipore; Cellometer®K2 was purchased from Nexcelom Bioscience; Infinite 200 PRO microplate reader was purchased from Tecan; and MTS2/4 orbital shaker was purchased from IKA Labortechnik.

Step 1: Cell culture and plating

Caco-2 cells were cultured in a culture flask. The incubator was set at 37 °C, with 5% $CO_2$ and a guaranteed relative humidity of 95%. Cells at a confluency of 70-90% can be used to inoculate the Transwell. Prior to cell inoculation, 50 μL of cell culture medium was added to each well of the upper Transwell chamber and 25 mL of cell culture medium

was added to the lower plate. The plate was incubated in an incubator (37 °C, 5% $CO_2$) for 1 h, and then inoculated with cells. After cell digestion, the cell suspension was pipetted and transferred to a round bottom centrifuge tube for centrifugation at 120 g for 5 min. The cells were resuspended in the medium to a final concentration of 6.86 × $10^5$ cells/mL. The cell suspension was then added to the upper chamber of the 96-well Transwell plate at 50 μL/well, with a final inoculation density of 2.4 × $10^5$cells/$cm^2$. The culture solution was changed 24 h after inoculation, and the medium was changed every other day after 14-18 days of culture. The medium was changed as follows: transwell chambers were separated from the receiver plate, medium in the receiver plate was discarded, then medium in the Transwell chambers was discarded, and finally, 75 μL of fresh medium was added to each chamber, and 25 mL of fresh medium was added to the receiver plate.

Step 2: Evaluation of integrity of a monolayer cell membrane

After approximately 14 days of culture, the Caco-2 cells reached the confluency and completed differentiation. In this case, the cells can be applied to permeability assay. The monolayer resistance was measured with a resistance meter (Millipore, USA) and the resistance per well was recorded. After the measurement, the Transwell plate was put back to the incubator. Calculation of resistance: measured resistance value (ohms) × membrane area ($cm^2$) = TEER value (ohm· $cm^2$); if TEER value < 230 ohms- $cm^2$, the well can not be used for permeability assay.

Step 3: Preparation of solution

In 900 mL of purified water, 2.38 g HEPES and 0.35 g sodium bicarbonate were dissolved, then 100 mLof 10× HBSS was added and stirred well, pH was adjusted to 7.4, and finally, the resulting solution was filtered to obtain 1 L of transport buffer (HBSS, 10 mM HEPES, pH 7.4).

A DMSO stock solution of 1 mM of compound to be tested was diluted with the transport buffer to obtain 5 μM test solution. The control compound digoxin or minoxidil was diluted with DMSO to 2 mM, and diluted with the transport buffer described above to 10 μM to obtain a control compound test solution. In addition, DMSO was also diluted with the transport buffer described above to a receiving end solution containing 0.5% DMSO.

Step 4: Drug permeability assay

The Transwell plate was removed from the incubator. Monolayer cell membranes were rinsed twice with the transport buffer (10 mM HEPES, pH 7.4) and incubated at 37 °C for 30 min.

The transport rate of the compound from the apical side to the basolateral side was determined. To each well of the upper chamber (apical side), 125 μL of test solution was added, and then 50 μL of solution was immediately transferred from the apical side to 200 μL of acetonitrile containing an internal standard (0.1 μM tolbutamide), as an initial sample from the apical side to the basolateral side. To each well of the lower chamber (basolateral side), 235 μL of receiving end solution was added.

The transport rate of the compound from the basolateral side to the apical side was determined. To each well of the upper chamber (apical side), 285 μL of receiving end solution was added, and then 50 μL of solution was immediately transferred from the apical side to 200 μL of acetonitrile containing an internal standard (0.1 μM tolbutamide), as an initial sample from the basolateral side to the apical side. To each well of the lower chamber (basolateral side), 75 μL of test solution was added.

The upper and lower transporters were merged and incubated at 37 °C for 2 h.

After incubation, sample from the upper and lower chambers of the Transwell plate was added to a new sample tube at 50 μL/well. To the sample tube, 200 μL of acetonitrile containing an internal standard (0.1 μM tolbutamide) was added, then the sample tube was vortexed for 10 min, and centrifuged at 3220 g for 40 min. Then 150 μL of supernatant was pipetted and diluted with 150 μL of water for LC-MS/MS. All samples were prepared in triplicate.

The integrity of monolayer cell membranes after 2 h of incubation was evaluated by leakage of fluorescein, and the fluorescein stock solution was diluted with the transport buffer (10 mM HEPES, pH 7.4) to a final concentration of 100 μM. To each well of the upper Transwell insert, 100μL of fluorescein solution was added, and 300 μL of transport buffer (10 mM HEPES, pH 7.4) was added to each well of the lower receiver plate. After incubation at 37 °C for 30 min, 80 μL of solution was pipetted from the upper and lower layers of each well into a new 96-well plate, respectively. Fluoremetry was performed using a microplate reader at an excitation wavelength of 485 nm and an emission wavelength of 530 nm.

Step 5: Data analysis

All calculations were performed by using Microsoft Excel. Peak areas were determined by using extracted ion

chromatograms.

The **apparent permeability coefficient** ($P_{app}$, unit: cm/s $\times 10^{-6}$) was calculated according to the following equation:

$$P_{app}=\frac{\text{volume of receiving end solution}}{\text{area of membrane}\times\text{time for incubation}}\times\frac{\text{concentration of drug at receiving end}}{\text{initial concentration of drug at administration end}}$$

where: the volume of the receiving end solution: Ap→Bl is 0.3 mL, and Bl→Ap is 0.1 mL; the membrane area of the Transwell-96-well plate is 0.143 $cm^2$; and the incubation time is in seconds. The **efflux ratio** was calculated according to the following equation:

$$\text{Efflux ratio}=\frac{P_{app\ (B\rightarrow A)}}{P_{app\ (A\rightarrow B)}}$$

where: $P_{app\ (B\text{-}A)}$ is the apparent permeability coefficient from the basolateral side to the apical side; $P_{app\ (A\text{-}B)}$ is the apparent permeability coefficient from the apical side to the basolateral side. The **recovery rate** was calculated according to the following equation:

$$\text{recovery rate (\%)}=\frac{\text{concentration of drug at receiving end}\times\text{volume of receiving end solution}+\text{concentration of drug at administration end}\times\text{volume of administration end solution}}{\text{initial concentration of drug at administration end}\times\text{volume of administration end solution}}\times 100\%$$

The **leakage rate LY (%)** was calculated according to the following equation:

$$\textbf{leakage rate}\ \text{LY (\%)}=\left(\frac{I_{\text{receiving end}}\times 0.3}{I_{\text{receiving end}}\times 0.3+I_{\text{administration end}}\times 0.1}\right)\times 100\%$$

where: $I_{receiving\ end}$ refers to the fluorescence intensity of receiver wells (0.3 mL), and $I_{administration\ end}$ refers to the fluorescence intensity of dosing wells (0.1 mL). LY < 1.5% indicates that the monolayer cell membranes are intact. For individual LY > 1.5% cases, the final data can be accepted based on scientific judgment if $P_{app}$ is close to that in other replicates.

Caco-2 permeability data obtained by testing the compounds provided in the present application are shown in Table 4.

Table 4 Caco-2 permeability data for compounds provided in the present application

| Compound No. | $P_{app\ (A\text{-}B)}$ ($10^{-6}$, cm/s) | $P_{app\ (B\text{-}A)}$ ($10^{-6}$, cm/s) | Efflux Ratio |
|---|---|---|---|
| Compound **11** | 4.9 | 17.6 | 3.6 |
| Compound **50** | 7.5 | 17.3 | 2.3 |

**Test Example 5: Assay for metabolic stability *in vitro* in rat hepatocytes**

The concentration of each compound in the reaction system was measured by LC/MS/MS to calculate the intrinsic clearance of the compounds to be tested, and evaluate the metabolic stability *in vitro* in rat hepatocytes.

To the incubation plate, 198 μL of 0.5 × $10^6$ cells/mL rat hepatocyte mixture and 2.0 μL of 100 μM compound to be tested or positive control were added to initiate reaction. Incubation was performed at 900 rpm at 37 °C. To a stop plate (150 μL of acetonitrile containing 100 nM alprazolam, 200 nM caffeine, and 100 nM toluenesulfonamide per well), 25μL of incubation system was transferred at 0, 15, 30, 60, 90, and 120 min, respectively. Then the mixture was vortex-mixed for 5 min. The stop plate was centrifuged at 3220 g for 45 min. Then 100 μL of supernatant of each compound was transferred to a 96-well sample plate, and 100 μL of purified water was added to dilute the sample. The resulting samples were quantified by ion chromatograms. The residual rate was calculated from peak areas of the compounds to be tested or the positive control. The slope k was determined using Microsoft Excel by linear regression of natural logarithm of the residual rate to the incubation time. The intrinsic clearance (*in vitro* $CL_{int}$, μL/min/$10^6$ cells) was calculated from the slope value according to the following equation:

$$in\ vitro\ CL_{int} = -kV/N$$

V = incubation volume (0.25 mL);
N = number of cells per well (0.125 × $10^6$ cells)

The measured intrinsic clearance in rat hepatocytes is shown in Table 5.

Table 5 Intrinsic clearance of compounds provided in the present application in rat hepatocytes

| Compound No. | Intrinsic clearance (μL/min/$10^6$ cells) |
|---|---|
| Verapamil | 125.7 |
| Compound **11** | < 1 |
| Compound **22** | 6.4 |
| Compound **26** | 4.2 |
| Compound **28** | 6.3 |
| Compound **33** | 4.3 |
| Compound **50** | 4.0 |

**Test Example 6: Assay for metabolic stability *in vitro* in human hepatocytes**

The concentration of each compound in the reaction system was measured by LC/MS/MS to calculate the intrinsic clearance of the compounds to be tested, and evaluate the metabolic stability *in vitro* in human hepatocytes.
To the incubation plate, 198 μL of 0.5 × $10^6$ cells/mL human hepatocyte mixture and 2.0 μL of 100 μM compound to be tested or positive control were added to initiate reaction. Incubation was performed at 900 rpm at 37 °C. To a stop plate (150 μL of acetonitrile containing 100 nM alprazolam, 200 nM caffeine, and 100 nM toluenesulfonamide per well), 25μL of incubation system was transferred at 0, 15, 30, 60, 90, and 120 min, respectively. Then the mixture was vortex-mixed for 5 min. The stop plate was centrifuged at 3220 g for 45 min. Then 100 μL of supernatant of each compound was transferred to a 96-well sample plate, and 100 μL of purified water was added to dilute the sample.
The resulting samples were quantified by ion chromatograms. The residual rate was calculated from peak areas of the compounds to be tested or the positive control. The slope k was determined using Microsoft Excel by linear regression of natural logarithm of the residual rate to the incubation time. The intrinsic clearance (*in vitro* $CL_{int}$, μL/min/$10^6$ cells) was calculated from the slope value according to the following equation:

$$in\ vitro\ CL_{int} = -kV/N$$

V = incubation volume (0.25 mL);
N = number of cells per well (0.125 × $10^6$ cells)

The measured intrinsic clearance in human hepatocytes is shown in Table 6.

Table 6 Intrinsic clearance of compounds provided in the present application in human hepatocytes

| Compound No. | Intrinsic clearance (μL/min/$10^6$ cells) |
|---|---|
| Verapamil | 37.3 |
| Compound **11** | < 1 |
| Compound **22** | 4.85 |
| Compound **45** | 4.26 |
| Compound **50** | < 1 |

**Test Example 7: Assay for solubility (PBS pH 7.4) of compound solids**

The solubility (PBS pH 7.4) of compound solids to be tested was determined by LC/MS/MS.

Approximately 1 mg of each compound powder was accurately weighed into a glass vial, and DMSO was added in a volume of 1 mL per milligram of compound. A stir bar was added to each vial, and the solubility sample vial was shaken at 1100 rpm at 25 °C for 2 h to completely dissolve the powder to prepare 1 mg/mL solution of the sample to be tested. In 490 μL of water and acetonitrile (1:1) containing an internal standard, 5 μL of 1 mg/mL solution and 5 μL of PBS pH 7.4 solution were mixed to prepare 10 μg/mL standard concentration solution of the sample to be tested. Then 10 μL of 10 μg/mL solution was diluted in 90 μL of water and acetonitrile (1:1) containing an internal standard to prepare 1 μg/mL standard concentration solution of the sample to be tested. The dilution factor of the standard solution can be adjusted according to the strength of response signals in LC/MS. Approximately 1 mg of each compound powder was accurately weighed into a glass vial, and PBS pH 7.4 solution was added in a volume of 1 mL per milligram of compound. A stir bar was added to each vial, and the solubility sample vial was shaken at 1100 rpm at 25 °C for 24 h. After shaking, the stir bar was removed, and the sample was transferred to a filter plate and filtered using a vacuum manifold. The filtered filtrate was diluted with a mixture of water and acetonitrile (1:1) containing an internal standard. The dilution factor can be adjusted according to the solubility value and the strength of response signals in LC/MS.

The resulting samples were determined by LC/MS/MS. The sample solubility was calculated from peak areas of the solution of the compound to be tested and the standard concentration solution, according to the following equation:

$$[\mathbf{Sample}] = \frac{Area\ ratio_{\ \mathrm{sample}} \times INJ\ VOL\ STD \times DF_{\ \mathrm{Sample}} \times [STD]}{Area\ ratio\ STD \times INJ\ VOL_{\ \mathrm{Sample}}}$$

[Sample] is the solubility of the sample to be tested;

*Area ratio* $_{sample}$ is the ratio of the peak area of sample in the sample to be tested to the peak area of the internal standard;

*INJ VOL STD* is the injection volume of the standard concentration solution;

$DF_{\mathrm{sample}}$ is the dilution factor of the solution of the sample to be tested;

[*STD*] is the concentration of the standard concentration solution;

*INJ VOL* $_{sample}$ is the injection volume of the solution of the sample to be tested; and

*Area ratio STD* is the ratio of the peak area of sample in the standard concentration solution to the peak area of the internal standard.

The solubility of the compound solids determined by this method is shown in Table 7.

Table 7 Solubility (PBS pH 7.4) of compound solids provided in the present application

| Compound No. | Solubility (μg/mL) | Calculated solubility (μM) |
|---|---|---|
| Compound **11** | 99.7 | 181.0 |

**Test Example 8: Assay for solubility (PBS pH 7.4) of compounds**

The solubility (PBS pH 7.4) of a compound to be tested was determined by LC/MS/MS.

6 μL of 10 mM DMSO solution of the compound to be tested was mixed with 194 μL of DMSO to prepare 300 μM compound solution. In 490 μL of water and acetonitrile (1:1) containing an internal standard, 5 μL of the solution and 5 μL of PBS pH 7.4 solution were mixed to prepare 3 μM standard concentration solution of the sample to be tested. The dilution factor of the standard solution can be adjusted according to the strength of response signals in LC/MS.

To 485 μL of PBS pH 7.4 solution, 15 μL of 10 mM DMSO solution of the compound to be tested was added. A stir bar was added, and the solubility sample vial was shaken at 1100 rpm at 25 °C for 2 h. After shaking, the stir bar was removed, and the sample was transferred to a filter plate and filtered using a vacuum manifold. In 490 μL of water and acetonitrile (1:1) containing an internal standard, 5 μL of filtrate and 5 μL of PBS pH 7.4 solution were mixed to prepare a solution to be tested. The dilution factor can be adjusted according to the solubility value and the strength of response signals in LC/MS.

The resulting samples were determined by LC/MS/MS. The sample solubility was calculated from peak areas of the solution of the compound to be tested and the standard concentration solution, according to the following equation:

$$[Sample] = \frac{Area\ ratio_{sample} \times INJ\ VOL\ STD \times DF_{Sample} \times [STD]}{Area\ ratio\ STD \times INJ\ VOL_{Sample}}$$

[Sample] is the solubility of the sample to be tested;
*Area ratio* $_{sample}$ is the ratio of the peak area of sample in the sample to be tested to the peak area of the internal standard;
*INJ VOL STD* is the injection volume of the standard concentration solution;
$DF_{sample}$ is the dilution factor of the solution of the sample to be tested;
[*STD*] is the concentration of the standard concentration solution;
*INJ VOL* $_{sample}$ is the injection volume of the solution of the sample to be tested; and
*Area ratio STD* is the ratio of the peak area of sample in the standard concentration solution to the peak area of the internal standard.

The solubility of the compounds determined by this method is shown in Table 8.

Table 8 Solubility (PBS pH 7.4) of compounds provided in the present application

| Compound No. | Calculated solubility (μM) |
|---|---|
| Compound **11** | 214 |
| Compound **16** | 284 |
| Compound **24** | 228 |
| Compound **26** | 115 |
| Compound **28** | 73 |
| Compound **30** | 225 |
| Compound **40** | 122 |
| Compound **41** | 88 |
| Compound **45** | 88 |
| Compound **46** | 50 |
| Compound **66** | 115 |
| Compound **70** | 98 |
| Compound **80** | 72 |

**Test Example 9: Assay for *in vivo* pharmacokinetics of compounds provided in the present application in rats**

Using SD rats as test animals, drug concentrations in plasma of the rats at different times after intravenous injection and intragastric administration of the compounds provided in the present application were determined by LC/MS/MS. *In vivo* pharmacokinetic behaviors of the compounds provided in the present application in rats were investigated to evaluate pharmacokinetic characteristics of the compounds.

There were three healthy 6-8-week-old male SD rats in each group.

Intravenous administration: A certain amount of drug was weighed, and then 10 vol% *N,N*-dimethylacetamide, 33 vol% triethylene glycol, and 57 vol% physiological saline were added to prepare 1 mg/mL colorless clear and transparent liquid;
Intragastric administration: A certain amount of drug was weighed, and then 0.5 wt% hydroxypropyl methylcellulose, 0.1 vol% tween 80, and 99.6 vol% physiological saline were added to prepare 1 mg/mL white suspension.
After fasting overnight, the SD rats were dosed intravenously or intragastrically.
The compounds provided in the present application were administered intravenously to the rats, then 0.2 mL of blood was collected from jugular veins respectively at 0.083, 0.25, 0.5, 1, 2, 4, 8, and 24 h after the administration, and placed in a test tube containing EDTA-K2. The test tube was then centrifuged at 4000 rpm at 4 °C for 5 min to separate plasma, and the plasma was stored at -75 °C. Alternatively, the compounds provided in the present application

were administered intragastrically to the rats, then 0.2 mL of blood was collected from jugular veins respectively at 0.25, 0.5, 1, 2, 4, 8, and 24 h after the administration, and placed in a test tube containing EDTA-K2. The test tube was then centrifuged at 3500 rpm at 4 °C for 10 min to separate plasma, and the plasma was stored at - 75 °C.

The content of the compounds to be tested in rat plasma after intravenous administration or intragastric administration at different concentrations was determined: 30 μL of rat plasma at each time after administration was taken, 200 μL (50 ng/mL) of solution of internal standard dexamethasone in acetonitrile was added, then the resulting mixture was vortex-mixed for 30 s, and centrifuged at 4700 rpm at 4 °C for 15 min, the supernatant of plasma samples was diluted three times with water, and 2.0 μL of diluted solution was taken for LC-MS/MS.

The *in vivo* pharmacokinetic parameters for the compounds provided in the present application in rats are shown in Table 9.

Table 9 *In vivo* pharmacokinetic results for compounds rovided in the resent alication in SD rats

| Compound No. and method/dose of administration | | Pharmacokinetic experiment | | | | | |
|---|---|---|---|---|---|---|---|
| | | Maximum plasma concentration $C_{max}$ (ng /mL) | Area under unit dose curve $AUC_{last}/D$ (h*mg/mL) | Half-life period $T_{1/2}$(h) | Clearance CL_obs (mL/min/kg) | Apparent volume of distribution $V_{ss}$_obs (L/kg) | Bioavailability F (%) |
| **11** | IV 1 mg/kg | - | 1024 | 3.2 | 16.5 | 3.2 | 96.3 |
| | PO 5 mg/kg | 1010 | 980 | - | - | - | |
| **50** | IV 2 mg/kg | - | 1397 | 6.0 | 11.4 | 4.1 | 47.2 |
| | PO 5 mg/kg | 235 | 659 | - | - | - | |

**Test Example 10: *In vitro* evaluation of potential for PXR activation in DPX2 cells**

DMSO solutions of the compounds to be tested and the control compound rifampicin solution were prepared, and diluted with Puracyp dosing medium (Puracyp, Cat. No: D-500-100) at 37 °C to respective test concentrations. The final concentrations of the compounds to be tested were 30 μM, 10 μM, and 1 μM, respectively, and the final concentration of rifampicin was 20 μM. The final concentration of DMSO in the test solution was 0.1%. In addition, 0.1% DMSO solution prepared with Puracyp dosing medium was used as a control.

DPX2 cells were suspended in Puracyp Culture medium (Puracyp, Cat No.: C-500-100) at a cell density of 4.5 $\times 10^5$ cell/mL. Then, the suspension was placed on a 96-well culture plate at 100 μL/well for incubation at 37 °C for 24 h. The medium in appropriate wells of the 96-well culture plate was replaced (three times per well) by 100 μL of solution of the compounds to be tested and a control compound solution. After replacement, the plate was incubated at 37 °C for 24 h. The medium was then replaced (three times per well) by a newly prepared test solution of the compounds to be tested and a rifampicin test solution, and the plate was incubated at 37 °C for 24 h.

The medium in the 96-well culture plate was pipetted and washed twice with PBS. To each well, 50 μL of reagent diluted as required by CellTiter-Fluor™ Cell Viability Assay Kit (Promega, Cat. No.: G6082) was added, and incubated at 37 °C for 0.5 h. The 96-well plate was cooled to room temperature, and the fluorescence value of each well at 505 nm was measured in a fluorescence mode with a microplate reader at an excitation wavelength of 400 nm. Then, 50 μL of reagent prepared as required by One-Glo Luciferase Assay System (Promega, Cat. No.: E6120) was added to each well, and the mixture was incubated at room temperature for 5 min. The luminescence value of each well was read by using a lumen meter.

Normalized luciferase activity was determined by RLU/RFU, and the RLU and RFU of the samples were mean values of duplicate wells, respectively.

$$Fold\ of\ activation = \frac{RLU_{test}/RFU_{test}}{RLU_{vehicle}/RFU_{vehicle}}$$

Fold of activation: fold of activation of normalized luciferase;
$RLU_{test}$: measured luminescence value of test wells at each test dose of test compounds;
$RLU_{vehicle}$: measured luminescence value of test wells when tested with the control DMSO solution;
$RFU_{test}$: measured fluorescence value of test wells at each test dose of test compounds;
$RFU_{vehicle}$: measured fluorescence value of test wells when tested with the control DMSO solution.

The percentage of positive control was calculated as follows:

% Positive control = (fold activation $_{\text{test compound}}$ -1)/(fold activation $_{\text{positive control compound}}$ -1) × 100%
% Positive control: percentage of PXR activation compared with 20 μM rifampicin activation at each test dose of test compounds;
Fold activation$_{\text{test compound}}$: fold of activation of normalized luciferase at each test dose of test compounds;
Fold activation$_{\text{positive control compound}}$: fold of activation of normalized luciferase of test compounds under 20 μM rifampicin.

The measured percentage of positive (rifampicin) control of PXR activation for the compounds provided in the present application is shown in Table 10.

Table 10 Percentage of positive control of PXR activation for compounds provided in the present application

| Compound No. | Concentration (μM) | PXR activation (% Positive control) |
|---|---|---|
| Compound 11 | 30 | 14.0 |
| Compound 43 | 10 | 3.2 |
| Compound 50 | 30 | 32.8 |
| Compound 52 | 10 | 20.9 |
| Compound 74 | 10 | 13.2 |

## Claims

**1.** A compound of formula (I) or a pharmaceutically acceptable salt thereof,

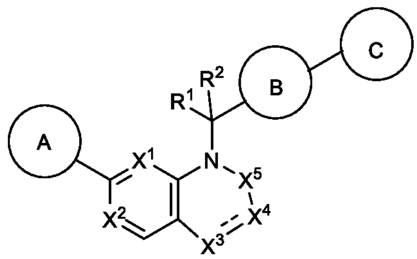

(I)

wherein,

$X^1$ is selected from $CR^3$ and N;
$X^2$ is selected from N;
$X^3$ and $X^4$ are each independently selected from $C(R^4)(R^5)$, $CR^4$, $NR^6$, N, O, S, S=O, and $S(=O)_2$;
$X^5$ is independently selected from $C(R^4)(R^5)$, $NR^6$, and O;
$R^3$, $R^4$, $R^5$, and $R^6$ are each independently selected from H, halogen, CN, OH, $NH_2$, $C_1$-$C_6$ alkyl, $C_2$-$C_6$ alkenyl, $C_2$-$C_6$ alkynyl, -C(O)-$C_1$-$C_6$ alkyl, -C(O)O-$C_1$-$C_6$ alkyl, $C_3$-$C_{10}$ cycloalkyl, and 4- to 7-membered heterocyclyl, wherein the OH, $NH_2$, $C_1$-$C_6$ alkyl, $C_2$-$C_6$ alkenyl, $C_2$-$C_6$ alkynyl, $C_3$-$C_{10}$ cycloalkyl, or 4- to 7-membered heterocyclyl is optionally substituted with $R^a$,
or $R^4$ and $R^5$ are merged into =O or =S; or $R^4$ and $R^5$ together with the C to which they are attached form $C_3$-$C_{10}$ cycloalkyl, wherein the $C_3$-$C_{10}$ cycloalkyl is optionally substituted with $R^a$; or $R^4$ and $R^5$ together with the atoms to which they are attached form $C_3$-$C_{10}$ heterocyclyl, wherein the $C_3$-$C_{10}$ heterocyclyl is optionally substituted

with $R^a$; or $R^4$ and $R^6$ at different positions in the ring, together with the atoms to which they are attached form $C_3$-$C_{10}$ heterocyclyl, wherein the $C_3$-$C_{10}$ heterocyclyl is optionally substituted with $R^a$;
ring A is selected from aryl and 5- to 10-membered heteroaryl, wherein the aryl or the 5- to 10-membered heteroaryl is optionally substituted with $R^b$;
ring B is selected from aryl, 5- to 10-membered heteroaryl, 4- to 10-membered heterocyclyl, $C_3$-$C_{10}$ cycloalkyl, and $C_3$-$C_{10}$ cycloalkenyl, wherein the aryl, 5- to 10-membered heteroaryl, 4- to 10-membered heterocyclyl, $C_3$-$C_{10}$ cycloalkyl, or $C_3$-$C_{10}$ cycloalkenyl is optionally substituted with $R^c$;
$R^b$ and $R^c$ are each independently selected from halogen, CN, OH, $NH_2$, SH, $C_1$-$C_6$ alkyl, $C_3$-$C_{10}$ cycloalkyl or 4- to 7-membered heterocyclyl,

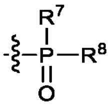 , 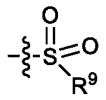 , 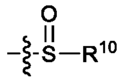 , 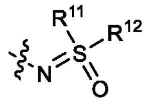 ,

and

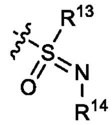 ,

wherein the OH, $NH_2$, SH, $C_1$-$C_6$ alkyl, $C_3$-$C_{10}$ cycloalkyl, or 4- to 7-membered heterocyclyl is optionally substituted with $R^a$;
$R^7$, $R^8$, $R^9$, $R^{10}$, $R^{11}$, $R^{12}$, $R^{13}$, and $R^{14}$ are each independently selected from $NH_2$, $C_1$-$C_6$ alkyl, $C_3$-$C_{10}$ cycloalkyl, and 4- to 7-membered heterocyclyl, wherein the $NH_2$, $C_1$-$C_6$ alkyl, $C_3$-$C_{10}$ cycloalkyl, or 4- to 7-membered heterocyclyl is optionally substituted with $R^a$;
or $R^7$ and $R^8$ together with the P to which they are attached form 4- to 7-membered heterocyclyl, wherein the 4- to 7-membered heterocyclyl is optionally substituted with $R^a$;
or $R^{13}$ and $R^{14}$ together with the atoms to which they are attached form 4- to 7-membered heterocyclyl, wherein the 4- to 7-membered heterocyclyl is optionally substituted with $R^a$;
ring C is selected from aryl, 5- to 10-membered heteroaryl, and 4- to 10-membered heterocyclyl, wherein the aryl, 5- to 10-membered heteroaryl, or 4- to 10-membered heterocyclyl is optionally substituted with $R^d$;
$R^d$ is selected from halogen, CN, OH, $NH_2$, $C_1$-$C_6$ alkyl, $C_3$-$C_{10}$ cycloalkyl, and 4- to 7-membered heterocyclyl, wherein the OH, $NH_2$, $C_1$-$C_6$ alkyl, $C_3$-$C_{10}$ cycloalkyl, or 4- to 7-membered heterocyclyl is optionally substituted with $R^a$; or $R^c$ and $R^d$ together with the atoms to which they are attached form 5- to 8-membered heterocyclyl or 5- to 6-membered heteroaryl, wherein the 5- to 8-membered heterocyclyl or the 5- to 6-membered heteroaryl is optionally substituted with $R^a$;
$R^1$ and $R^2$ are each independently selected from H, halogen, CN, OH, $NH_2$, $C_1$-$C_6$ alkyl, $C_3$-$C_{10}$ cycloalkyl, and 4- to 7-membered heterocyclyl, wherein the OH, $NH_2$, $C_1$-$C_6$ alkyl, $C_3$-$C_{10}$ cycloalkyl, or 4- to 7-membered heterocyclyl is optionally substituted with $R^a$,
or $R^1$ and $R^2$ together with the atoms to which they are attached form $C_3$-$C_{10}$ cycloalkyl or 4- to 7-membered heterocyclyl, wherein the $C_3$-$C_{10}$ cycloalkyl or the 4- to 7-membered heterocyclyl is optionally substituted with $R^a$;
each $R^a$ is independently selected from halogen, CN, =O, OH, $NH_2$, $C_1$-$C_6$ alkyl, $C_3$-$C_{10}$ cycloalkyl, and 4- to 7-membered heterocyclyl, wherein the OH, $NH_2$, $C_1$-$C_6$ alkyl, $C_3$-$C_{10}$ cycloalkyl, or 4- to 7-membered heterocyclyl is optionally substituted with $R^e$;
$R^e$ is selected from halogen, CN, =O, OH, $NH_2$, $C_1$-$C_6$ alkyl, $C_3$-$C_6$ cycloalkyl, and 4- to 7-membered heterocyclyl, wherein the OH, $NH_2$, $C_1$-$C_6$ alkyl, $C_3$-$C_6$ cycloalkyl, or 4- to 7-membered heterocyclyl is optionally substituted with $R^f$; and
$R^f$ is selected from halogen, CN, OH, $NH_2$, $C_1$-$C_6$ alkyl, $C_3$-$C_6$ cycloalkyl, and 4- to 7-membered heterocyclyl.

**2.** The compound of formula (I) or the pharmaceutically acceptable salt thereof according to claim 1, wherein $X^1$ is selected from N, and $X^2$ is selected from N; or $X^1$ is selected from $CR^3$, and $X^2$ is selected from N; or $X^1$ is selected from CH, and $X^2$ is selected from N; or $X^1$ is selected from $CR^3$ and N, wherein $R^3$ is H, and $X^2$ is selected from N.

**3.** The compound of formula (I) or the pharmaceutically acceptable salt thereof according to claim 1 or 2, wherein $R^3$, $R^4$, $R^5$, and $R^6$ are each independently selected from H, halogen, CN, OH, $NH_2$, $C_1$-$C_6$ alkyl, $C_3$-$C_{10}$ cycloalkyl, and

4- to 7-membered heterocyclyl, wherein the OH, $NH_2$, $C_1$-$C_6$ alkyl, $C_3$-$C_{10}$ cycloalkyl, or 4- to 7-membered heterocyclyl is optionally substituted with $R^a$; or $R^4$ and $R^5$ are merged into =O; or $R^4$ and $R^5$ together with the C to which they are attached form $C_3$-$C_{10}$ cycloalkyl, wherein the $C_3$-$C_{10}$ cycloalkyl is optionally substituted with $R^a$; or $R^4$ and $R^5$ together with the atoms to which they are attached form $C_3$-$C_{10}$ heterocyclyl, wherein the $C_3$-$C_{10}$ heterocyclyl is optionally substituted with $R^a$; or $R^4$ and $R^6$ at different positions in the ring, together with the atoms to which they are attached form $C_3$-$C_{10}$ heterocyclyl, wherein the $C_3$-$C_{10}$ heterocyclyl is optionally substituted with $R^a$; or

$R^3$, $R^4$, $R^5$, and $R^6$ are each independently selected from H, halogen, CN, $C_1$-$C_6$ alkyl, $C_2$-$C_6$ alkenyl, -C(O)-$C_1$-$C_6$ alkyl, -C(O)O-$C_1$-$C_6$ alkyl, $C_3$-$C_{10}$ cycloalkyl, and 4- to 7-membered heterocyclyl, wherein the $C_1$-$C_6$ alkyl, $C_2$-$C_6$ alkenyl, $C_3$-$C_{10}$ cycloalkyl, or 4- to 7-membered heterocyclyl is optionally substituted with $R^a$; or
$R^3$, $R^4$, $R^5$, and $R^6$ are each independently selected from H, halogen, $C_1$-$C_6$ alkyl, $C_2$-$C_6$ alkenyl, - C(O)-$C_1$-$C_6$ alkyl, -C(O)O-$C_1$-$C_6$ alkyl, and $C_3$-$C_{10}$ cycloalkyl, wherein the $C_1$-$C_6$ alkyl, $C_2$-$C_6$ alkenyl, or $C_3$-$C_{10}$ cycloalkyl is optionally substituted with $R^a$; or
$R^3$ is H;
$R^4$ and $R^5$ are each independently selected from H, halogen, $C_1$-$C_6$ alkyl, and $C_2$-$C_6$ alkynyl, wherein the $C_1$-$C_6$ alkyl or the $C_2$-$C_6$ alkynyl is optionally substituted with $R^a$; or $R^4$ and $R^5$ are merged into =O or =S; or $R^4$ and $R^5$ together with the C to which they are attached form $C_3$-$C_{10}$ cycloalkyl, wherein the $C_3$-$C_{10}$ cycloalkyl is optionally substituted with $R^a$; or $R^4$ and $R^5$ together with the atoms to which they are attached form $C_3$-$C_{10}$ heterocyclyl, wherein the $C_3$-$C_{10}$ heterocyclyl is optionally substituted with $R^a$; or $R^4$ and $R^6$ at different positions in the ring, together with the atoms to which they are attached form $C_3$-$C_{10}$ heterocyclyl, wherein the $C_3$-$C_{10}$ heterocyclyl is optionally substituted with $R^a$; or
$R^4$ and $R^5$ are each independently selected from H, halogen, $C_1$-$C_6$ alkyl, and $C_2$-$C_6$ alkynyl, wherein the $C_1$-$C_6$ alkyl is optionally substituted with $R^a$; or $R^4$ and $R^5$ are merged into =O or =S; or $R^4$ and $R^5$ together with the C to which they are attached form $C_3$-$C_{10}$ cycloalkyl; or $R^4$ and $R^5$ together with the atoms to which they are attached form $C_3$-$C_{10}$ heterocyclyl; or $R^4$ and $R^6$ at different positions in the ring, together with the atoms to which they are attached form $C_3$-$C_{10}$ heterocyclyl; or
$R^4$ and $R^5$ are each independently selected from H, halogen, $C_1$-$C_4$ alkyl and $C_2$-$C_3$ alkynyl, wherein the $C_1$-$C_4$ alkyl is optionally substituted with $R^a$; or $R^4$ and $R^5$ are merged into =O or =S; or $R^4$ and $R^5$ together with the C to which they are attached form $C_3$-$C_7$ cycloalkyl; or $R^4$ and $R^5$ together with the atoms to which they are attached form $C_3$-$C_6$ heterocyclyl; or $R^4$ and $R^6$ at different positions in the ring, together with the atoms to which they are attached form $C_3$-$C_6$ heterocyclyl; and
$R^6$ is selected from H, $C_1$-$C_6$ alkyl, $C_2$-$C_6$ alkenyl, -C(O)-$C_1$-$C_6$ alkyl, -C(O)O-$C_1$-$C_6$ alkyl, and $C_3$-$C_{10}$ cycloalkyl, wherein the $C_1$-$C_6$ alkyl, $C_2$-$C_6$ alkenyl, or $C_3$-$C_{10}$ cycloalkyl is optionally substituted with $R^a$; or
$R^6$ is selected from H, $C_1$-$C_6$ alkyl, and $C_3$-$C_{10}$ cycloalkyl, wherein the $C_1$-$C_6$ alkyl is optionally substituted with $R^a$; or
$R^6$ is selected from H, $C_1$-$C_4$ alkyl, and $C_3$-$C_6$ cycloalkyl, wherein the $C_1$-$C_4$ alkyl is optionally substituted with $R^a$; or
$R^6$ is selected from H, $C_1$-$C_4$ alkyl, and $C_3$-$C_4$ cycloalkyl, wherein the $C_1$-$C_4$ alkyl is optionally substituted with $R^a$; or
when $X^4$ is $NR^6$ and $X^5$ is $C(R^4)(R^5)$, $R^4$ and $R^6$ together with the atoms to which they are attached form $C_3$-$C_6$ heterocyclyl, wherein the $C_3$-$C_6$ heterocyclyl is optionally substituted with $R^a$.

**4.** The compound of formula (I) or the pharmaceutically acceptable salt thereof according to any one of claims 1 to 3, wherein $X^3$ is selected from $C(R^4)(R^5)$, $CR^4$, $NR^6$, N, O, and S; or $X^3$ and $X^4$ are each independently selected from $C(R^4)(R^5)$, $CR^4$, $NR^6$, and O; or $X^3$ is selected from $C(R^4)(R^5)$, $NR^6$, and O; or $X^3$ is selected from S.

**5.** The compound of formula (I) or the pharmaceutically acceptable salt thereof according to any one of claims 1 to 4, wherein $X^4$ is selected from $C(R^4)(R^5)$, $CR^4$, $NR^6$, and O; or $X^4$ is selected from $C(R^4)(R^5)$, $NR^6$, and O.

**6.** The compound of formula (I) or the pharmaceutically acceptable salt thereof according to any one of claims 1 to 5, wherein $X^5$ is selected from $C(R^4)(R^5)$ and $NR^6$; or $X^5$ is selected from $C(R^4)(R^5)$.

**7.** The compound of formula (I) or the pharmaceutically acceptable salt thereof according to any one of claims 1 to 6, wherein ring A is selected from phenyl and 5- to 6-membered heteroaryl, wherein the phenyl or the 5- to 6-membered heteroaryl is optionally substituted with $R^b$; or

ring A is selected from phenyl, pyridyl, pyrimidinyl, and pyrazolyl, wherein the phenyl, pyridyl, pyrimidinyl, or pyrazolyl is optionally substituted with $R^b$; or

ring A is selected from phenyl, pyridyl, and pyrimidinyl, wherein the phenyl, pyridyl, or pyrimidinyl is optionally substituted with $R^b$; or
ring A is selected from

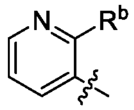, 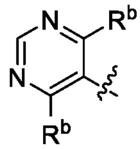, 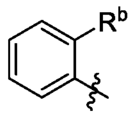 and 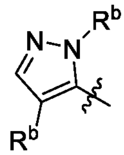;

or
ring A is selected from

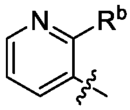, 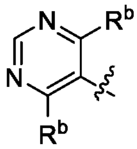, and 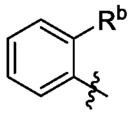.

**8.** The compound of formula (I) or the pharmaceutically acceptable salt thereof according to any one of claims 1 to 7, wherein ring B is selected from aryl, 5- to 6-membered heteroaryl, 4- to 10-membered heterocyclyl, $C_3$-$C_{10}$ cycloalkyl, and $C_3$-$C_{10}$ cycloalkenyl, wherein the aryl, 5- to 6-membered heteroaryl, 4- to 10-membered heterocyclyl, $C_3$-$C_{10}$ cycloalkyl, or $C_3$-$C_{10}$ cycloalkenyl is optionally substituted with $R^c$; or

ring B is selected from phenyl, 5- to 6-membered heteroaryl, 4- to 6-membered heterocyclyl, $C_3$-$C_8$ cycloalkyl, and $C_4$-$C_6$ cycloalkenyl, wherein the phenyl, 5- to 6-membered heteroaryl, 4- to 6-membered heterocyclyl, $C_3$-$C_8$ cycloalkyl, or $C_4$-$C_6$ cycloalkenyl is optionally substituted with $R^c$; or ring B is selected from phenyl, 4- to 6-membered heterocyclyl, $C_3$-$C_8$ cycloalkyl, and $C_4$-$C_6$ cycloalkenyl, wherein the phenyl, 4- to 6-membered heterocyclyl, $C_3$-$C_8$ cycloalkyl, or $C_4$-$C_6$ cycloalkenyl is optionally substituted with $R^c$; or
ring B is selected from

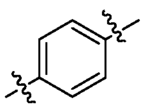, 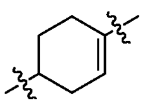, 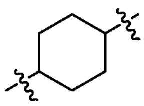, 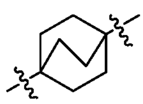, 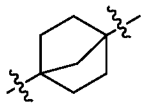, 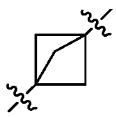,

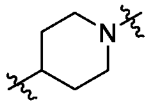, 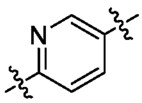, and 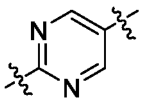,

wherein the

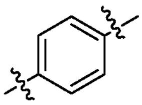, 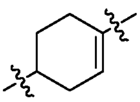, 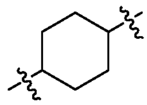,

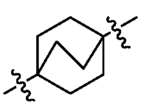, 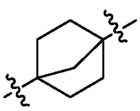, 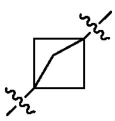, 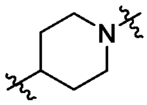, 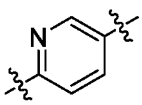, or 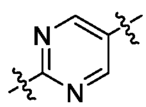

is optionally substituted with $R^c$; or
ring B is selected from

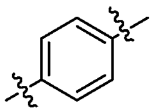, 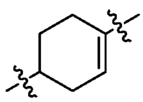, 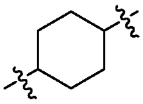, 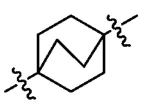, 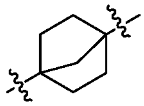, 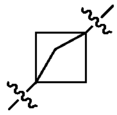,

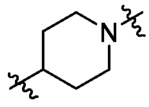, and 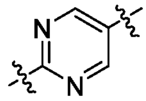,

wherein the

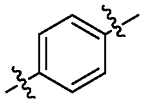, 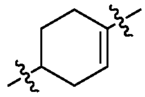, 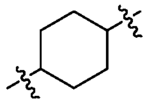, 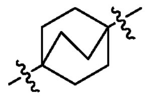,

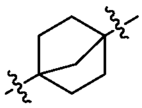, 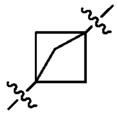, 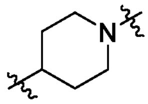, or 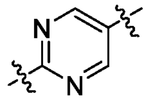

is optionally substituted with $R^c$.

**9.** The compound of formula (I) or the pharmaceutically acceptable salt thereof according to any one of claims 1 to 8, wherein $R^b$ and $R^c$ are each independently selected from halogen, OH, $NH_2$, SH, $C_1$-$C_6$ alkyl, $C_3$-$C_{10}$ cycloalkyl, 4- to 7-membered heterocyclyl,

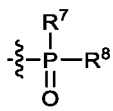, 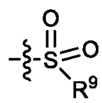, 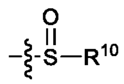,

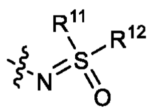, and 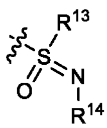,

wherein the OH, $NH_2$, SH, $C_1$-$C_6$ alkyl, $C_3$-$C_{10}$ cycloalkyl, or 4- to 7-membered heterocyclyl is optionally substituted with $R^a$, wherein $R^7$, $R^8$, $R^9$, $R^{10}$, $R^{11}$, $R^{12}$, $R^{13}$, and $R^{14}$ are each independently selected from $C_1$-$C_6$ alkyl, $C_3$-$C_{10}$ cycloalkyl, and 4- to 7-membered heterocyclyl, wherein the $C_1$-$C_6$ alkyl, $C_3$-$C_{10}$ cycloalkyl, or 4- to 7-membered heterocyclyl is optionally substituted with $R^a$; or $R^7$ and $R^8$ together with the P to which they are attached form 4- to 7-membered heterocyclyl, wherein the 4- to 7-membered heterocyclyl is optionally substituted with $R^a$; or $R^{13}$ and $R^{14}$ together with the atoms to which they are attached form 4- to 7-membered heterocyclyl, wherein the 4- to 7-membered heterocyclyl is optionally substituted with $R^a$; or

$R^b$ and $R^c$ are each independently selected from

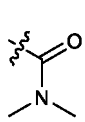, 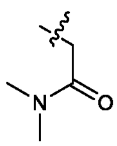, 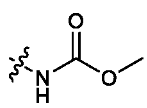, 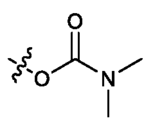, 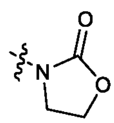,

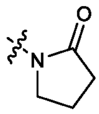, 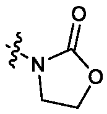, 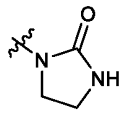, 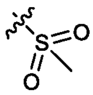, 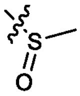, 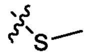, 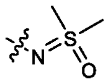, 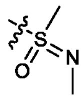, 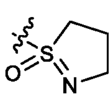, 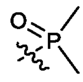,

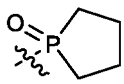, 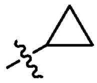, and 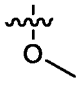;

or

$R^b$ is selected from halogen, OH, $C_1$-$C_6$ alkyl, $C_3$-$C_{10}$ cycloalkyl, and

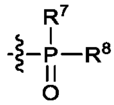,

wherein the OH, $C_1$-$C_6$ alkyl, or $C_3$-$C_{10}$ cycloalkyl is optionally substituted with $R^a$; or

$R^b$ is selected from halogen, OH, $C_1$-$C_4$ alkyl, and $C_3$-$C_6$ cycloalkyl, wherein the OH, $C_1$-$C_4$ alkyl, or $C_3$-$C_6$ cycloalkyl is optionally substituted with $R^a$; or

$R^b$ is selected from F, Cl, OH, $C_1$-$C_3$ alkyl, and $C_3$ cycloalkyl, wherein the OH is substituted with $R^a$; or

$R^c$ is selected from halogen and $C_1$-$C_6$ alkyl, wherein the $C_1$-$C_6$ alkyl is optionally substituted with $R^a$; or

$R^c$ is selected from halogen and $C_1$-$C_4$ alkyl, wherein the $C_1$-$C_4$ alkyl is optionally substituted with $R^a$; or

$R^c$ is selected from F, Cl, and $C_1$-$C_4$ alkyl, wherein the $C_1$-$C_4$ alkyl is optionally substituted with $R^a$; or

$R^c$ is selected from F, Cl, and $C_1$-$C_2$ alkyl, wherein the $C_1$-$C_2$ alkyl is optionally substituted with $R^a$.

**10.** The compound of formula (I) or the pharmaceutically acceptable salt thereof according to any one of claims 1 to 9, wherein ring C is selected from aryl and 5- to 10-membered heteroaryl, wherein the aryl or the 5- to 10-membered heteroaryl is optionally substituted with $R^d$; or

ring C is selected from 5- to 6-membered heteroaryl, wherein the 5- to 6-membered heteroaryl is optionally substituted with $R^d$; or

ring C is selected from 4- to 10-membered heterocyclyl, wherein the 4- to 10-membered heterocyclyl is optionally substituted with $R^d$; or

ring C is selected from

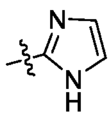, 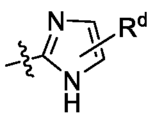, 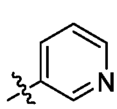, 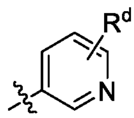, 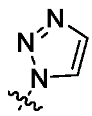, 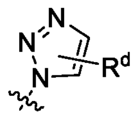, 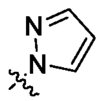,

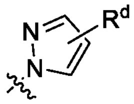, 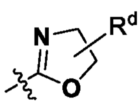, 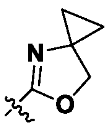, 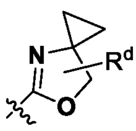, 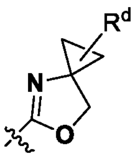, 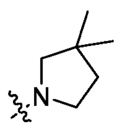, 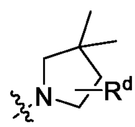, 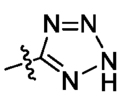,

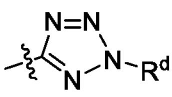, 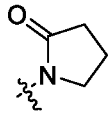, and 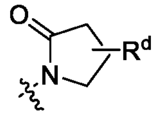;

or

ring C is selected from

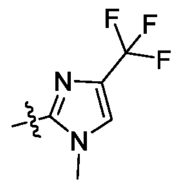
;

or

ring C is selected from

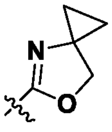 , 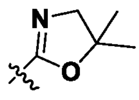 , 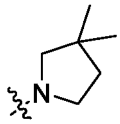 , 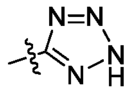 , 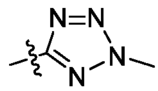 , and 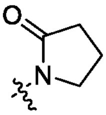 .

**11.** The compound of formula (I) or the pharmaceutically acceptable salt thereof according to any one of claims 1 to 10, wherein $R^d$ is selected from halogen, CN, OH, $NH_2$, $C_1$-$C_6$ alkyl, and $C_3$-$C_{10}$ cycloalkyl, wherein the OH, $NH_2$, $C_1$-$C_6$ alkyl, or $C_3$-$C_{10}$ cycloalkyl is optionally substituted with $R^a$; or $R^c$ and $R^d$ together with the atoms to which they are attached form 6- to 7-membered heterocyclyl or 5- to 6-membered heteroaryl, wherein the 6- to 7-membered heterocyclyl or the 5- to 6-membered heteroaryl is optionally substituted with $R^a$; or

$R^d$ is selected from $C_1$-$C_6$ alkyl and $C_3$-$C_{10}$ cycloalkyl, wherein the $C_1$-$C_6$ alkyl or the $C_3$-$C_{10}$ cycloalkyl is optionally substituted with $R^a$; or
$R^d$ is selected from $C_1$-$C_4$ alkyl and $C_3$-$C_6$ cycloalkyl, wherein the $C_1$-$C_4$ alkyl is optionally substituted with $R^a$; or
$R^d$ is $C_1$-$C_4$ alkyl optionally substituted with $R^a$.

**12.** The compound of formula (I) or the pharmaceutically acceptable salt thereof according to any one of claims 1 to 11, wherein $R^1$ and $R^2$ are each independently selected from H, halogen, CN, OH, $NH_2$, and $C_1$-$C_6$ alkyl, wherein the OH, $NH_2$, or $C_1$-$C_6$ alkyl is optionally substituted with $R^a$; or $R^1$ and $R^2$ together with the atoms to which they are attached form $C_3$-$C_6$ cycloalkyl or 4- to 7-membered heterocyclyl, wherein the $C_3$-$C_6$ cycloalkyl or the 4- to 7-membered heterocyclyl is optionally substituted with $R^a$; or

$R^1$ and $R^2$ are each independently selected from H, methyl, and ethyl; or $R^1$ and $R^2$ together with the atoms to which they are attached form the following rings:

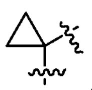 , 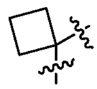 , and 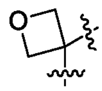 ,

wherein the

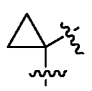 , 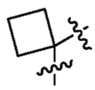 , and 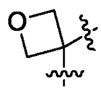

are optionally substituted with $R^a$; or
$R^1$ and $R^2$ are each independently selected from H; or $R^1$ and $R^2$ together with the atoms to which they are attached form the following rings:

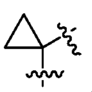 , 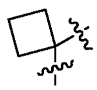 , and 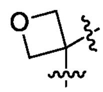 ,

wherein the

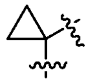

,

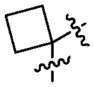

, and

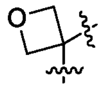

are optionally substituted with $R^a$; or both $R^1$ and $R^2$ are H.

**13.** The compound of formula (I) or the pharmaceutically acceptable salt thereof according to any one of claims 1 to 12, wherein each $R^a$ is independently selected from halogen, =O, OH, $NH_2$, $C_1$-$C_6$ alkyl, $C_3$-$C_6$ cycloalkyl, and 4- to 7-membered heterocyclyl, wherein the OH, $NH_2$, $C_1$-$C_6$ alkyl, $C_3$-$C_6$ cycloalkyl, or 4- to 7-membered heterocyclyl is optionally substituted with $R^e$; or

each $R^a$ is independently selected from halogen, OH, $C_1$-$C_6$ alkyl, and $C_3$-$C_6$ cycloalkyl, wherein the OH, $C_1$-$C_6$ alkyl, or $C_3$-$C_6$ cycloalkyl is optionally substituted with $R^e$; or
each $R^a$ is independently selected from F, Cl, OH, $C_1$-$C_6$ alkyl, and $C_3$-$C_6$ cycloalkyl, wherein the OH or the $C_1$-$C_6$ alkyl is optionally substituted with $R^e$.

**14.** The compound of formula (I) or the pharmaceutically acceptable salt thereof according to any one of claims 1 to 13, wherein $R^e$ is selected from halogen, =O, OH, $NH_2$, $C_1$-$C_6$ alkyl, $C_3$-$C_6$ cycloalkyl, and 4- to 7-membered heterocyclyl, wherein the OH, $NH_2$, $C_1$-$C_6$ alkyl, $C_3$-$C_6$ cycloalkyl, or 4- to 7-membered heterocyclyl is optionally substituted with $R^f$; or
$R^e$ is halogen, such as F or Cl.

**15.** The compound of formula (I) or the pharmaceutically acceptable salt thereof according to any one of claims 1 to 14, wherein $R^f$ is selected from halogen, OH, $NH_2$, $C_1$-$C_6$ alkyl, $C_3$-$C_6$ cycloalkyl, and 4- to 7-membered heterocyclyl.

**16.** The compound of formula (I) or the pharmaceutically acceptable salt thereof according to any one of claims 1 to 15, wherein the compound of formula (I) or the pharmaceutically acceptable salt thereof is selected from a compound of formula (II) and a pharmaceutically acceptable salt thereof,

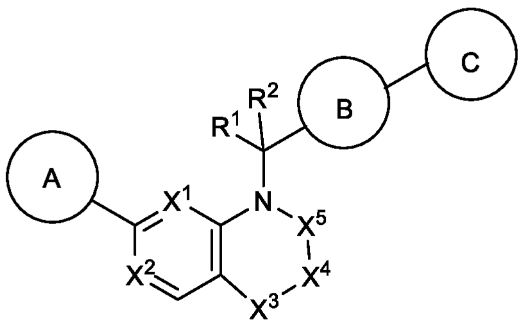

(II)

wherein $X^3$ and $X^4$ are independently selected from $C(R^4)(R^5)$, $NR^6$, O, S, and $S(=O)_2$; and
ring A, ring B, ring C, $X^1$, $X^2$, $X^5$, $R^1$, $R^2$, $R^4$, $R^5$, and $R^6$ are as defined in formula (I).

**17.** The compound of formula (I) or the pharmaceutically acceptable salt thereof according to claim 1, wherein the compound of formula (I) or the pharmaceutically acceptable salt thereof is selected from the following compounds and pharmaceutically acceptable salts thereof,

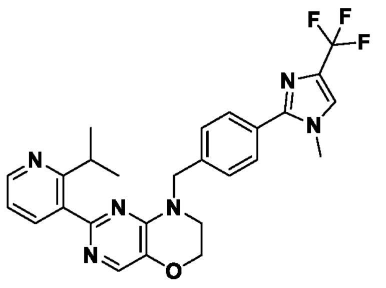 , 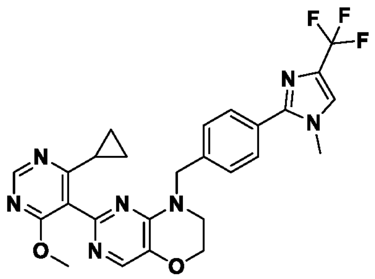 , 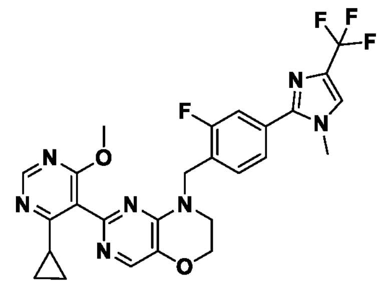 ,

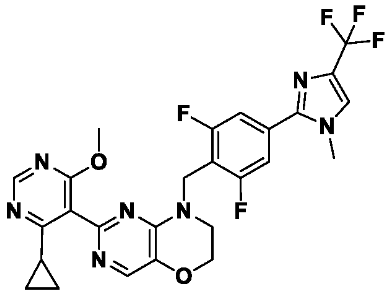 , 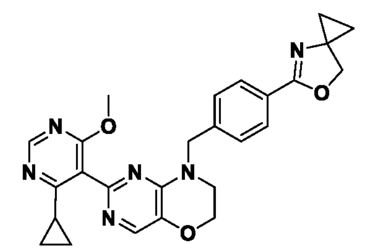 , 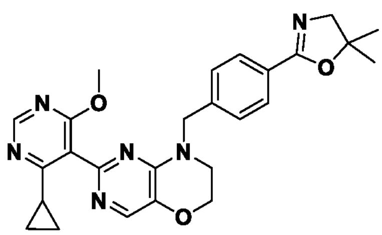 ,

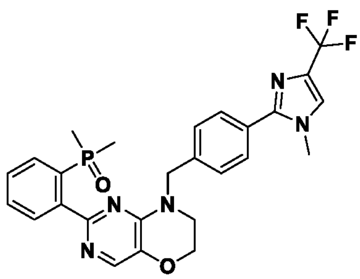 , 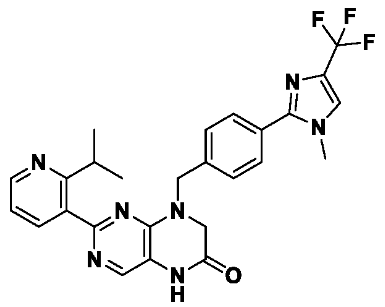 , 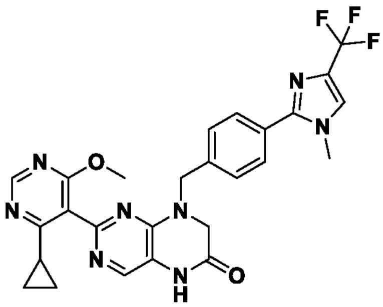 ,

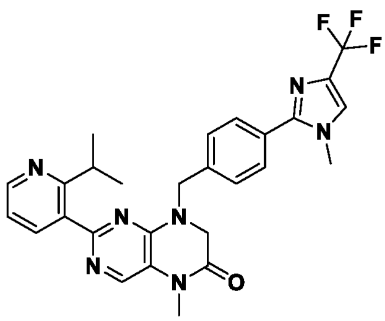 , 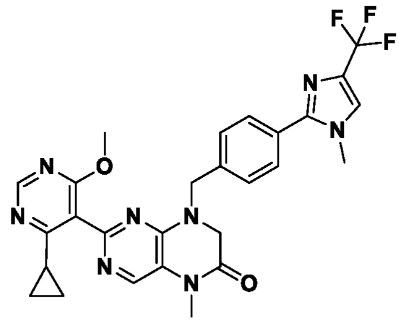 , 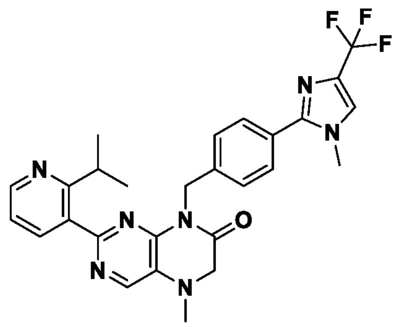 ,

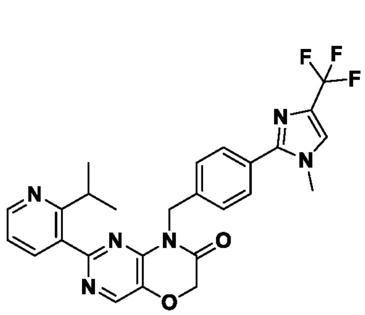 , 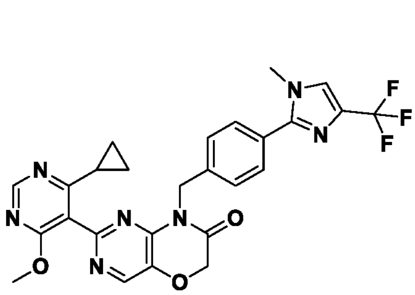 , 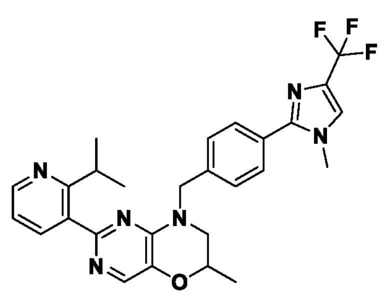 ,

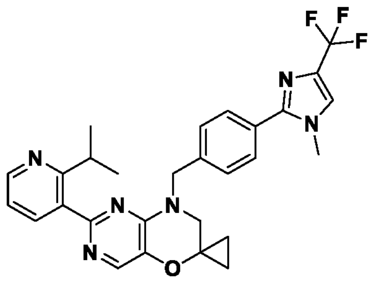,
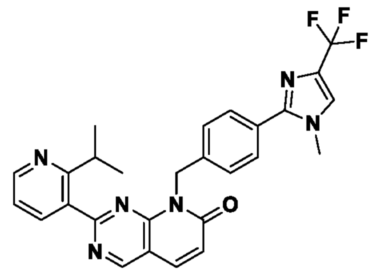,
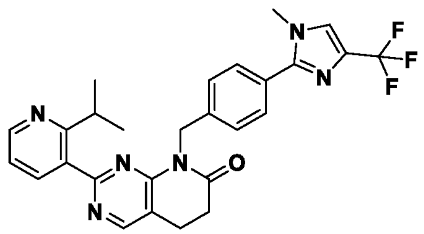,

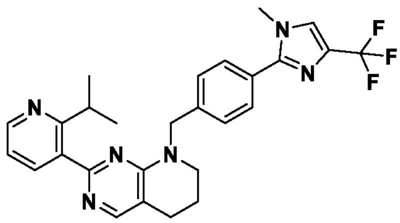,
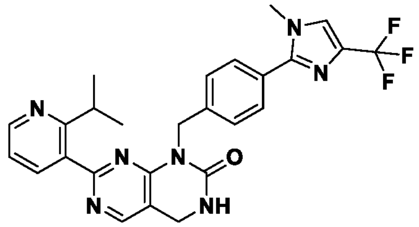,

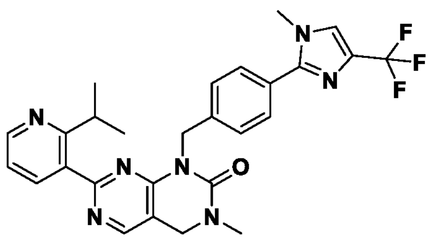,
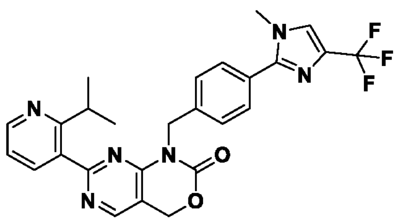,
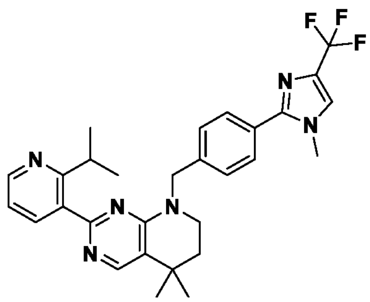,

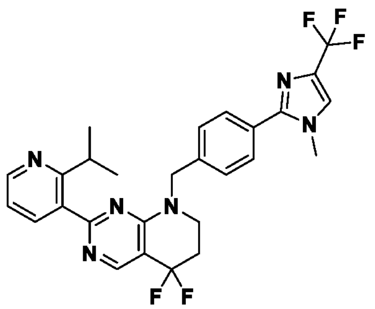,
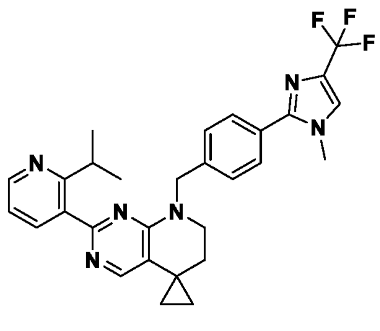,
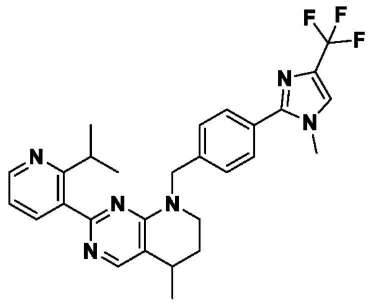,

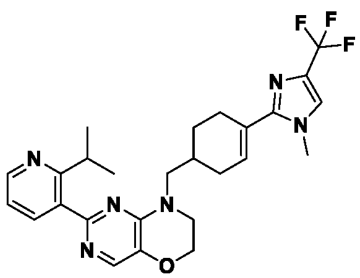,
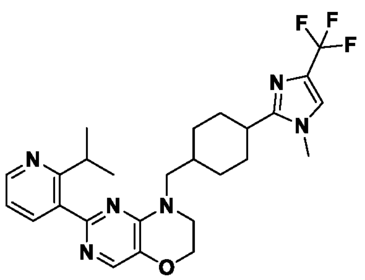,
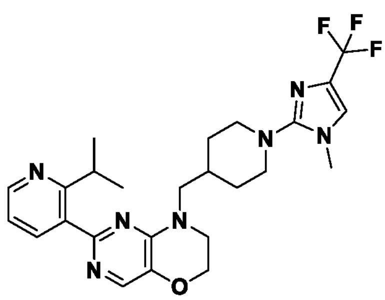,

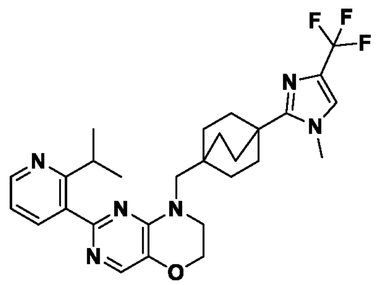 , 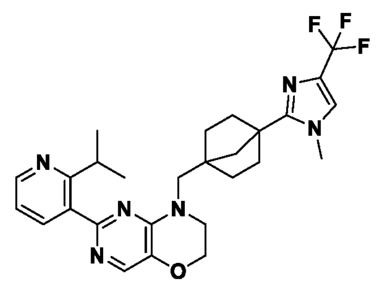 , 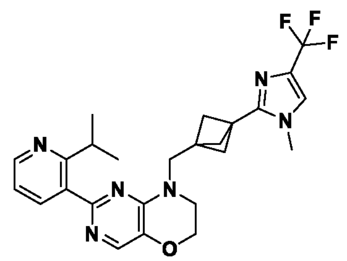 ,

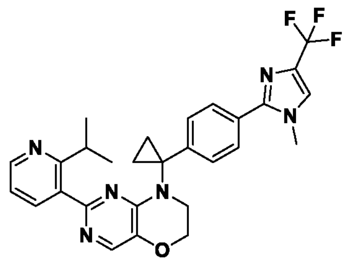 , 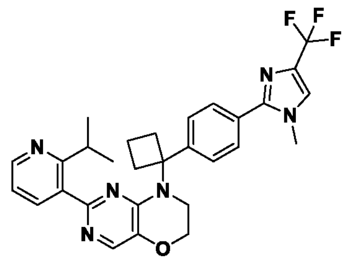 , 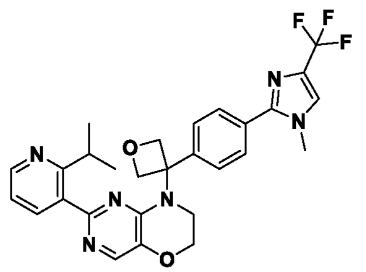 ,

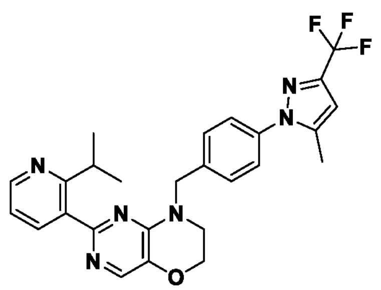 , 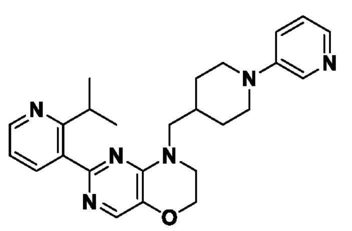 , 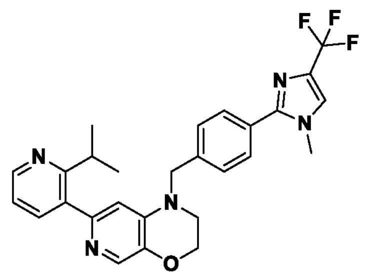 ,

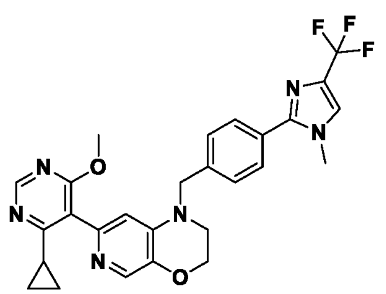 , 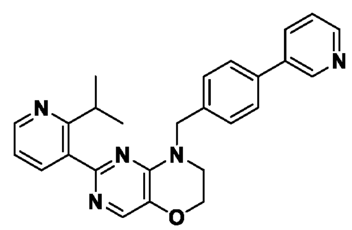 , 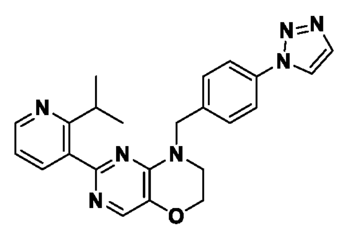 ,

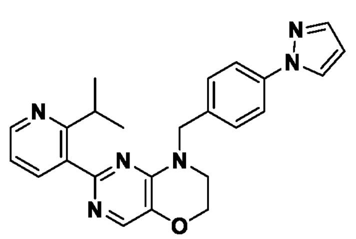 , 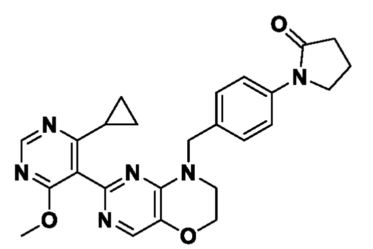 , 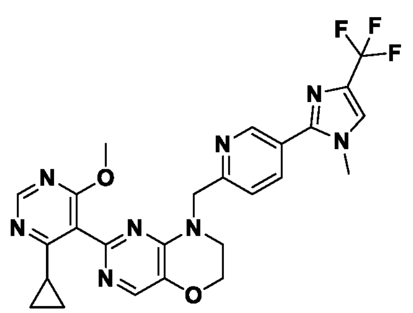 ,

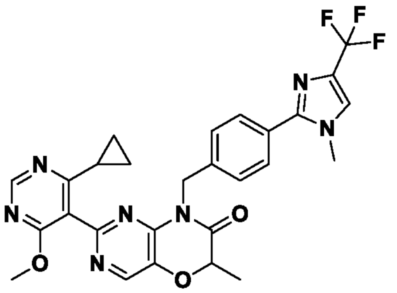, 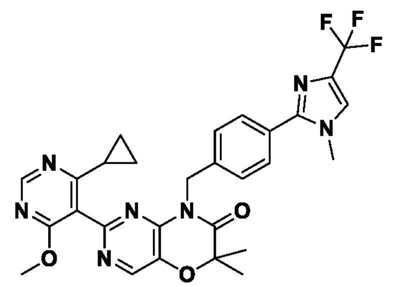, 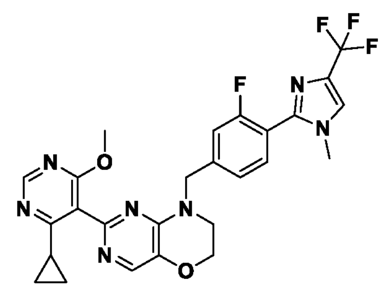,

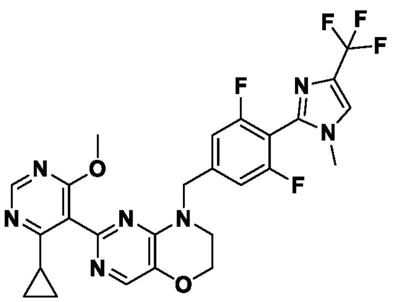, 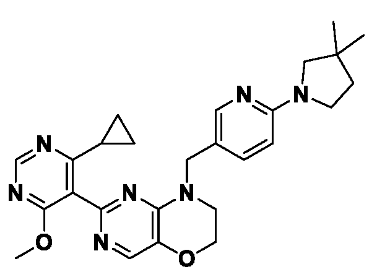, 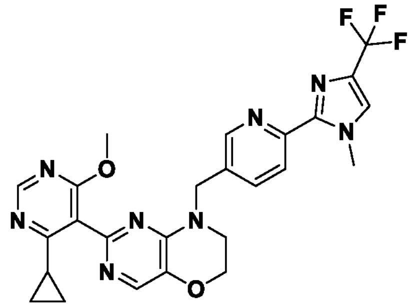,

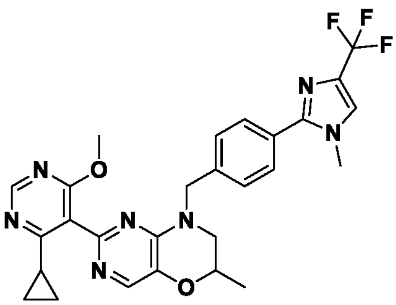, 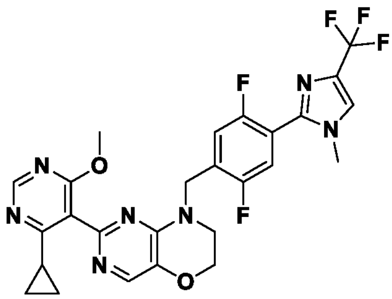, 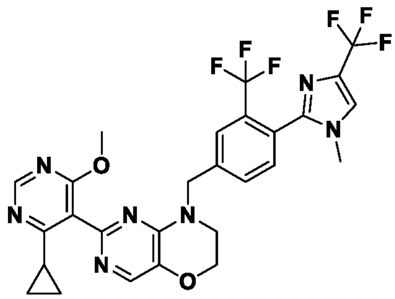,

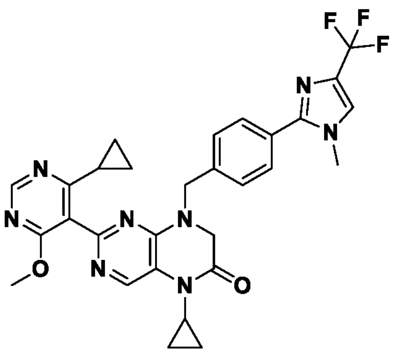, 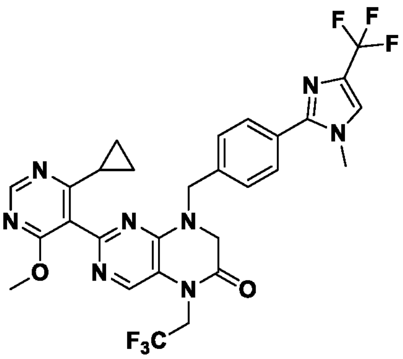, 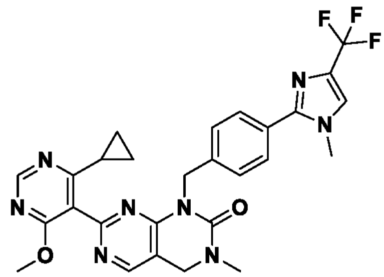,

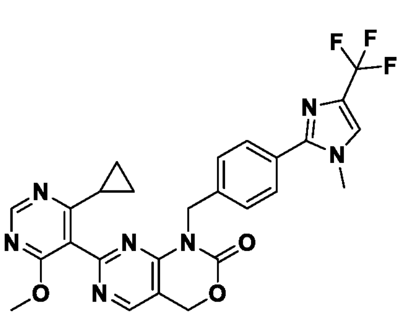, 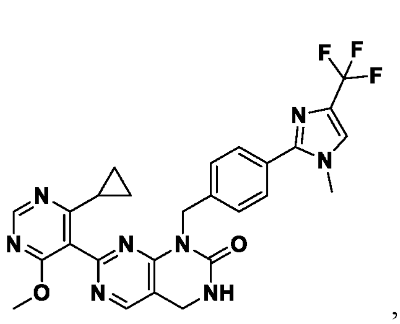, 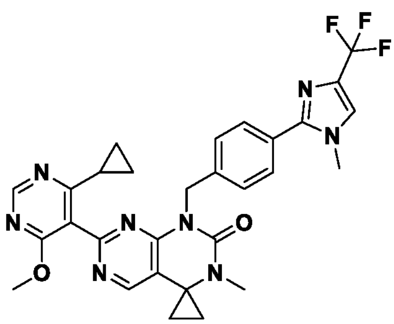,

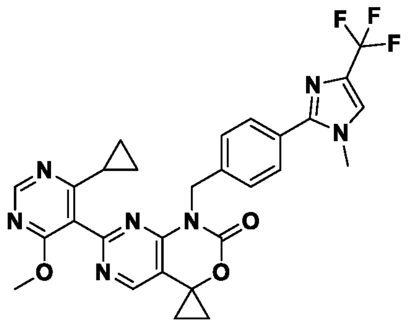

,

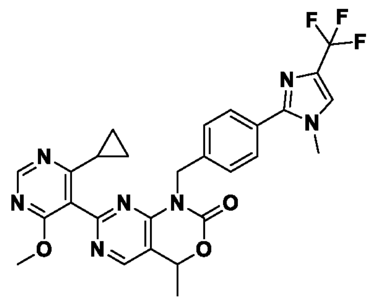

,

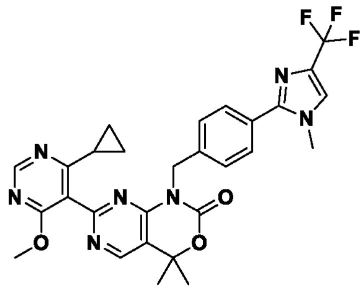

,

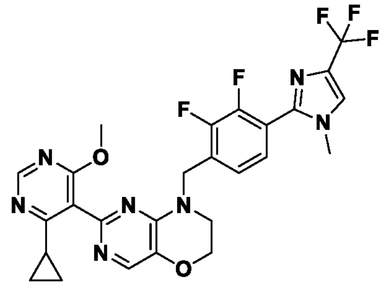

,

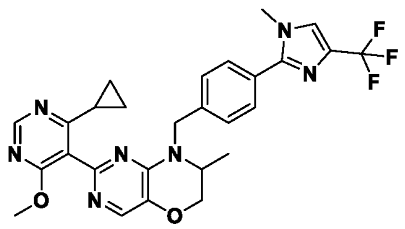

,

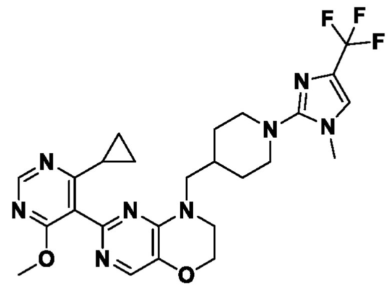

,

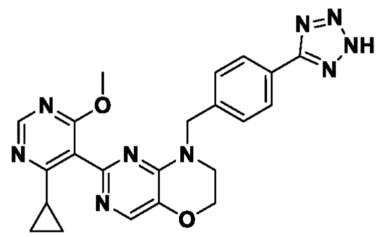

,

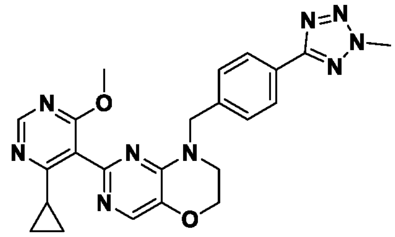

,

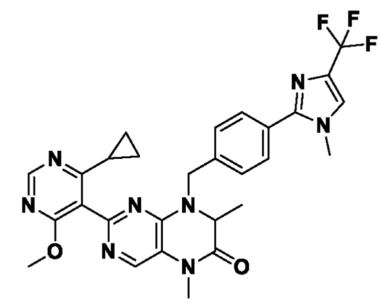

,

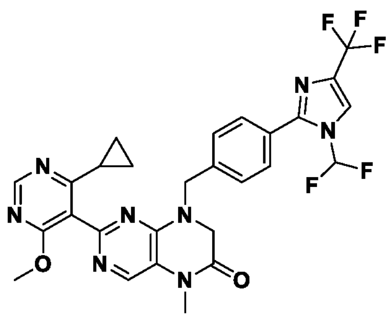

,

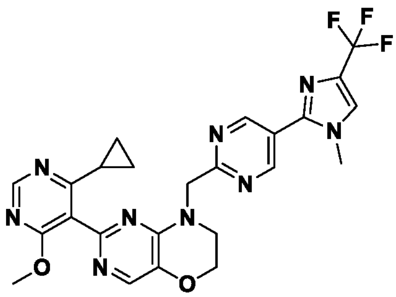

,

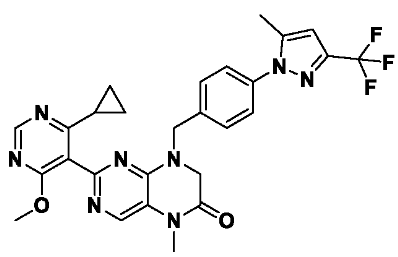

,

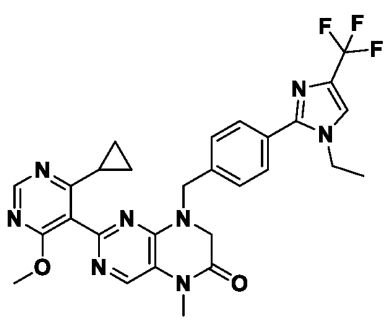

,

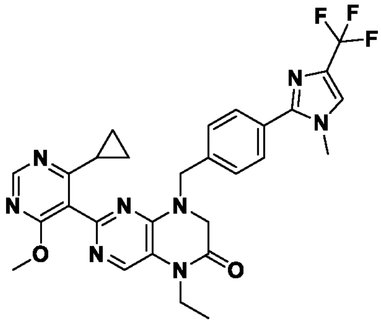

,

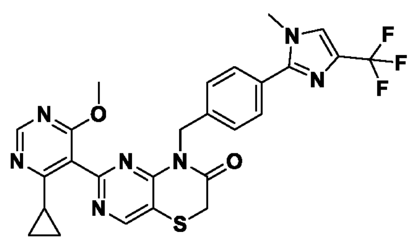

,

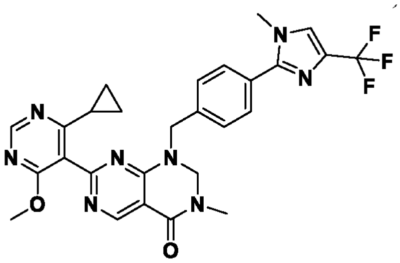
,
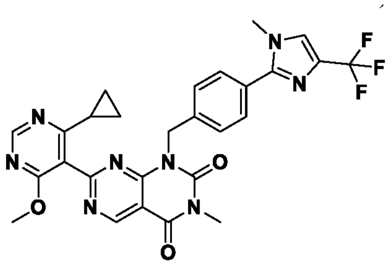
,
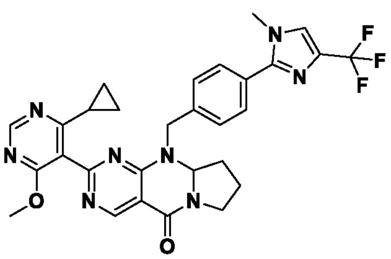
,
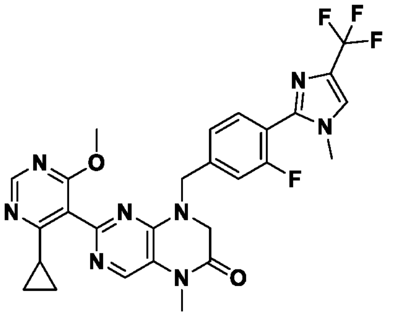
,
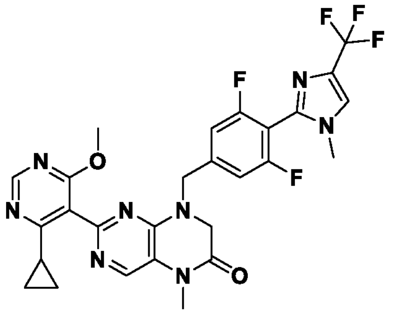
,
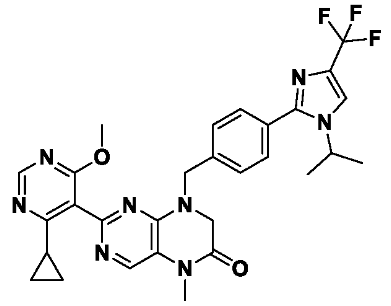
,
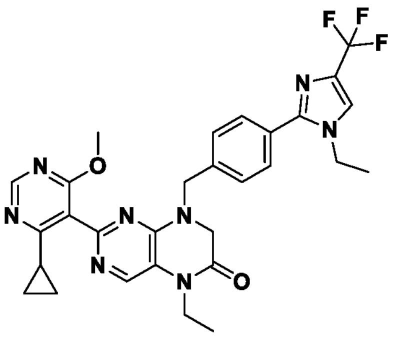
,
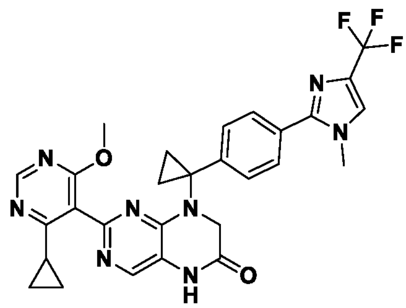
,
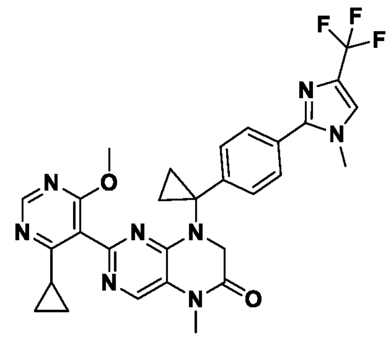
,
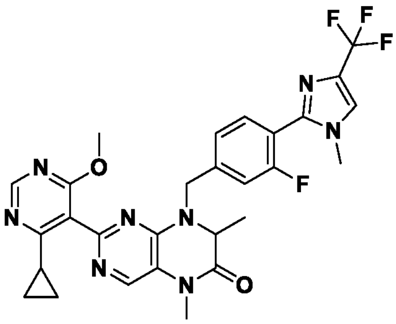
,
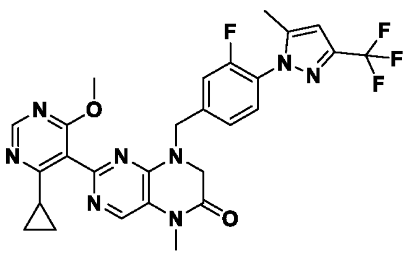
,
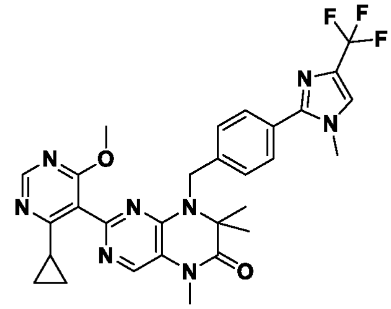
,
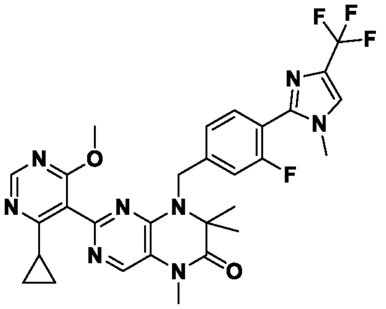
,
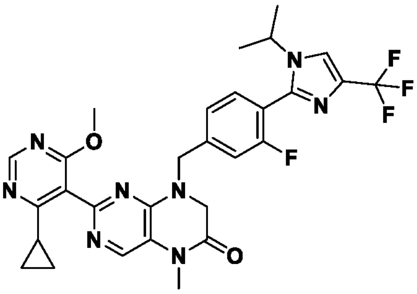
,
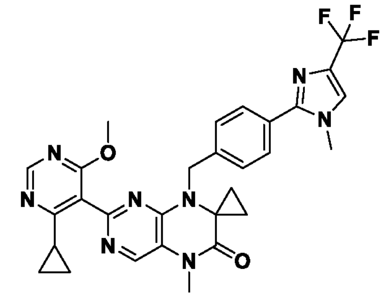
,

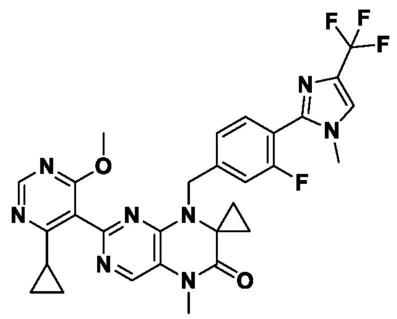 , 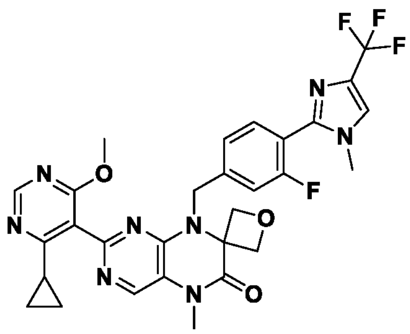 , 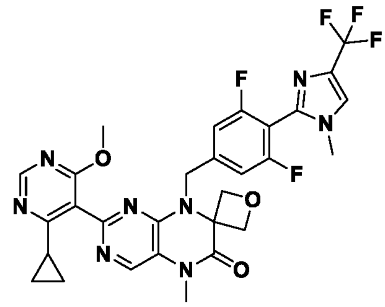 ,

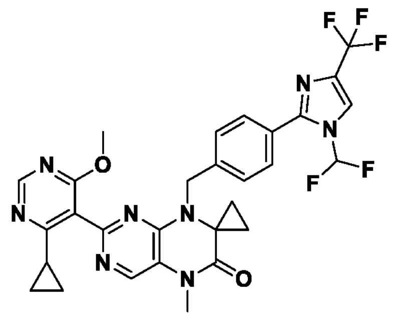 , 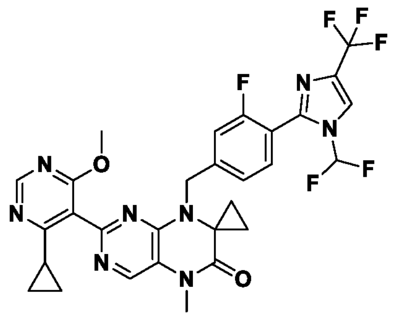 , 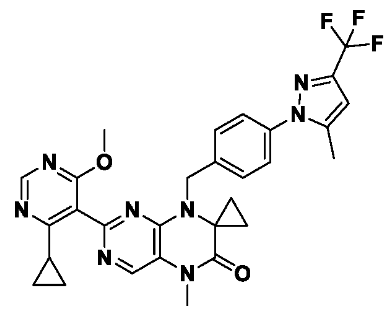 ,

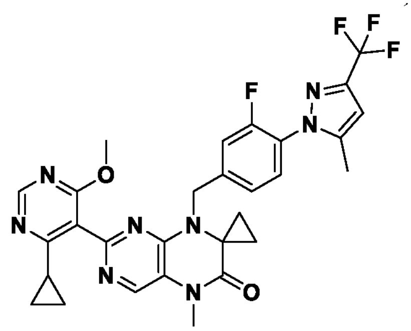 , 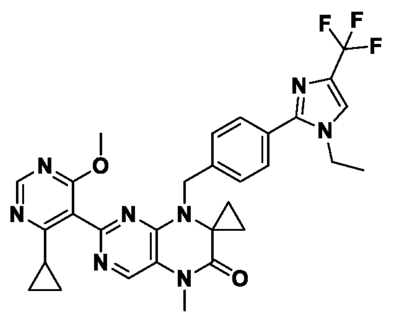 , 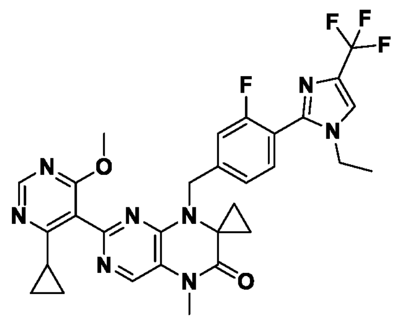 ,

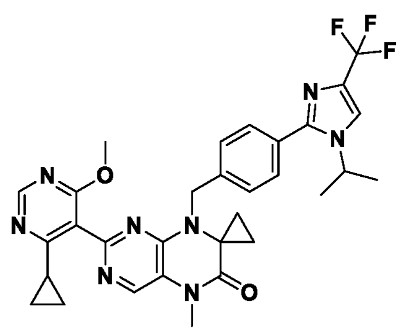 , 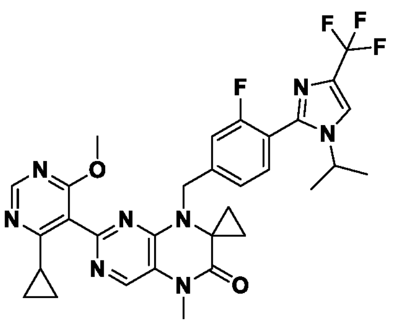 , 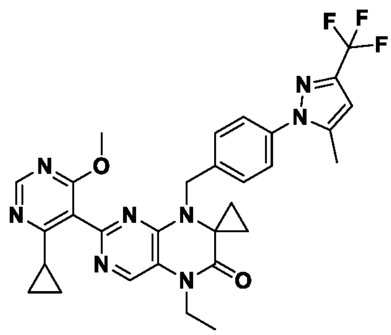 ,

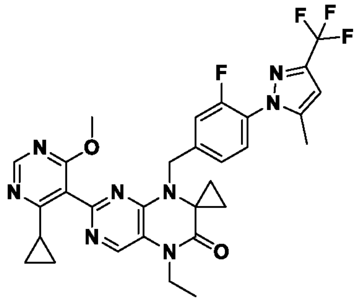,

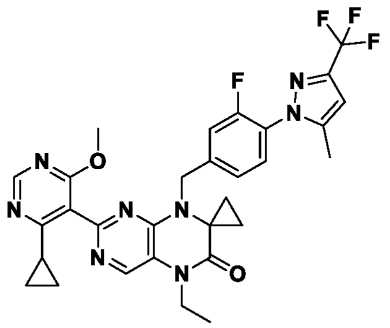,

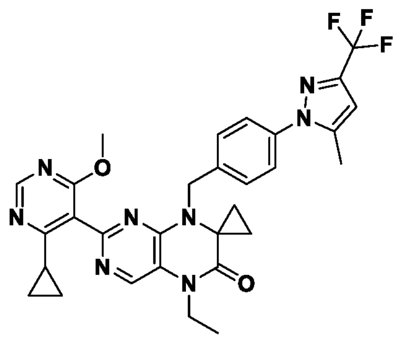,

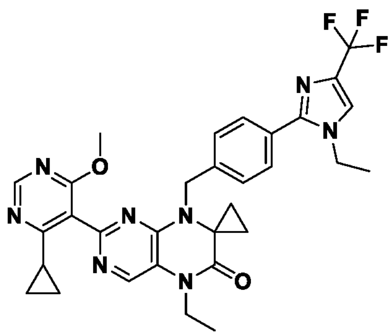,

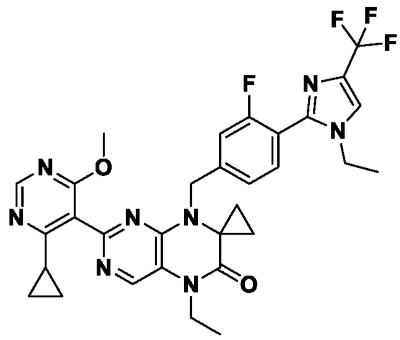,

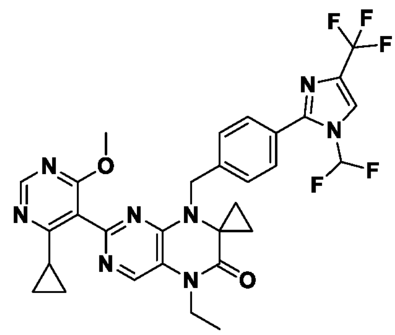,

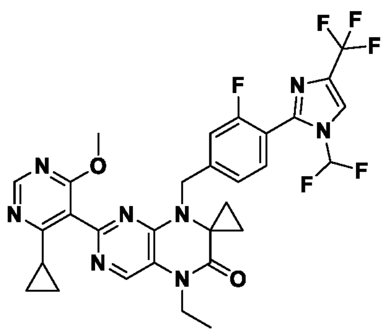,

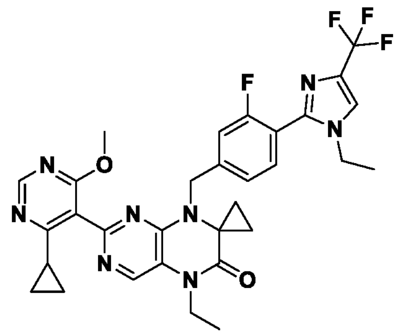,

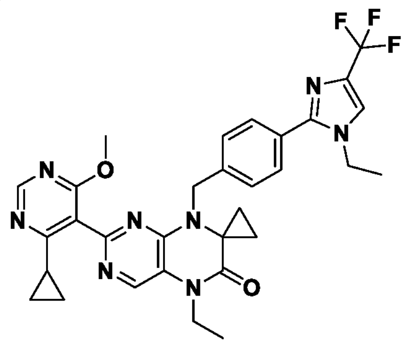,

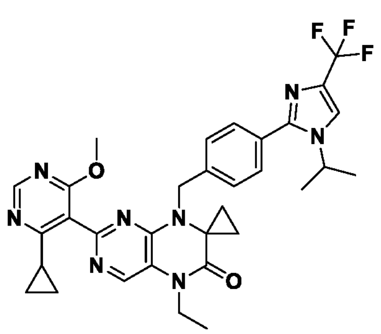,

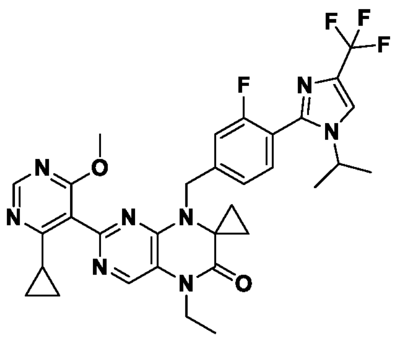,

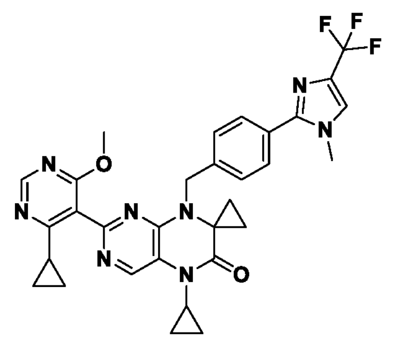,

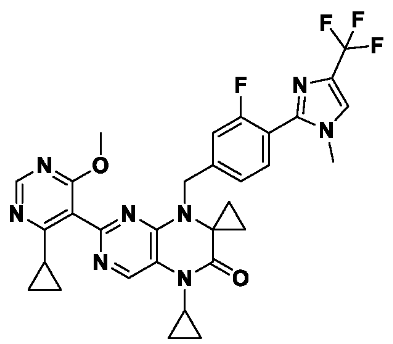
,
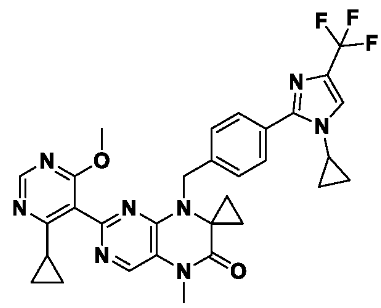
,
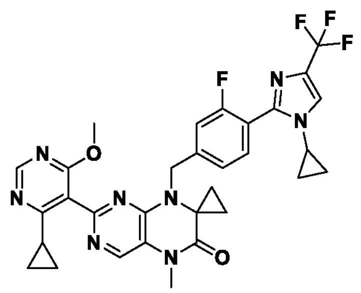
,

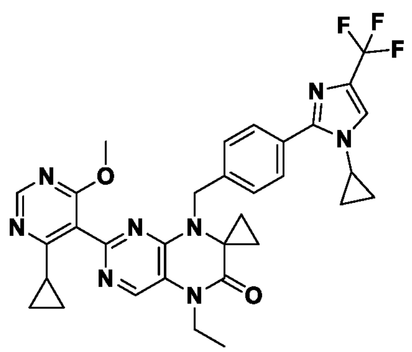
,
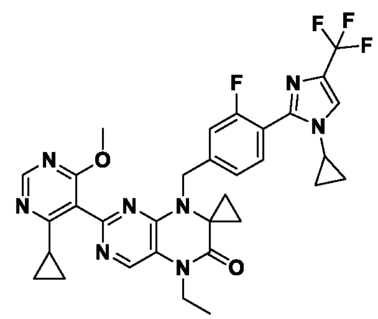
,
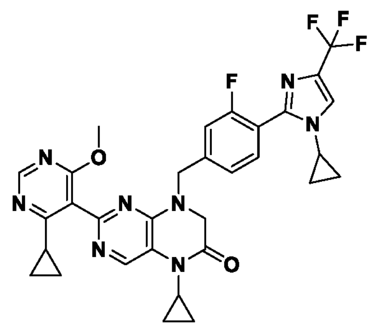
,

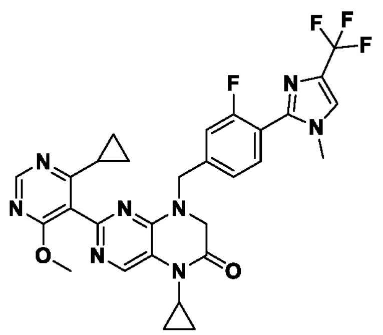
,
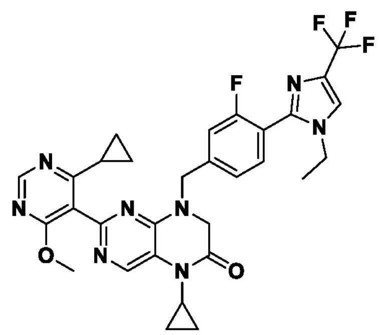
,
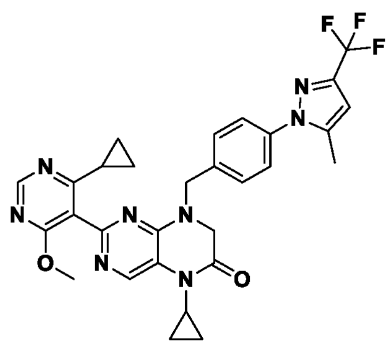
,

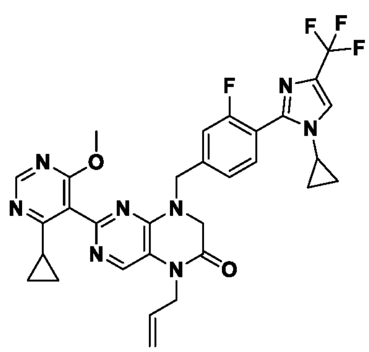
,
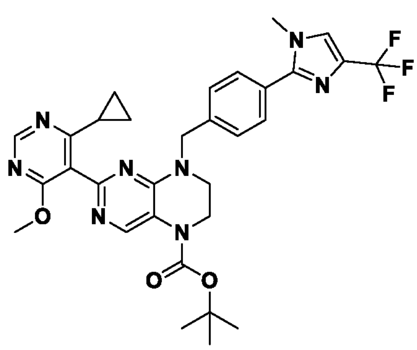
,
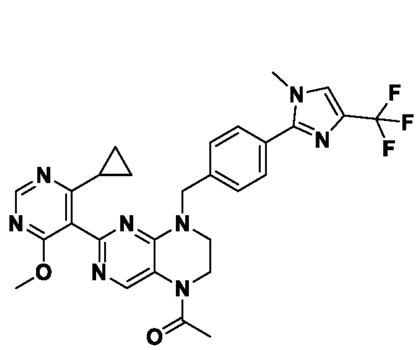
,

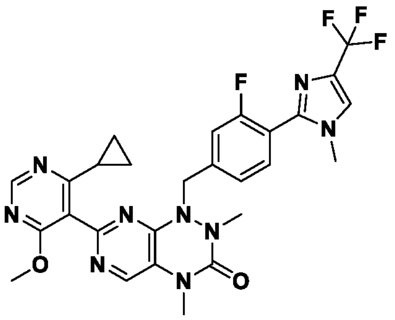

,

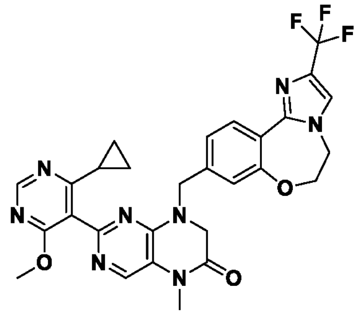

,

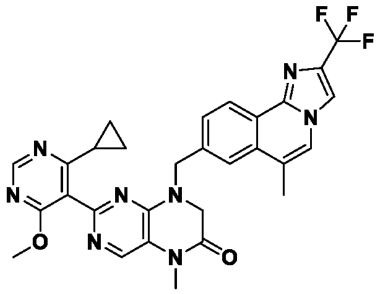

,

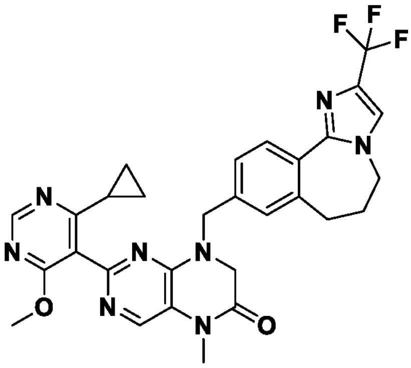

,

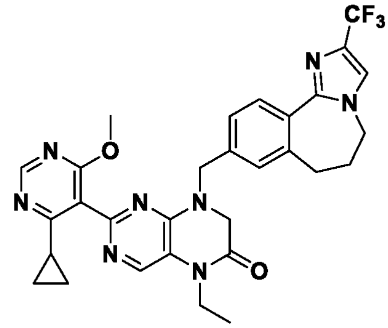

,

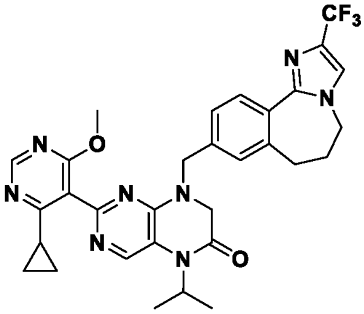

,

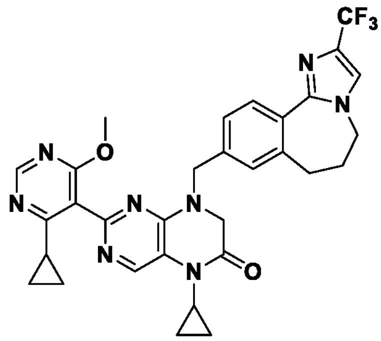

,

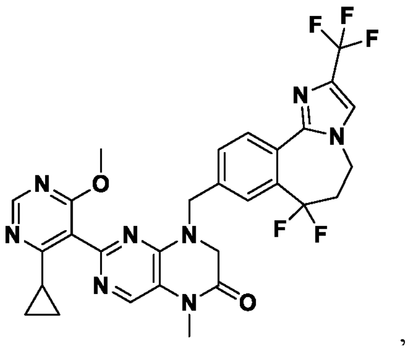

,

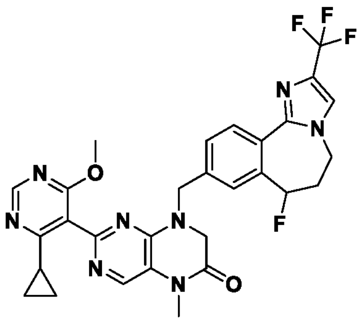

,

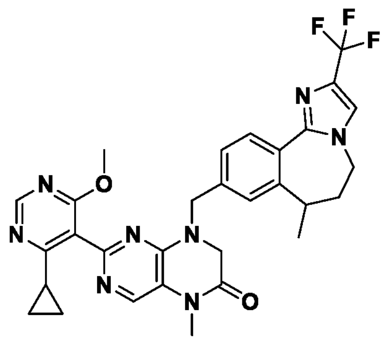

,

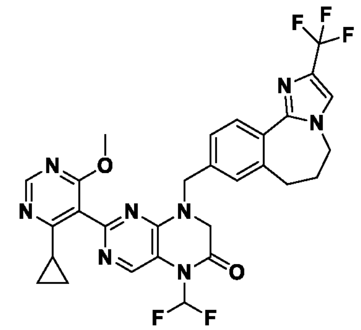

,

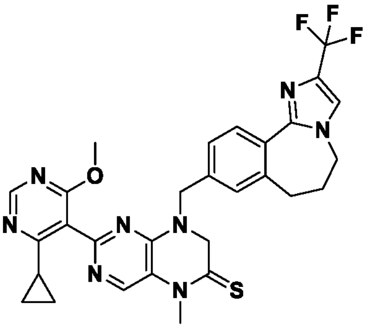

,

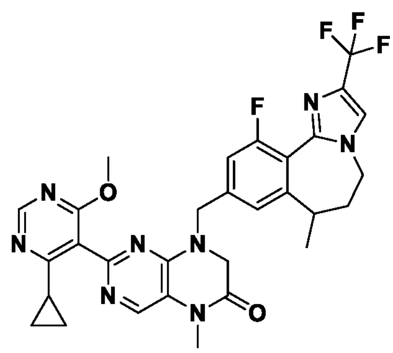
,
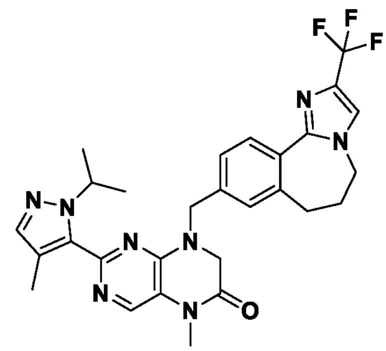
,
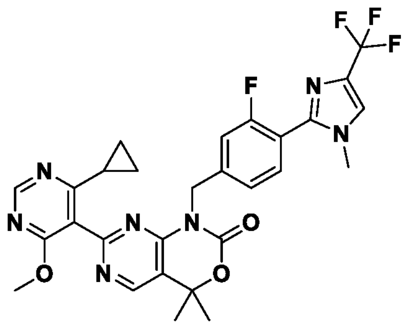
,

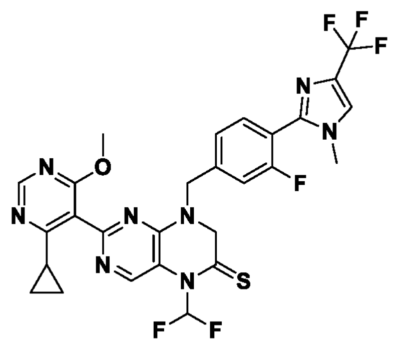
,
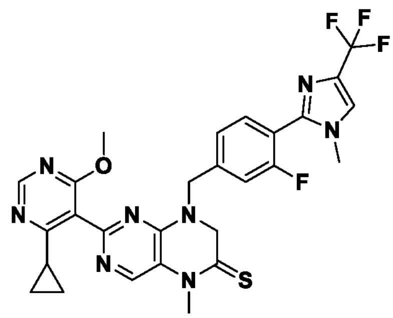
,
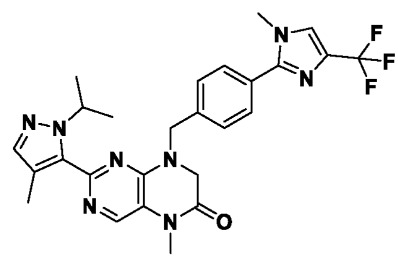
,

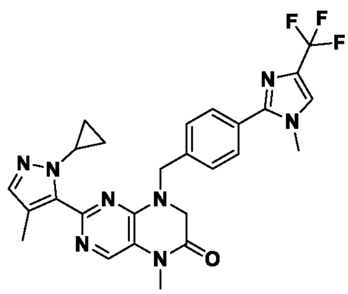
,
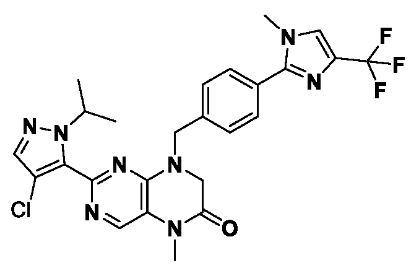
,
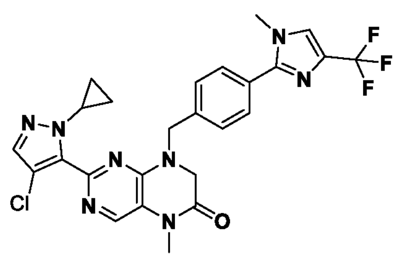
,

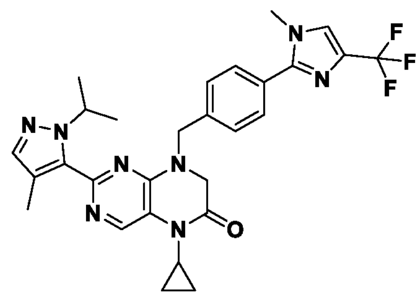
,
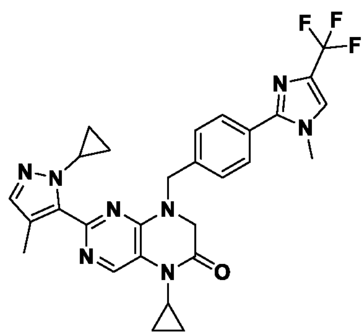
,
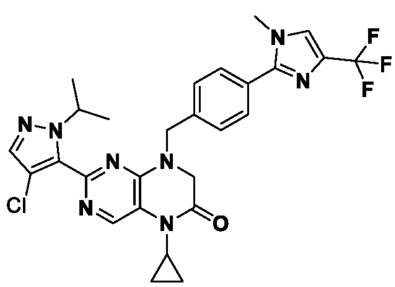
,

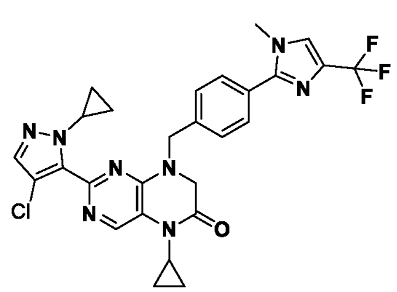,
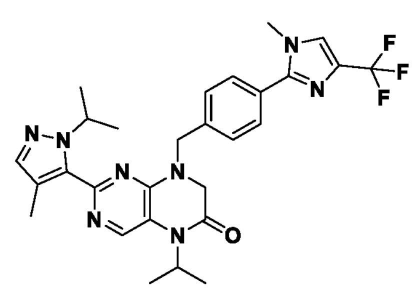,
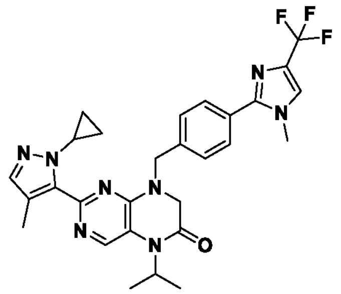,

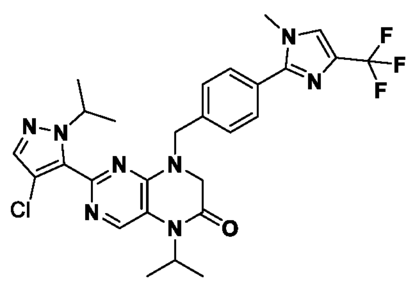,
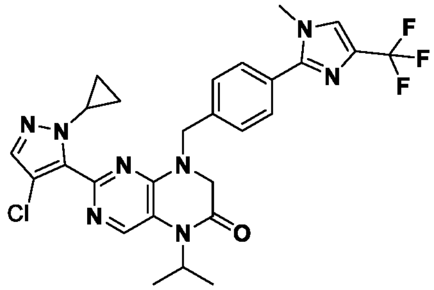

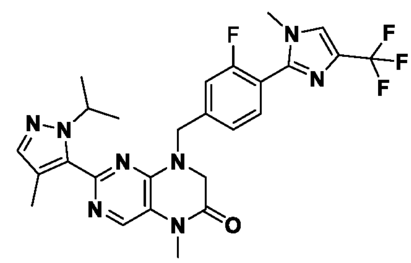,
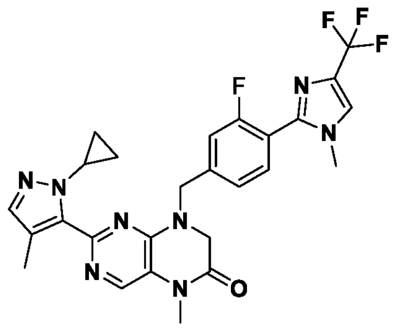,
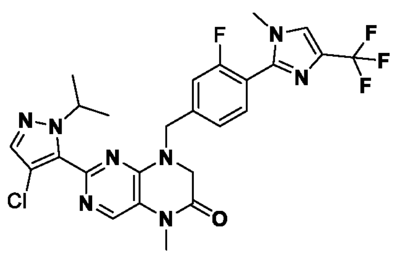,

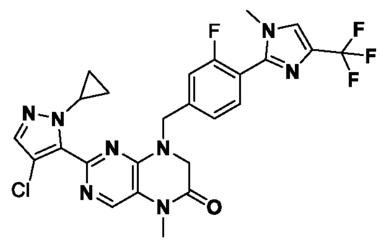,
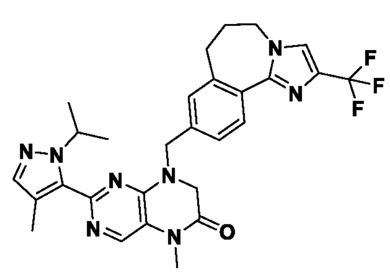,
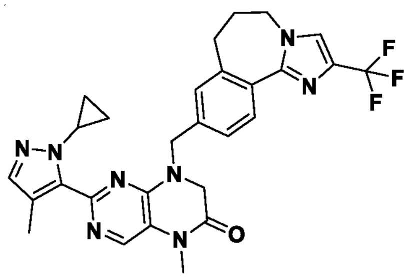,

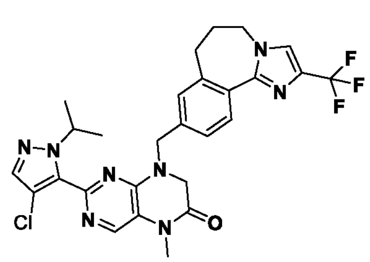,
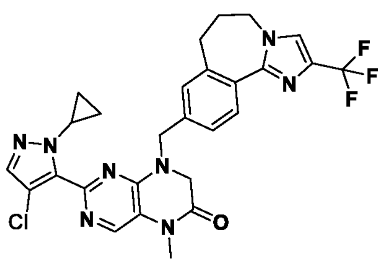,
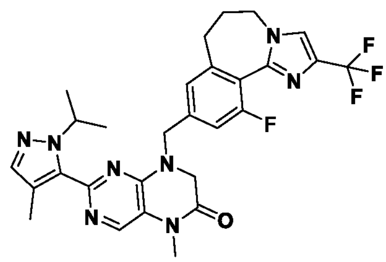,

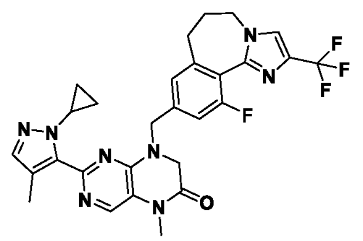,
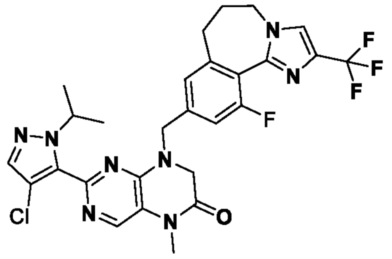,
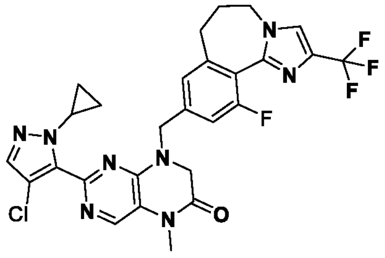,

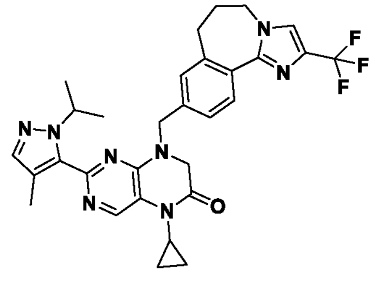,
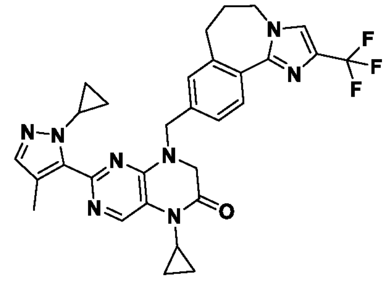,
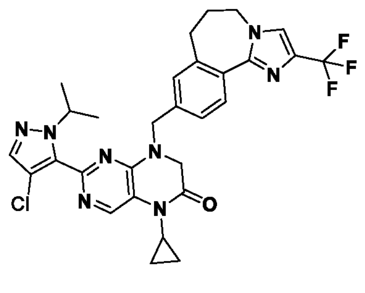,

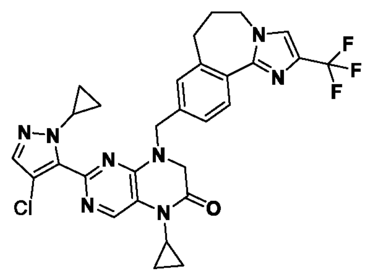,
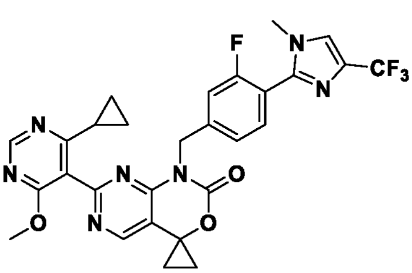,
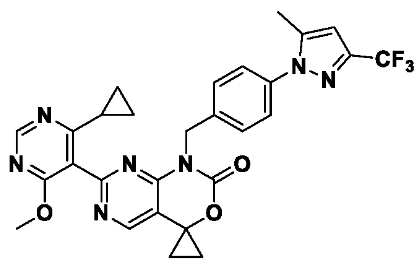,

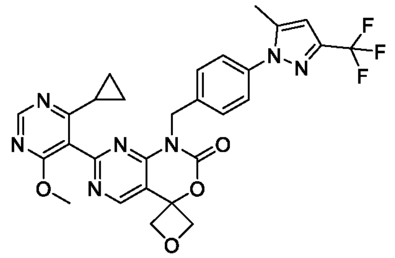,
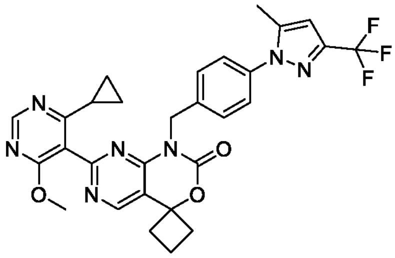,
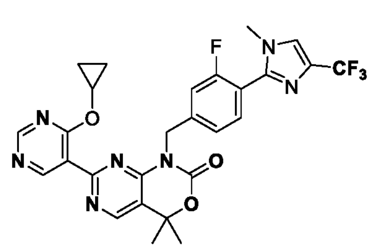,

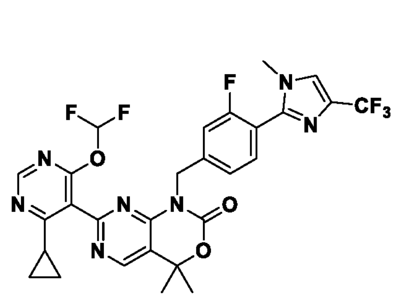,
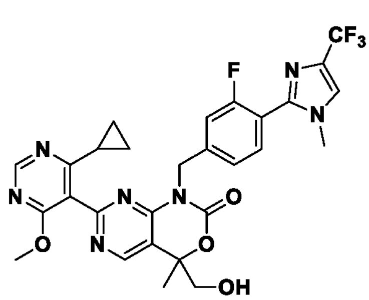,
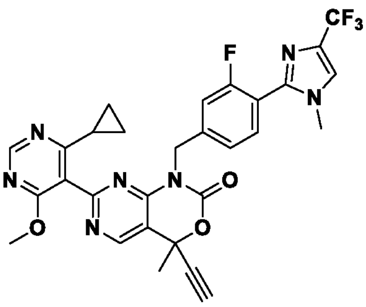,

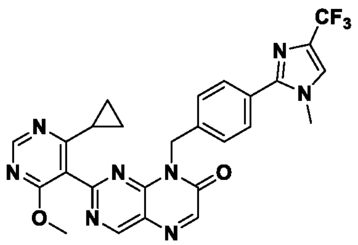
,
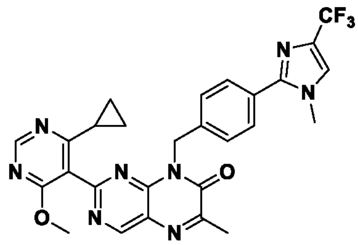
,
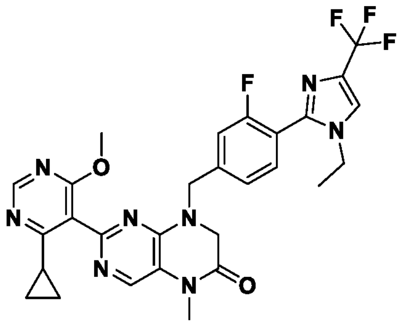
,
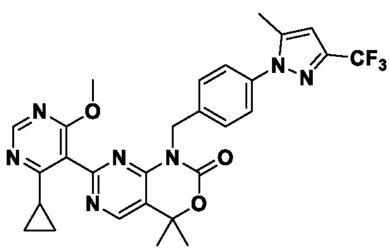
,
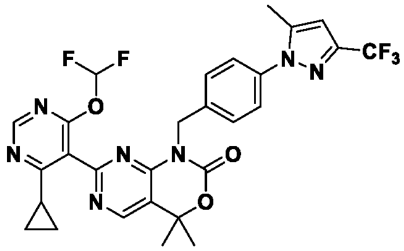
,
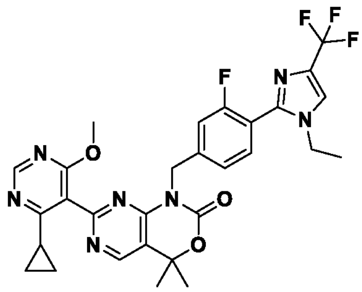
,
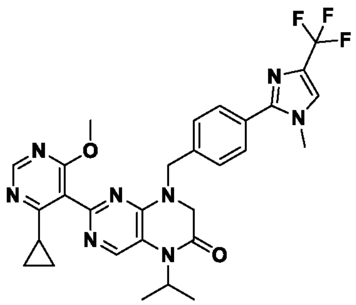
,
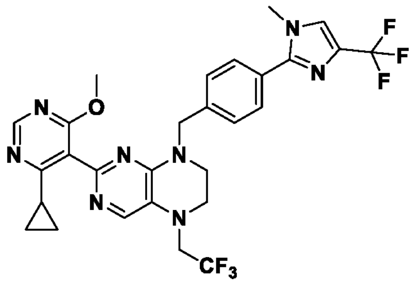
, or
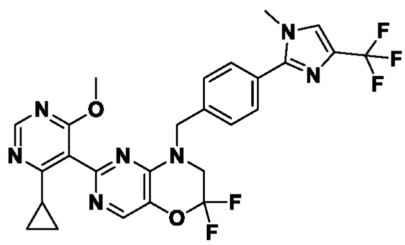
.

**18.** A pharmaceutical composition, comprising the compound of formula (I) or the pharmaceutically acceptable salt thereof according to any one of claims 1 to 17 and a pharmaceutically acceptable excipient.

**19.** A method for treating a USP1-mediated disease in a mammal, comprising administering to the mammal, preferably a human, in need of the treatment a therapeutically effective amount of the compound of formula (I) or the pharmaceutically acceptable salt thereof according to any one of claims 1 to 17 or the pharmaceutical composition according to claim 18, wherein the USP1-mediated disease is preferably a tumor.

**20.** Use of the compound of formula (I) or the pharmaceutically acceptable salt thereof according to any one of claims 1 to 17 or the pharmaceutical composition according to claim 18 in preparing a medicament for use in preventing or treating a USP1-mediated disease, wherein the USP1-mediated disease is preferably a tumor.

## INTERNATIONAL SEARCH REPORT

International application No.
**PCT/CN2022/085703**

**A. CLASSIFICATION OF SUBJECT MATTER**

C07D 417/14(2006.01)i; C07D 291/08(2006.01)i; C07D 273/00(2006.01)i; A61K 31/519(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNABS, CNTXT, SIPOABS, DWPI, WOTXT, EPTXT, USTXT, CNKI, 万方, WANFANG, 读秀, DUXIU, Pubmed, ISI_web of Science, Science Direct, STNext: 海南药臻生物医药科技有限公司, 祝伟, 结构检索, 癌症, USP1, structure search, cancer

**C. DOCUMENTS CONSIDERED TO BE RELEVANT**

| Category* | Citation of document, with indication, where appropriate, of the relevant passages | Relevant to claim No. |
|---|---|---|
| X | CN 109311868 A (SHY THERAPEUTICS LLC.) 05 February 2019 (2019-02-05) abstract, and description, paragraphs 1059, 1938-1939, and 2018-2023 | 1-16, 18-20 |
| X | CN 111032662 A (SHY THERAPEUTICS LLC.) 17 April 2020 (2020-04-17) abstract, embodiment 13, and claims 1-217 | 1-16, 18-20 |
| A | CA 2082668 A1 (IMPERIAL CHEMICAL INDUSTRIES PLC.) 05 June 1993 (1993-06-05) entire document | 1-20 |
| A | CN 106714800 A (GILEAD SCIENCES INC.) 24 May 2017 (2017-05-24) entire document | 1-20 |
| A | WO 9637481 A1 (CHUGOKU KAYAKU KABUSHIKI KAISHA et al.) 28 November 1996 (1996-11-28) entire document | 1-20 |
| A | WO 2014086737 A1 (BAYER CROPSCIENCE AG.) 12 June 2014 (2014-06-12) entire document | 1-20 |
| A | US 5620978 A (THE UNIVERSITY OF OREGON et al.) 15 April 1997 (1997-04-15) entire document | 1-20 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **24 June 2022** | **06 July 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

INTERNATIONAL SEARCH REPORT

International application No.
PCT/CN2022/085703

C. DOCUMENTS CONSIDERED TO BE RELEVANT

| Category* | Citation of document, with indication, where appropriate, of the relevant passages | Relevant to claim No. |
|---|---|---|
| A | US 5330989 A (AMERICAN HOME PRODUCTS CORPORATION) 19 July 1994 (1994-07-19)<br>entire document | 1-20 |
| A | CN 109311868 A (SHY THERAPEUTICS LLC.) 05 February 2019 (2019-02-05)<br>abstract, and description, paragraphs 1059, 1938-1939, and 2018-2023 | 17 |
| A | CN 111032662 A (SHY THERAPEUTICS LLC.) 17 April 2020 (2020-04-17)<br>abstract, embodiment 13, and claims 1-217 | 17 |

Form PCT/ISA/210 (second sheet) (January 2015)

INTERNATIONAL SEARCH REPORT

International application No.
PCT/CN2022/085703

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **19**
because they relate to subject matter not required to be searched by this Authority, namely:

[1] Claim 19 sets forth a method for treating a USP1-mediated disease in a mammal, which contains a treatment solution for a human body or an animal, and thus claim 19 does not comply with PCT Rule 39.1(iv). The research for claim 19 was made on the basis of the following amendment: a use of a compound of formula (I) or a pharmaceutically acceptable salt according to any one of claims 1-17 or a pharmaceutical composition according to claim 18 in preparation of a drug for treating a USP1-mediated disease in a mammal.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2022/085703**

| Patent document cited in search report | Publication date (day/month/year) | Patent family member(s) | Publication date (day/month/year) |
|---|---|---|---|
| CN 109311868 A | 05 February 2019 | BR 112018012914 A2 | 19 March 2019 |
| | | IL 259950 D0 | 31 July 2018 |
| | | US 2017174699 A1 | 22 June 2017 |
| | | AU 2016378723 A1 | 28 June 2018 |
| | | EP 3394056 A1 | 31 October 2018 |
| | | JP 2019500377 A | 10 January 2019 |
| | | WO 2017112777 A1 | 29 June 2017 |
| | | US 2019218229 A1 | 18 July 2019 |
| | | SG 11201804901W A | 30 July 2018 |
| CN 111032662 A | 17 April 2020 | EP 3642209 A1 | 29 April 2020 |
| | | US 11213515 B1 | 04 January 2022 |
| | | KR 20200041294 A | 21 April 2020 |
| | | JP 2020524703 A | 20 August 2020 |
| | | WO 2018237084 A1 | 27 December 2018 |
| | | US 2020061037 A1 | 27 February 2020 |
| | | US 2020054614 A1 | 20 February 2020 |
| | | US 11026930 B1 | 08 June 2021 |
| | | US 2020121660 A1 | 23 April 2020 |
| | | AU 2018288841 A1 | 02 January 2020 |
| | | MA 49458 A | 29 April 2020 |
| | | CA 3066939 A1 | 27 December 2018 |
| | | EA 201992780 A1 | 02 June 2020 |
| | | BR 112019027640 A2 | 07 July 2020 |
| | | IL 271230 D0 | 30 January 2020 |
| | | SG 11201911929X A | 30 January 2020 |
| | | US 2019022074 A1 | 24 January 2019 |
| CA 2082668 A1 | 05 June 1993 | NL 9202091 A | 01 July 1993 |
| | | DE 4239440 A1 | 09 June 1993 |
| | | ZA 9208742 B | 09 June 1993 |
| | | FR 2684672 A1 | 11 June 1993 |
| | | IE 922785 A1 | 16 June 1993 |
| | | IT MI922661 D0 | 20 November 1992 |
| | | BE 1005473 A3 | 03 August 1993 |
| | | GB 9125842 D0 | 05 February 1992 |
| | | JP H05255327 A | 05 October 1993 |
| | | GB 9225283 D0 | 27 January 1993 |
| CN 106714800 A | 24 May 2017 | MX 2017000026 A | 01 May 2017 |
| | | US 2016008374 A1 | 14 January 2016 |
| | | JP 2022044706 A | 17 March 2022 |
| | | AU 2022200792 A1 | 24 February 2022 |
| | | SG 11201700070Q A | 27 February 2017 |
| | | KR 20190104438 A | 09 September 2019 |
| | | EA 201790024 A1 | 31 July 2017 |
| | | UY 36212 A | 29 January 2016 |
| | | JP 2018090645 A | 14 June 2018 |
| | | JP 2017521434 A | 03 August 2017 |
| | | KR 20170029578 A | 15 March 2017 |
| | | CN 113577081 A | 02 November 2021 |
| | | AU 2019279900 A1 | 02 January 2020 |
| | | JP 2020183407 A | 12 November 2020 |

Form PCT/ISA/210 (patent family annex) (January 2015)

**INTERNATIONAL SEARCH REPORT**
**Information on patent family members**

International application No.
**PCT/CN2022/085703**

| Patent document cited in search report | | | Publication date (day/month/year) | Patent family member(s) | | | Publication date (day/month/year) |
|---|---|---|---|---|---|---|---|
| | | | | TW | 201617079 | A | 16 May 2016 |
| | | | | AU | 2018203738 | A1 | 21 June 2018 |
| | | | | WO | 2016007765 | A1 | 14 January 2016 |
| | | | | NZ | 728072 | A | 29 June 2018 |
| | | | | US | 2022062293 | A1 | 03 March 2022 |
| | | | | CA | 2954056 | A1 | 14 January 2016 |
| | | | | BR | 112017000528 | A2 | 14 November 2017 |
| | | | | AU | 2015287773 | A1 | 02 February 2017 |
| | | | | EP | 3166607 | A1 | 17 May 2017 |
| | | | | JP | 2019163303 | A | 26 September 2019 |
| | | | | TW | 202203927 | A | 01 February 2022 |
| | | | | MA | 40238 | A | 17 May 2017 |
| WO | 9637481 | A1 | 28 November 1996 | AU | 5780196 | A | 11 December 1996 |
| | | | | JP | H08325248 | A | 10 December 1996 |
| | | | | EP | 0838458 | A1 | 29 April 1998 |
| | | | | CN | 1189158 | A | 29 July 1998 |
| WO | 2014086737 | A1 | 12 June 2014 | None | | | |
| US | 5620978 | A | 15 April 1997 | CA | 2180122 | A1 | 13 July 1995 |
| | | | | JP | H09510695 | A | 28 October 1997 |
| | | | | WO | 9518616 | A2 | 13 July 1995 |
| | | | | AU | 1599395 | A | 01 August 1995 |
| | | | | JP | 2005247864 | A | 15 September 2005 |
| | | | | EP | 0743855 | A1 | 27 November 1996 |
| | | | | US | 5863916 | A | 26 January 1999 |
| | | | | AU | 1999015993 | A1 | 12 August 1999 |
| | | | | IL | 112235 | D0 | 30 March 1995 |
| US | 5330989 | A | 19 July 1994 | None | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202110403760
- CN 202110829701
- CN 202111166322X
- CN 202111591747
- CN 202210242231

### Non-patent literature cited in the description

- **HUSSAIN, S.** DUBs and cancer: The role of deubiquitinating enzymes as oncogenes, non-oncogenes and tumor suppressors. *Cell Cycle,* 2009, vol. 8, 1688-1697
- *CHEMICAL ABSTRACTS,* 301224-40-8